(19) Europäisches Patentamt
European Patent Office
Office européen des brevets

(11) EP 4 647 433 A1

(12) **EUROPEAN PATENT APPLICATION**
published in accordance with Art. 153(4) EPC

(43) Date of publication:
**12.11.2025 Bulletin 2025/46**

(21) Application number: **24738771.5**

(22) Date of filing: **05.01.2024**

(51) International Patent Classification (IPC):
***C07D 513/10*** *(2006.01)* ***A61K 31/517*** *(2006.01)*
***A61K 31/497*** *(2006.01)* ***A61P 35/00*** *(2006.01)*
***A61P 35/04*** *(2006.01)* ***A61P 9/00*** *(2006.01)*
***A61P 37/00*** *(2006.01)* ***A61P 27/02*** *(2006.01)*
***C07D 471/10*** *(2006.01)* ***C07D 401/14*** *(2006.01)*

(52) Cooperative Patent Classification (CPC):
**A61K 31/497; A61K 31/517; A61P 9/00; A61P 27/02; A61P 35/00; A61P 35/04; A61P 37/00; C07D 401/14; C07D 471/10; C07D 513/10**

(86) International application number:
**PCT/KR2024/000298**

(87) International publication number:
**WO 2024/147703 (11.07.2024 Gazette 2024/28)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **05.01.2023 KR 20230001842**

(71) Applicant: **Kanaph Therapeutics Inc.**
**Seoul 04348 (KR)**

(72) Inventors:
- **KIM, Miyeon**
  **Yongin-si Gyeonggi-do 16941 (KR)**
- **KIM, Dongsu**
  **Hwaseong-si Gyeonggi-do 18443 (KR)**
- **YOON, Kyeongjin**
  **Seoul 06065 (KR)**
- **CHOI, Sungpil**
  **Anyang-si Gyeonggi-do 14074 (KR)**
- **JEON, Yeejin**
  **Gwangmyeong-si Gyeonggi-do 14295 (KR)**
- **LEE, Mijung**
  **Hwaseong-si Gyeonggi-do 18452 (KR)**
- **JEON, Dahye**
  **Ansan-si Gyeonggi-do 15468 (KR)**
- **JANG, Soyeon**
  **Seoul 06998 (KR)**
- **YOO, Jakyung**
  **Yongin-si Gyeonggi-do 17097 (KR)**

(74) Representative: **HGF**
**HGF Limited**
**1 City Walk**
**Leeds LS11 9DX (GB)**

(54) **SHP2 INHIBITOR AND USES THEREOF**

(57) The present invention relates to an inhibitor of SHP2, a pharmaceutical composition for preventing or treating a disease related to SHP2 comprising the same, a method for treating and preventing a disease using the same, and a use thereof. Accordingly, the present invention can effectively prevent or treat diseases related to SHP2.

EP 4 647 433 A1

## Description

### Technical Field

The present invention relates to a compound as a SHP2 inhibitor, a stereoisomer, isotope-labeled compound or solvate thereof, or a pharmaceutically acceptable salt thereof, and its use for the prevention or treatment of a disease associated with the abnormal activity of SHP2.

The present study was supported by the National New Drug Development Project Support (HN22C0066) of the KOREA DRUG DEVELOPMENT FUND funded by the Ministry of Science and ICT, the Ministry of Trade, Industry and Energy, and the Ministry of Health and Welfare.

### Background Art

Src homology region 2 domain-containing phosphatase-2 (SHP2) is a protein tyrosine phosphatase (PTP), also referred to as PTPN11 (protein tyrosine phosphatase non-receptor type 11), PTP-1D (protein-tyrosine phosphatase 1D), or PTP-2C (protein-tyrosine phosphatase 2C). SHP2 is known as a signaling molecule that regulates various cellular functions, including cell growth, differentiation, cell cycle, and oncogenic transformation. SHP2, together with SHP1, consists of two tandem SH2 domains at the N-terminus. In the inactive state, the SH2 domain at the N-terminus binds to the PTP domain and blocks the substrate from binding to the active site, thereby inhibiting SHP2. Upon binding of the phospho-tyrosyl residue, the SH2 domain at the N-terminus is released from the PTP domain and the enzyme is activated.

Mutations in SHP2 are known to cause Noonan syndrome and Leopard syndrome and are known to be associated with cancer such as juvenile myelomonocytic leukemia, neuroblastoma, melanoma, acute myeloid leukemia, breast cancer, esophageal cancer, lung cancer, large intestine cancer, head cancer, squamous cell carcinoma of the head and neck, gastric carcinoma, anaplastic large cell lymphoma, glioblastoma, pancreatic cancer, biliary tract cancer, uterine cancer, endometrial cancer, liver cancer, and neurofibromatosis type 1.

In addition, SHP2 inhibitors are considered as substances that can effectively penetrate the blood-brain barrier (BBB). Therefore, SHP2 inhibitors are expected to be therapeutic agents for primary and metastatic malignant brain tumors based on their excellent brain penetration ability.

Therefore, there is a need to develop a novel compound capable of inhibiting the activity of SHP2, a method for preparing the same, a pharmaceutical composition comprising the same, and a method for preventing or treating a disease associated with the abnormal activity of SHP2 using the same.

### Detailed Description of Invention

### Technical Problem

There is provided a novel compound capable of inhibiting the activity of SHP2, a stereoisomer, isotope-labeled compound or solvate thereof, or a pharmaceutically acceptable salt thereof.

There is provided a pharmaceutical composition for preventing or treating a disease associated with the abnormal activity of SHP2 using a novel compound capable of inhibiting the activity of SHP2, a stereoisomer, isotope-labeled compound or solvate thereof, or a pharmaceutically acceptable salt thereof.

There is provided a method for preventing or treating a disease associated with the abnormal activity of SHP2 using a novel compound capable of inhibiting the activity of SHP2, a stereoisomer, isotope-labeled compound or solvate thereof, or a pharmaceutically acceptable salt thereof.

There is provided a use of a novel compound capable of inhibiting the activity of SHP2, a stereoisomer, isotope-labeled compound or solvate thereof, or a pharmaceutically acceptable salt thereof.

### Solution to Problem

Each description and embodiment disclosed herein may also apply to each other description and embodiment. That is, all combinations of the various elements disclosed herein fall within the scope of the present application. In addition, it should not be construed that the scope of the present application is limited by the specific description set forth below.

In one aspect, there is provided a compound represented by Formula 1, or a stereoisomer, solvate or isotope-labeled compound thereof, or a pharmaceutically acceptable salt thereof:

[Formula 1]

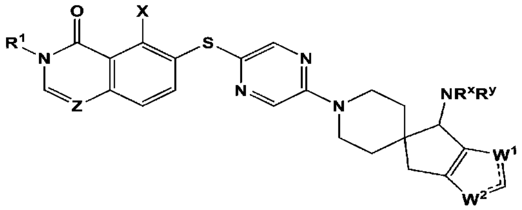

In Formula 1, Z is N or CH; X is H or halogen; one of $W^1$ and $W^2$ is S, and the other is N. $R^1$ is H, $C_{6-10}$ aryl, or $C_{1-6}$ alkyl optionally substituted with $C_{1-6}$ alkoxy; $R^x$ and $R^y$ are each independently H or $C_{1-6}$ alkyl,

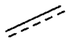

is a single bond or a double bond.

In some embodiments, the compound represented by Formula 1 may be represented by the following Formula 1A:

[Formula 1A]

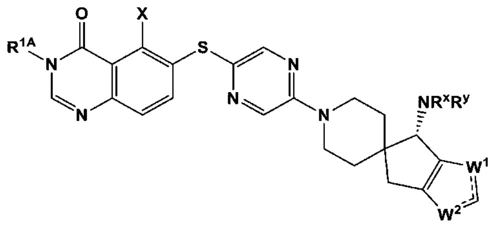

In Formula 1A, X is H or halogen; one of $W^1$ and $W^2$ is S, and the other is N. $R^{1A}$ is H, $C_{6-10}$ aryl, or $C_{1-6}$ alkyl optionally substituted with $C_{1-6}$ alkoxy; $R^x$ and $R^y$ are each independently H or $C_{1-6}$ alkyl. For example, $W^1$ may be S, and $W^2$ may be N. Alternatively, $W^1$ may be N, and $W^2$ may be S. In this case, 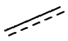 connected to N may be a double bond, and 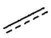 connected to S may be a single bond.

In Formula 1A, X is H or halogen. In Formula 1A, X may be H, F, Cl, Br or I. For example, X may be H or Cl.

In Formula 1A, $R^{1A}$ is H, $C_{6-10}$ aryl, or $C_{1-6}$ alkyl optionally substituted with $C_{1-6}$ alkoxy. The $C_{1-6}$ alkyl optionally substituted with $C_{1-6}$ alkoxy may include $C_{1-6}$ alkyl, $C_{1-5}$ alkyl, $C_{3-6}$ alkyl substituted with $C_{1-6}$ alkoxy, $C_{4-6}$ alkyl substituted with $C_{1-6}$ alkoxy, branched $C_{3-6}$ alkyl substituted with $C_{1-6}$ alkoxy, or branched $C_{4-6}$ alkyl substituted with $C_{1-6}$ alkoxy.

In Formula 1A, $R^{1A}$ is H, $C_{6-10}$ aryl, or $C_{1-6}$ alkyl optionally substituted with $C_{1-6}$ alkoxy. In some embodiments, $R^{1A}$ may be selected from the group consisting of H; phenyl; naphthyl; and methyl, ethyl, propyl, butyl, pentyl, or hexyl, optionally substituted with methoxy, ethoxy, propoxy, or butoxy. For example, it may be selected from the group consisting of H, phenyl, $CH_3$ , $CH_2CH_3$,

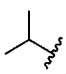 , 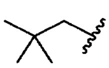 and 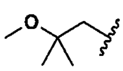 .

In some embodiments, $R^{1A}$ may be other than

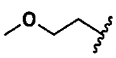 .

In Formula 1A, $R^x$ and $R^y$ are each independently H or $C_{1-6}$ alkyl. In some embodiments, $R^x$ and $R^y$ may be the same or different. In some embodiments, $R^x$ and $R^y$ may each independently be H or $C_{1-4}$ alkyl. In some embodiments, $R^x$ and $R^y$ may each independently be H or $C_{1-3}$ alkyl. For example, $R^x$ and $R^y$ may each be H.

In some embodiments, in Formula 1A, X is H or Cl, $R^{1A}$ is selected from the group consisting of H, phenyl, $CH_3$,

$CH_2CH_3$,

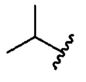, 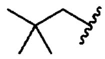 and 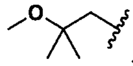,

and $R^x$ and $R^y$ may each be H.

In some embodiments, the compound represented by Formula 1A may be represented by the following Formula 1A-1.

[Formula 1A-1]

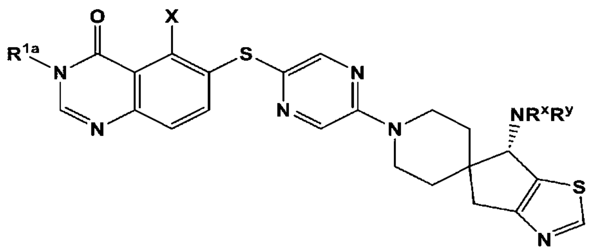

In Formula 1A-1, X is H or halogen; $R^{1a}$ is H or $C_{1-6}$ alkyl; and $R^x$ and $R^y$ may each independently be H or $C_{1-6}$ alkyl.

In Formula 1A-1, X is H or halogen. In some embodiments, X may be H, F, Cl, Br, or I. For example, X may be H or Cl.

In Formula 1A-1, $R^{1a}$ may be H or $C_{1-6}$ alkyl. In some embodiments, $R^{1a}$ may be H, methyl, ethyl, propyl, butyl, pentyl, or hexyl. For example, $R^{1a}$ may be selected from the group consisting of H, $CH_3$, $CH_2CH_3$,

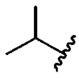, and 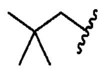.

In Formula 1A-1, $R^x$ and $R^y$ are each independently H or $C_{1-6}$ alkyl. In some embodiments, $R^x$ and $R^y$ may be the same or different. In some embodiments, $R^x$ and $R^y$ may each independently be H or $C_{1-4}$ alkyl. In some embodiments, $R^x$ and $R^y$ may each independently be H or $C_{1-3}$ alkyl. For example, $R^x$ and $R^y$ may each be H.

In some embodiments, in Formula 1A-1, X is H or Cl, $R^{1A}$ is selected from the group consisting of H, $CH_3$, $CH_2CH_3$,

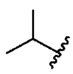 and 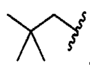,

and $R^x$ and $R^y$ may each be H.

In some embodiments, the compound represented by Formula 1A or Formula 1A-1 may be selected from compounds represented by the following formulas.

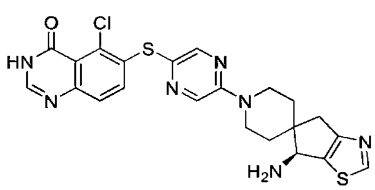

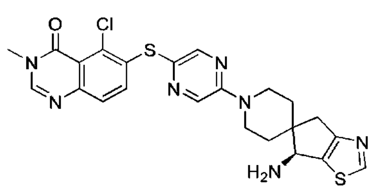

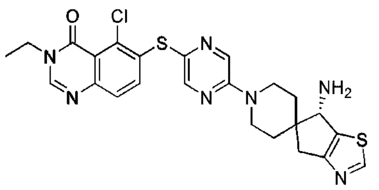

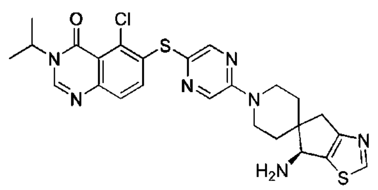

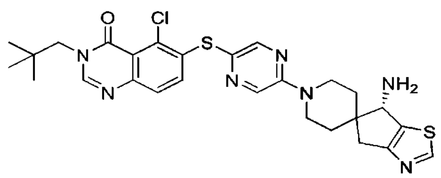

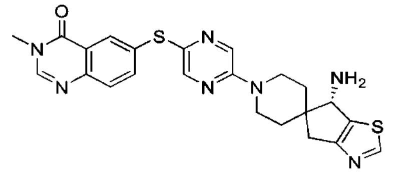

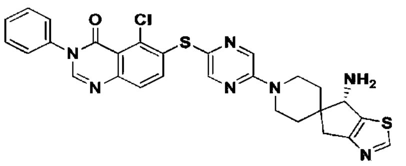

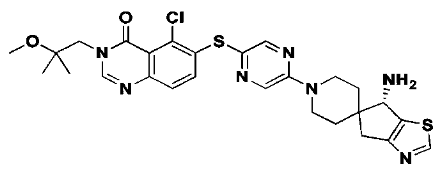

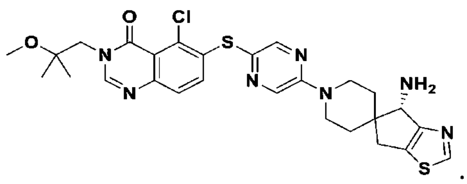
.

In some embodiments, the compound represented by Formula 1 may be represented by the following Formula 1B.

[Formula 1B]

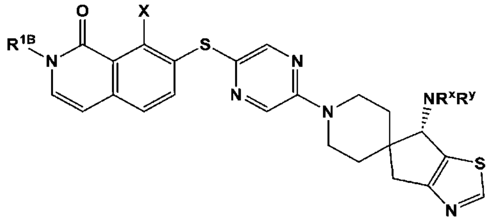

In Formula 1B, X is H or halogen; $R^{1B}$ is H or $C_{1-6}$ alkyl; and $R^x$ and $R^y$ are each independently H or $C_{1-6}$ alkyl.

In Formula 1B, X is H or halogen. In some embodiments, X may be halogen. For example, X may be Cl.

In Formula 1B, $R^{1B}$ is H or $C_{1-6}$ alkyl. The $C_{1-6}$ alkyl may be $C_{1-4}$ alkyl or $C_{1-3}$ alkyl. In some embodiments, $R^{1B}$ may be $C_{1-6}$ alkyl. In some embodiments, $R^{1B}$ may be methyl or propyl. In some embodiments, the propyl may be isopropyl.

In Formula 1B, $R^x$ and $R^y$ are each independently H or $C_{1-6}$ alkyl. In some embodiments, $R^x$ and $R^y$ may be the same or different. In some embodiments, $R^x$ and $R^y$ may each independently be H or $C_{1-4}$ alkyl. In some embodiments, $R^x$ and $R^y$ may each independently be H or $C_{1-3}$ alkyl. For example, $R^x$ and $R^y$ may each be H.

In some embodiments, in Formula 1B, X is halogen, $R^{1B}$ is $C_{1-6}$ alkyl, and $R^x$ and $R^y$ may each be H.

In some embodiments, the compound represented by Formula 1B may be a compound represented by the following chemical formula.

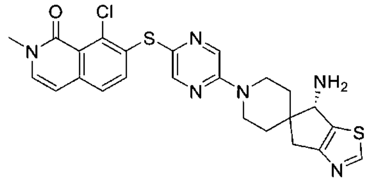

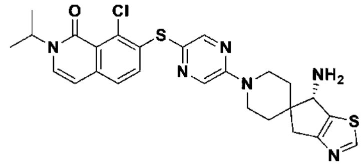
.

In one aspect, there is provided a compound represented by the following Formula 2, a stereoisomer, solvate or isotope-labeled compound thereof, or a pharmaceutically acceptable salt thereof:

[Formula 2]

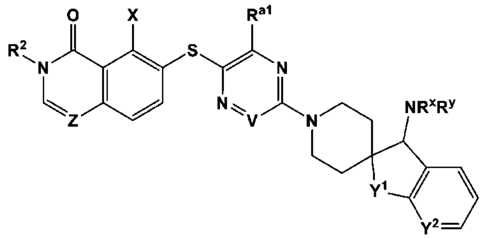

In Formula 2, Z is N or CH; V is N or $CR^{a2}$; X is H or halogen. $R^{a1}$ is H, $NR^iR^{ii}$ or $C_{1-6}$ alkyl; $R^{a2}$ is H, or $C_{1-6}$ alkyl substituted with OH or $C_{1-6}$ alkoxy. $Y^1$ is O or $CH_2$; $Y^2$ is N or CH. $R^2$ is selected from H, $C_{6-10}$ aryl, 5- to 7-membered heteroaryl containing a heteroatom selected from N and O, and $C_{1-6}$ alkyl optionally substituted with OH or $C_{1-6}$ alkoxy; $R^i$ and $R^{ii}$ are each independently H or $C_{1-6}$ alkyl; $R^x$ and $R^y$ are each independently H or $C_{1-6}$ alkyl.

In some embodiments, the compound represented by Formula 2 may be represented by the following Formula 2A.

[Formula 2A]

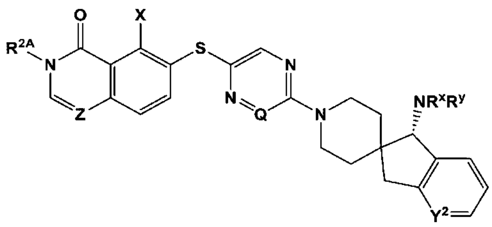

In Formula 2A, Z is N or CH; Q is N or CH; X is H or halogen; $Y^2$ is N or CH. $R^{2A}$ is H, $C_{6-10}$ aryl, 5- to 7-membered heteroaryl containing a heteroatom selected from N and O, or $C_{1-6}$ alkyl optionally substituted with OH or $C_{1-6}$ alkoxy; and $R^x$ and $R^y$ are each independently H or $C_{1-6}$ alkyl.

In Formula 2A, Q is N or CH. In some embodiments, when Q is N, Z may be N. In some embodiments, when Q is N, $Y^2$ may be CH.

In Formula 2A, X is H or halogen. In some embodiments, X may be halogen. In some embodiments, X may be F, Cl, Br, or I. For example, X may be Cl.

In Formula 2A, $R^{2A}$ is H, $C_{6-10}$ aryl, 5- to 7-membered heteroaryl containing a heteroatom selected from N and O, or $C_{1-6}$ alkyl optionally substituted with OH or $C_{1-6}$ alkoxy. In some embodiments, the $C_{1-6}$ alkyl optionally substituted with OH or $C_{1-6}$ alkoxy may include $C_{1-6}$ alkyl, $C_{1-4}$ alkyl, $C_{1-3}$ alkyl, $C_{1-6}$ alkyl substituted with OH, branched $C_{3-6}$ alkyl substituted with OH, branched $C_{4-6}$ alkyl substituted with OH, $C_{1-6}$ alkyl substituted with $C_{1-6}$ alkoxy, $C_{3-6}$ alkyl substituted with $C_{1-6}$ alkoxy, $C_{4-6}$ alkyl substituted with $C_{1-6}$ alkoxy, branched $C_{3-6}$ alkyl substituted with $C_{1-6}$ alkoxy, or branched $C_{4-6}$ alkyl substituted with $C_{1-6}$ alkoxy.

In some embodiments, $R^{2A}$ may be H, phenyl, naphthyl, 5- to 7-membered heteroaryl containing N as a heteroatom, or $C_{1-6}$ alkyl optionally substituted with OH or $C_{1-6}$ alkoxy. For example, $R^{2A}$ may be methyl, phenyl, pyridinyl,

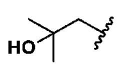 , 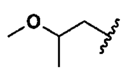 or 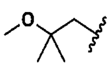 .

In some embodiments, $R^{2A}$ may be other than

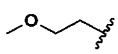 , 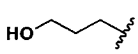 or 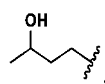 .

In some embodiments, when Z is N, $R^{2A}$ may be other than

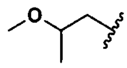.

In Formula 2A, $R^x$ and $R^y$ are each independently H or $C_{1-6}$ alkyl. In some embodiments, $R^x$ and $R^y$ may be the same or different. In some embodiments, $R^x$ and $R^y$ may each independently be H or $C_{1-4}$ alkyl. In some embodiments, $R^x$ and $R^y$ may each independently be H or $C_{1-3}$ alkyl. For example, $R^x$ and $R^y$ may each be H.

In some embodiments, in Formula 2A, X is halogen, $R^{2A}$ is methyl, phenyl, pyridinyl,

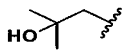, 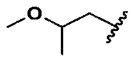 or 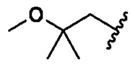,

and $R^x$ and $R^y$ may each independently be H.

In some embodiments, the compound represented by Formula 2A may be represented by the following Formula 2A-1.

[Formula 2A-1]

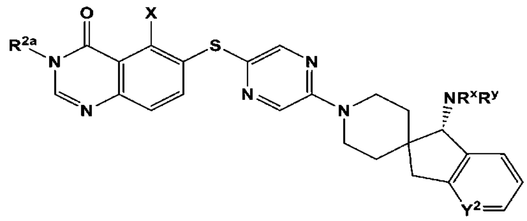

In Formula 2A-1, X is H or halogen; $Y^2$ is N or CH; $R^{2a}$ is H or $C_{1-6}$ alkyl; $R^x$ and $R^y$ may each independently be H or $C_{1-6}$ alkyl.

In Formula 2A-1, X is H or halogen. In some embodiments, X may be halogen. In some embodiments, X may be F, Cl, Br, or I. For example, X may be Cl.

In Formula 2A-1, $Y^2$ is N or CH.

In Formula 2A-1, $R^{2a}$ is H or $C_{1-6}$ alkyl. In some embodiments, $R^{2a}$ may be $C_{1-6}$ alkyl. In some embodiments, $R^{2a}$ may be methyl, ethyl, propyl, butyl, pentyl, or hexyl. In some embodiments, $R^{2a}$ may be $C_{1-4}$ alkyl or $C_{1-3}$ alkyl. For example, $R^{2a}$ may be $CH_3$.

In Formula 2A-1, $R^x$ and $R^y$ are each independently H or $C_{1-6}$ alkyl. In some embodiments, $R^x$ and $R^y$ may be the same or different. In some embodiments, $R^x$ and $R^y$ may each independently be H or $C_{1-4}$ alkyl. In some embodiments, $R^x$ and $R^y$ may each independently be H or $C_{1-3}$ alkyl. For example, $R^x$ and $R^y$ may each be H.

In some embodiments, in Formula 2A-1, X is halogen, $R^{2a}$ is $C_{1-6}$ alkyl, and $R^x$ and $R^y$ may each independently be H.

In some embodiments, the compound represented by Formula 2A or Formula 2A-1 may be selected from compounds represented by the following formulas.

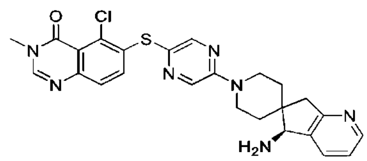

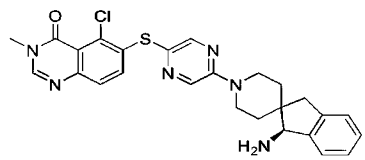

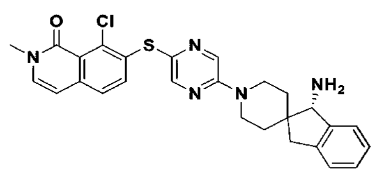

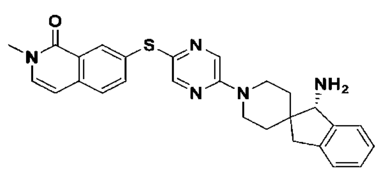

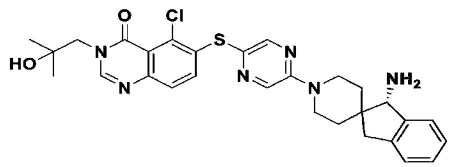

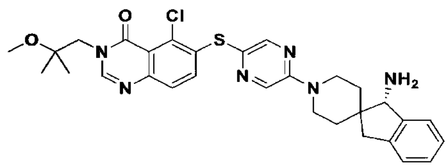

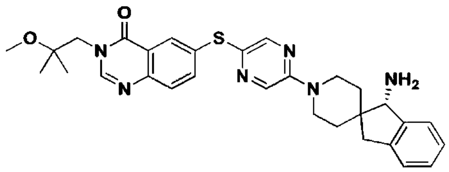

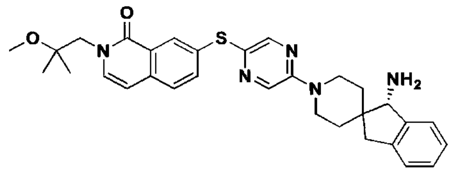

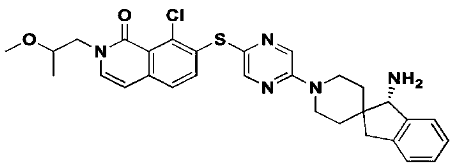

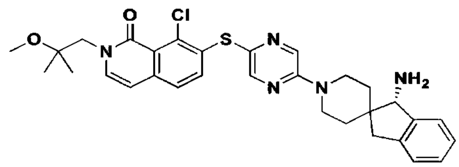

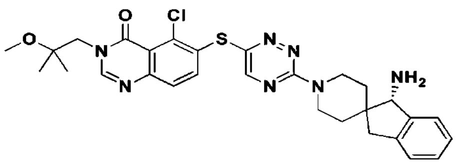

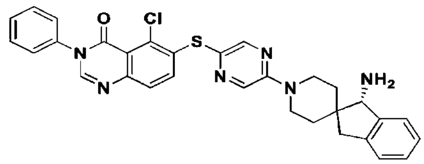

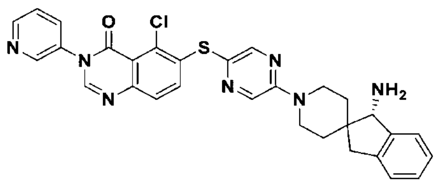.

In some embodiments, the compound represented by Formula 2 may be represented by the following Formula 2B:

[Formula 2B]

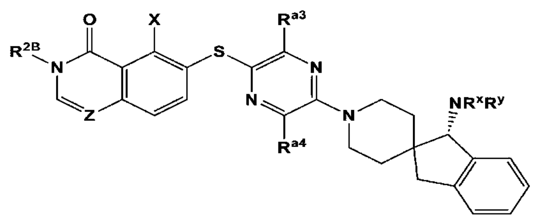

In Formula 2B, Z is N or CH; X is H or halogen. $R^{a3}$ is $NR^iR^{ii}$ or $C_{1-6}$ alkyl. $R^{a4}$ is H, or $C_{1-6}$ alkyl substituted with OH or $C_{1-6}$ alkoxy. $R^{2B}$ is selected from H, and $C_{1-6}$ alkyl optionally substituted with OH or $C_{1-6}$ alkoxy. $R^i$ and $R^{ii}$ are each independently H or $C_{1-6}$ alkyl. $R^x$ and $R^y$ are each independently H or $C_{1-6}$ alkyl.

In Formula 2B, X is H or halogen. In some embodiments, X may be halogen. In some embodiments, X may be F, Cl, Br, or I. For example, X may be Cl.

In Formula 2B, $R^{2B}$ is selected from H, and $C_{1-6}$ alkyl optionally substituted with OH or $C_{1-6}$ alkoxy. The $C_{1-6}$ alkoxy may be methoxy, ethoxy, propoxy, butoxy, pentoxy, or hexoxy. In some embodiments, the $C_{1-6}$ alkoxy may be $C_{1-4}$ alkoxy or $C_{1-3}$ alkoxy. For example, the $C_{1-6}$ alkoxy may be methoxy. The $C_{1-6}$ alkyl may be methyl, ethyl, propyl, butyl, pentyl, or hexyl. For example, the $C_{1-6}$ alkyl may be methyl, ethyl, propyl, or isobutyl. In some embodiments, $R^{2B}$ may be $C_{1-6}$ alkyl optionally substituted with OH or $C_{1-6}$ alkoxy. For example, $R^{2B}$ may be selected from the group consisting of $CH_3$,

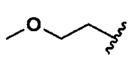, 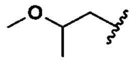, 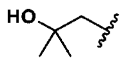, and 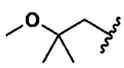.

In Formula 2B, $R^{a3}$ is $NR^iR^{ii}$ or $C_{1-6}$ alkyl. $R^{a4}$ is H, or $C_{1-6}$ alkyl substituted with OH or $C_{1-6}$ alkoxy. In some embodiments, $R^{a3}$ may be $NR^iR^{ii}$, and $R^{a4}$ may be H. Alternatively, $R^{a3}$ may be $C_{1-6}$ alkyl, and $R^{a4}$ may be $C_{1-6}$ alkyl substituted with OH or $C_{1-6}$ alkoxy. In some embodiments, $R^{a3}$ may be $NH_2$, and $R^{a4}$ may be H. Additionally, $R^{a3}$ may be $C_{1-4}$ alkyl, and $R^{a4}$ may be $C_{1-4}$ alkyl substituted with OH or $C_{1-6}$ alkoxy. For example, $R^{a3}$ may be $NH_2$ and $R^{a4}$ may be H. Further, $R^{a3}$ may be methyl, and $R^{a4}$ may be hydroxymethyl.

In Formula 2B, $R^i$ and $R^{ii}$ are each independently H or $C_{1-6}$ alkyl. In some embodiments, $R^i$ and $R^{ii}$ may be the same or different. In some embodiments, $R^i$ and $R^{ii}$ may each independently be H or $C_{1-4}$ alkyl. In some embodiments, $R^i$ and $R^{ii}$ may each independently be H or $C_{1-3}$ alkyl. For example, $R^i$ and $R^{ii}$ may each be H.

In Formula 2B, $R^x$ and $R^y$ are each independently H or $C_{1-6}$ alkyl. In some embodiments, $R^x$ and $R^y$ may be the same or different. In some embodiments, $R^x$ and $R^y$ may each independently be H or $C_{1-4}$ alkyl. In some embodiments, $R^x$ and $R^y$ may each independently be H or $C_{1-3}$ alkyl. For example, $R^x$ and $R^y$ may each be H.

In some embodiments, in Formula 2B, X is halogen, $R^{2B}$ may be selected from the group consisting of $CH_3$,

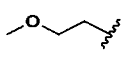, 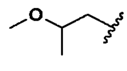, 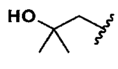, and 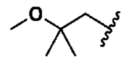,

and $R^i$, $R^{ii}$ , $R^x$, and $R^y$ may each independently be H.

In some embodiments, the compound represented by Formula 2B may be represented by the following Formula 2B-1.

[Formula 2B-1]

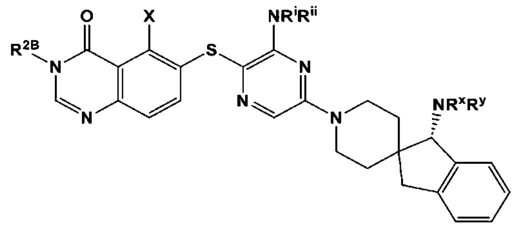

In Formula 2B-1, X is H or halogen; $R^{2B}$ is selected from H, and $C_{1-6}$ alkyl optionally substituted with OH or $C_{1-6}$ alkoxy; $R^i$ and $R^{ii}$ are each independently H or $C_{1-6}$ alkyl; $R^x$ and $R^y$ are each independently H or $C_{1-6}$ alkyl.

In Formula 2B-1, X is H or halogen. In some embodiments, X may be halogen. In some embodiments, X may be F, Cl, Br, or I. For example, X may be Cl.

In Formula 2B-1, $R^{2B}$ is selected from H, and $C_{1-6}$ alkyl optionally substituted with OH or $C_{1-6}$ alkoxy. The $C_{1-6}$ alkoxy may be methoxy, ethoxy, propoxy, butoxy, pentoxy, or hexoxy. In some embodiments, the $C_{1-6}$ alkoxy may be $C_{1-4}$ alkoxy or $C_{1-3}$ alkoxy. For example, the $C_{1-6}$ alkoxy may be methoxy. The $C_{1-6}$ alkyl may be methyl, ethyl, propyl, butyl, pentyl, or hexyl. For example, the $C_{1-6}$ alkyl may be methyl, ethyl, propyl, or isobutyl. In some embodiments, $R^{2B}$ may be $C_{1-6}$ alkyl optionally substituted with OH or $C_{1-6}$ alkoxy. For example, $R^{2B}$ may be selected from the group consisting of $CH_3$,

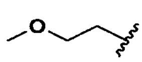, 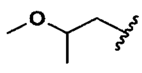, and 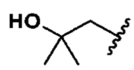.

In Formula 2B-1, $R^i$ and $R^{ii}$ are each independently H or $C_{1-6}$ alkyl. In some embodiments, $R^i$ and $R^{ii}$ may be the same or different. In some embodiments, $R^i$ and $R^{ii}$ may each independently be H or $C_{1-4}$ alkyl. In some embodiments, $R^i$ and $R^{ii}$ may each independently be H or $C_{1-3}$ alkyl. For example, $R^i$ and $R^{ii}$ may each be H.

In Formula 2B-1, $R^x$ and $R^y$ are each independently H or $C_{1-6}$ alkyl. In some embodiments, $R^x$ and $R^y$ may be the same or different. In some embodiments, $R^x$ and $R^y$ may each independently be H or $C_{1-4}$ alkyl. In some embodiments, $R^x$ and $R^y$ may each independently be H or $C_{1-3}$ alkyl. For example, $R^x$ and $R^y$ may each be H.

In some embodiments, in Formula 2B-1, X may be halogen, $R^{2B}$ may be selected from the group consisting of $CH_3$,

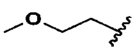, 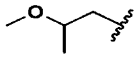, and 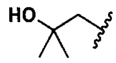,

and $R^i$, $R^{ii}$, $R^x$, and $R^y$ may each independently be H.

In some embodiments, the compound represented by Formula 2B or Formula 2B-1 may be selected from compounds represented by the following formulas.

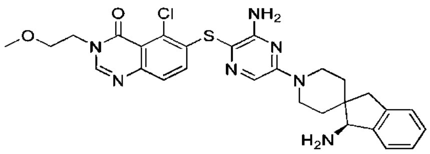

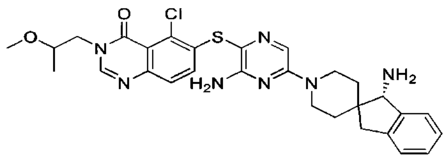

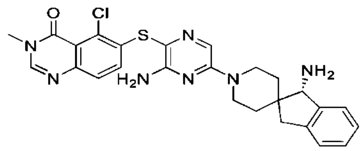

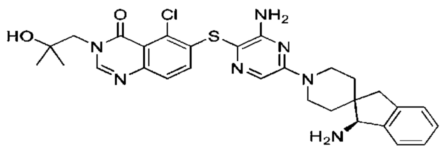

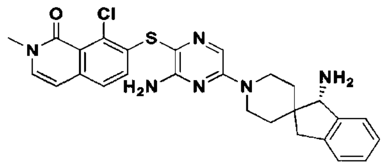

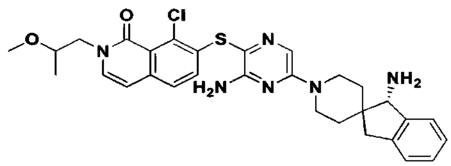

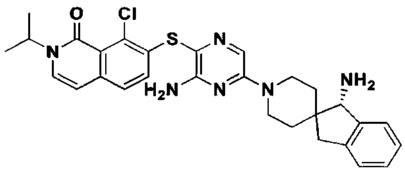

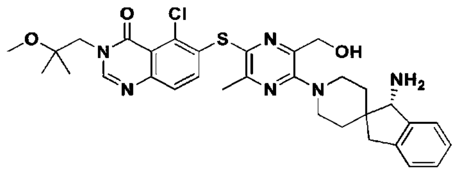

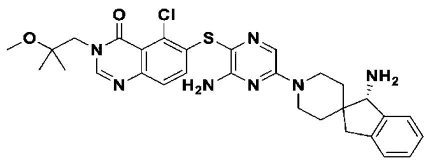.

In some embodiments, the compound represented by Formula 2 may be represented by the following Formula 2C.

[Formula 2C]

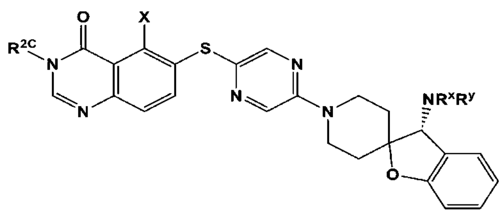

In Formula 2C, X is H or halogen; $R^{2C}$ is selected from H, $C_{6-10}$ aryl, and $C_{1-6}$ alkyl optionally substituted with OH or $C_{1-6}$ alkoxy. $R^x$ and $R^y$ are each independently H or $C_{1-6}$ alkyl.

In Formula 2C, X is H or halogen. In some embodiments, X may be halogen. In some embodiments, X may be F, Cl, Br or I. For example, X may be Cl.

In Formula 2C, $R^{2C}$ is selected from H, $C_{6-10}$ aryl, and $C_{1-6}$ alkyl optionally substituted with OH or $C_{1-6}$ alkoxy. The $C_{1-6}$ alkoxy can be methoxy, ethoxy, propoxy, butoxy, pentoxy, or hexoxy. In some embodiments, the $C_{1-6}$ alkoxy can be

$C_{1-4}$ alkoxy or $C_{1-3}$ alkoxy. For example, the $C_{1-6}$ alkoxy may be methoxy. The $C_{1-6}$ alkyl may be methyl, ethyl, propyl, butyl, pentyl, or hexyl. In some embodiments, the $C_{1-6}$ alkyl may be $C_{1-4}$ alkyl. For example, the $C_{1-6}$ alkyl may be methyl, ethyl, or butyl (e.g., n-butyl, isobutyl, or tert-butyl). In some embodiments, $R^{2C}$ may be $C_{6-10}$ aryl, or $C_{1-6}$ alkyl optionally substituted with OH or $C_{1-6}$ alkoxy. For example, $R^{2C}$ may be

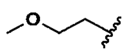 , 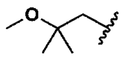 ,

methyl, $CD_3$ or phenyl.

In Formula 2C, $R^x$ and $R^y$ are each independently H or $C_{1-6}$ alkyl. In some embodiments, $R^x$ and $R^y$ may be the same or different. In some embodiments, $R^x$ and $R^y$ may each independently be H or $C_{1-4}$ alkyl. In some embodiments, $R^x$ and $R^y$ may each independently be H or $C_{1-3}$ alkyl. For example, $R^x$ and $R^y$ may each be H.

In some embodiments, in Formula 2C, X may be halogen, $R^{2C}$ may be $C_{1-6}$ alkyl substituted with OH or $C_{1-6}$ alkoxy, and $R^x$ and $R^y$ may each independently be H.

In some embodiments, the compound represented by Formula 2C may be a compound represented by the following formula.

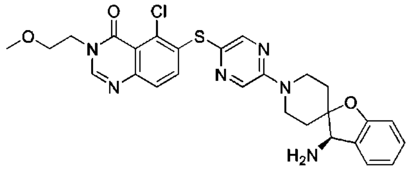

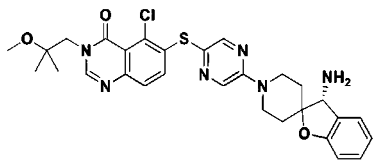

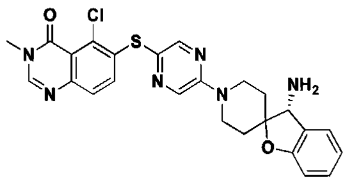

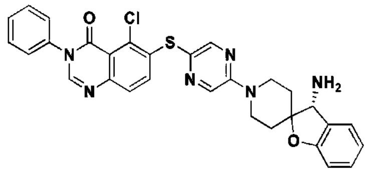

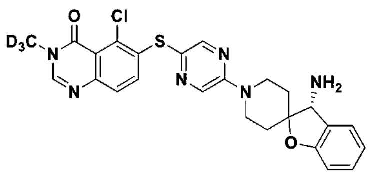

In one aspect, there is provided a compound represented by the following Formula 3, a stereoisomer, solvate or isotope-labeled compound thereof, or a pharmaceutically acceptable salt thereof:

[Formula 3]

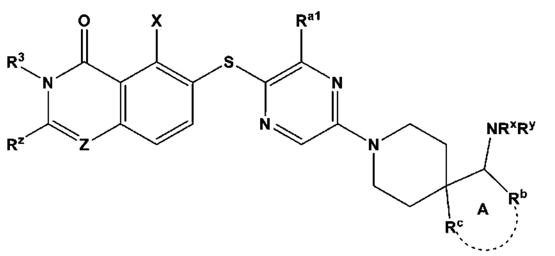

In Formula 3, Z is N or CH; $R^z$ is H or $C_{1-6}$ alkyl; X is H or halogen. $R^3$ is H, -($C_{1-6}$ alkylene)-oxanyl substituted with $C_{1-6}$ alkyl, or $C_{1-6}$ alkyl, -($C_{1-6}$ alkylene)-($C_{3-6}$ cycloalkyl) or - ($C_{1-6}$ alkylene)-piperidinyl, each optionally substituted with one to three occurences of $R^{31}$; $R^{31}$ is halogen, cyano, OH, $C_{1-6}$ alkoxy or $C_{6-12}$ aryl. $R^{a1}$ is H, $NH_2$, $C_{1-6}$ alkylamino or di($C_{1-6}$ alkyl)amino. $R^b$ and $R^c$ are each independently H or $C_{1-6}$ alkyl, or are connected to each other, together with the carbon atom to which they are attached, to form Ring A which is a 5-membered ring optionally containing O. Ring A may be optionally substituted with $R^A$. $R^A$ is H or $C_{1-6}$ alkyl. $R^x$ and $R^y$ are each independently H or $C_{1-6}$ alkyl.

In some embodiments, the compound represented by Formula 3 may be represented by the following Formula

3A.

[Formula 3A]

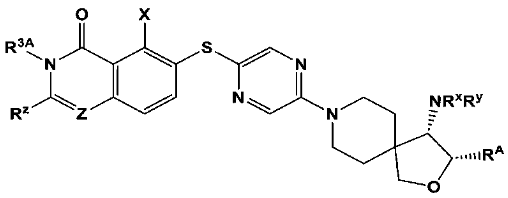

In Formula 3A, Z is N or CH; X is H or halogen; $R^z$ is H or $C_{1-6}$ alkyl. $R^{3A}$ is H, -($C_{1-6}$ alkylene)-oxanyl substituted with $C_{1-6}$ alkyl, or $C_{1-6}$ alkyl, -($C_{1-6}$ alkylene)-($C_{3-6}$ cycloalkyl) or - ($C_{1-6}$ alkylene)-piperidinyl, each optionally substituted with one to three occurences of $R^{31A}$. $R^{31A}$ is halogen, cyano, or $C_{1-6}$ alkoxy. $R^A$ is H or $C_{1-6}$ alkyl. $R^x$ and $R^y$ are each independently H or $C_{1-6}$ alkyl.

In Formula 3A, X is H or halogen. In some embodiments, X may be halogen. In some embodiments, X may be F, Cl, Br, or I. For example, X may be Cl.

In Formula 3A, $R^z$ is H or $C_{1-6}$ alkyl. In some embodiments, $R^z$ may be H or $C_{1-4}$ alkyl. In some embodiments, $R^z$ may be H or $C_{1-3}$ alkyl. For example, $R^z$ may be H or methyl. In some embodiments, when $R^z$ is $C_{1-6}$ alkyl, Z may be N.

In Formula 3A, $R^{3A}$ is H, -($C_{1-6}$ alkylene)-oxanyl substituted with $C_{1-6}$ alkyl, or $C_{1-6}$ alkyl, -($C_{1-6}$ alkylene)-($C_{3-6}$ cycloalkyl) or -($C_{1-6}$ alkylene)-piperidinyl, each optionally substituted with one to three occurences of $R^{31A}$. $R^{31A}$ is halogen, cyano, OH or $C_{1-6}$ alkoxy. Two or more $R^{31A}$ may be the same or different. The $C_{1-6}$ alkyl may be methyl, ethyl, propyl, butyl, pentyl or hexyl. For example, the $C_{1-6}$ alkyl may be methyl, ethyl, n-propyl, isopropyl, or isobutyl. The $C_{3-6}$ cycloalkyl may be cyclopropyl, cyclobutyl, cyclopentyl or cyclohexyl. For example, the $C_{3-6}$ cycloalkyl may be cyclopropyl. In some embodiments, the -($C_{1-6}$ alkyl)-($C_{3-6}$ cycloalkyl) may be -($C_{1-4}$ alkyl)-($C_{3-6}$ cycloalkyl) or -($C_{1-3}$ alkyl)-($C_{3-6}$ cycloalkyl). For example, the -($C_{1-6}$ alkyl)-($C_{3-6}$ cycloalkyl) may be methyl substituted with cyclopropyl (cyclopropyl-methyl-). The -($C_{1-6}$ alkylene)-oxanyl substituted with $C_{1-6}$ alkyl may be -($C_{1-4}$ alkylene)-oxanyl substituted with $C_{1-6}$ alkyl or -($C_{1-3}$ alkylene)-oxanyl substituted with $C_{1-6}$ alkyl. For example, the -($C_{1-6}$ alkylene)-oxanyl substituted with $C_{1-6}$ alkyl may be oxanylmethyl. The -($C_{1-6}$ alkylene)-piperidinyl may be -($C_{1-4}$ alkylene)-piperidinyl or -($C_{1-3}$ alkylene)-piperidinyl. For example, the -($C_{1-6}$ alkylene)-piperidinyl may be piperidinylmethyl.

In some embodiments, $R^{31A}$ is halogen, cyano, or $C_{1-6}$ alkoxy. In some embodiments, $R^{31A}$ may be halogen, cyano, or $C_{1-4}$ alkoxy. In some embodiments, $R^{31A}$ may be halogen, cyano, or $C_{1-3}$ alkoxy. For example, $R^{31A}$ may be cyano, F, or methoxy.

For example, $R^{3A}$ may be selected from the group consisting of $CH_3$,

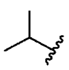, 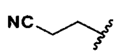, 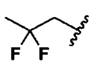, 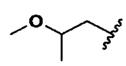, 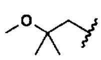, 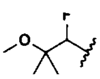, 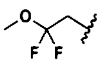, 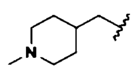, 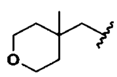

and

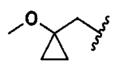.

In some embodiments, $R^{3A}$ may be other than H,

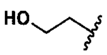,

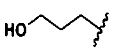, 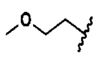, 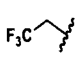, 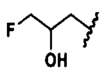, 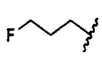, 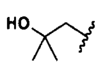, 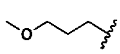, 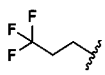, 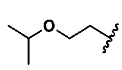, 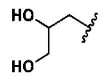

or

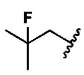.

In some embodiments, when Z is N, $R^{3A}$ may be other than

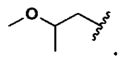.

In Formula 3A, $R^A$ is H or $C_{1-6}$ alkyl. The $C_{1-6}$ alkyl may be methyl, ethyl, propyl, butyl, pentyl, or hexyl. In some embodiments, $R^A$ may be $C_{1-6}$ alkyl. In some embodiments, the $C_{1-6}$ alkyl may be $C_{1-4}$ alkyl or $C_{1-3}$ alkyl. For example, $R^A$ may be methyl.

In Formula 3A, $R^x$ and $R^y$ are each independently H or $C_{1-6}$ alkyl. In some embodiments, $R^x$ and $R^y$ may be the same or different. In some embodiments, $R^x$ and $R^y$ may each independently be H or $C_{1-4}$ alkyl. In some embodiments, $R^x$ and $R^y$ may each independently be H or $C_{1-3}$ alkyl. For example, $R^x$ and $R^y$ may each be H.

In some embodiments, in Formula 3A, X may be halogen, $R^{3A}$ may be selected from the group consisting of $CH_3$,

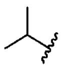, 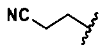, 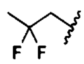, 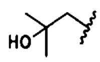, 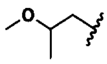, 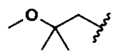, 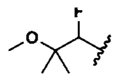,

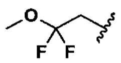, 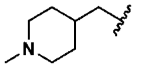, 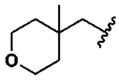 and 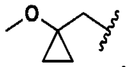,

$R^A$ may be $C_{1-6}$ alkyl, and $R^x$ and $R^y$ may each independently be H.

In some embodiments, the compound represented by Formula 3A may be represented by the following Formula 3A-1.

[Formula 3A-1]

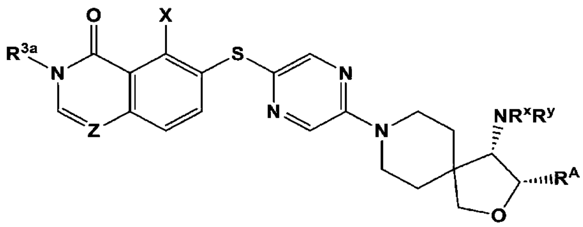

In Formula 3A-1, Z is N or CH; X is H or halogen; $R^{3a}$ is H, or $C_{1-6}$ alkyl or -($C_{1-6}$ alkyl)-($C_{3-6}$ cycloalkyl), each optionally substituted with one to three occurences of $R^{31a}$; $R^{31a}$ is halogen, OH or $C_{1-6}$ alkoxy; $R^A$ is H or $C_{1-6}$ alkyl; $R^x$ and $R^y$ are each independently H or $C_{1-6}$ alkyl.

In Formula 3A-1, X is H or halogen. In some embodiments, X may be halogen. In some embodiments, X may be F, Cl, Br or I. For example, X may be Cl.

In Formula 3A-1, $R^{3a}$ is H, or $C_{1-6}$ alkyl or -($C_{1-6}$ alkyl)-($C_{3-6}$ cycloalkyl), each optionally substituted with one to three occurences of $R^{31a}$, and $R^{31a}$ is halogen, OH or $C_{1-6}$ alkoxy. Two or more $R^{31a}$ may be the same or different. The $C_{1-6}$ alkyl may be methyl, ethyl, propyl, butyl, pentyl or hexyl. For example, the $C_{1-6}$ alkyl may be methyl, ethyl, propyl or isobutyl. The $C_{3-6}$ cycloalkyl may be cyclopropyl, cyclobutyl, cyclopentyl or cyclohexyl. For example, the $C_{3-6}$ cycloalkyl may be cyclopropyl. In some embodiments, the -($C_{1-6}$ alkyl)-($C_{3-6}$ cycloalkyl) may be -($C_{1-4}$ alkyl)-($C_{3-6}$ cycloalkyl) or -($C_{1-3}$ alkyl)-($C_{3-6}$ cycloalkyl). For example, the -($C_{1-6}$ alkyl)-($C_{3-6}$ cycloalkyl) may be methyl substituted with cyclopropyl (cyclopropylmethyl-). In some embodiments, $R^{31a}$ may be halogen or $C_{1-6}$ alkoxy. In some embodiments, $R^{31a}$ may be halogen or $C_{1-4}$ alkoxy. In some embodiments, $R^{31a}$ may be halogen or $C_{1-3}$ alkoxy. For example, $R^{3a}$ may be selected from the group consisting of $CH_3$,

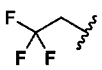, 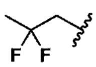, 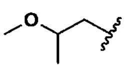', 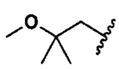, 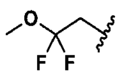 and 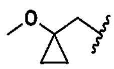.

In some embodiments, Z may be N, $R^{3a}$ may be $C_{1-6}$ alkyl or -($C_{1-6}$ alkyl)-($C_{3-6}$ cycloalkyl), each substituted with one to three occurrences of $R^{3a}$, and $R^{3a}$ may be halogen, OH, or $C_{1-6}$ alkoxy.

In some embodiments, Z may be CH, and $R^{3a}$ may be H, or $C_{1-6}$ alkyl optionally substituted with $R^{3a}$. In this case, $R^{3a}$ may be OH or $C_{1-6}$ alkoxy.

In Formula 3A-1, $R^A$ is H or $C_{1-6}$ alkyl. The $C_{1-6}$ alkyl may be methyl, ethyl, propyl, butyl, pentyl, or hexyl. In some embodiments, $R^A$ can be $C_{1-6}$ alkyl. In some embodiments, the $C_{1-6}$ alkyl may be $C_{1-4}$ alkyl or $C_{1-3}$ alkyl. For example, $R^A$ may be methyl.

In Formula 3A-1, $R^x$ and $R^y$ are each independently H or $C_{1-6}$ alkyl. In some embodiments, $R^x$ and $R^y$ may be the same or different. In some embodiments, $R^x$ and $R^y$ may each independently be H or $C_{1-4}$ alkyl. In some embodiments, $R^x$ and $R^y$ may each independently be H or $C_{1-3}$ alkyl. For example, $R^x$ and $R^y$ may each be H.

In some embodiments, in Formula 3A-1, X may be halogen, $R^{3A}$ may be selected from the group consisting of $CH_3$,

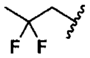, 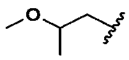, 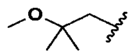, 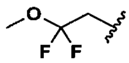 and 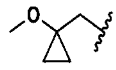,

$R^A$ may be $C_{1-6}$ alkyl, and $R^x$ and $R^y$ may each independently be H.

In some embodiments, the compound represented by Formula 3A or Formula 3A-1 may be selected from compounds represented by the following formulas.

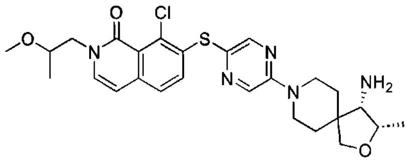
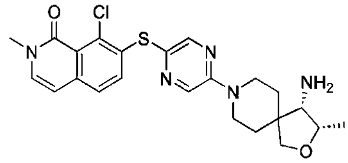

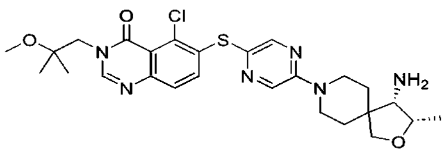
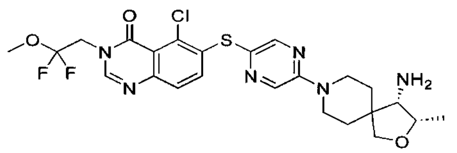

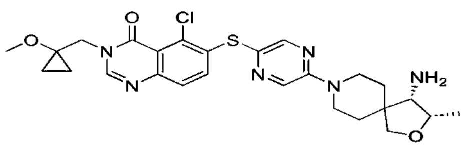
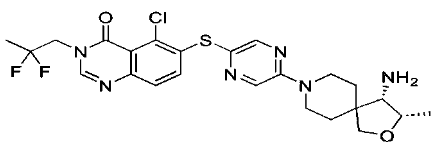

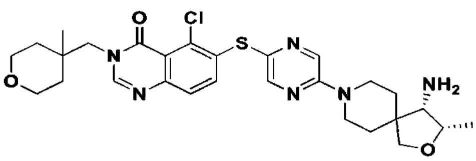
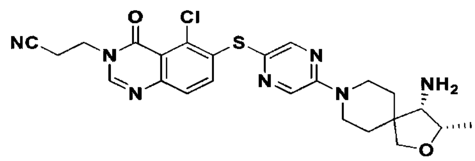

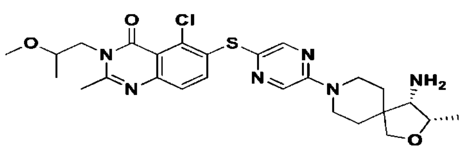
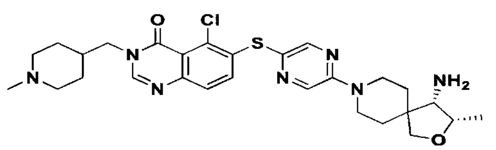

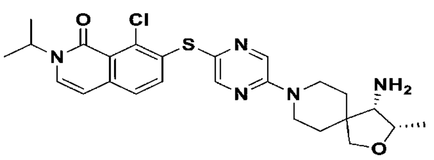
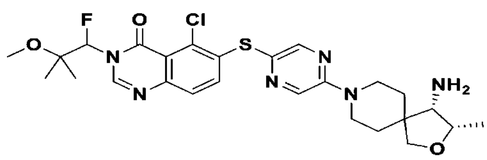.

In some embodiments, the compound represented by Formula 3 may be represented by the following Formula 3B:

[Formula 3B]

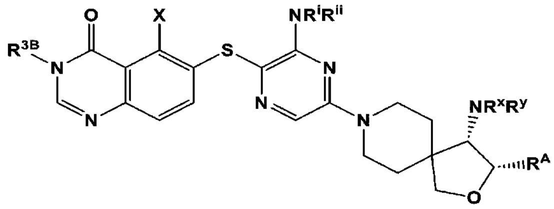

In Formula 3B, X is H or halogen. $R^{3B}$ is $C_{1-6}$ alkyl optionally substituted with OH, $C_{1-6}$ alkoxy or $C_{6-10}$ aryl. $R^A$ is H or $C_{1-6}$ alkyl; $R^i$ and $R^{ii}$ are each independently H or $C_{1-6}$ alkyl; and $R^x$ and $R^y$ are each independently H or $C_{1-6}$ alkyl.

In Formula 3B, X is H or halogen. In some embodiments, X may be halogen. In some embodiments, X may be F, Cl, Br, or I. For example, X may be Cl.

In Formula 3B, $R^{3B}$ is $C_{1-6}$ alkyl optionally substituted with OH, $C_{1-6}$ alkoxy or $C_{6-10}$ aryl. The $C_{1-6}$ alkoxy may be $C_{1-4}$ alkoxy or $C_{1-3}$ alkoxy. For example, the $C_{1-6}$ alkoxy may be methoxy. The $C_{6-10}$ aryl may be phenyl or naphthyl. For example, the $C_{6-10}$ aryl may be phenyl. The $C_{1-6}$ alkyl can be methyl, ethyl, propyl, butyl, pentyl or hexyl. In some embodiments, the $C_{1-6}$ alkyl may be $C_{1-4}$ alkyl or $C_{1-3}$ alkyl. For example, the $C_{1-6}$ alkyl may be methyl or isobutyl. In some embodiments, $R^{3B}$ may be $C_{1-6}$ alkyl substituted with $C_{1-6}$ alkoxy or $C_{6-10}$ aryl. For example, $R^{3B}$ may include $C_{3-6}$ alkyl substituted with $C_{1-6}$ alkoxy, $C_{4-6}$ alkyl substituted with $C_{1-6}$ alkoxy, branched $C_{3-6}$ alkyl substituted with $C_{1-6}$ alkoxy, branched $C_{4-6}$ alkyl substituted with $C_{1-6}$ alkoxy, or $C_{1-6}$ alkyl substituted with $C_{6-10}$ aryl. In some embodiments, $R^{3B}$ may be $C_{1-4}$ alkyl substituted with $C_{1-4}$ alkoxy or $C_{6-10}$ aryl. For example, $R^{3B}$ may be phenylmethyl (benzyl) or

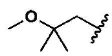.

In some embodiments, $R^{3B}$ may be other than

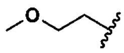.

In Formula 3B, $R^A$ is H or $C_{1-6}$ alkyl. The $C_{1-6}$ alkyl may be methyl, ethyl, propyl, butyl, pentyl, or hexyl. In some embodiments, $R^A$ may be $C_{1-6}$ alkyl. In some embodiments, the $C_{1-6}$ alkyl can be $C_{1-4}$ alkyl or $C_{1-3}$ alkyl. For example, $R^A$ may be methyl.

In Formula 3B, $R^i$ and $R^{ii}$ are each independently H or $C_{1-6}$ alkyl. In some embodiments, $R^i$ and $R^{ii}$ may be the same or different. In some embodiments, $R^i$ and $R^{ii}$ may each independently be H or $C_{1-4}$ alkyl. In some embodiments, $R^i$ and $R^{ii}$ may each independently be H or $C_{1-3}$ alkyl. For example, $R^i$ and $R^{ii}$ may each be H.

In Formula 3B, $R^x$ and $R^y$ are each independently H or $C_{1-6}$ alkyl. In some embodiments, $R^x$ and $R^y$ may be the same or different. In some embodiments, $R^x$ and $R^y$ may each independently be H or $C_{1-4}$ alkyl. In some embodiments, $R^x$ and $R^y$ may each independently be H or $C_{1-3}$ alkyl. For example, $R^x$ and $R^y$ may each be H.

In some embodiments, X may be halogen, $R^{3B}$ may be $C_{1-6}$ alkyl substituted with $C_{6-10}$ aryl, $R^A$ may be $C_{1-6}$ alkyl, and $R^i$, $R^{ii}$, $R^x$, and $R^y$ may each independently be H.

In some embodiments, the compound represented by Formula 3B may be a compound represented by the following formula.

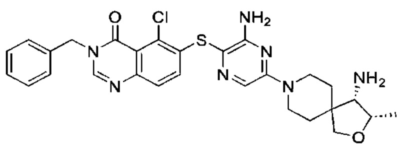

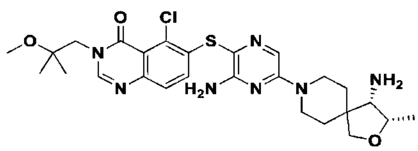.

In some embodiments, the compound represented by Formula 3 may be represented by the following Formula 3C.

[Formula 3C]

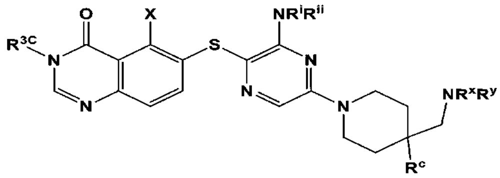

In Formula 3C, X is H or halogen; $R^{3C}$ is $C_{1-6}$ alkyl optionally substituted with $C_{6-10}$ aryl; $R^c$ is H or $C_{1-6}$ alkyl; $R^i$ and $R^{ii}$ are each independently H or $C_{1-6}$ alkyl; and $R^x$ and $R^y$ are each independently H or $C_{1-6}$ alkyl.

In Formula 3C, X is H or halogen. In some embodiments, X may be halogen. In some embodiments, X may be F, Cl, Br, or I. For example, X may be Cl.

In Formula 3C, $R^{3C}$ is $C_{1-6}$ alkyl optionally substituted with $C_{6-10}$ aryl. The $C_{6-10}$ aryl may be phenyl or naphthyl. For example, the $C_{6-10}$ aryl may be phenyl. The $C_{1-6}$ alkyl may be methyl, ethyl, propyl, butyl, pentyl or hexyl. In some embodiments, the $C_{1-6}$ alkyl may be $C_{1-4}$ alkyl or $C_{1-3}$ alkyl. For example, the $C_{1-6}$ alkyl may be methyl. For example, $R^{3C}$ may be phenylmethyl (benzyl).

In Formula 3C, $R^c$ is H or $C_{1-6}$ alkyl. The $C_{1-6}$ alkyl may be methyl, ethyl, propyl, butyl, pentyl, or hexyl. In some embodiments, $R^A$ may be $C_{1-6}$ alkyl. In some embodiments, the $C_{1-6}$ alkyl may be $C_{1-4}$ alkyl or $C_{1-3}$ alkyl. For example, $R^c$ may be methyl.

In Formula 3C, $R^i$ and $R^{ii}$ are each independently H or $C_{1-6}$ alkyl. In some embodiments, $R^i$ and $R^{ii}$ may be the same or different. In some embodiments, $R^i$ and $R^{ii}$ may each independently be H or $C_{1-4}$ alkyl. In some embodiments, $R^i$ and $R^{ii}$ may each independently be H or $C_{1-3}$ alkyl. For example, $R^i$ and $R^{ii}$ may each be H.

In Formula 3C, $R^x$ and $R^y$ are each independently H or $C_{1-6}$ alkyl. In some embodiments, $R^x$ and $R^y$ may be the same or different. In some embodiments, $R^x$ and $R^y$ may each independently be H or $C_{1-4}$ alkyl. In some embodiments, $R^x$ and $R^y$ may each independently be H or $C_{1-3}$ alkyl. For example, $R^x$ and $R^y$ may each be H.

In some embodiments, the compound represented by Formula 3C may be a compound represented by the following formula.

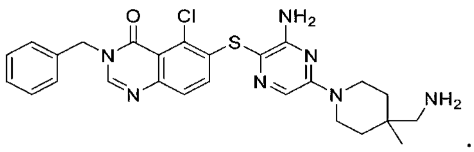

As used herein, the term "halogen" or "halogen atom" refers to an atom belonging to Group 17 of the periodic table. Halogen atoms include F, Cl, Br, I and the like.

The term "alkyl" refers to a fully saturated branched or unbranched (or straight-chain or linear) hydrocarbon. The alkyl may be a substituted or unsubstituted alkyl. The $C_1$-$C_{20}$ alkyl may be, for example, $C_1$-$C_{15}$, $C_1$-$C_{10}$, or $C_1$-$C_6$ alkyl. The $C_1$-$C_6$ alkyl may be $C_1$-$C_5$, $C_1$-$C_4$, $C_1$-$C_3$, or $C_1$-$C_2$ alkyl. The alkyl may be, for example, methyl, ethyl, n-propyl, isopropyl, n-butyl, isobutyl, sec-butyl, n-pentyl, isopentyl, neopentyl, iso-amyl, or n-hexyl.

The term "haloalkyl" refers to an alkyl substituted with at least one halogen.

The term "hydroxy" refers to an -OH functional group (hydroxyl group).

The term "carbonyl" refers to -C(=O)-.

The term "alkoxy" refers to an alkyl bound to an oxygen atom. The $C_1$-$C_{20}$ alkoxy may be, for example, $C_1$-$C_{15}$, $C_1$-$C_{10}$, or $C_1$-$C_6$ alkoxy. The $C_1$-$C_6$ alkoxy may be $C_1$-$C_5$, $C_1$-$C_4$, $C_1$-$C_3$, or $C_1$-$C_2$ alkoxy. The alkoxy may be methoxy, ethoxy, propoxy, butoxy, and the like.

The term "alkoxyalkyl" refers to alkoxy bound to an alkyl. The $C_2$-$C_{20}$ alkoxyalkyl may be, for example, $C_2$-$C_{15}$, $C_2$-$C_{10}$, or $C_2$-$C_6$ alkoxyalkyl. For example, $C_2$-$C_{20}$ alkoxyalkyl may be ($C_1$-$C_{10}$ alkoxy)-($C_1$-$C_{10}$ alkyl), ($C_1$-$C_6$ alkoxy)-($C_1$-$C_6$ alkyl) and the like, and the number of carbon atoms in the alkoxy group and the alkyl group may be the same or different. The alkoxy may be, for example, methoxymethyl, methoxyethyl, ethoxymethyl, ethoxyethyl, methoxypropyl, ethoxypropyl, and the like.

The term "amino" refers to $-NH_2$.

The term "amine group" refers to a substituent in which one, two or all three hydrogens of ammonia are replaced with an organic functional group and includes all of primary amines, secondary amines and tertiary amines, also including an amino group.

The term "alkylamine" refers to an amine in which one H of the amino ($-NH_2$) is replaced with alkyl.

The term "di(alkyl)amine" refers to an amine in which both H of the amino ($-NH_2$) are replaced with alkyls. The two alkyls in the di(alkyl)amine may be the same or different.

The term "nitro" refers to $-NO_2$.

The term "cyano" refers to -CN, which is a functional group consisting of a triple bond between a carbon atom and a nitrogen atom.

The term "carboxy" refers to -COOH. A salt of carboxy refers to a conjugate base of a carboxylic acid.

The term "sulfonyl" refers to a $-SO_2-$ group.

The term "cycloalkyl" refers to a saturated non-aromatic monocyclic, bicyclic or tricyclic hydrocarbon group. The cycloalkyl may contain 3 to 20 carbon atoms, for example, 5 to 10, 3 to 8, or 3 to 6 carbon atoms. The monocyclic cycloalkyl can be, for example, cyclopropyl, cyclobutyl, cyclopentyl, or cyclohexyl, and the like. The bicyclic cycloalkyl may be, for example, bornyl, decahydronaphthyl, bicyclo[2.1.1]hexyl, bicyclo[2.2.1]heptyl, or bicyclo[2.2.2]octyl and the like. The tricyclic cycloalkyl may be, for example, adamantyl.

The term "cycloalkane ring" refers to a saturated non-aromatic monocyclic, bicyclic or tricyclic hydrocarbon ring. The cycloalkane ring can be a full (non-radical) functional group of the aforementioned cycloalkyl. The cycloalkane ring may contain 3 to 20 carbon atoms, for example 5 to 10, 3 to 8, or 3 to 6 carbon atoms. The monocyclic cycloalkane ring can be, for example, a cyclopropane ring, a cyclobutane ring, a cyclopentane ring, or a cyclohexane ring, and the like.

The term "aryl" also includes a group in which an aromatic ring is fused to one or more carbon rings. The $C_6$-$C_{30}$ aryl may be, for example, $C_6$-$C_{15}$, or $C_6$-$C_{10}$ aryl. Aryl may be phenyl, naphthyl, or tetrahydronaphthyl.

The term "arylalkyl" refers to an alkyl substituted with an aryl.

The term "aryloxy" refers to an aryl bound to an oxygen atom.

The term "heteroaryl" refers to a monocyclic or bicyclic aromatic compound containing one or more heteroatoms, the remaining ring atoms being carbon. The heteroaryl may include, for example, 1 to 5, 1 to 3, 1 or 2 heteroatoms, and may include 5 to 10 ring members. "Heteroaryl" may be, for example, pyrrolyl, furanyl, thiophenyl, pyrazolyl, imidazolyl, isoxazolyl, oxazolyl, thiazolyl, isothiazolyl, pyridinyl, pyrimidinyl, pyrazinyl, pyridazinyl, indolyl, benzofuranyl, benzothiophenyl, benzopyrazolyl, benzimidazolyl, benzooxazolyl, benzoisoxazolyl, benzothiazolyl, or benzoisothiazolyl, and the like.

The term "heterocycloalkyl" or "heterocyclyl" refers to a saturated or partially unsaturated cyclic hydrocarbon containing at least one heteroatom. The heterocyclyl ring group may be a single ring group, a two ring group, or a three ring group. The two ring groups may be a spiro-ring group, a bridged-ring group, and a fused-ring group. A heterocyclyl ring group may contain 3 to 20, 3 to 10, 3 to 8, 3 to 7, 5 to 7, 4 to 6, or 5 to 6 ring atoms. The heteroatom may be any one or more, for example, 1, 2 or 3 heteroatoms selected from the group consisting of N, O and S. For example, the heterocycloalkyl may be aziridinyl, oxiranyl, oxetanyl, azetidinyl, pyrrolidinyl, tetrahydrofuranyl, tetrahydrothiophenyl, piperidinyl, tetrahydropyranyl, dihydropyranyl, morpholinyl, thiomorpholinyl or oxazolidinyl, and the like.

The heteroatom may be any one or more selected from the group consisting of N, O, P, and S. The heteroatom may be 1, 2, or 3 heteroatoms selected from the group consisting of N, O and S.

The term "substituted" included in "substituted or unsubstituted" refers to replacement of hydrogen atoms and introduction of other atomic groups when a derivative is formed by substituting one or more hydrogen atoms in an organic compound with other atomic groups, and the term "substituent" refers to an atomic group as introduced. "Substituted" used herein without any limitation of a substituent may mean substitution with, for example, a halogen atom, $C_1$-$C_{20}$ alkyl substituted with a halogen atom (for example, $CCF_3$, $CHCF_2$, $CH_2F$, $CCl_3$ etc.), $C_1$-$C_{20}$ alkoxy, $C_2$-$C_{20}$ alkoxyalkyl, a hydroxy group, $-NH_2$, =NH, nitro, cyano, amidino, hydrazine, hydrazone, carboxy or a salt thereof, sulfonyl, sulfamoyl, a sulfonic acid group or a salt thereof, phosphoric acid or a salt thereof, $C_1$-$C_{20}$ alkyl, $C_2$-$C_{20}$ alkenyl, $C_2$-$C_{20}$ alkynyl, $C_6$-$C_{20}$ aryl, $C_6$-$C_{20}$ arylalkyl, $C_6$-$C_{20}$ heteroaryl, $C_7$-$C_{20}$ heteroarylalkyl, $C_6$-$C_{20}$ heteroaryloxy, $C_6$-$C_{20}$ heteroaryloxyalkyl, or $C_6$-$C_{20}$ heteroarylalkyl, and the like.

The term "isomer" included in "stereoisomer" refers to a compound that has the same molecular formula but differs in atomic connectivity or spatial arrangement of constituent atoms in the molecule. Isomers include, for example, structural isomers and stereoisomers. The stereoisomer may be a diastereomer or an enantiomer. Enantiomers refer to a pair of isomers that are non-superimposable mirror images, such as the relationship between the left and right hands, and are also called optical isomers. Enantiomers are divided into R(Rectus: clockwise) and S(Sinister: counterclockwise) when 4 or more substituents differ from each other at the chiral center carbon. Diastereomers refer to non-mirror image stereoisomers and are isomers generated by different spatial arrangement of atoms. The diastereomers may be divided into cis-trans isomers, and conformational isomers or conformers.

The term "solvate" refers to a compound solvated in an organic or inorganic solvent. The solvate is, for example, a hydrate.

The term "isotope" refers to elements of the same element but with different mass numbers, that is, elements with the same number of protons but a different number of neutrons. For example, the isotope may include $^{2}H$, $^{3}H$, $^{11}C$, $^{13}C$, $^{14}C$, $^{13}N$, $^{15}N$, $^{17}O$, $^{18}O$, $^{18}F$, $^{31}P$, $^{32}P$, $^{35}S$, $^{36}Cl$, $^{125}I$, and the like, but is not limited thereto. Isotope-labeled compounds may be advantageous in that they have improved stability in the body and a longer half-life.

The term "salt" refers to inorganic and organic acid addition salts of compounds. The pharmaceutically acceptable salt may be a salt that does not cause serious irritation to an organism administered with the compound

and does not impair the biological activity and properties of the compound. The inorganic acid salt may be hydrochloride, bromate, phosphate, sulfate, or disulfate. The organic acid salt may be formate, acetate, propionate, lactate, oxalate, tartrate, malate, maleate, citrate, fumarate, besylate, camsylate, edicylate, trichloroacetate, trifluoroacetate, benzoate, gluconate, methanesulfonate, glycolate, succinate, 4-toluenesulfonate, galacturonate, embonate, glutamate, ethanesulfonate, benzenesulfonate, p-toluenesulfonate, or aspartate. The metal salt may be a calcium salt, a sodium salt, a magnesium salt, a strontium salt, or a potassium salt.

The compound of any one or more of Formulae 1 to 3 may be an inhibitor for Src homology region 2 domain-containing phosphatase-2(SHP2). Src homology region 2 domain-containing phosphatase-2(SHP2) may be a protein belonging to protein tyrosine phosphatase(PTP). The SHP2 may also be referred to as tyrosine-protein phosphatase non-receptor type 11(PTPN11), protein-tyrosine phosphatase 1D(PTP-1D), or protein-tyrosine phosphatase 2C(PTP-2C). The SHP2 may include two tandem SH2 domains at the N-terminus, together with SPP1. The SHP2 may be a protein comprising the amino acid sequence of Uniprot No. Q06124 in humans or the amino acid sequence of Uniprot No. P35235 in mice. The SHP2 may be a wild-type SHP2 or a SHP2 mutant. The inhibitor for SHP2 may be an inhibitor that inhibits the expression or activity of SHP2.

In another aspect, there is provided a pharmaceutical composition comprising the compound according to one aspect, a stereoisomer, solvate or isotope-labeled compound thereof, or a pharmaceutically acceptable salt thereof.

In another aspect, there is provided a pharmaceutical composition for preventing or treating a disease associated with the abnormal activity of Src homology region 2 domain-containing phosphatase-2 (SHP2), comprising the compound according to one aspect, a stereoisomer, solvate or isotope-labeled compound thereof, or a pharmaceutically acceptable salt thereof.

The above compound, stereoisomer, solvate, isotope-labeled compound, pharmaceutically acceptable salt, and SHP2 are as described above.

The disease associated with the abnormal activity of SHP2 may be selected from the group consisting of cancer, cancer metastasis, cardiovascular disease, immune disorder, fibrosis, and ocular disorder.

The cancer may be selected from the group consisting of ovarian cancer, cervical cancer, endometrial cancer, uterine sarcoma, vulvar cancer, breast cancer, skin cancer, head and neck cancer, pancreatic cancer, lung cancer, large intestine cancer, colorectal cancer, gastric cancer, prostate cancer, bladder cancer, urethral cancer, liver cancer, kidney cancer, skin cancer, cerebrospinal tumor, malignant brain tumor (glioblastoma, anaplastic astrocytoma, low-grade glioma, etc.), thymoma, mesothelioma, bronchial cancer, nasopharyngeal cancer, laryngeal cancer, esophageal cancer, biliary tract cancer, testicular cancer, germ cell tumor, thyroid cancer, parathyroid cancer, lymphoma, myelodysplastic syndrome (MDS), myelofibrosis, acute leukemia, chronic leukemia, multiple myeloma, endocrine system cancer, and sarcoma.

The cancer metastasis refers to the spread of a tumor from a primary site to another body site, where it settles and proliferates. The cancer metastasis can cause cancer cells to spread to blood vessels, lymphatic vessels, or tissues.

In particular, the compound of the present disclosure may exhibit improved blood-brain barrier (BBB) penetration properties. Specifically, the compound of the present disclosure may have a blood-brain concentration ratio of at least 0.05, at least 0.1, at least 0.15, at least 0.2, or at least 0.25. Accordingly, the compound of the present disclosure may exhibit excellent therapeutic effects on brain tumor, such as glioblastoma, anaplastic astrocytoma, low-grade glioma, and cancer originating in a site other than the brain and metastasizing to the brain, for example, lung cancer, breast cancer, melanoma, etc., metastasized to the brain.

In some embodiments, the disease associated with abnormal activity of SHP2 may be EGFR/RAS pathway-dependent brain metastasis or glioblastoma. For example, brain metastasis may occur in non-small cell lung cancer (NSCLC) patients treated with EGFR inhibitors.

The cardiovascular disease refers to a disease occurring in the heart or major arteries (for example, aorta, pulmonary arteries, carotid arteries, cerebral blood vessels, renal arteries, lower extremity arteries). The cardiovascular disease may be selected from the group consisting of hypertension, ischemic heart disease, coronary artery disease, angina pectoris, myocardial infarction, atherosclerosis (arteriosclerosis), cerebrovascular disease, stroke, arrhythmia, acute heart failure, chronic heart failure, and hypotension.

The immune disorder refers to a state in which a normal immune response is not made. The immune disorder may be selected from the group consisting of acquired immunodeficiency, autoimmune disease, systemic lupus erythematosus, scleroderma, Sjogren's syndrome, polymyositis, dermatomyositis, polymyalgia rheumatica, temporal arteritis, polyarteritis nodosa, and Behcet's syndrome.

The fibrosis refers to a symptom in which fibrous tissue is excessively increased in a part of a tissue or an organ. The fibrosis may be selected from the group consisting of hepatic fibrosis, cystic fibrosis, myelofibrosis, endocardial myocardial fibrosis, and retroperitoneal fibrosis.

The ocular disorder refers to a disorder affecting various structures of the eye. The ocular disorder may be selected from the group consisting of endophthalmitis, degenerative myopia, degenerative disorders of the eye, hypotonia of the eye, intraocular foreign body, hemophthalmos, and dislocation of the eye.

The disease associated with the abnormal activity of SHP2 may be selected from the group consisting of Noonan

syndrome, Leopard syndrome, juvenile myelomonocytic leukemia (JMML), neuroblastoma, melanoma, acute myeloid leukemia, breast cancer, esophageal cancer, lung cancer, large intestine cancer, head cancer, malignant brain tumor (glioblastoma, anaplastic astrocytoma, low-grade glioma, etc.), squamous cell carcinoma of the head and neck, gastric carcinoma, anaplastic large cell lymphoma, pancreatic cancer, biliary tract cancer, uterine cancer, endometrial cancer, liver cancer, and neurofibromatosis type 1.

The term "prevention" refers to any action that inhibits the occurrence of or delays the onset of a disease associated with SHP2 by administration of the pharmaceutical composition. The term "treatment" refers to any action that improves or beneficially changes the symptoms of a disease associated with SHP2 by administration of the pharmaceutical composition.

The pharmaceutical composition may include a pharmaceutically acceptable carrier. The carrier is used in the sense of including an excipient, a diluent or an adjuvant. For example, the carrier may be selected from the group consisting of lactose, dextrose, sucrose, sorbitol, mannitol, xylitol, erythritol, maltitol, starch, gum acacia, alginate, gelatin, calcium phosphate, calcium silicate, cellulose, methyl cellulose, polyvinyl pyrrolidone, water, physiological saline, buffers such as PBS, methyl hydroxybenzoate, propyl hydroxybenzoate, talc, magnesium stearate, and mineral oil. The composition may include a filler, an anti-aggregation agent, a lubricant, a wetting agent, a flavoring agent, an emulsifying agent, a preservative, or a combination thereof.

The pharmaceutical composition may be prepared in any formulation according to a conventional method. The composition may be formulated, for example, as an oral dosage form (for example, a powder, a tablet, a capsule, a syrup, a pill, or a granule) or a parenteral dosage form (for example, an injection). In addition, the composition may be prepared as a systemic formulation or as a topical formulation.

In the pharmaceutical composition, the solid preparation for oral administration may be a tablet, a pill, a powder, a granule, or a capsule. The solid preparation may further include an excipient. The excipient may be, for example, starch, calcium carbonate, sucrose, lactose, or gelatin. In addition, the solid preparation may further include a lubricant such as magnesium stearate or talc. In the pharmaceutical composition, the liquid preparation for oral administration may be a suspension, an internal solution, an emulsion, or a syrup. The liquid preparation may include water or liquid paraffin. The liquid preparation may include an excipient, for example, a wetting agent, a sweetening agent, a perfuming agent, or a preservative. In the pharmaceutical composition, the preparation for parenteral administration may be a sterile aqueous solution, a non-aqueous solution, a suspension, an emulsion, a freeze-dried preparation or a suppository. A non-aqueous solution or suspension may include a vegetable oil or an ester. The vegetable oil may be, for example, propylene glycol, polyethylene glycol, or olive oil. The ester may be, for example, ethyl oleate. The base of the suppository may be witepsol, macrogol, tween 61, cacao butter, laurin butter, or glycerogelatin.

The pharmaceutical composition comprises the compound according to one aspect, a stereoisomer, solvate or isotope-labeled compound thereof, or a pharmaceutically acceptable salt thereof as an active ingredient of the pharmaceutical composition. "Active ingredient" refers to a physiologically active substance used to achieve a pharmacological activity (for example, treatment of a disease associated with the abnormal activity of SHP2).

The pharmaceutical composition may comprise the compound according to one aspect, a stereoisomer, solvate or isotope-labeled compound thereof, or a pharmaceutically acceptable salt thereof in an effective amount. The term "effective amount" refers to an amount sufficient to exhibit the effect of preventing or treating a disease when administered to a subject in need thereof. The effective amount may be appropriately selected by one of ordinary skill in the art depending on the cell or subject to be selected. The preferred dosage of the pharmaceutical composition varies depending on the condition and body weight of the subject, the severity of the disease, the drug form, the route and duration of administration, but may be appropriately selected by one of ordinary skill in the art. The effective amount may be about 0.5 μg to about 2 g, about 1 μg to about 1 g, about 10 μg to about 500 mg, about 100 μg to about 100 mg, or about 1 mg to about 50 mg per the pharmaceutical composition. However, the compound, stereoisomer, solvate, isotope-labeled compound, or pharmaceutically acceptable salt thereof may be administered in an amount of, for example, about 0.0001 mg/kg to about 100 mg/kg, or about 0.001 mg/kg to about 100 mg/kg, which may be administered in divided doses 1 to 24 times a day, 1 to 7 times per 2 days to 1 week, or 1 to 24 times per 1 month to 12 months. In the pharmaceutical composition, the compound, stereoisomer, solvate, isotope-labeled compound, or pharmaceutically acceptable salt thereof may be included in an amount of from about 0.0001% by weight to about 10% by weight, or from about 0.001% by weight to about 1% by weight, based on the total weight of the entire composition.

The administration method may be oral or parenteral administration. The administration method may be, for example, oral, transdermal, subcutaneous, rectal, intravenous, intraarterial, intraperitoneal, intramuscular, intrasternal, topical, intranasal, intratracheal, or intradermal route. The composition may be administered systemically or locally, alone or in combination with other pharmaceutically active compounds.

In another aspect, there is provided a method for preventing or treating a disease associated with the abnormal activity of SHP2, comprising administering to a subject the compound according to one aspect, a stereoisomer, solvate or isotope-labeled compound thereof, or a pharmaceutically acceptable salt thereof.

The above compound, stereoisomer, solvate, isotope-labeled compound, pharmaceutically acceptable salt,

SHP2, disease associated with the abnormal activity of SHP2, prevention and treatment are as described above.

The subject may be a mammal, such as a human, mouse, rat, cow, horse, pig, dog, monkey, sheep, goat, ape, or cat. The subject may be a subject suffering from, or likely to suffer from, a symptom associated with a disease associated with the abnormal activity of SHP2.

The method may further comprise administering to the subject an active ingredient known to have an effect of preventing or treating a disease associated with SHP2. The known active ingredient may be administered to the subject simultaneously, separately, or sequentially with the compound according to one aspect, a stereoisomer, solvate or isotope-labeled compound thereof, or a pharmaceutically acceptable salt thereof.

The administration method may be oral or parenteral administration. The administration method may be, for example, oral, transdermal, subcutaneous, rectal, intravenous, intraarterial, intraperitoneal, intramuscular, intrasternal, topical, intranasal, intratracheal, or intradermal route. The pharmaceutical composition may be administered systemically or locally, alone or in combination with other pharmaceutically active compounds.

The preferred dosage of the pharmaceutical composition varies depending on the condition and body weight of the patient, the severity of the disease, the drug form, the route and duration of administration, but may be appropriately selected by one of ordinary skill in the art. The dosage may be, for example, in the range of about 0.001 mg/kg to about 100 mg/kg, about 0.01 mg/kg to about 10 mg/kg, or about 0.1 mg/kg to about 1 mg/kg, for an adult. The administration may be performed once a day, 2 to 24 times a day, 1 to 2 times per 3 days, 1 to 6 times a week, 1 to 10 times per 2 weeks, 1 to 15 times per 3 weeks , 1 to 3 times per 4 weeks, or 1 to 12 times a year.

In another aspect, there is provided the compound according to one aspect, a stereoisomer, solvate or isotope-labeled compound thereof, or a pharmaceutically acceptable salt thereof, for use in preventing or treating a disease associated with the abnormal activity of SHP2.

The above compound, stereoisomer, solvate, isotope-labeled compound, pharmaceutically acceptable salt, SHP2, disease associated with the abnormal activity of SHP2, prevention, and treatment are as described above.

In another aspect, there is provided a use of the compound according to one aspect, a stereoisomer, solvate or isotope-labeled compound thereof, or a pharmaceutically acceptable salt thereof, for use in the manufacture of a medicament for the prevention or treatment of a disease associated with the abnormal activity of SHP2.

The above compound, stereoisomer, solvate, isotope-labeled compound, pharmaceutically acceptable salt, SHP2, disease associated with the abnormal activity of SHP2, prevention, and treatment are as described above.

## Effects of Invention

A disease associated with SHP2 can be effectively prevented or treated by a SHP2 inhibitor, a pharmaceutical composition for preventing or treating a disease associated with SHP2 comprising the same, a method for treating and preventing a disease using the same, and a use thereof.

## Detailed Description for Carrying out the Invention

Hereinafter, the present invention will be described in more detail by way of the following examples. However, the following examples are only for illustrating the present invention, and the scope of the present invention is not limited thereto.

### Preparation Example 1: tert-butyl 2-chloro-6-oxo-spiro[4H-cyclopenta[d]thiazole-5,4'-piperidine]-1'-carboxylate (Intermediate 1-1)

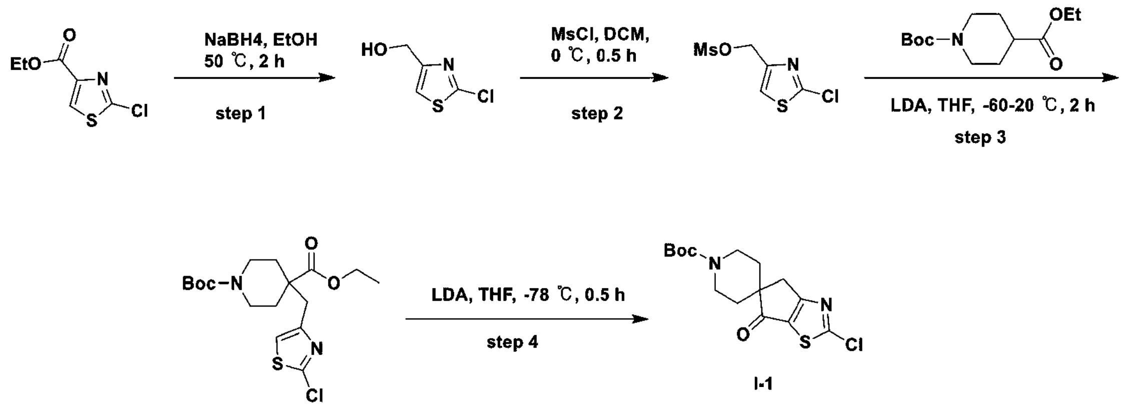


I-1

**Step 1: (2-chlorothiazol-4-yl)methanol**

To a solution of ethyl 2-chlorothiazole-4-carboxylate (30 g, 157 mmol) in EtOH (300 mL) $NaBH_4$ (41.5 g, 1.10 mol) was added, and the mixture was stirred at 50 °C for 2 hours. The reaction mixture was quenched with aqueous ammonium chloride (100 mL) at 0 °C, diluted in water (100 ml), and then extracted with EA (200 mL x 3). The combined organic layers were washed with brine (200 mL x 3), dried over anhydrous sodium sulfate, and then filtered and concentrated under reduced pressure. The residue was purified by column chromatography to obtain (2-chlorothiazol-4-yl)methanol (22 g, 94% yield) as a colorless oil.

$^1H$ NMR (400 MHz, $CDCl_3$) δ= 7.12 (s, 1H), 4.73 - 4.70 (m, 2H), 2.52 - 2.34 (m, 1H); MS (EI) m/z: 150.5 $[M+H]^+$.

**Step 2: (2-chlorothiazol-4-yl)methylmethanesulfonate**

To a solution of ethyl 2-chlorothiazole-4-carboxylate (30 g, 157 mmol) in DCM (300 mL), TEA (27.1 g, 267 mmol) and MsCl (21.4 g, 187 mmol) were added at 0 °C, and the mixture was stirred at 0 °C for 0.5 hours. The reaction mixture was quenched with aqueous $NaHCO_3$ solution (100 mL) at 25 °C, diluted in water (100 ml), and then extracted with DCM (200 mL x 3). The combined organic layers were washed with brine (100 mL x 3), dried over anhydrous sodium sulfate, and then filtered and concentrated under reduced pressure to obtain (2-chlorothiazol-4-yl)methylmethanesulfonate (28 g, crude product) as a yellow oil.

$^1H$ NMR (400 MHz, $CDCl_3$) δ= 7.29 (s, 1H), 5.19 (s, 2H), 3.01 (s, 3H)

**Step 3: O1-tert-butyl O4-ethyl 4-[(2-chlorothiazol-4-yl)methyl]piperidine-1,4-dicarboxylate**

A solution of ethyl 2-chlorothiazole-4-carboxylate (30 g, 157 mmol) in THF (30 mL) was stirred at -60°C under nitrogen, and then LDA (2 M, 81.6 mL) was added thereto dropwise. The reaction mixture was stirred at -60°C for 0.5 hours and added dropwise with (2-chlorothiazol-4-yl)methylmethanesulfonate (26.5 g, 117 mmol) in THF (15 ml). The reaction mixture was stirred at -60°C for 0.5 hours, and then slowly warmed to room temperature and stirred for 2 hours. The reaction mixture was quenched with aqueous ammonium chloride solution (100 mL), diluted in water (200 ml), and then extracted with EA (300 mL x 3). The combined organic layers were washed with brine (150 mL x 3), dried over anhydrous sodium sulfate, and then filtered and concentrated under reduced pressure. The residue was purified by column chromatography (0.1% FA) to obtain O1-tert-butyl O4-ethyl 4-[(2-chlorothiazol-4-yl)methyl]piperidine-1,4-dicarboxylate (14 g, 31% yield) as a yellow oil.

$^1H$ NMR (400 MHz, $CDCl_3$) δ= 6.80 (s, 1H), 4.15 (q, J = 7.2 Hz, 2H), 3.89 (s, 2H), 2.94 - 2.84 (m, 4H), 2.11 (d, J = 13.2 Hz, 2H), 1.52 - 1.47 (m, 2H), 1.45 (s, 9H), 1.24 (t, J = 7.2 Hz, 3H).

**Step 4: tert-butyl 2-chloro-6-oxo-spiro[4H-cyclopenta[d]thiazole-5,4'-piperidine]-1'-carboxylate**

To a solution of O1-tert-butyl O4-ethyl 4-[(2-chlorothiazol-4-yl)methyl]piperidine-1,4-dicarboxylate (13.8 g, 35.5 mmol) in THF (300 mL), LDA (2 M, 44.4 mL) was added dropwise, and the reaction mixture was stirred at 70°C for 0.5 hours. The reaction mixture was quenched with aqueous ammonium chloride solution (100 mL) at 0°C, diluted in water (100 ml), and then extracted with EA (200 mL x 3). The combined organic layers were washed with brine (200 mL x 3), dried over anhydrous sodium sulfate, and then filtered and concentrated under reduced pressure. The residue was purified by column chromatography to obtain Intermediate I-1 (6.1 g, 50% yield) as a yellow oil.

$^1H$ NMR (400 MHz, $CDCl_3$) δ= 4.16 (br s, 2H), 3.09 - 3.02 (s, 2H), 3.01 - 2.90 (m, 2H), 2.02 - 1.92 (m, 2H), 1.52 - 1.48 (m, 11H).

**Preparation Example 2: (R)-2-methyl-N-[(6S)-spiro[4,6-dihydrocyclopenta[d]thiazole-5,4'-piperidin]-6-yl]oropane-2-sulfinamide (Intermediate 1-2)**

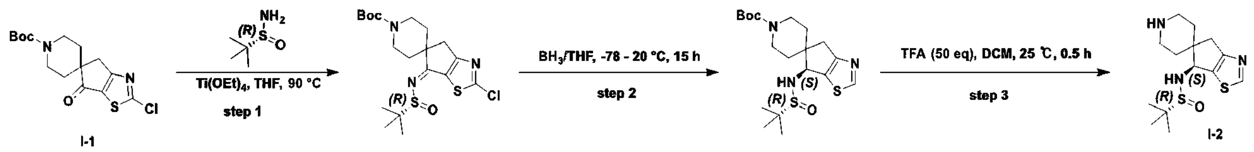


**Step 1: tert-butyl (6Z)-6-[(R)-tert-butylsulfinyl]imino-2-chloro-spiro[4H-cyclopenta[d]thiazole-5,4'-piperidine]-1'-carboxylate**

To a solution of tert-butyl 2-chloro-6-oxo-spiro[4H-cyclo[d]thiazole-5,4'-piperidine]-1'-carboxylate (3.0 g, 8.75 mmol) and (R)-2-methylpropane-2-sulfinamide (4.24 g, 35.0 mmol) in THF (30 mL), $Ti(OEt)_4$ (29.9 g, 131 mmol) was added dropwise, and the reaction mixture was stirred at 90°C for 12 hours. The reaction mixture was added with EA (300 mL), diluted in water (50 ml), and then extracted with EA (100 mL x 3). The combined organic layers were washed with brine (100 mL x 3), dried over anhydrous sodium sulfate, and then filtered and concentrated under reduced pressure. The residue was purified by column chromatography to obtain tert-butyl (6Z)-6-[(R)-tert-butylsulfinyl]imino-2-chloro-spiro[4H-cyclopenta[d]thiazole-5,4'-piperidine]-1'-carboxylate (3.5 g, 90% yield) as a yellow solid.

$^1$H NMR (400 MHz, $CDCl_3$) δ= 4.28 - 4.14 (m, 2H), 2.96 - 2.84 (m, 4H), 2.05 - 1.91 (m, 2H), 1.61 - 1.55 (m, 2H), 1.49 (s, 9H), 1.28 (s, 9H); MS (EI) m/z: 446.0 $[M+H]^+$.

**Step 2: tert-butyl (6S)-6-[[(R)-tert-butylsulfinyl]amino]spiro[4,6-dihydrocyclopenta[d]thiazole-5,4'-piperidine]-1'-carboxylate**

To a solution of tert-butyl (6Z)-6-[(R)-tert-butylsulfinyl]imino-2-chloro-spiro[4H-cyclopenta[d]thiazole-5,4'-piperidine]-1'-carboxylate (3.5 g, 7.85 mmol) in THF (30 mL), $BH_3$.THF (1 M, 31.4 mL) was added, and the reaction mixture was stirred at -70°C for 2 hours. The reaction mixture was quenched with MeOH (10 mL) at 0°C, diluted in water (100 ml), and then extracted with EA(100 mL x 3). The combined organic layers were washed with brine (50 mL x 3), dried over anhydrous sodium sulfate, and then filtered and concentrated under reduced pressure. The residue was purified by column chromatography to obtain tert-butyl (6S)-6-[[(R)-tert-butylsulfinyl]amino]spiro[4,6-dihydrocyclopenta[d]thiazole-5,4'-piperidine]-1'-carboxylate (660 mg, 20% yield) as a yellow solid.

$^1$H NMR (400 MHz, $CDCl_3$) δ= 8.78 (s, 1H), 4.57 (d, J = 8.6 Hz, 1H), 4.13 - 3.95 (m, 2H), 3.65 - 3.52 (m, 1H), 3.06 - 2.98 (m, 1H), 2.95 - 2.85 (m, 2H), 1.91 - 1.78 (m, 2H), 1.64 (d, J = 14.0 Hz, 2H), 1.47 (s, 9H), 1.24 - 1.21 (s, 9H).

**Step 3: (R)-2-methyl-N-[(6S)-spiro[4,6-dihydrocyclopenta[d]thiazole-5,4'-piperidin]-6-yl]propane-2-sulfinamide**

To a solution of tert-butyl (6S)-6-[[(R)-tert-butylsulfinyl]amino]spiro[4,6-dihydrocyclopenta[d]thiazole-5,4'-piperidine]-1'-carboxylate (200 mg, 484 μmol) in DCM (6 mL), TFA (2.76 g, 24.2 mmol) was added, and the reaction mixture was stirred at 25°C for 0.5 hours. The reaction mixture was quenched with saturated aqueous $K_2CO_3$ solution (20 mL) at 25°C, diluted in water (30 ml), and then extracted with DCM (50 mL x 3). The combined organic layers were washed with brine (20 mL x 3), dried over anhydrous sodium sulfate, and then filtered and concentrated under reduced pressure to obtain Intermediate 1-2 (140 mg, crude product) as a yellow solid.

$^1$H NMR (400 MHz, $CDCl_3$) δ= 8.77 (s, 1H), 4.58 (d, J = 8.8 Hz, 1H), 3.67 (d, J = 8.8 Hz, 1H), 3.16 - 3.05 (m, 2H), 2.90 - 2.84 (m, 2H), 2.60 - 2.36 (m, 2H), 1.93 - 1.85 (m, 2H), 1.69 - 1.61 (m, 1H), 1.59 - 1.53 (m, 1H), 1.24 (s, 9H).

**Preparation Example 3: 6-(5-bromopyrazin-2-yl)sulfanyl-5-chloro-3H-quinazolin-4-one (Intermediate 1-3)**

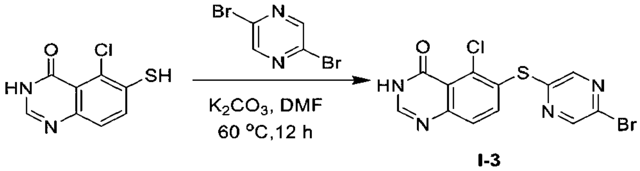

To a solution of 5-chloro-6-mercaptoquinazolin-4(3H)-one (880 mg, 3.75 mmol) in DMF (20 mL), $K_2CO_3$ (1.56 g, 11.3 mmol) and 2,5-dibromopyrazine (3.57 g, 15.00 mmol) were added, and the mixture was stirred at 0°C for 2 hours. The reaction mixture was quenched by addition of aqueous ammonium chloride (50 mL) at 25°C, diluted with water (50 mL), and extracted with EA (100 mL x 3). The combined organic layers were washed with brine (50 mL x 3), dried over anhydrous sodium sulfate, and then filtered and concentrated under reduced pressure to obtain a residue. The residue was purified by column chromatography (PE/EA=1/1 to 0:1) to obtain Intermediate I-3 (500 mg, 36% yield) as a yellow solid.

$^1H$ NMR (400 MHz, DMSO-$d_6$) δ= 12.50 (br s, 1H), 8.71 (d, J = 1.0 Hz, 1H), 8.46 - 8.43 (m, 1H), 8.15 (s, 1H), 7.98 (d, J = 8.8 Hz, 1H), 7.61 (d, J = 8.8 Hz, 1H); MS (EI) m/z: 371.1 $[M+H]^+$.

**Preparation Example 4: (R)-N-[(6S)-1'-[5-[(5-chloro-4-oxo-3H-quinazolin-6-yl)sulfanyl]pyrazin-2-yl]spiro[4,6-dihydrocyclopenta[d]thiazole-5,4'-piperidin]-6-yl]-2-methyl-propane-2-sulfinamide (Intermediate 1-4)**

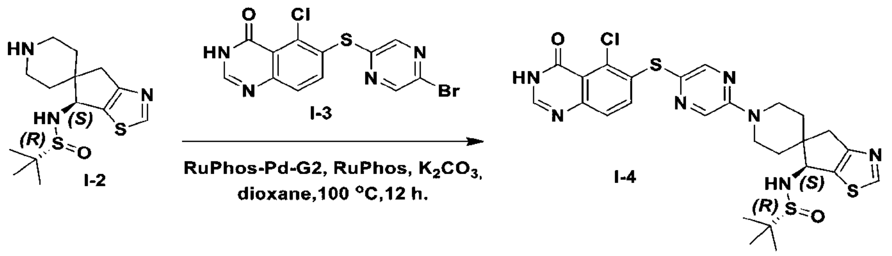


To a solution of Intermediate 1-3 (402 mg, 1.09 mmol) in dioxane (5 mL), Intermediate I-2 (310 mg, 989 μmol), RuPhos (92.3 mg, 198 μmol), RuPhos-Pd-G2 (76.8 mg, 98.9 μmol) and $K_2CO_3$ (410 mg, 2.97 mmol) were added, and the mixture was stirred at 100 °C for 16 hours. The reaction mixture was concentrated under reduced pressure, and then the residue was purified by column chromatography to obtain Intermediate 1-4 (300 mg, 53% yield) as a yellow solid.

$^1H$ NMR (400 MHz, $CDCl_3$) δ= 10.74 - 10.64 (m, 1H), 8.82 (s, 1H), 8.26 (s, 1H), 8.23 (s, 1H), 7.98 - 7.92 (m, 1H), 7.48 (d, J = 8.8 Hz, 1H), 7.29 (s, 1H), 4.68 (d, J = 8.8 Hz, 1H), 4.41 (d, J = 13.2 Hz, 1H), 4.35 - 4.26 (m, 1H), 3.29 - 3.16 (m, 2H), 3.11 - 3.02 (m, 1H), 2.99 - 2.93 (m, 1H), 2.18 - 1.99 (m, 2H), 1.87 - 1.75 (m, 2H), 1.27 (s, 9H).

**Preparation Example 5: 6-((5-bromopyrazin-2-yl)thio)-5-chloro-3-(2-methoxyethyl)quinazolin-4(3H)-one (Intermediate I-5)**

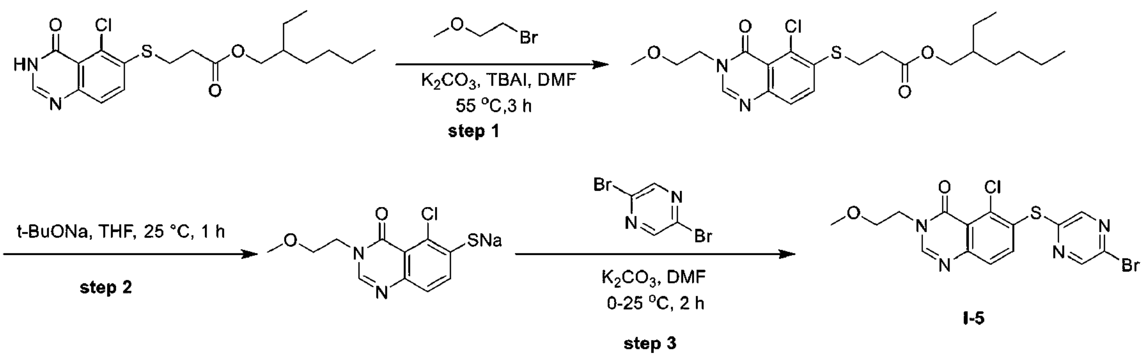


**Step 1: 2-ethylhexyl 3-((5-chloro-3-(2-methoxyethyl)-4-oxo-3,4-dihydroquinazolin-6-yl)thio)propanoate**

To a solution of ethyl 3-((5-chloro-4-oxo-3,4-dihydroquinazolin-6-yl)thio)propionate (10g, 25.2mmol) in DMF (100 mL), 1-bromo-2-methoxy-ethane (4.2g, 30.2mmol), $K_2CO_3$ (6.96 g, 50.4 mmol) and TBAI (931 mg, 2.52 mmol) were added, and the mixture was stirred at 55°C for 3 hours. The reaction mixture was quenched with aqueous ammonium chloride solution (50 mL), diluted in water (50 mL), and then extracted with EA (100 mL x 3). The combined organic layers were washed with brine (50 mL x 3), dried over anhydrous sodium sulfate, and then filtered and concentrated under reduced pressure. The residue was purified by column chromatography (0.1% formic acid) to obtain 2-ethylhexyl 3-(5-chloro-3-(2-methoxyethyl)-4-oxo-3,4-dihydroquinazolin-6-yl)thio)propanoate (6.8 g, 59% yield) as a yellow solid.

$^1$H NMR (400 MHz, $CDCl_3$) δ = 8.04 (s, 1H), 7.67 - 7.59 (m, 2H), 4.15 (t, J = 4.8 Hz, 2H), 4.05 (dd, J = 2.0, 5.8 Hz, 2H), 3.71 - 3.65 (m, 2H), 3.33 (s, 3H), 3.30 - 3.25 (m, 2H), 2.71 (t, J = 7.6 Hz, 2H), 1.62 - 1.54 (m, 1H), 1.40 - 1.34 (m, 2H), 1.31 - 1.26 (m, 6H), 0.91 - 0.85 (m, 6H); MS (EI) m/z: 455.3 $[M+H]^+$.

**Step 2 and Step 3: 6-((5-bromopyrazin-2-yl)thio)-5-chloro-3-(2-methoxyethyl)quinazolin-4(3H)-one**

Intermediate I-5 (1.4 g, 22%) was obtained as a yellow solid in the same method as in steps 3 and 4 of Preparation Example 9, using 2-ethylhexyl 3-(5-chloro-3-(2-methoxyethyl)-4-oxo-3,4-dihydroquinazolin-6-yl)thio)propanoate as a starting material.

$^1$H NMR (400 MHz, $CDCl_3$) δ= 8.44 (s, 1H), 8.19 (s, 1H), 8.13 (s, 1H), 7.92 (d, J = 8.4 Hz, 1H), 7.65 (d, J = 8.4 Hz, 1H), 4.17 (t, J = 4.8 Hz, 2H), 3.73 - 3.66 (m, 2H), 3.35 (s, 3H); MS (EI) m/z: 429.1 $[M+H]^+$.

**Preparation Example 6: 6-(3-amino-5-chloro-pyrazin-2-yl)sulfanyl-5-chloro-3-(2-methoxyethyl)quinazolin-4-one (Intermediate 1-6)**

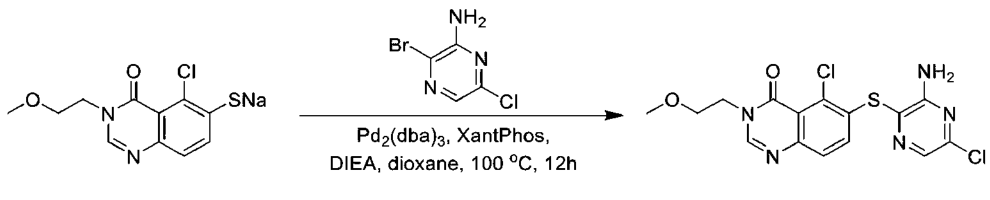


I-6

A mixture of [5-chloro-3-(2-methoxyethyl)-4-oxo-quinazolin-6-yl]sulfanyl sodium (630 mg, 2.15 mmol) obtained in step 2 of Preparation Example 14, 3-bromo-6-chloro-pyrazine-2-amine (538 mg, 2.58 mmol), $Pd_2(dba)_3$ (197 mg, 215 μmol), XantPhos (249 mg, 431 μmo) and DIEA (835 mg, 6.46 mmol) in dioxane (20 mL) was degassed, and purged 3 times with $N_2$, and then the mixture was stirred under $N_2$ atmosphere at 100°C for 12 hours. The reaction mixture was filtered, and the filtrate was concentrated under reduced pressure to obtain a residue. The residue was purified by reverse phase flash column chromatography (0.1% FA) to obtain Intermediate I-6 (120 mg, 14% yield) as a yellow solid.

$^1$H NMR (400 MHz, $CDCl_3$) δ= 8.08 (s, 1H), 7.92 (s, 1H), 7.57 - 7.53 (m, 1H), 7.49 - 7.45 (m, 1H), 5.16 (br s, 2H), 4.16 (t, J = 4.8 Hz, 2H), 3.68 (t, J = 4.8 Hz, 2H), 3.33 (s, 3H); MS (EI) m/z: 398.0 $[M+H]^+$.

**Preparation Example 7: 6-(3-amino-5-chloro-pyrazin-2-yl)sulfanyl-5-chloro-3-(2-methoxypropyl)quinazolin-4-one (Intermediate I-7)**

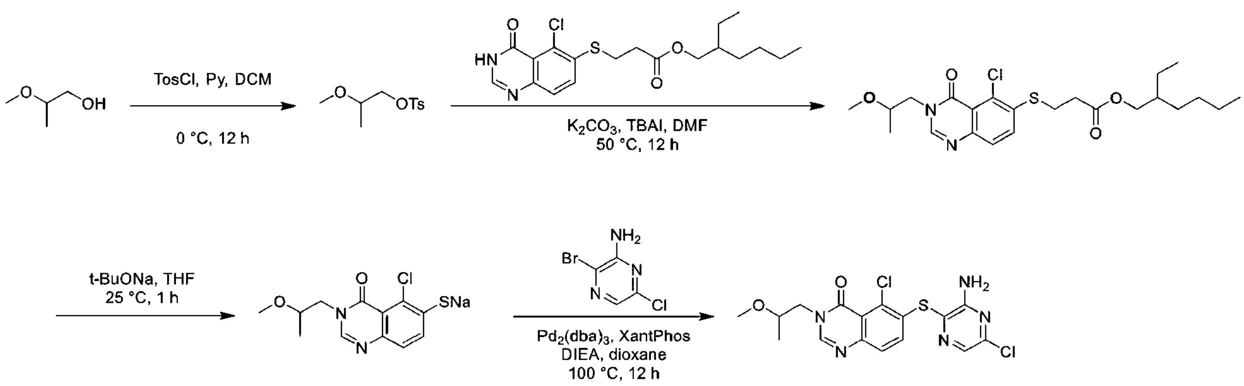


I-7

Step **1: 2-methoxypropyl 4-methylbenzenesulfonate**

To a solution of 2-methoxypropane-1-ol (3.80 g, 42.1 mmol) in DCM (40 mL), TosCl (12.0 g, 63.2 mmol) and Py (10.0 g, 126 mmol, 10.2 mL) were added. The mixture was stirred at 0 °C for 12 hours. Upon completion of the reaction, the

reaction mixture was quenched by addition of aqueous ammonium chloride (20 mL) at 25 °C, diluted with water (30 mL) and extracted with EA (3 x 50 mL). The combined organic layers were washed with brine (3 x 20 mL), dried over anhydrous sodium sulfate, and then filtered and concentrated under reduced pressure to obtain a residue. The residue was purified by column chromatography ($SiO_2$, petroleum ether/ethyl acetate = 15/1 - 5/1) to obtain 2-methoxypropyl 4-methylbenzenesulfonate (9.80 g, 95% yield) as a yellow solid.

$^1H$ NMR (400 MHz, $CDCl_3$) $\delta$= 7.77 (d, J = 8.0 Hz, 2H), 7.33 (d, J = 8.0 Hz, 2H), 3.93 (dd, J = 1.6, 4.8 Hz, 2H), 3.55 - 3.47 (m, 1H), 3.26 (s, 3H), 2.42 (s, 3H), 1.08 (d, J = 6.4 Hz, 3H).

Step 2: **2-ethylhexyl 3-[5-chloro-3-(2-methoxypropyl)-4-oxo-quinazolin-6-yl]sulfanylpropanoate**

To a solution of 2-ethylhexyl 3-[(5-chloro-4-oxo-3H-quinazolin-6-yl)sulfanyl]propanoate (3.00 g, 7.56 mmol) and 2-methoxypropyl 4-methylbenzenesulfonate (2.40 g, 9.83 mmol) in DMF (30 mL), $K_2CO_3$ (3.13 g, 22.6 mmol) and TBAI (279 mg, 755 $\mu$mol) were added. The mixture was stirred at 50 °C for 12 hours. Upon completion of the reaction, the reaction mixture was quenched by addition of aqueous ammonium chloride (20 mL) at 25 °C, diluted with water (30 mL) and extracted with EA (3 x 50 mL). The combined organic layers were washed with brine (3 x 30 mL), dried over anhydrous sodium sulfate, and filtered and concentrated under reduced pressure to obtain a residue. The residue was purified by column chromatography ($SiO_2$, petroleum ether/ethyl acetate = 15/1 - 3/1) to obtain 2-ethylhexyl 3-[5-chloro-3-(2-methoxypropyl)-4-oxo-quinazolin-6-yl]sulfanylpropanoate (1.00 g, 28% yield) as a red solid.

MS (EI) m/z: 469.2 $[M+H]^+$.

**Step 3: [5-chloro-3-(2-methoxypropyl)-4-oxo-quinazolin-6-yl]sulfanylsodium**

To a solution of 2-ethylhexyl 3-[5-chloro-3-(2-methoxypropyl)-4-oxo-quinazolin-6-yl]sulfanylpropanoate (500 mg, 1.07 mmol) in THF (10 mL), t-BuONa (153 mg, 1.60 mmol) was added, and the mixture was stirred at 25 °C for 1 hour. Upon completion of the reaction, the reaction mixture was filtered and concentrated under reduced pressure to obtain [5-chloro-3-(2-methoxypropyl)-4-oxo-quinazolin-6-yl]sulfanylsodium (300 mg, 92% yield) as a yellow solid.

MS (EI) m/z: 285.1 $[M+H]^+$.

**Step 4: 6-(3-amino-5-chloro-pyrazin-2-yl)sulfanyl-5-chloro-3-(2-methoxypropyl)quinazolin-4-one**

A mixture of [5-chloro-3-(2-methoxypropyl)-4-oxo-quinazolin-6-yl]sulfanylsodium (327 mg, 1.07 mmol), 3-bromo-6-chloro-pyrazine-2-amine (222 mg, 1.07 mmol), (5-diphenylphosphanyl-9,9-dimethyl-xanthen-4-yl)-diphenyl-phosphane (123 mg, 213 $\mu$mol), $Pd_2(dba)_3$ (97.6 mg, 106 $\mu$mol) and DIEA (413 mg, 3.20 mmol, 557 $\mu$L) in dioxane (10 mL) was degassed, and purged 3 times with $N_2$, and then the mixture was stirred under $N_2$ atmosphere at 100 °C for 12 hours. Upon completion of the reaction, the reaction mixture was quenched by addition of aqueous ammonium chloride (10 mL) at 25 °C, and then diluted with water (10 mL) and extracted with EA (3 x 10 mL). The combined organic layers were washed with brine (3 x 10 mL), dried over anhydrous sodium sulfate, and filtered and concentrated under reduced pressure to obtain a residue. The residue was purified by column chromatography ($SiO_2$, petroleum ether/ethyl acetate = 3/1 to 1/2) to obtain Intermediate I-7 (100 mg, 23% yield) as a black solid.

MS (EI) m/z: 411.9 $[M+H]^+$.

**Preparation Example 8: sodium 5-((3S,4S)-4-(tert-butoxycarbonyl)amino)-3-methyl-2-oxa-8-azaspiro[4.5]decan-8-yl]pyrazine-2-thiolate (Intermediate I-8)**

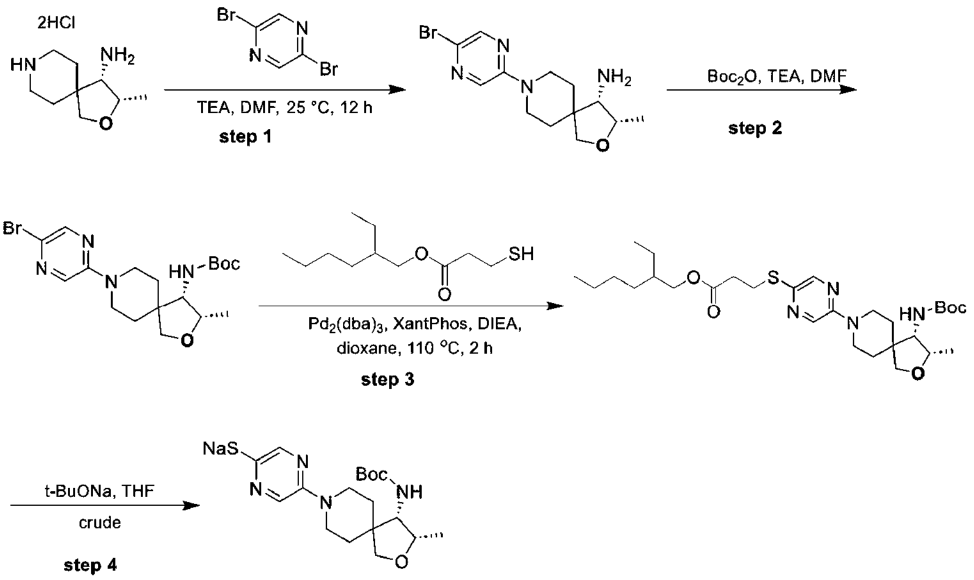


I-8

**Step 1: (3S,4S)-8-(5-bromopyrazin-2-yl)-3-methyl-2-oxa-8-azaspiro[4.5]decan-4-amine**

To a solution of (3S,4S)-3-methyl-2-oxa-8-azaspiro[4.5]decan-4-amine (5.00 g, 20.6 mmol, 2HCl) in DMF (50 mL), TEA (6.24 g, 61.7 mmol) and 2,5-dibromopyrazine (4.89 g, 20.6 mmol) were added, and the mixture was stirred at 25 °C for 12 hours. The reaction mixture was quenched by addition of aqueous ammonium chloride (100 mL) at 25 °C, and then diluted with water (100 mL) and extracted with EA (3 x 200 mL). The combined organic layers were washed with brine (3 x 100 mL), dried over anhydrous sodium sulfate, and then filtered and concentrated under reduced pressure to obtain (3S,4S)-8-(5-bromopyrazin-2-yl)-3-methyl-2-oxa-8-azaspiro[4.5]decan-4-amine (6.7 g, crude product) as a yellow oil, which was used directly in the next step.

MS (EI) m/z: 329.1 $[M+H]^+$.

**Step 2: tert-butyl N-[(3S,4S)-8-(5-bromopyrazin-2-yl)-3-methyl-2-oxa-8-azaspiro[4.5]decan-4-yl]carbamate**

To a solution of (3S,4S)-8-(5-bromopyrazin-2-yl)-3-methyl-2-oxa-8-azaspiro[4.5]decan-4-amine (6.7 g, 20.5 mmol) in DMF (50 mL), Boc$_2$O (6.70 g, 30.7 mmol) and TEA (3.11 g, 30.7 mmol) were added, and the mixture was stirred at 25°C for 2 hours. The reaction mixture was quenched by addition of aqueous ammonium chloride (100 mL) at 25°C, and then diluted with water (100 mL) and extracted with EA (3 x 200 mL). The combined organic layers were washed with brine (3 x 100 mL), dried over anhydrous sodium sulfate, and then filtered and concentrated under reduced pressure to obtain a residue. The residue was purified by column chromatography(PE/EA = 5:1) to obtain tert-butyl N-[(3S,4S)-8-(5-bromopyrazin-2-yl)-3-methyl-2-oxa-8-azaspiro[4.5]decan-4-yl]carbamate (5.5 g, 63% yield) as a yellow solid. MS (EI) m/z: 429.1 $[M+H]^+$.

**Step 3: 2-ethylhexyl 3-((5-((3S,4S)-4-((tert-butoxycarbonyl)amino)-3-methyl-2-oxa-8-azaspiro[4.5]decan-8-yl)pyrazin-2-yl)thio)propanoate**

A mixture of tert-butyl N-[(3S,4S)-8-(5-bromopyrazin-2-yl)-3-methyl-2-oxa-8-azaspiro[4.5]decan-4-yl]carbamate (5.50 g, 12.9 mmol), 2-ethylhexyl 3-sulfanylpropanoate (4.22 g, 19.3 mmol), Pd$_2$(dba)$_3$ (1.18 g, 1.29 mmol), XantPhos (1.49 g, 2.57 mmol) and DIEA (4.96 g, 38.6 mmol) in dioxane (80 mL) was degassed and purged 3 times with $N_2$, and then the mixture was stirred under $N_2$ atmosphere at 110°C for 12 hours. The reaction mixture was filtered and concentrated under reduced pressure to obtain a residue. The residue was purified by column chromatography (PE/EA = 3:1) to obtain 2-ethylhexyl 3-[5-[(3S,4S)-4-(tert-butoxycarbonylamino)-3-methyl-2-oxa-8-azaspiro[4.5]decan-8-yl]pyrazin-2-yl]sulfanylpropanoate (5.00 g, 69% yield) as a yellow oil.

$^1H$ NMR (400 MHz, $CDCl_3$) $\delta$= 8.08 (s, 1H), 8.06 (s, 1H), 4.63 (d, J = 10.8 Hz, 1H), 4.23 - 4.13 (m, 1H), 4.01 (dd, J =5.8, 2.6 Hz, 2H), 3.82 - 3.59 (m, 4H), 3.57 - 3.46 (m, 1H), 3.44 - 3.33 (m, 1H), 3.28 (t, J = 7.2 Hz, 2H), 2.69 (t, J = 7.2 Hz, 2H), 1.92 - 1.71 (m, 4H), 1.62 - 1.52 (m, 2H), 1.45 (s, 9H), 1.41 - 1.32 (m, 3H), 1.30 - 1.27 (m, 6H), 1.21 (d, J =6.4 Hz, 2H), 0.93 - 0.86 (m, 6H); MS (EI) m/z: 565.4 $[M+H]^+$.

**Step 4: sodium 5-((3S,4S)-4-(tert-butoxycarbonyl)amino)-3-methyl-2-oxa-8-azaspiro[4.5]decan-8-yl]pyrazine-2-thiolate**

To a solution of 2-ethylhexyl 3-[5-[(3S,4S)-4-(tert-butoxycarbonylamino)-3-methyl-2-oxa-8-azaspiro[4.5]decan-8-yl]pyrazin-2-yl]sulfanylpropanoate (270 mg, 478 μmol) in THF (5 mL), t-BuONa (68.9 mg, 717 μmol) was added, and the mixture was stirred at 25°C for 0.5 hours. The residue was triturated with PE (10 mL) to obtain Intermediate I-8 (150 mg, crude product) as a yellow solid, which was used directly in the next step.
MS (EI) m/z: 381.1 $[M+H]^+$.

**Preparation Example 9: 7-bromo-8-chloroisoquinolin-1(2H)-one (Intermediate 1-9)**

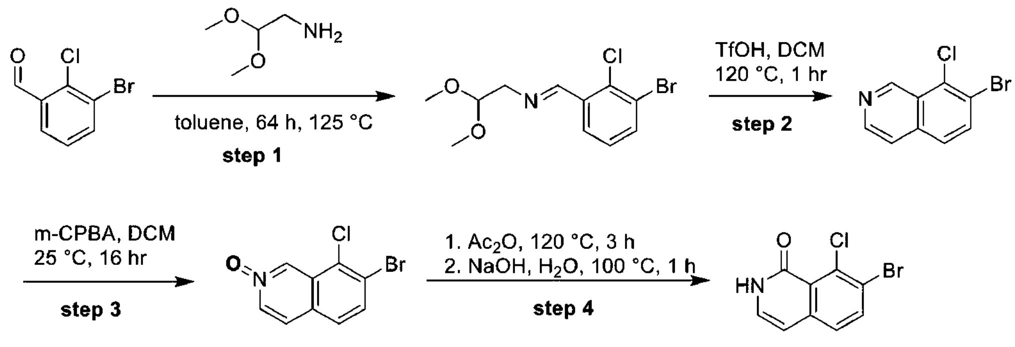


I-9

**Step 1: (E)-1-(3-bromo-2-chloro-phenyl)-N-(2,2-dimethoxyethyl)methanimine**

To a mixture of 3-bromo-2-chloro-benzaldehyde (14 g, 63.7 mmol) in toluene (200 mL), 2,2-dimethoxyethanamine (6.71 g, 63.7 mmol) was added, and the mixture was stirred at 125 °C for 64 hours. Upon completion of the reaction, the reaction mixture was concentrated under reduced pressure to obtain (E)-1-(3-bromo-2-chloro-phenyl)-N-(2,2-dimethoxyethyl)methanimine (19 g, 97% yield) as a yellow oil.
$^1H$ NMR (400 MHz, DMSO-$d_6$) δ = 8.68 (s, 1H), 7.94 - 7.86 (m, 2H), 7.34 (t, J = 7.6 Hz, 1H), 4.64 (t, J = 5.4 Hz, 1H), 3.78 (d, J = 5.4 Hz, 2H), 3.30 (s, 6H).

**Step 2: 7-bromo-8-chloro-isoquinoline**

To a mixture of (E)-1-(3-bromo-2-chloro-phenyl)-N-(2,2-dimethoxyethyl)methanimine (2g, 6.52 mmol) in DCM (20 mL), trifluoromethanesulfonic acid (20 mL) was added, and the mixture was stirred at 120 °C for 0.5 hours. Thereafter, the reaction mixture was cooled to 25 °C and stirred at 25 °C for 0.5 hours. Upon completion of the reaction, MeOH (200 mL) was added to the reaction mixture. The mixture was poured into $NH_3 \cdot H_2O$ (200 mL) and extracted with EA (3 X 300 mL). The organic phase was concentrated under reduced pressure. The residue was triturated with water at 25 °C for 30 minutes to obtain 7-bromo-8-chloro-isoquinoline (820 mg, 51% yield) as a brown solid.
$^1H$ NMR (400 MHz, DMSO-$d_6$) δ = 9.56 (s, 1H), 8.68 (d, J = 5.6 Hz, 1H), 8.06 (d, J = 8.8 Hz, 1H), 7.96 - 7.94 (m, 2H).

**Step 3: 7-bromo-8-chloro-2-oxido-isoquinolin-2-ium**

To a mixture of 7-bromo-8-chloro-isoquinoline (820 mg, 3.38 mmol) in DCM (20 mL), m-CPBA (1.37 g, 6.76 mmol, 85% purity) was added, and the mixture was stirred at 25 °C for 16 hours. Upon completion of the reaction, $Na_2O_3S_2$ (50 ml) was added to the mixture. The mixture was concentrated under reduced pressure. The residue was extracted with EA (3 x 40 mL). The organic phase was concentrated under reduced pressure. The residue was purified by column chromatography ($SiO_2$, PE/EA/MeOH= 1/0/0 to 0/10/1) to obtain 7-bromo-8-chloro-2-oxido-isoquinolin-2-ium (690 mg, 78% yield) as a yellow solid.
$^1H$ NMR (400 MHz, DMSO-$d_6$) δ= 8.88 (s, 1H), 8.32 (d, J = 7.2 Hz, 1H), 8.06 (d, J = 7.2 Hz, 1H), 7.90 (s, 2H).

**Step 4: 7-bromo-8-chloroisoquinolin-1(2H)-one**

A mixture of 7-bromo-8-chloro-2-oxido-isoquinolin-2-ium (640 mg, 2.48 mmol) in $Ac_2O$ (6 mL) was stirred at 120

°C for 3 hours. Upon completion of the reaction, the mixture was concentrated under reduced pressure to obtain (7-bromo-8-chloro-1-isoquinolyl) acetate (640 mg, crude product). A mixture of (7-bromo-8-chloro-1- isoquinolyl) acetate (640 mg, 2.13 mmol) in a mixture of NaOH (2 M, 32.00 mL) and $H_2O$ (8 mL) was stirred at 100 °C for 1 hour. Upon completion of the reaction, the mixture was acidified to pH 6 with citric acid (5% aqueous solution) and extracted with DCM (3 X 100 mL). The combined organic phase was concentrated under reduced pressure to obtain Intermediate 1-9 (320 mg, 58% yield) as a yellow solid.

$^1$H NMR (400 MHz, $CDCl_3$) δ= 10.76 - 10.32 (m, 1H), 7.86 (d, J = 8.4 Hz, 1H), 7.32 (d, J = 8.4 Hz, 1H), 7.19 (d, J = 6.8 Hz, 1H), 6.48 (d, J = 7.2 Hz, 1H).

**Preparation Example 10: sodium 5-((S)-6-(((R)-tert-butylsulfinyl)amino)-4,6-dihydrospiro[cyclopenta[d]thiazole-5,4'-piperidin]-1'-yl)pyrazine-2-thiolate (Intermediate I-10)**

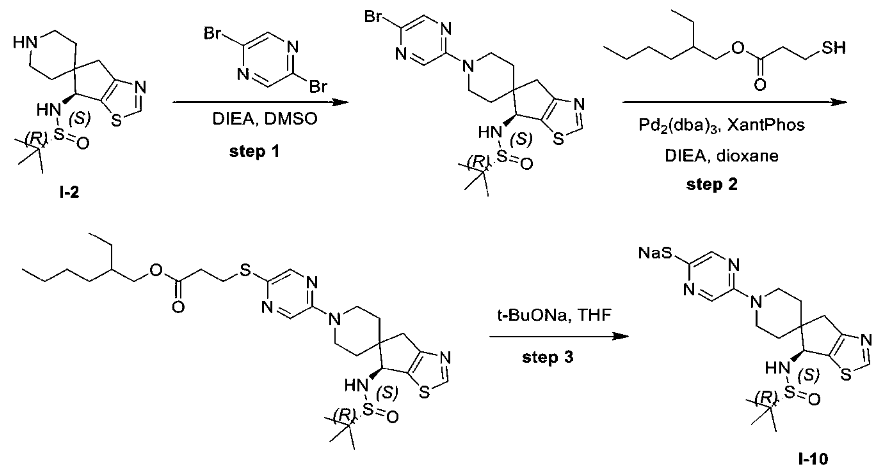


**Step 1: (R)-N-((S)-1'-(5-bromopyrazin-2-yl)-4,6-dihydrospiro[cyclopenta[d]thiazole-5,4'-piperidin]-6-yl)-2-methylpropane-2-sulfinamide**

To a solution of Intermediate I-2 (500 mg, 1.59 mmol) in DMF (8 mL), DIEA (2.06 g, 16.0 mmol) and 2,5-dibromopyrazine (1.14 g, 4.78 mmol) were added. The mixture was stirred at 100 °C for 2 hours. Upon completion of the reaction, the reaction mixture was quenched by addition of aqueous ammonium chloride (20 mL) at 25 °C, and then diluted with water (30 mL) and extracted with EA (3 x 50 mL). The combined organic layers were washed with brine (20 mL), dried over anhydrous sodium sulfate, and filtered and concentrated under reduced pressure to obtain a residue. The residue was purified by column chromatography to obtain (R)-N-((S)-1'-(5-bromopyrazin-2-yl)-4,6-dihydrospiro[cyclopenta[d]thiazole-5,4'-piperidin]-6-yl)-2-methylpropane-2-sulfinamide (230 mg, 31% yield) as a yellow solid.

$^1$H NMR (400 MHz, $CDCl_3$) δ= 8.80 (s, 1H), 8.12 (d, J = 1.2 Hz, 1H), 7.90 (d, J = 1.2 Hz, 1H), 4.63 (d, J = 9.2 Hz, 1H), 4.27 - 4.12 (m, 2H), 3.66 (d, J = 9.2 Hz, 1H), 3.22 - 3.08 (m, 2H), 3.04 - 2.99 (m, 1H), 2.94 (s, 1H), 2.07 - 1.96 (m, 2H), 1.80 (dd, J = 2.4, 13.2 Hz, 1H), 1.72 (dd, J = 2.4, 13.2 Hz, 1H), 1.23 (s, 9H); MS (EI) m/z: 472.2 $[M+H]^+$.

**Step 2: 2-ethylhexyl 3-((5-((S)-6-(((R)-tert-butylsulfinyl)amino)-4,6-dihydrospiro[cyclopenta[d]thiazole-5,4'-piperidin]-1'-yl)pyrazin-2-yl)thio)propanoate**

A mixture of (R)-N-((S)-1'-(5-bromopyrazin-2-yl)-4,6-dihydrospiro[cyclopenta[d]thiazole-5,4'-piperidin]-6-yl)-2-methylpropane-2-sulfinamide (230 mg, 489 μmol), 2-ethylhexyl 3-sulfanylpropanoate (128 mg, 587 μmol), $Pd_2(dba)_3$ (44.8 mg, 48.9 μmol), Xantphos (56.6 mg, 97.8 μmol) and DIEA (190 mg, 1.47 mmol) in dioxane (5 mL) was degassed and purged 3 times with $N_2$, and then the mixture was stirred under $N_2$ atmosphere at 100 °C for 3 hours. Upon completion of the reaction, the reaction mixture was filtered, and the filtrate was concentrated under reduced pressure to obtain a residue. The residue was purified by column chromatography ($SiO_2$, PE/EA = 3/1 to 1/5) to obtain 2-ethylhexyl 3-((5-((S)-6-(((R)-tert-butylsulfinyl)amino)-4,6-dihydrospiro[cyclopenta[d]thiazole-5,4'-piperidin]-1'-yl)pyrazin-2-yl)thio) propanoate (240 mg, 81% yield) as a yellow solid.

$^1$H NMR (400 MHz, $CDCl_3$) δ= 8.80 (s, 1H), 8.09 (d, J = 1.2 Hz, 1H), 8.06 (d, J = 1.2 Hz, 1H), 4.63 (d, J = 8.8 Hz, 1H), 4.26 - 4.14 (m, 2H), 4.13 - 4.08 (m, 2H), 4.01 (dd, J = 2.8, 5.6 Hz, 2H), 3.67 (d, J = 8.8 Hz, 1H), 3.28 (t, J = 7.2 Hz, 2H), 3.22 - 3.07 (m, 2H), 3.04 - 2.89 (m, 2H), 2.04 - 1.95 (m, 2H), 1.83 - 1.71 (m, 2H), 1.37 - 1.27 (m, 9H), 1.22 (s, 9H), 0.91 - 0.86 (m,

6H).

**Step 3: sodium 5-((S)-6-(((R)-tert-butylsulfinyl)amino)-4,6-dihydrospiro[cyclopenta[d]thiazole-5,4'-piperidin]-1'-yl)pyrazine-2-thiolate**

To a solution of 2-ethylhexyl 3-((5-((S)-6-(((R)-tert-butylsulfinyl)amino)-4,6-dihydrospiro[cyclopenta[d]thiazole-5,4'-piperidin]-1'-yl)pyrazin-2-yl)thio)propanoate (230 mg, 378 μmol) in THF (5 mL), t-BuONa (54.5 mg, 568 μmol) was added, and the mixture was stirred at 25 °C for 0.5 hours. Upon completion of the reaction, the reaction mixture was concentrated and the residue was triturated with PE (30 mL) to obtain Intermediate I-10 (170 mg, crude product) as a yellow solid.

MS (EI) m/z: 424.2 $[M-Na]^+$.

**Preparation Example 11: 7-bromo-8-chloro-2-methylisoquinolin-1(2H)-one (Intermediate 1-11)**

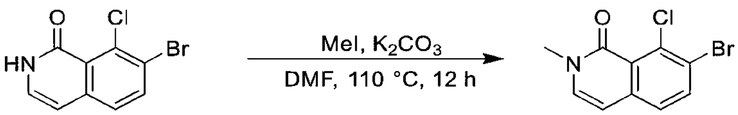


I-9 I-11

To a mixture of Intermediate I-9 (100 mg, 387 μmol) in DMF (5 mL), $K_2CO_3$ (160 mg, 1.16 mmol) and MeI (82.4 mg, 580 μmol, 36.1 μL) were added. The mixture was stirred at 25 °C for 16 hours. Upon completion of the reaction, the mixture was added with water (20 mL) and extracted with EA (3 x 30 mL). The combined organic phase was washed with brine (3 x 30 mL) and concentrated under reduced pressure. The residue was purified by Prep-HPLC to obtain Intermediate I-11 (60 mg, 57% yield) as a white solid.

$^1H$ NMR (400 MHz, $CDCl_3$) δ= 7.83 (d, J = 8.4 Hz, 1H), 7.28 (s, 1H), 7.17 (d, J = 7.2 Hz, 1H), 6.41 (d, J = 7.2 Hz, 1H), 3.60 (s, 3H).

**Preparation Example 12: (R)-2-methyl-N-[(5S)-spiro[5,7-dihydrocyclopenta[b]pyridine-6,4'-piperidin]-5-yl]propane-2-sulfinamide (Intermediate I-12)**

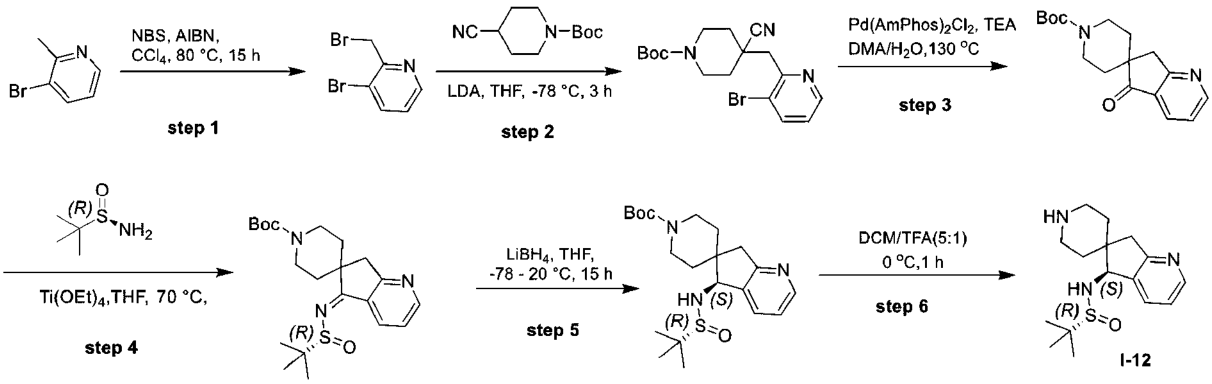


**Step 1: 3-bromo-2-(bromomethyl)pyridine**

To a solution of 3-bromo-2-methyl-pyridine (20.0 g, 116 mmol) in $CCl_4$ (200 mL), N-bromosuccinimide(NBS) (22.8 g, 128 mmol) and azobisisobutyronitrile(AIBN) (1.91 g, 11.6 mmol) were added, and the mixture was stirred at 80°C for 12 hours. The reaction mixture was filtered and concentrated under reduced pressure to obtain a residue. The residue was purified by MPLC (PE/EA=30:1) to obtain 3-bromo-2-(bromomethyl)pyridine (12.5 g, 42% yield) as a brown solid.

$^1H$ NMR (400 MHz, DMSO-$d_6$) δ= 8.60 - 8.53 (m, 1H), 8.16 - 8.08 (m, 1H), 7.38 - 7.26 (m, 1H), 4.80 - 4.70 (m, 2H); MS (EI) m/z: 251.8 $[M+H]^+$.

**Step 2: tert-butyl 4-[(3-bromo-2-pyridyl)methyl]-4-cyano-piperidine-1-carboxylate**

To a solution of tert-butyl 4-cyanopiperidine-1-carboxylate (10.1 g, 47.8 mmol) in THF (100 mL), lithium diisopropylamide(LDA) (2M, 23.9 mL) was added, followed by stirring at -78°C for 0.5 hours. Then, the reaction mixture was slowly added with 3-bromo-2-(bromomethyl)pyridine (10.0 g, 39.9 mmol) in THF (100 mL) and then stirred at -78°C for 2.5 hours. The reaction mixture was quenched by addition of aqueous ammonium chloride (100 mL) at 0°C, diluted with water (100 mL), and extracted with EA (3 X 200 mL). The combined organic layers were washed with brine (3 x 100 mL), dried over anhydrous sodium sulfate, and then filtered and concentrated under reduced pressure to obtain a residue. The residue was purified by reverse phase flash column chromatography (0.1% FA) to obtain tert-butyl 4-[(3-bromo-2-pyridyl) methyl]-4-cyano-piperidine-1-carboxylate (13.5 g, 89% yield) as a brown solid. $^1$H NMR (400 MHz, DMSO-$d_6$) δ= 8.60 - 8.53 (m, 1H), 8.14 - 8.07 (m, 1H), 7.32 - 7.24 (m, 1H), 4.01 - 3.90 (m, 2H), 3.25 (s, 2H), 3.01 - 2.78 (m, 2H), 2.12 - 2.03 (m, 2H), 1.73 - 1.56 (m, 2H), 1.44 - 1.35 (m, 9H); MS (EI) m/z: 402.0 $[M+23]^+$.

**Step 3: tert-butyl 5-oxospiro[7H-cyclopenta[b]pyridine-6,4'-piperidine]-1'-carboxylate**

A mixture of tert-butyl 4-[(3-bromo-2-pyridyl)methyl]-4-cyano-piperidine-1-carboxylate (5.90 g, 15.5 mmol), 4-di-tert-butylphosphanyl-N,N-dimethyl-aniline;dichloropalladium (Pd(AmPhos)$Cl_2$) (1.10 g, 1.55 mmol), TEA (6.28 g, 62.1 mmol) in dimethylacetamide(DMA) (120 mL) and $H_2O$ (12 mL) was degassed and then purged 3 times with $N_2$. The mixture was stirred under $N_2$ atmosphere at 130°C for 12 hours. The reaction mixture was quenched by addition of aqueous ammonium chloride (50 mL) at 25°C, and then diluted with water (50 mL), and extracted with EA (3 x 100 mL). The combined organic layers were washed with brine (3 x 50 mL), dried over anhydrous sodium sulfate, and then filtered and concentrated under reduced pressure to obtain a residue. The residue was purified by silica gel chromatography (PE/EA=3:1) to obtain tert-butyl 5-oxospiro[7H-cyclopenta[b]pyridine-6,4'-piperidine]-1'-carboxylate (3.2 g, 68% yield) as a brown solid.

$^1$H NMR (400 MHz, $CDCl_3$) δ= 8.89 - 8.79 (m, 1H), 8.09 - 8.00 (m, 1H), 7.40 - 7.32 (m, 1H), 4.22 - 4.05 (m, 2H), 3.10 (s, 2H), 3.12 - 3.02 (m, 2H), 2.01 - 1.90 (m, 2H), 1.51 - 1.44 (m, 11H); MS (EI) m/z: 303.4 $[M+H]^+$.

**Step 4: tert-butyl (5Z)-5-[(R)-tert-butylsulfinyl]iminospiro[7H-cyclopenta[b]pyridine-6,4'-piperidine]-1'-carboxylate**

To a solution of tert-butyl 5-oxospiro[7H-cyclopenta[b]pyridine-6,4'-piperidine]-1'-carboxylate (3.00 g, 9.92 mmol) in THF (30 mL), $Ti(OEt)_4$ (34.0 g, 149 mmol) and (R)-2-methylpropane-2-sulfinamide (4.81 g, 39.7 mmol) were added. The mixture was stirred at 70°C for 12 hours. The reaction mixture was diluted with EA (300 mL) and quenched with water (50 mL), and then filtered and extracted with EA (3 x 100 mL). The combined organic layers were washed with brine (3 x 50 mL), dried over anhydrous sodium sulfate, and then filtered and concentrated under reduced pressure to obtain a residue. The residue was purified by reverse phase flash column chromatography (0.1% ammonium hydroxide) to obtain tert-butyl (5Z)-5-[(R)-tert-butylsulfinyl]iminospiro[7H-cyclopenta[b]pyridine-6,4'-piperidine]-1'-carboxylate (3.35 g, 83% yield) as a yellow solid.

$^1$H NMR (400 MHz, DMSO-$d_6$) δ8.79 - 8.74 (m, 1H), 8.69 - 8.61 (m, 1H), 7.50 - 7.44 (m, 1H), 4.00 - 3.93 (m, 2H), 3.00 (s, 2H), 3.02 - 2.89 (m, 2H), 1.81 - 1.68 (m, 2H), 1.58 - 1.50 (m, 2H), 1.44 - 1.42 (m, 9H), 1.27 - 1.22 (m, 9H); MS (EI) m/z: 406.2 $[M+H]^+$.

**Step 5: tert-butyl (5S)-5-[[(R)-tert-butylsulfinyl]amino]spiro[5,7-dihydrocyclopenta[b]pyridine-6,4'-piperidine]-1'-carboxylate**

To a solution of tert-butyl (5Z)-5-[(R)-tert-butylsulfinyl]iminospiro[7H-cyclopenta[b]pyridine-6,4'-piperidine]-1'-carboxylate (2.00 g, 4.93 mmol) in THF (20 mL), $LiBH_4$ (537 mg, 24.7 mmol) was added, and the mixture was stirred at -70°C for 2 hours. The reaction mixture was quenched by addition of aqueous ammonium chloride (20 mL) at 0°C, and then diluted with water (30 mL) and extracted with EA (3 x 50 mL). The combined organic layers were washed with brine (3 x 20 mL), dried over anhydrous sodium sulfate, and then filtered and concentrated under reduced pressure to obtain a residue. The residue was purified by Prep-HPLC (column: Welch Ultimate XB-CN 250*50*10 um, mobile phase: [hexane-EtOH(0.1% $NH_3 \cdot H_2O$)], B%: 10%-50%, 15 minutes) to obtain tert-butyl (5S)-5-[[(R)-tert-butylsulfinyl]amino]spiro[5,7-dihydrocyclopenta[b]pyridine-6,4'-piperidine]-1'-carboxylate (630 mg, 31 % yield) as a yellow solid.

$^1$H NMR (400 MHz, $CDCl_3$) δ= 8.56 - 8.51 (m, 1H), 8.33 - 8.26 (m, 1H), 7.38 - 7.32 (m, 1H), 4.56 - 4.51 (m, 1H), 4.07 - 3.96 (m, 2H), 3.60 - 3.51 (m, 1H), 3.36 - 3.27 (m, 1H), 2.98 (s, 2H), 1.83 - 1.71 (m, 1H), 1.50 - 1.47 (m, 11H), 1.34 - 1.26 (m, 11H); MS (EI) m/z: 408.3 $[M+H]^+$.

**Step 6: (R)-2-methyl-N-[(5S)-spiro[5,7-dihydrocyclopenta[b]pyridine-6,4'-piperidin]-5-yl]propane-2-sulfinamide**

To a solution of tert-butyl (5S)-5-[[(R)-tert-butylsulfinyl]amino]spiro[5,7-dihydrocyclopenta[b]pyridine-6,4'-piperidine]-1'-carboxylate (200 mg, 491 μmol) in DCM (5 mL), TFA (1.68 g, 14.7 mmol) was added, and the mixture was stirred at 0°C for 1 hour. The reaction mixture was poured into aqueous $K_2CO_3$ (20 mL) at 25°C, and then diluted with water (30 mL) and extracted with DCM (3 x 50 mL). The combined organic layers were washed with brine (3 x 20 mL), dried over anhydrous sodium sulfate, and then filtered and concentrated under reduced pressure to obtain Intermediate I-12 (140 mg, 92% yield) as a yellow solid.

$^1$H NMR (400 MHz, $CDCl_3$) = 8.49 - 8.42 (m, 1H), 8.12 - 8.04 (m, 1H), 7.25 - 7.19 (m, 1H), 4.55 - 4.50 (m, 1H), 3.38 - 3.32 (m, 2H), 3.26 - 3.06 (m, 1H), 2.85 - 2.81 (m, 2H), 2.30 - 2.14 (m, 2H), 1.99 - 1.82 (m, 2H), 1.37 - 1.31 (m, 11H); MS (EI) m/z: 308.4 $[M+H]^+$.

**Preparation Example 13: N-((S)-1,3-dihydrospiro[indene-2,4'-piperidin]-1-yl)-2-methylpropane-2-sulfinamide (Intermediate I-13)**

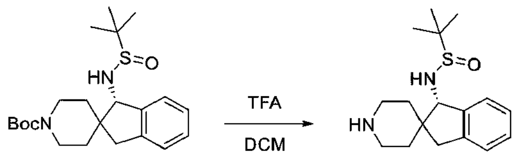


I-13

Tert-butyl (1S)-1-((tert-butylsulfinyl)amino)-1,3-dihydrospiro[indene-2,4'-piperidine]-1'-carboxylate (0.5 g, 1.23 mmol) was dissolved in DCM (6.9 ml, 0.18 M). The reaction mixture was slowly added dropwise with TFA (1.3 ml, 0.095 M) and then stirred at room temperature for 3 hours. Upon completion of the reaction, the resulting product was concentrated to obtain Intermediate I-13.

MS (EI) m/z: 307.4 $[M+H]^+$.

**Preparation Example 14: 6-((5-bromopyrazin-2-yl)thio)-5-chloro-3-methylquinazolin-4(3H)-one (Intermediate 1-14)**

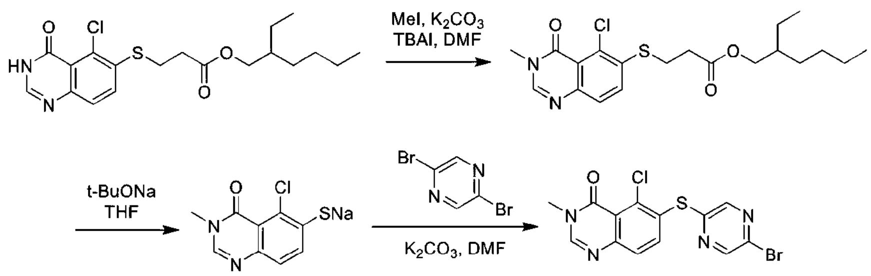


I-14

**Step 1: 2-ethylhexyl 3-((5-chloro-3-methyl-4-oxo-3,4-dihydroquinazolin-6-yl)thio)propanoate**

To a solution of 2-ethylhexyl 3-[(5-chloro-4-oxo-3H-quinazolin-6-yl)sulfanyl]propanoate (1.00 g, 2.52 mmol) and iodomethane (536 mg, 3.78 mmol) in DMF (10 mL), TBAI (93.0 mg, 251 μmol) and $K_2CO_3$ (1.04 g, 7.56 mmol) were added. The mixture was stirred at 25 °C for 12 hours. Upon completion of the reaction, the reaction mixture was quenched by addition of aqueous ammonium chloride (30 mL) at 25 °C, and then diluted with water (50 mL) and extracted with EA (3 x 80 mL). The combined organic layers were washed with brine (50 mL), dried over anhydrous sodium sulfate, and filtered and concentrated under reduced pressure to obtain a residue. The residue was purified by column chromatography ($SiO_2$, petroleum ether/ethyl acetate = 5/1 to 2/1) to obtain 2-ethylhexyl 3-(5-chloro-3-methyl-4-oxo-quinazolin-6-yl)sulfanylpropanoate (850 mg, 82% yield) as a yellow solid.

$^1$H NMR (400 MHz, DMSO-$d_6$) δ= 8.36 (s, 1H), 7.79 (d, J = 8.8 Hz, 1H), 7.60 (d, J = 8.8 Hz, 1H), 3.96 (d, J = 5.6 Hz, 2H), 3.44 (s, 3H), 3.30 (d, J = 6.8 Hz, 2H), 2.73 (d, J = 5.2 Hz, 2H), 1.55 - 1.49 (m, 1H), 1.31 - 1.26 (m, 2H), 1.24 - 1.23 (m, 1H),

1.22 (d, J = 2.0 Hz, 4H), 1.21 - 1.15 (m, 2H), 0.83 (d, J = 7.2 Hz, 6H).

**Step 2: sodium 5-chloro-3-methyl-4-oxo-3,4-dihydroquinazolin-6-thiolate**

To a solution of 2-ethylhexyl 3-(5-chloro-3-methyl-4-oxo-quinazolin-6-yl)sulfanylpropanoate (750 mg, 1.83 mmol) in THF (20 mL), t-BuONa (263 mg, 2.74 mmol) was added. The mixture was stirred at 25 °C for 1 hour. Upon completion of the reaction, the reaction mixture was dissolved in PE (50 mL) and filtered. The filter cake was dried to obtain sodium 5-chloro-3-methyl-4-oxo-3,4-dihydroquinazolin-6-thiolate (400 mg, crude product) as a yellow solid, which was used in the next step without further purification.

**Step 3: 6-((5-bromopyrazin-2-yl)thio)-5-chloro-3-methylquinazolin-4(3H)-one**

To a solution of sodium 5-chloro-3-methyl-4-oxo-3,4-dihydroquinazolin-6-thiolate (400 mg, 1.61 mmol) and 2,5-dibromopyrazine (1.15 g, 4.83 mmol) in DMF (30 mL), $K_2CO_3$ (666 mg, 4.83 mmol) was added. The mixture was stirred at 0 °C for 4 hours. Upon completion of the reaction, the reaction mixture was quenched by addition of aqueous ammonium chloride (20 mL) at 25 °C, and then diluted with water (50 mL) and extracted with EA (3 x 50 mL). The combined organic layers were washed with brine (20 mL), dried over anhydrous sodium sulfate, and filtered and concentrated under reduced pressure to obtain a residue. The residue was purified by column chromatography ($SiO_2$, petroleum ether/ethyl acetate = 5/1 to 2/1) to obtain Intermediate I-14 (180 mg, 29% yield) as a white solid.

$^1H$ NMR (400 MHz, DMSO-$d_6$) $\delta$ = 8.72 (d, J = 1.2 Hz, 1H), 8.48 - 8.47 (m, 2H), 7.98 (d, J = 8.4 Hz, 1H), 7.64 (d, J = 8.4 Hz, 1H), 3.47 (s, 3H).

**Preparation Example 15: 6-((3-amino-5-chloropyrazin-2-yl)thio)-5-chloro-3-methylquinazolin-4(3H)-one (Intermediate 1-15)**

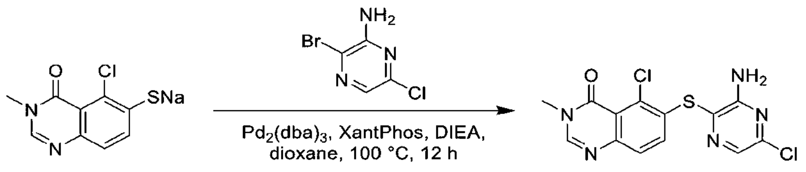


I-15

Intermediate I-15 (260 mg, 18% yield) was synthesized using sodium 5-chloro-3-methyl-4-oxo-3,4-dihydroquinazolin-6-thiolate instead of [5-chloro-3-(2-methoxyethyl)-4-oxo-quinazolin-6-yl]sulfanylsodium in Preparation Example 6.

MS (EI) m/z: 354.0 [M+H]$^+$.

**Preparation Example 16: 6-((3-amino-5-chloropyrazin-2-yl)thio)-3-benzyl-5-chloroquinazolin-4(3H)-one (Intermediate I-16)**

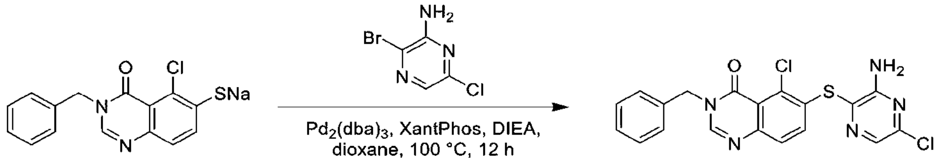


I-16

Intermediate I-16 (300 mg, 23% yield) was synthesized using 6-((3-amino-5-chloropyrazin-2-yl)thio)-3-benzyl-5-chloroquinazolin-4(3H)-one instead of 5-chloro-3-(2-methoxyethyl)-4-oxo-quinazolin-6-yl]sulfanylsodium in Preparation Example 6.

MS (EI) m/z: 430.0 [M+H]$^+$.

**Preparation Example 17: 6-((3-amino-5-chloropyrazin-2-yl)thio)-5-chloroquinazolin-4(3H)-one (Intermediate I-17)**

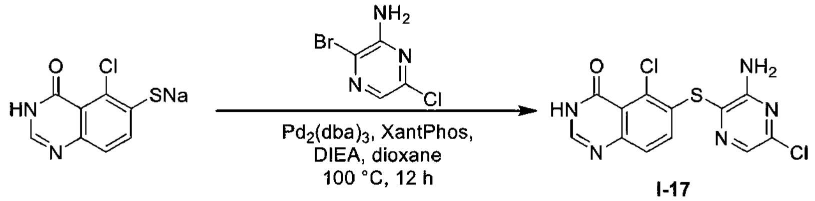


I-17

Intermediate I-17 (700 mg, 28% yield) was synthesized using sodium 5-chloro-4-oxo-3,4-dihydroquinazolin-6-thiolate instead of [5-chloro-3-(2-methoxyethyl)-4-oxo-quinazolin-6-yl]sulfanylsodium in Preparation Example 6.
$^1$H NMR (400 MHz, DMSO-$d_6$) $\delta$= 8.09 (s, 1H), 7.74 (s, 1H), 7.57 - 7.52 (m, 2H), 7.11 (s, 1H), 3.30 (s, 2H).

**Preparation Example 18: 6-((5-bromopyrazin-2-yl)thio)-3-methylquinazolin-4(3H)-one (Intermediate I-18)**

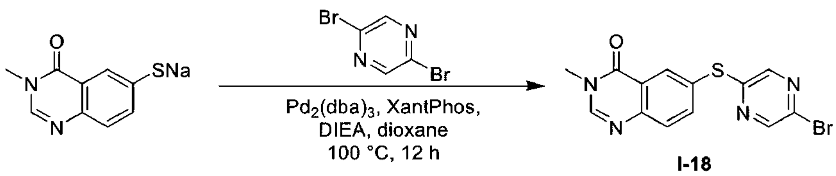


I-18

Intermediate I-18 (500 mg, 31% yield) was synthesized using sodium 3-methyl-4-oxo-3,4-dihydroquinazolin-6-thiolate instead of [5-chloro-3-(2-methoxyethyl)-4-oxo-quinazolin-6-yl]sulfanylsodium, and 2,5-dibromopyrazine instead of 3-bromo-6-chloro-pyrazine-2-amine in Preparation Example 6.
MS (EI) m/z: 349.2 [M+H]$^+$.

**Preparation Example 19: tert-butyl ((3S,4S)-8-(5-((5-chloro-4-oxo-3,4-dihydroquinazolin-6-yl)thio)pyrazin-2-yl)-3-methyl-2-oxa-8-azaspiro[4.5]decan-4-vl)carbamate (Intermediate I-19)**

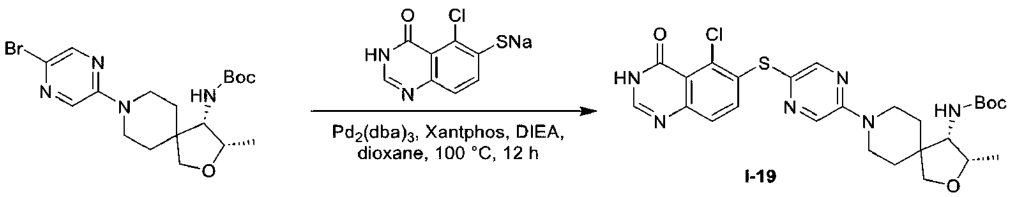


I-19

Intermediate I-19 (200 mg, 34% yield) was synthesized using sodium 5-chloro-4-oxo-3,4-dihydroquinazolin-6-thiolate instead of [5-chloro-3-(2-methoxyethyl)-4-oxo-quinazolin-6-yl]sulfanylsodium, and the resulting product from step 2 of Preparation Example 8 instead of 3-bromo-6-chloro-pyrazine-2-amine in Preparation Example 6.
$^1$H NMR (400 MHz, $CDCl_3$) $\delta$= 11.11 (br s, 1H), 8.24 (d, J = 1.2 Hz, 1H), 8.18 (d, J = 1.2 Hz, 1H), 7.98 (s, 1H), 7.47 (d, J = 8.8 Hz, 1H), 7.22 (d, J = 8.8 Hz, 1H), 4.30 - 4.22 (m, 2H), 4.20 - 4.12 (m, 1H), 3.93 (d, J = 9.2 Hz, 1H), 3.69 (d, J = 9.2 Hz, 1H), 3.64 - 3.53 (m, 2H), 3.21 - 3.10 (m, 2H), 2.11 - 1.97 (m, 2H), 1.76 - 1.63 (m, 2H), 1.29 (s, 9H), 1.24 (d, J = 6.4 Hz, 3H); MS (EI) m/z: 559.1 [M+H]$^+$.

**Preparation Example 20: tert-butyl ((3S,4S)-8-(5-((5-chloro-4-oxo-3,4-dihydroquinazolin-6-yl)thio)pyrazin-2-yl)-3-methyl-2-oxa-8-azaspiro[4.5]decan-4-vl)carbamate (Intermediate I-20)**

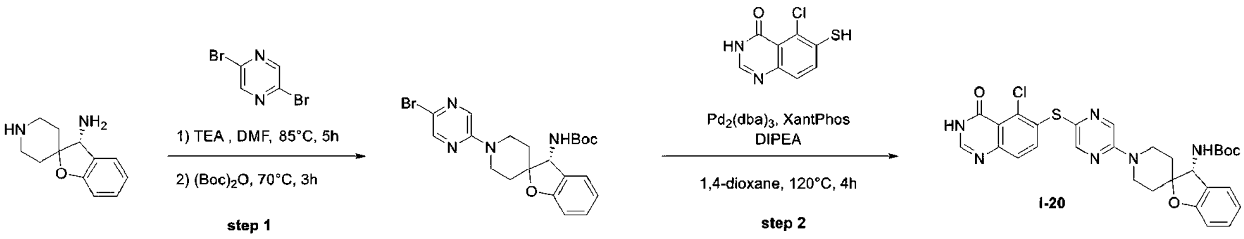


I-20

**Step 1: tert-butyl (R)-(1'-(5-bromopyrazin-2-yl)-3H-spiro[benzofuran-2,4'-piperidin]-3-yl)carbamate**

In a round bottom flask, (R)-3H-spiro[benzofuran-2,4'-piperidine]-3-amine (200mg, 0.72mmol), 2,5-dibromopyrazine (296mg, 1.08mmol) and TEA (0.8ml, 3.6mmol) were dissolved in DMF (3.6ml, 0.2M), and then stirred at 90 °C for 5 hours. The reaction mixture was added with di-tert-butyl dicarbonate (0.25ml, 1.08mmol) and stirred at 90 °C for 5 hours. The reaction was terminated with $H_2O$, and the mixture was extracted with EA. The EA layer was dried over $MgSO_4$, filtered and concentrated. The resulting product was separated by MPLC (EA:Hx=1:10) and concentrated to obtain tert-butyl (R)-(1'-(5-bromopyrazin-2-yl)-3H-spiro[benzofuran-2,4'-piperidin]-3-yl)carbamate (200mg, 60%).

Step 2: **tert-butyl (R)-(1'-(5-((5-chloro-4-oxo-3,4-dihydroquinazolin-6-yl)thio)pyrazin-2-yl)-3H-spiro[benzofuran-2,4'-piperidin]-3-yl)carbamate**

In a round bottom flask, tert-butyl (R)-(1'-(5-bromopyrazin-2-yl)-3H-spiro[benzofuran-2,4'-piperidin]-3-yl)carbamate (200mg, 0.43mmol), Intermediate I-8 (164mg, 0.65mmol), $Pd_2(dba)_3$ (40mg, 0.043mmol), XantPhos (25mg, 0.043mmol) and DIPEA (0.15ml, 0.87mmol) were dissolved in 1,4-dioxane (1.8ml, 0.25M) and purged with nitrogen. The reaction mixture was stirred at 120 °C for 4 hours. The reaction was terminated with $H_2O$, and the mixture was extracted with EA. The EA layer was dried over $MgSO_4$, filtered and concentrated to obtain Intermediate I-20 (243mg, 94%).

**Preparation Example 21: 6-bromo-5-chloroquinazolin-4(3H)-one (Intermediate 1-21)**

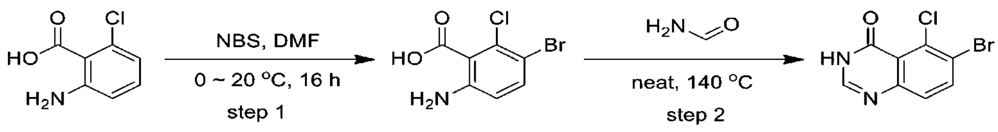


I-21

**Step 1: 6-amino-3-bromo-2-chlorobenzoic acid**

To a solution of 2-amino-6-chloro-benzoic acid (100 g, 583 mmol) in DMF (1000 mL), NBS (114 g, 641 mmol) was added, and the mixture was stirred at 0°C for 2 hours. Upon completion of stirring, the reaction mixture was quenched with water (3 L) and extracted with ethyl acetate (1.5 L x 3). The combined organic layers were washed with brine (1 L x 3), dried over anhydrous sodium sulfate, and then filtered and concentrated under reduced pressure to obtain a residue. The residue was triturated with EA (500 mL) to obtain 6-amino-3-bromo-2-chlorobenzoic acid (113 g, 77% yield) as a brown solid.

MS (EI) m/z: $[M+H]^+$ 251.9, $^1H$ NMR (400 MHz, DMSO-$d_6$) $\delta$ = 7.40 (d, $J$= 8.8 Hz, 1H), 6.64 (d, $J$= 8.8 Hz, 1H), 3.42 (br s, 2H).

**Step 2: 6-bromo-5-chloroquinazolin-4(3H)-one**

A solution of 6-amino-3-bromo-2-chloro-benzoic acid (80 g, 319 mmol) in formamide (216 g, 4.79 mol) was stirred at 140°C for 12 hours. Upon completion of stirring, the reaction mixture was poured into aqueous ammonium chloride (500 mL) at 25°C, and filtered and concentrated under reduced pressure to obtain a residue. The residue was triturated with MeOH (500 mL) to obtain Intermediate I-21 (68 g, 82% yield) as a yellow solid.

MS (EI) m/z: $[M+H]^+$ 261.2, $^1H$ NMR (400 MHz, DMSO-$d_6$) $\delta$ = 12.45 (br s, 1H), 8.16 - 8.06 (m, 2H), 7.54 (d, $J$= 8.8 Hz, 1H).

**Preparation Example 22: sodium 5-chloro-4-oxo-3,4-dihydroquinazolin-6-thiolate (Intermediate 1-22)**

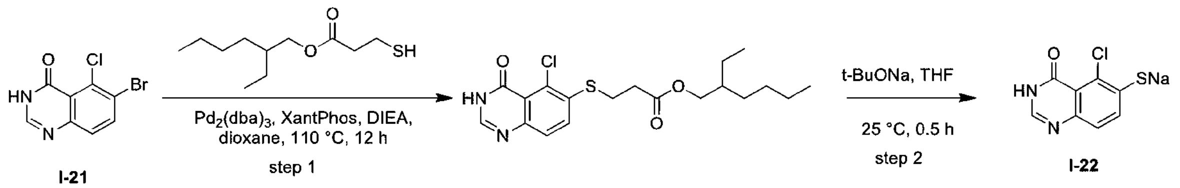


**Step 1: 2-ethylhexyl 3-((5-chloro-4-oxo-3,4-dihydroquinazolin-6-yl)thio)propanoate**

A mixture of Intermediate I-21 (64 g, 247 mmol), 2-ethylhexyl 3-sulfanylpropanoate (80.8 g, 370 mmol), $Pd_2(dba)_3$ (11.3 g, 12.3 mmol), Xantphos (14.3 g, 24.7 mmol) and DIEA (95.6 g, 740 mmol) in dioxane (1000 mL) was degassed and purged 3 times with $N_2$, and then the mixture was stirred under $N_2$ atmosphere at 100°C for 12 hours. Upon completion of stirring, the reaction mixture was filtered and concentrated under reduced pressure to obtain a residue. The residue was purified by MPLC (petroleum ether/ethyl acetate = 1/1 to 1:2) to obtain 2-ethylhexyl 3-[(5-chloro-4-oxo-3H-quinazolin-6-yl)sulfanyl]propanoate (81 g, 83% yield) as a brown solid.

MS (EI) m/z: $[M+H]^+$ 397.2, $^1H$ NMR (400 MHz, $CDCl_3$) $\delta$ = 11.42 - 10.88 (m, 1H), 8.10 - 8.04 (m, 1H), 7.71 - 7.65 (m, 2H), 4.09 - 4.03 (m, 2H), 3.30 (t, $J$ = 7.4 Hz, 2H), 2.73 (t, $J$ = 7.4 Hz, 2H), 1.42 - 1.25 (m, 9H), 0.94 - 0.85 (m, 6H).

**Step 2: sodium 5-chloro-4-oxo-3,4-dihydroquinazolin-6-thiolate**

To a solution of 2-ethylhexyl 3-[(5-chloro-4-oxo-3H-quinazolin-6-yl)sulfanyl]propanoate (42 g, 106 mmol) in THF (1000 mL), t-BuONa (25.4 g, 265 mmol) was added, and then the mixture was stirred at 25°C for 0.5 hours. Upon completion of stirring, the reaction mixture was concentrated under reduced pressure to obtain a residue. The residue was triturated with PE (2 L) to obtain Intermediate I-22 (24.5 g, crude product) as a yellow solid.

MS (EI) m/z: $[M+H]^+$ 213.3, $^1H$ NMR (400 MHz, MeOD) $\delta$ = 7.91 (s, 1H), 7.89 (d, $J$ = 8.6 Hz, 1H), 7.12 - 7.08 (d, $J$ = 8.6 Hz, 1H).

**Preparation Example 23: N-((3S,4S)-8-(5-((5-chloro-4-oxo-3,4-dihydroquinazolin-6-yl)thio)pyrazin-2-yl)-3-methyl-2-oxa-8-azaspiro[4.5]decan-4-yl)-2-methylpropane-2-sulfinamide (Intermediate 1-23)**

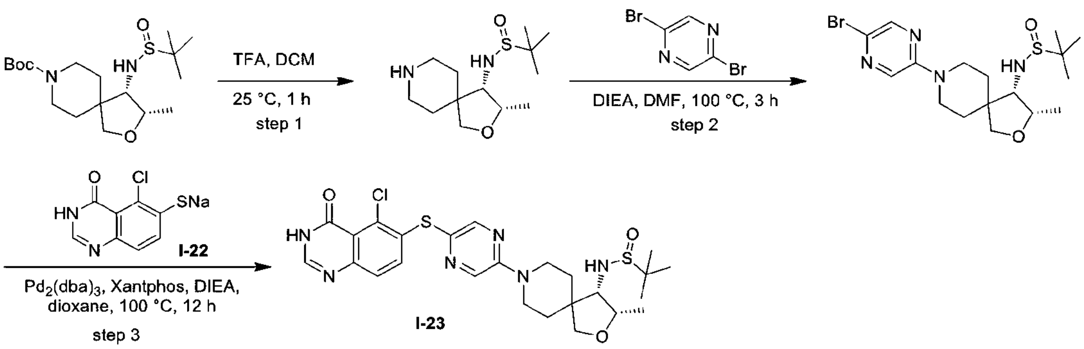


**Step 1: 2-methyl-N-((3S,4S)-3-methyl-2-oxa-8-azaspiro[4.5]decan-4-yl)propane-2-sulfinamide**

To a solution of tert-butyl (3S,4S)-4-(tert-butylsulfinylamino)-3-methyl-2-oxa-8-azaspiro[4.5]decan-8-carboxylate (40 g, 107 mmol) in DCM (400 mL), TFA (244 g, 2.14 mol) was added, and the mixture was stirred at 25°C for 2 hours. Upon completion of stirring, the reaction mixture was concentrated under reduced pressure to obtain 2-methyl-N-[(3S,4S)-3-methyl-2-oxa-8-azaspiro[4.5]decan-4-yl]propane-2-sulfinamide (29.3 g, crude product) as a colorless oil. MS (EI) m/z: $[M+H]^+$ 275.4

**Step 2: N-((3S,4S)-8-(5-bromopyrazin-2-yl)-3-methyl-2-oxa-8-azaspiro[4.5]decan-4-yl)-2-methylpropane-2-sulfinamide**

To a solution of 2-methyl-N-[(3S,4S)-3-methyl-2-oxa-8-azaspiro[4.5]decan-4-yl]propane-2-sulfinamide (29.3 g, 107 mmol) and 2,5-dibromopyrazine (50.8 g, 214 mmol) in DMF (200 mL), DIEA (138 g, 1.07 mol) was added, and the mixture was stirred at 100°C for 3 hours. Upon completion of stirring, the reaction mixture was quenched by addition of aqueous ammonium chloride (200 mL) at 25°C, and then diluted with water (300 mL) and extracted with EA (500 mL x 3). The combined organic layers were washed with brine (300 mL x 3), dried over anhydrous sodium sulfate, and then filtered and concentrated under reduced pressure to obtain a residue. The residue was purified by MPLC (petroleum ether/ethyl acetate=2/1 to 1:1) to obtain N-[(3S,4S)-8-(5-bromopyrazin-2-yl)-3-methyl-2-oxa-8-azaspiro[4.5]decan-4-yl]-2-methylpropane-2-sulfinamide (43 g, 93% yield) as a yellow solid.

MS (EI) m/z: $[M+H]^+$ 431.2, $^1H$ NMR (400 MHz, $CDCl_3$) δ = 8.10 (d, *J* = 1.2 Hz, 1H), 7.85 (d, *J* = 1.2 Hz, 1H), 4.26 - 4.20 (m, 1H), 4.10 (td, *J* = 4.4, 13.2 Hz, 1H), 4.01 (td, *J* = 4.4, 13.2 Hz, 1H), 3.87 (d, *J* = 9.2 Hz, 1H), 3.67 (d, *J* = 9.2 Hz, 1H), 3.51 (dd, *J* = 5.6, 10.4 Hz, 1H), 3.35 (d, *J* = 10.4 Hz, 1H), 3.18 - 3.04 (m, 2H), 2.07 - 1.93 (m, 2H), 1.74 - 1.69 (m, 1H), 1.50 (d, *J* = 7.6 Hz, 1H), 1.26 (s, 9H), 1.23 (d, *J* = 6.4 Hz, 3H).

**Step 3: N-((3S,4S)-8-(5-((5-chloro-4-oxo-3,4-dihydroquinazolin-6-yl)thio)pyrazin-2-yl)-3-methyl-2-oxa-8-azaspiro[4.5]decan-4-yl)-2-methylpropane-2-sulfinamide**

A mixture of Intermediate I-22 (24 g, 102 mmol), N-[(3S,4S)-8-(5-bromopyrazin-2-yl)-3-methyl-2-oxa-8-azaspiro [4.5]decan-4-yl]-2-methyl-propane-2-sulfinamide (35.3 g, 81.8 mmol), $Pd_2(dba)_3$ (9.37 g, 10.2 mmol), Xantphos (11.8 g, 20.5 mmol) and DIEA (39.7 g, 307 mmol) in dioxane (400 mL) was degassed and purged 3 times with $N_2$, and then the mixture was stirred under $N_2$ atmosphere at 110°C for 12 hours. Upon completion of stirring, the reaction mixture was filtered and concentrated under reduced pressure to obtain a residue. The residue was purified by column chromatography (petroleum ether/ethyl acetate=0/1 to EA/MeOH=9/1) to obtain a crude product. The residue was triturated with EA:MeOH=3:1 (400 mL) to obtain Intermediate I-23 (20 g, 34% yield) as a yellow solid.

MS (EI) m/z: $[M+H]^+$ 563.2, $^1H$ NMR (400 MHz, $CDCl_3$) δ = 11.11 (br s, 1H), 8.24 (d, *J* = 1.2 Hz, 1H), 8.18 (d, *J*= 1.2 Hz, 1H), 7.98 (s, 1H), 7.47 (d, *J*= 8.8 Hz, 1H), 7.22 (d, *J* = 8.8 Hz, 1H), 4.30 - 4.22 (m, 2H), 4.20 - 4.12 (m, 1H), 3.93 (d, *J*= 9.2 Hz, 1H), 3.69 (d, *J*= 9.2 Hz, 1H), 3.64 - 3.53 (m, 2H), 3.21 - 3.10 (m, 2H), 2.11 - 1.97 (m, 2H), 1.76 - 1.63 (m, 2H), 1.29 (s, 9H), 1.24 (d, *J*= 6.4 Hz, 3H).

**Preparation Example 24: sodium 5-((3S,4S)-4-((tert-butylsulfinyl)amino)-3-methyl-2-oxa-8-azaspiro[4.5]decan-8-yl)pyrazine-2-thiolate (Intermediate 1-24)**

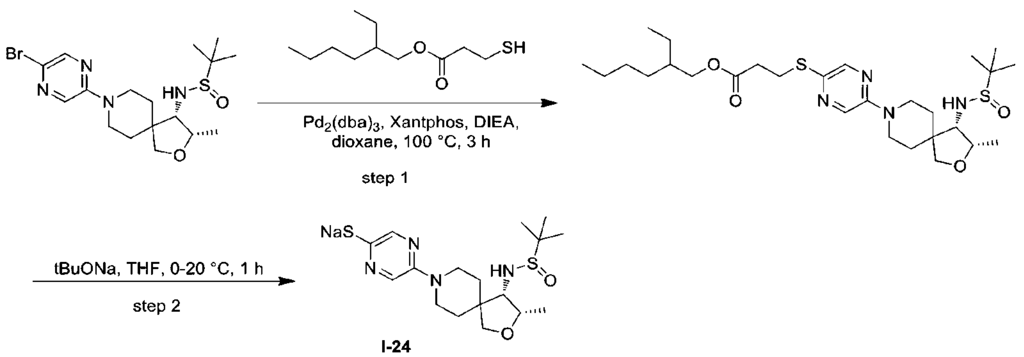


I-24

**Step 1: 2-ethylhexyl 3-((5-((3S,4S)-4-((tert-butylsulfinyl)amino)-3-methyl-2-oxa-8-azaspiro[4.5]decan-8-yl)pyrazin-2-yl)thio)propanoate**

A mixture of the resulting product of step 2 of Preparation Example 23 (6.0 g, 13.9 mmol), 2-ethylhexyl 3-sulfanylpropanoate (3.64 g, 16.7 mmol), $Pd_2(dba)_3$ (1.27 g, 1.39 mmol), Xantphos (1.61 g, 2.78 mmol) and DIPEA (3.60 g, 27.8 mmol) in dioxane (90 mL) was degassed and purged 3 times with $N_2$, and then the mixture was stirred under $N_2$ atmosphere at 100°C for 3 hours. Upon completion of stirring, the reaction mixture was concentrated under reduced pressure to obtain a residue. The residue was purified by flash silica gel chromatography to obtain 2-ethylhexyl 3-[5-[(3S,4S)-4-(tert-butylsulfinylamino)-3-methyl-2-oxa-8-azaspiro[4.5]decan-8-yl]pyrazin-2-yl]sulfanylpropanoate

(7.4 g, 91% yield) as a yellow oil.

MS (EI) m/z: $[M+H]^+$ 569.3.

$^1H$ NMR (400 MHz, $CDCl_3$) δ = 8.09 - 7.99 (m, 2H), 4.27 - 4.19 (m, 1H), 4.12 - 4.06 (m, 1H), 4.06 - 3.97 (m, 3H), 3.87 (d, *J* = 9.2 Hz, 1H), 3.68 (d, *J* = 9.2 Hz, 1H), 3.54 - 3.48 (m, 1H), 3.34 (d, *J* = 10.4 Hz, 1H), 3.27 (t, *J* = 7.2 Hz, 2H), 3.17 - 3.02 (m, 2H), 2.68 (t, *J* = 7.2 Hz, 2H), 2.04 - 1.93 (m, 2H), 1.72 (d, *J* = 13.2 Hz, 1H), 1.66 - 1.60 (m, 2H), 1.59 - 1.53 (m, 1H), 1.43 - 1.31 (m, 3H), 1.31 - 1.27 (m, 6H), 1.26 (s, 9H), 0.94 - 0.86 (m, 6H).

**Step 2: sodium 5-((3S,4S)-4-((tert-butylsulfinyl)amino)-3-methyl-2-oxa-8-azaspiro[4.5]decan-8-yl)pyrazine-2-thiolate**

To a solution of 2-ethylhexyl 3-[5-[(3S,4S)-4-(tert-butylsulfinylamino)-3-methyl-2-oxa-8-azaspiro[4.5]decan-8-yl]pyrazin-2-yl]sulfanylpropanoate (2.0 g, 3.52 mmol) in THF (20 mL), t-BuONa (507 mg, 5.27 mmol) was added. The mixture was stirred at 20°C for 1 hour. Upon completion of stirring, the reaction mixture was concentrated under reduced pressure to obtain a residue. The residue was triturated by PE (15 mL) at 20°C to obtain Intermediate I-24 (1.5 g, crude product) as a light yellow solid.

$^1H$ NMR (400 MHz, DMSO-$d_6$) δ = 7.73 (d, *J*= 1.6 Hz, 1H), 7.54 (s, 1H), 4.13 - 4.05 (m, 1H), 3.77 (d, *J* = 8.8 Hz, 1H), 3.69 - 3.63 (m, 1H), 3.63 - 3.57 (m, 2H), 3.48 - 3.42 (m, 1H), 3.36 (d, *J*= 6.4 Hz, 1H), 2.65 (d, *J*= 15.6 Hz, 1H), 1.78 - 1.74 (m, 2H), 1.56 - 1.52 (m, 1H), 1.24 (d, *J* = 2.0 Hz, 1H), 1.18 - 1.11 (m, 9H), 1.08 (d, *J*= 6.4 Hz, 3H).

**Preparation Example 25: (R)-N-((S)-1,3-dihydrospiro[indene-2,4'-piperidin]-1-yl)-2-methylpropane-2-sulfinamide (Intermediate 1-25)**

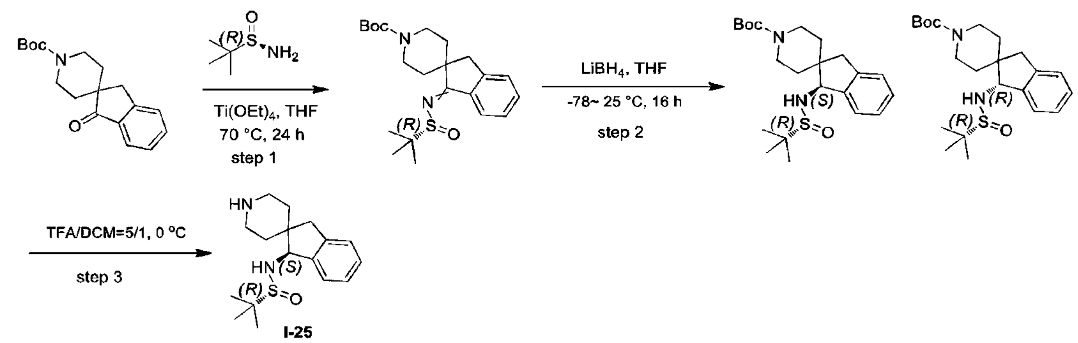


I-25

**Step 1: tert-butyl (R)-1-((tert-butylsulfinyl)imino)-1,3-dihydrospiro[indene-2,4'-piperidine]-1'-carboxylate**

A mixture of tert-butyl 1-oxospiro[indane-2,4'-piperidine]-1'-carboxylate (1.00 g, 3.32 mmol), (R)-2-methylpropane-2-sulfinamide (402 mg, 3.32 mmol) and $Ti(OEt)_4$ (2.27 g, 9.95 mmol) in THF (5 mL) was stirred at 70°C for 36 hours. Upon completion of stirring, the reaction mixture was poured into water (10 mL) at 0°C and filtered. The filtrate was diluted with water (10 ml) and extracted with EtOAc (2 X 20 ml). The combined organic layers were washed with brine (20 ml), dried over $Na_2SO_4$, and filtered and concentrated under reduced pressure to obtain a residue. The residue was purified by silica gel chromatography (PE:EtOAc=3:1) to obtain tert-butyl 1-[(R)-tert-butylsulfinyl]iminospiro[indane-2,4'-piperidine]-1'-carboxylate (900 mg, 67% yield) as a yellow solid.

$^1H$ NMR (400 MHz, $CDCl_3$) δ = 8.41 (d, *J*= 6.4 Hz, 1H), 7.55 - 7.48 (m, 1H), 7.43 - 7.35 (m, 2H), 4.18 - 4.08 (m, 2H), 3.06 (s, 2H), 3.00 - 2.86 (m, 2H), 2.04 - 1.90 (m, 2H), 1.48 (s, 9H), 1.42 (d, *J*= 14.4 Hz, 1H), 1.32 (s, 9H), 1.28 - 1.24 (m, 1H).

Step 2: **tert-butyl (S)-1-(((R)-tert-butylsulfinyl)amino)-1,3-dihydrospiro[indene-2,4'-piperidine]-1'-carboxylate**

To a solution of tert-butyl 1-[(R)-tert-butylsulfinyl]iminospiro[indane-2,4'-piperidine]-1'-carboxylate (800 mg, 1.98 mmol) in THF (10 mL), $LiBH_4$ (129 mg, 5.93 mmol) was added under $N_2$ at -78°C. The reaction mixture was stirred at -78~25°C for 16 hours. Upon completion of stirring, the reaction mixture was quenched by addition of $NH_4Cl$ (10 mL) at 0°C, and then diluted with water (10 mL) and extracted with EtOAc (2 X 20 mL). The combined organic layers were washed with brine (2 X 20 mL), dried over $Na_2SO_4$, and then filtered and concentrated under reduced pressure to obtain a residue. The residue was purified by Prep-HPLC (Column: Welch Ultimate XB-CN 250*70*10 μm; mobile phase: [hexane-EtOH(0.1% $NH_3 \cdot H_2O$)]; B%: 1%-35%, 15 minutes) to obtain tert-butyl (1S)-1-[[(R)-tert-butylsulfinyl]amino]spiro[indane-2,4'-piperidine]-1'-carboxylate (540 mg, 67% yield) as a white solid, and tert-butyl (1R)-1-[[(R)-tert-butylsulfinyl]amino]spiro[indane-2,4'-piperidine]-1'-carboxylate (130 mg, 16% yield) as a white solid.

tert-butyl (1S)-1-[[(R)-tert-butylsulfinyl]amino]spiro[indane-2,4'-piperidine]-1'-carboxylate was used in the next reaction.

$^1$H NMR (400 MHz, $CDCl_3$) δ = 7.23 (d, *J*= 6.4 Hz, 1H), 7.18 - 7.12 (m, 3H), 4.43 (d, *J*= 9.2 Hz, 1H), 4.02 - 3.87 (m, 2H), 3.57 - 3.38 (m, 1H), 3.11 - 2.74 (m, 4H), 2.69 - 2.58 (m, 1H), 2.09 - 1.96 (m, 1H), 1.47 - 1.42 (m, 2H), 1.39 (s, 9H), 1.22 (s, 9H).

**Step 3: (R)-N-((S)-1,3-dihydrospiro[indene-2,4'-piperidin]-1-yl)-2-methylpropane-2-sulfinamide**

A mixture of tert-butyl (1S)-1-[[(R)-tert-butylsulfinyl]amino]spiro[indane-2,4'-piperidine]-1'-carboxylate (100 mg, 245 μmol) and TFA (1 mL) in DCM (5 mL) was stirred at 0°C for 1 hour. Upon completion of stirring, the reaction mixture was diluted with saturated aqueous $NaHCO_3$ (10 mL) and extracted with EtOAc (2 x 10 mL). The combined organic layers were washed with brine (2 x 10 mL), dried over $Na_2SO_4$, and then filtered and concentrated under reduced pressure to obtain Intermediate I-25 (70 mg, 92% yield) as a colorless oil.

$^1$H NMR (400 MHz, $CDCl_3$) δ = 7.29 (s, 1H), 7.26 - 7.19 (m, 3H), 4.49 (d, *J*= 10.4 Hz, 1H), 4.01 (br s, 1H), 3.63 (d, *J* = 10.4 Hz, 1H), 3.20 - 3.02 (m, 3H), 2.94 - 2.66 (m, 3H), 2.23 - 2.21 (m, 1H), 1.72 - 1.66 (m, 1H), 1.60 - 1.57 (m, 1H), 1.33 (s, 9H), 1.19 (d, *J*= 1.6 Hz, 1H).

**Preparation Example 26: sodium 5-((S)-1-(((R)-tert-butylsulfinyl)amino)-1,3-dihydrospiro[indene-2,4'-piperidin]-1'-yl)pyrazine-2-thiolate (Intermediate 1-26)**

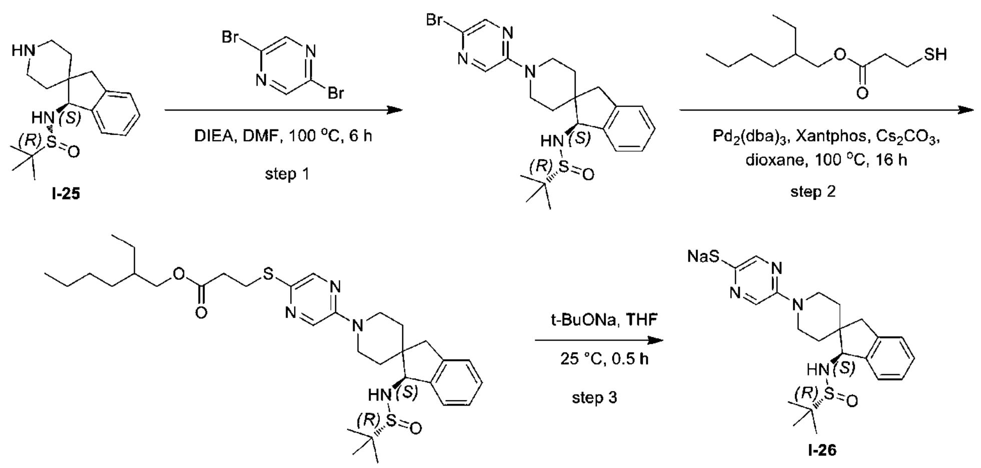


**Step 1: (R)-N-((S)-1'-(5-bromopyrazin-2-yl)-1,3-dihydrospiro[indene-2,4'-piperidin]-1-yl)-2-methylpropane-2-sulfinamide**

To a solution of Intermediate I-25 (2.3 g, 7.50 mmol) in DMF (20 mL), 2,5-dibromopyrazine (5.36 g, 22.5 mmol) and DIEA (9.70 g, 75.0 mmol) were added. The mixture was stirred at 100°C for 6 hours. Upon completion of stirring, the reaction mixture was diluted with $H_2O$ (10 mL) and extracted with EtOAc (3 x 200 mL). The combined organic layers were washed with brine (3 x 200 mL) and dried under reduced pressure to obtain a residue. The residue was purified by flash silica gel chromatography (ISCO® 40 g SepaFlash® silica flash column, eluent: 40~50% ethyl acetate/petroleum ether, gradient @ 40 mL/min) to obtain (R)-N-[(1S)-1'-(5-bromopyrazin-2-yl)spiro[indane-2,4'-piperidin]-1-yl]-2-methyl-propane-2-sulfinamide (2 g, 55% yield) as a light yellow solid.

MS (EI) m/z: $[M+H]^+$ 465.3; $^1$H NMR (400 MHz, DMSO-$d_6$) δ = 8.20 (d, *J* = 1.2 Hz, 1H), 8.16 (d, *J*= 1.2 Hz, 1H), 7.28 - 7.24 (m, 1H), 7.24 - 7.19 (m, 3H), 5.65 (d, *J* = 10.4 Hz, 1H), 4.42 (d, *J* = 10.4 Hz, 1H), 4.27 - 4.15 (m, 2H), 3.14 (d, *J* = 16.0 Hz, 1H), 3.11 - 2.99 (m, 2H), 2.74 - 2.66 (m, 1H), 2.08 - 1.99 (m, 1H), 1.75 - 1.64 (m, 1H), 1.59 (d, *J* = 12.8 Hz, 1H), 1.30 - 1.24 (m, 1H), 1.19 (s, 9H).

**Step 2: 2-ethylhexyl 3-((5-((S)-1-(((R)-tert-butylsulfinyl)amino)-1,3-dihydrospiro[indene-2,4'-piperidin]-1'-yl) pyrazin-2-yl)thio)propanoate**

A mixture of (R)-N-[(1S)-1'-(5-bromopyrazin-2-yl)spiro[indane-2,4'-piperidin]-1-yl]-2-methyl-propane-2-sulfinamide (1 g, 2.16 mmol), 2-ethylhexyl 3-sulfanylpropanoate (565 mg, 2.59 mmol), $Pd_2(dba)_3$ (197 mg, 215 μmol), Xantphos (249 mg, 431 μmol) and DIPEA (836 mg, 6.47 mmol) in dioxane (10 mL) was degassed and purged 3 times with $N_2$, and then the mixture was stirred under $N_2$ atmosphere at 100°C for 16 hours. Upon completion of stirring, the reaction mixture was concentrated under reduced pressure to obtain a residue. The residue was purified by flash silica gel chromatography (ISCO® 40 g SepaFlash® silica flash column, eluent: 35-40% ethyl acetate/petroleum ether, gradient @ 60 mL/min) to obtain 2-ethylhexyl 3-[5-[(1S)-1-[[(R)-tert-butylsulfinyl]amino]spiro[indane-2,4'-piperidin]-1'-yl]pyrazin-2-yl]sulfanylpropanoate (1.2 g, 90% yield) as a light yellow oil.

$^1$H NMR (400 MHz, $CDCl_3$) δ = 8.10 - 8.03 (m, 2H), 7.35 - 7.31 (m, 1H), 7.27 - 7.22 (m, 3H), 4.57 (d, *J*= 12.0 Hz, 1H), 4.23 - 4.16 (m, 2H), 4.04 - 3.99 (m, 2H), 3.58 (d, *J* = 12.0 Hz, 1H), 3.27 (t, *J*= 7.2 Hz, 2H), 3.18 - 3.03 (m, 3H), 2.78 (d, *J* = 15.6 Hz, 1H), 2.76 - 2.60 (m, 2H), 2.37 - 2.27 (m, 1H), 1.95 - 1.85 (m, 1H), 1.74 - 1.66 (m, 1H), 1.59 - 1.54 (m, 1H), 1.46 - 1.31 (m, 5H), 1.30 (s, 9H), 1.29 - 1.24 (m, 4H), 0.92 - 0.88 (m, 6H).

**Step 3: sodium 5-((S)-1-(((R)-tert-butylsulfinyl)amino)-1,3-dihydrospiro[indene-2,4'-piperidin]-1'-yl)pyrazine-2-thiolate**

To a mixture of 2-ethylhexyl 3-[5-[(1S)-1-[[(R)-tert-butylsulfinyl]amino]spiro[indane-2,4'-piperidin]-1'-yl]pyrazin-2-yl]sulfanylpropanoate (200 mg, 332 μmol) in THF (2 mL), t-BuONa (47.9 mg, 499 μmol) was added, and then the mixture was stirred at 25°C for 0.5 hours. Upon completion of stirring, the reaction mixture is concentrated under reduced pressure to obtain a residue. The residue was triturated with PE (15 mL) at 20°C to obtain Intermediate I-26 (110 mg, crude product) as a yellow solid.
MS (EI) m/z: $[M+H]^+$ 438.5

<u>Preparation Example 27: (4-methyltetrahydro-2H-pyran-4-yl)methyl 4-methylbenzenesulfonate (Intermediate 1-27)</u>

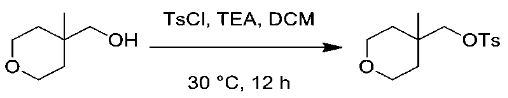


**I-27**

To a solution of (4-methyltetrahydropyran-4-yl)methanol (400 mg, 3.07 mmol) in DCM (5 mL), TEA (932 mg, 9.22 mmol) and 4- methylbenzenesulfonyl chloride (878 mg, 4.61 mmol) were added. The mixture was stirred at 30°C for 12 hours. Upon completion of stirring, the reaction mixture was filtered, and the filtrate was concentrated under reduced pressure to obtain a residue. The residue was purified by column chromatography ($SiO_2$, petroleum ether/ethyl acetate = 10/1 to 4/1) to obtain Intermediate I-27 (800 mg, 91% yield) as a white solid.

$^1$H NMR (400 MHz, $CDCl_3$) δ = 7.79 (d, *J*= 8.4 Hz, 2H), 7.36 (d, *J* = 8.0 Hz, 2H), 3.77 (s, 2H), 3.68 - 3.52 (m, 4H), 2.46 (s, 3H), 1.50 (dd, *J= 4.4,* 13.6 Hz, 2H), 1.34 - 1.23 (m, 2H), 1.03 (s, 3H).

<u>**Preparation Example** 28: **6-bromo-5-chloro-3-(2-hydroxy-2-methylpropyl)quinazolin-4(3H)-one (Intermediate 1-28)**</u>

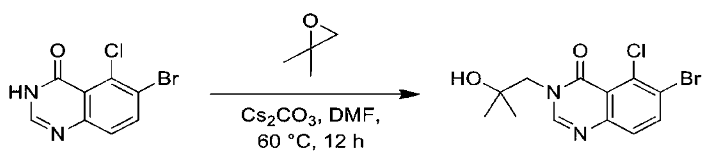


**I-21** **I-28**

To a solution of **Intermediate I-21** (1 g, 3.85 mmol) in DMF (20 mL), $Cs_2CO_3$ (3.77 g, 11.6 mmol) and 2,2-dimethyloxirane (2.78 g, 38.5 mmol) were added. The mixture was stirred at 60°C for 12 hours. Upon completion of stirring, the reaction mixture was quenched by addition of aqueous ammonium chloride (20 mL) at 25°C, and then diluted with water (30 mL) and extracted with EA (3 x 50 mL). The combined organic layers were washed with brine (3 x 20 mL), dried over anhydrous sodium sulfate, and filtered and concentrated under reduced pressure to obtain a residue. The residue was purified by reverse phase flash column chromatography (0.1% FA) to obtain **Intermediate I-28** (540 mg, 42% yield) as a white solid.

$^1H$ NMR (400 MHz, $CDCl_3$) $\delta$ = 8.20 (s, 1H), 7.97 (d, *J*= 8.8 Hz, 1H), 7.51 (d, *J* = 8.8 Hz, 1H), 4.08 (s, 2H), 2.16 - 2.12 (m, 1H), 1.33 (s, 6H).

**Preparation Example** 29: **6-bromo-5-chloro-3-(2-methoxy-2-methylpropyl)quinazolin-4(3H)-one (Intermediate 1-29)**

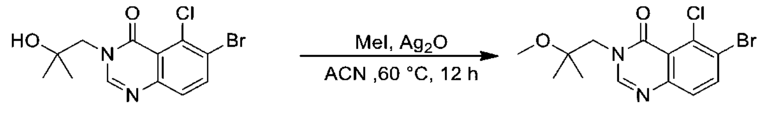


**I-28** **I-29**

To a solution of Intermediate I-28 (350 mg, 1.06 mmol) in ACN (8 mL), $Ag_2O$ (1.22 g, 5.28 mmol) and MeI (749 mg, 5.28 mmol) were added. The mixture was stirred at 25°C for 12 hours. Upon completion of stirring, the reaction mixture was filtered, and the filtrate was concentrated under reduced pressure to obtain a residue. The residue was purified by reverse phase flash column chromatography (0.1% FA) to obtain **Intermediate** I-29 (70 mg, 19% yield) as a white solid.

$^1H$ NMR (400 MHz, $CDCl_3$) $\delta$ = 8.22 (s, 1H), 7.94 (d, *J* = 8.8 Hz, 1H), 7.49 (d, *J* = 8.8 Hz, 1H), 4.07 (s, 2H), 3.21 (s, 3H), 1.21 (s, 6H).

**Preparation Example 30: 6-bromo-5-chloro-3-(1-fluoro-2-methoxy-2-methylpropyl)quinazolin-4(3H)-one (Intermediate I-30)**

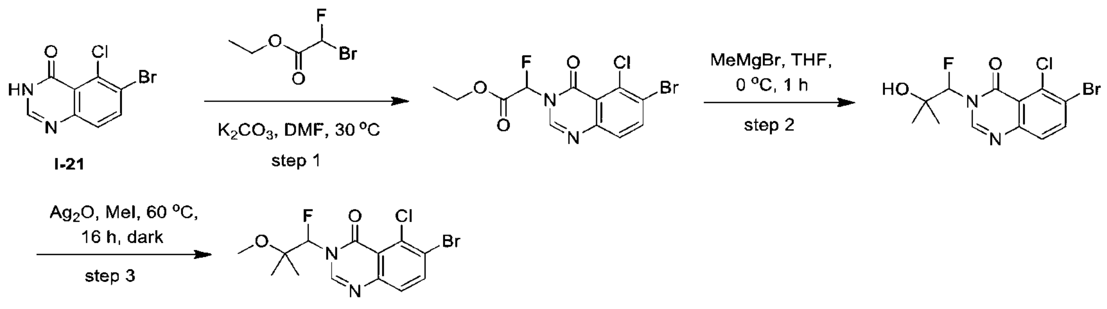


**I-21**

**I-30**

**Step 1: ethyl 2-(6-bromo-5-chloro-4-oxoquinazolin-3(4H)-yl)-2-fluoroacetate**

To a solution of **Intermediate I-21** (16 g, 61.6 mmol) and $K_2CO_3$ (25.5 g, 184 mmol) in DMF (200 mL), ethyl 2-bromo-2-fluoro-acetate (12.5 g, 67.8 mmol) was added. The reaction mixture was stirred at 30°C for 2 hours. Upon completion of stirring, the reaction mixture was added to water (1 L) and extracted with EA (3 x 200 ml). The combined organic layers were washed with brine (2 x 200 ml), dried over $Na_2SO_4$ and concentrated under reduced pressure to obtain ethyl 2-(6-bromo-5-chloro-4-oxo-quinazolin-3-yl)-2-fluoro-acetate (16 g, 71% yield) as a yellow solid.

$^1H$ NMR (400 MHz, DMSO-$d_6$) $\delta$ = 8.61 (s, 1H), 8.24 (d, *J*= 8.8 Hz, 1H), 7.63 (d, *J* = 8.8 Hz, 1H), 6.96 - 6.74 (m, 1H), 4.35 - 4.20 (m, 2H), 1.20 (t, *J* = 7.2 Hz, 3H).

**Step 2: 6-bromo-5-chloro-3-(1-fluoro-2-hydroxy-2-methylpropyl)quinazolin-4(3H)-one**

To a solution of ethyl 2-(6-bromo-5-chloro-4-oxo-quinazolin-3-yl)-2-fluoro-acetate (10 g, 27.5 mmol) in THF (100 mL), MeMgBr (3 M, 18.3 mL) was added at 0°C. The reaction mixture was stirred at 0°C for 1 hour. Upon completion of stirring, the reaction mixture was added to saturated $NH_4Cl$ solution (500ml) and extracted with EA (3 x 100ml). The combined organic layers were dried over $Na_2SO_4$ and concentrated under reduced pressure. The crude product was purified by reverse phase flash column chromatography (0.1% FA) to obtain 6-bromo-5-chloro-3-(1-fluoro-2-hydroxy-2-methyl-propyl)quinazolin-4-one (1.4 g, 14% yield) as a yellow solid.

$^1H$ NMR (400 MHz, DMSO-$d_6$) $\delta$ = 8.48 (s, 1H), 8.19 (d, *J* = 8.8 Hz, 1H), 7.59 (d, *J* = 8.8 Hz, 1H), 6.69 - 6.53 (m, 1H), 5.53 (s, 1H), 1.39 (s, 3H), 1.01 (d, *J* = 1.6 Hz, 3H).

**Step 3: 6-bromo-5-chloro-3-(1-fluoro-2-methoxy-2-methylpropyl)quinazolin-4(3H)-one**

A solution of 6-bromo-5-chloro-3-(1-fluoro-2-hydroxy-2-methyl-propyl)quinazolin-4-one (0.3 g, 858 μmol), $Ag_2O$ (994 mg, 4.29 mmol) and MeI (609 mg, 4.29 mmol) in ACN (6 mL) was stirred in the dark at 60°C for 16 hours. Upon completion of stirring, the reaction mixture was filtered, and the filtrate was concentrated under reduced pressure. The residue was purified by column chromatography ($SiO_2$, petroleum ether/ethyl acetate = 1/0 to 10/1). Thereafter, the residue was purified by Prep-TLC ($SiO_2$, PE: EA = 6: 1) to obtain **Intermediate I-30** (0.1 g, 32% yield) as a white solid.
$^1$H NMR (400 MHz, DMSO-$d_6$) δ = 8.40 (s, 1H), 8.19 (d, *J*= 8.8 Hz, 1H), 7.58 (d, *J* = 8.8 Hz, 1H), 6.89 - 6.56 (m, 1H), 3.25 (s, 3H), 1.41 (s, 3H), 1.06 (d, *J*= 1.6 Hz, 3H).

**Preparation Example 31: 6-bromo-3-(2-methoxy-2-methylpropyl)quinazolin-4(3H)-one (Intermediate 1-31)**

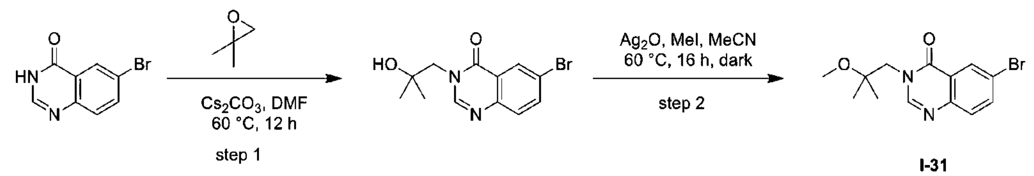


I-31

**Step 1: 6-bromo-3-(2-hydroxy-2-methylpropyl)quinazolin-4(3H)-one**

To a solution of 6-bromo-3H-quinazolin-4-one (1.00 g, 4.44 mmol) and 2,2-dimethyloxirane (3.20 g, 44.4 mmol) in DMF (10 mL), $Cs_2CO_3$ (4.34 g, 13.3 mmol) was added. The mixture was stirred at 60°C for 12 hours. Upon completion of stirring, the reaction mixture was diluted with 30 mL of water and extracted with EA (3 x 50 mL). The combined organic layers were washed with aqueous NaCl solution (3 x 30 mL), dried over $Na_2SO_4$, and then filtered and concentrated under reduced pressure to obtain a residue. The residue was purified by column chromatography ($SiO_2$, petroleum ether/ethyl acetate = 3/1 to 1/1) to obtain 6-bromo-3-(2-hydroxy-2-methyl-propyl)quinazolin-4-one (1.10 g, 83% yield) as a yellow solid.

MS (EI) m/z: $[M+H]^+$ 299.0
$^1$H NMR (400 MHz, $CDCl_3$) δ = 8.37 (d, *J*= 2.0 Hz, 1H), 8.12 (s, 1H), 7.77 (dd, *J* = 2.4, 8.4 Hz, 1H), 7.53 (d, *J*= 8.4 Hz, 1H), 4.01 (s, 2H), 1.24 (s, 6H).

**Step 2: 6-bromo-3-(2-methoxy-2-methylpropyl)quinazolin-4(3H)-one**

To a solution of 6-bromo-3-(2-hydroxy-2-methyl-propyl)quinazolin-4-one (300 mg, 1.01 mmol), MeI (716 mg, 5.05 mmol, 314 μL) in ACN (5 mL), $Ag_2O$ (1.17 g, 5.05 mmol) was added. The mixture was stirred at 60°C for 16 hours. Upon completion of stirring, the reaction mixture was dissolved in EA (50 mL) and filtered. The filter liquor was concentrated under reduced pressure to obtain a residue. The residue was purified by flash silica gel chromatography (column: Welch Ultimate XB-CN 250*50*10 μm; mobile phase: [Hexane-EtOH (0.1% $NH_3.H_2O$)] ; concentration gradient: 1%-30% B over 15 min) and Prep-TLC ($SiO_2$, PE/EA = 1/1) to obtain **Intermediate I-31** (85.0 mg, 27% yield) as a white solid.
MS (EI) m/z: $[M+H]^+$ 311.0.
$^1$H NMR (400 MHz, MeOD) δ = 8.24 - 8.22 (m, 1H), 8.13 - 8.11 (m, 1H), 7.84 - 7.80 (m, 1H), 7.51 - 7.47 (m, 1H), 3.26 - 3.16 (m, 2H), 3.14 (s, 3H), 1.10 (s, 6H).

Preparation Example 32: 6-bromo-5-chloro-3-phenylquinazolin-4(3H)-one (Intermediate 1-32)

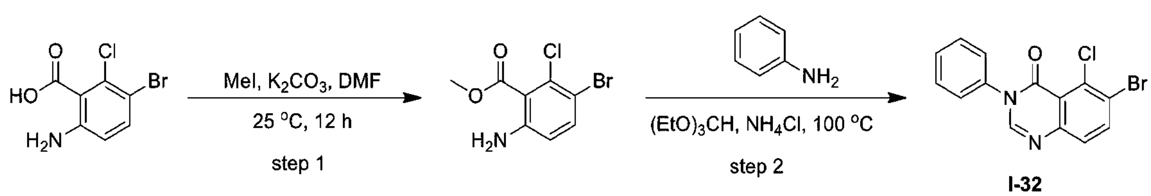


I-32

**Step 1: methyl 6-amino-3-bromo-2-chlorobenzoate**

To a solution of 6-amino-3-bromo-2-chloro-benzoic acid (5 g, 20.0 mmol) in DMF (50 mL), $K_2CO_3$ (3.31 g, 24.0 mmol) and MeI (3.40 g, 24.0 mmol) were added. The mixture was stirred at 25°C for 12 hours. Upon completion of stirring, the reaction mixture was quenched by addition of 20 mL of aqueous ammonium chloride at 25°C, diluted with water (30 mL) and extracted with ethyl acetate (3 x 100 mL). The combined organic layers were washed with brine (3 x 50 mL), dried over anhydrous sodium sulfate, and then filtered and concentrated under reduced pressure to obtain a residue. The residue was purified by column chromatography ($SiO_2$, petroleum ether/ethyl acetate = 20/1 to 10/1) to obtain methyl 6-amino-3-bromo-2-chloro-benzoate (5 g, 95% yield) as a yellow oil.

$^1H$ NMR (400 MHz, $CDCl_3$) $\delta$ = 7.39 (d, $J$ = 8.8 Hz, 1H), 6.50 (d, $J$ = 8.8 Hz, 1H), 4.69 (s, 2H), 3.94 (s, 3H).

**Step 2: 6-bromo-5-chloro-3-phenylquinazolin-4(3H)-one**

To a solution of methyl 6-amino-3-bromo-2-chloro-benzoate (2.0 g, 7.56 mmol) in diethoxymethoxyethane (1.68 g, 11.3 mmol), aniline (845 mg, 9.07 mmol) and $NH_4Cl$ (324 mg, 6.05 mmol) were added. The mixture was stirred at 100°C for 12 hours. Upon completion of stirring, the reaction mixture was diluted with ethyl acetate (30 mL) and carefully poured into 50 mL of water. The suspension was filtered, and the filter cake was washed with 50 mL of MeOH and then dried under reduced pressure to obtain a yellow solid. The solid was triturated with EA (30 mL) to obtain **Intermediate I-32** (900 mg, 35% yield) as a yellow solid.

m/z ES+ $[M+H]^+$ 336.9.

$^1H$ NMR (400 MHz, DMSO-$d_6$) $\delta$ = 8.40 (s, 1H), 8.18 (d, $J$=8.8 Hz, 1H), 7.61 (d, $J$ = 8.8 Hz, 1H), 7.60 - 7.50 (m, 5H).

<u>**Preparation Example 33: 6-bromo-5-chloro-3-(pyridin-3-yl)quinazolin-4(3H)-one (Intermediate 1-33)**</u>

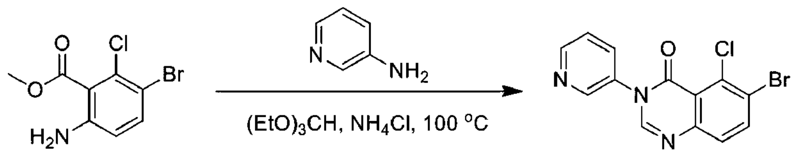


I-33

To a solution of the resulting product (1 g, 3.78 mmol) from **Step 1 of Preparation Example 32** in diethoxymethoxyethane (840 mg, 5.67 mmol), pyridine-3-amine (427 mg, 4.54 mmol) and $NH_4Cl$ (162 mg, 3.02 mmol) were added. The mixture was stirred at 100°C for 12 hours. Upon completion of stirring, the reaction mixture was diluted with ethyl acetate (50 mL) and carefully poured into 100 mL of water. The suspension was filtered and the filter cake was collected to obtain a yellow solid. The residue was purified by Prep-HPLC (column: Welch Ultimate XB-$NH_2$ 250*50*10 $\mu$m; mobile phase: [Hexane-EtOH (0.1% $NH_3.H_2O$) ]; concentration gradient: 1%-35% B over 25 min) to obtain **Intermediate I-33** (230 mg, 18% yield) as a yellow solid.

m/z ES+ $[M+H]^+$ 337.9.

$^1H$ NMR (400 MHz, $CDCl_3$) $\delta$ = 8.75 (d, $J$ = 4.8 Hz, 1H), 8.71 - 8.66 (m, 1H), 8.11 (s, 1H), 8.02 (d, $J$ = 8.8 Hz, 1H), 7.85 (dd, $J$ = 1.6, 8.0 Hz, 1H), 7.57 (d, $J$ = 8.8 Hz, 1H), 7.27 (s, 1H).

<u>**Preparation Example 34: 6-bromo-5-chloro-3-((1-methylpiperidin-4-yl)methyl)quinazolin-4(3H)-one (Intermediate 1-34)**</u>

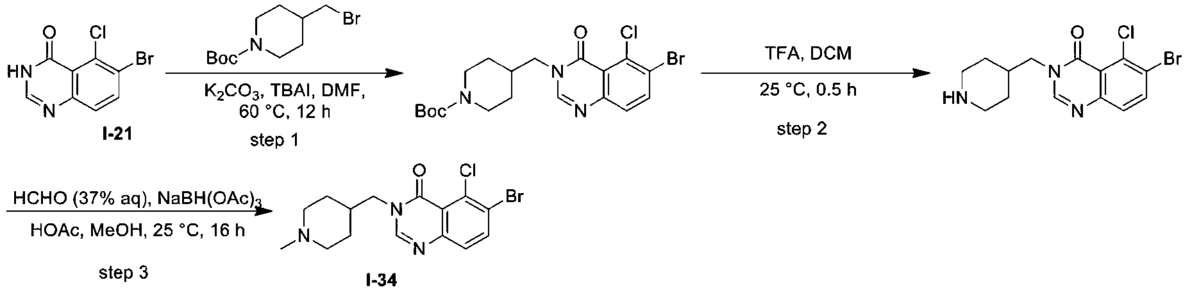


I-21

I-34

**Step 1: tert-butyl 4-((6-bromo-5-chloro-4-oxoquinazolin-3(4H)-yl)methyl)piperidine-1-carboxylate**

To a solution of **Intermediate I-21** (2.0 g, 7.71 mmol) and tert-butyl 4-(bromomethyl)piperidine-1-carboxylate (3.22 g, 11.6 mmol) in DMF (20 mL), $K_2CO_3$ (3.20 g, 23.1 mmol) and TBAI (569 mg, 1.54 mmol) were added. The mixture was stirred at 60°C for 12 hours. Upon completion of stirring, the reaction mixture was quenched by addition of aqueous ammonium chloride (50 mL) at 25°C, and then diluted with water (50 mL) and extracted with EA (3 x 100 mL). The collected organic layers were washed with brine (3 x 50 mL), dried over anhydrous sodium sulfate, and then filtered and concentrated under reduced pressure to obtain a residue. The residue was purified by column chromatography ($SiO_2$, petroleum ether/ethyl acetate = 1/1 to 1/5) to obtain tert-butyl 4-[(6-bromo-5-chloro-4-oxo-quinazolin-3-yl) methyl]piperidine-1-carboxylate (2.83 g, 80% yield) as a white solid.

$^1$H NMR (400 MHz, $CDCl_3$) δ = 7.98 - 7.94 (m, 2H), 7.49 (d, *J* = 8.8 Hz, 1H), 4.21 - 4.06 (m, 2H), 3.90 - 3.77 (m, 2H), 2.67 (t, *J*= 12.0 Hz, 2H), 2.17 - 2.06 (m, 1H), 1.71 - 1.64 (m, 2H), 1.45 (s, 9H), 1.27 - 1.17 (m, 2H).

**Step 2: 6-bromo-5-chloro-3-(piperidin-4-ylmethyl)quinazolin-4(3H)-one**

To a solution of tert-butyl 4-[(6-bromo-5-chloro-4-oxo-quinazolin-3-yl)methyl]piperidine-1-carboxylate (2.8 g, 6.13 mmol) in DCM (30 mL), TFA (14.0 g, 123 mmol) was added, and the mixture was stirred at 25°C for 0.5 hours. Upon completion of stirring, the reaction mixture was concentrated. The residue was basified to pH = 10 with saturated aqueous $K_2CO_3$ solution. The suspension was filtered, and the filter cake was washed with 30 mL of water and dried under reduced pressure to obtain 6-bromo-5-chloro-3-(4-piperidylmethyl)quinazolin-4-one (2 g, 83% yield) as a white solid.

MS (EI) m/z: $[M+H]^+$ 358.0, $^1$H NMR (400 MHz, DMSO-$d_6$) δ = 8.45 (s, 1H), 8.13 (d, *J* = 8.8 Hz, 1H), 7.55 (d, *J* = 8.8 Hz, 1H), 3.84 (d, *J= 7.2* Hz, 2H), 3.07 (d, *J* = 12.0 Hz, 2H), 2.57 (t, *J= 12.0* Hz, 2H), 2.00 - 1.91 (m, 1H), 1.59 (d, *J*= 13.2 Hz, 2H), 1.30 - 1.17 (m, 2H).

**Step 3: 6-bromo-5-chloro-3-((1-methylpiperidin-4-yl)methyl)quinazolin-4(3H)-one**

To a solution of 6-bromo-5-chloro-3-(4-piperidylmethyl)quinazolin-4-one (550 mg, 1.54 mmol) and HCHO (6.26 g, 77.1 mmol, 37% purity) in MeOH (5 mL), HOAc (278 mg, 4.63 mmol) and $NaBH(OAc)_3$ (980 mg, 4.63 mmol) were added. The mixture was stirred at 25°C for 2 hours. Upon completion of stirring, the reaction mixture was quenched by addition of aqueous $NaHCO_3$ (20 mL) at 25°C, and then diluted with water (30 mL) and extracted with DCM/MeOH(3/1) (3 x 50 mL). The combined organic layers were washed with brine (20 mL), dried over anhydrous sodium sulfate, and then filtered and concentrated under reduced pressure to obtain a residue. The residue was purified by column chromatography ($Al_2O_3$, petroleum ether/ethyl acetate = 0/1 to EA/MeOH = 10/1) to obtain **Intermediate I-34** (450 mg, 79% yield) as a white solid.

MS (EI) m/z: $[M+H]^+$ 371.8, $^1$H NMR (400 MHz, DMSO-$d_6$) δ = 8.43 (s, 1H), 8.14 - 8.07 (m, 1H), 7.54 *(d, J=* 8.8 Hz, 1H), 3.83 *(d, J=* 7.2 Hz, 2H), 2.73 *(d, J=* 10.8 Hz, 2H), 2.11 (s, 3H), 1.79 - 1.67 (m, 3H), 1.51 (d, *J* = 11.6 Hz, 2H), 1.31 - 1.17 (m, 2H).

**Preparation Example 35: (R)-N-((S)-4,6-dihydrospiro[cyclopenta[d]thiazole-5,4'-piperidin]-6-yl)-2-methylpropane-2-sulfinamide (Intermediate 1-35)**

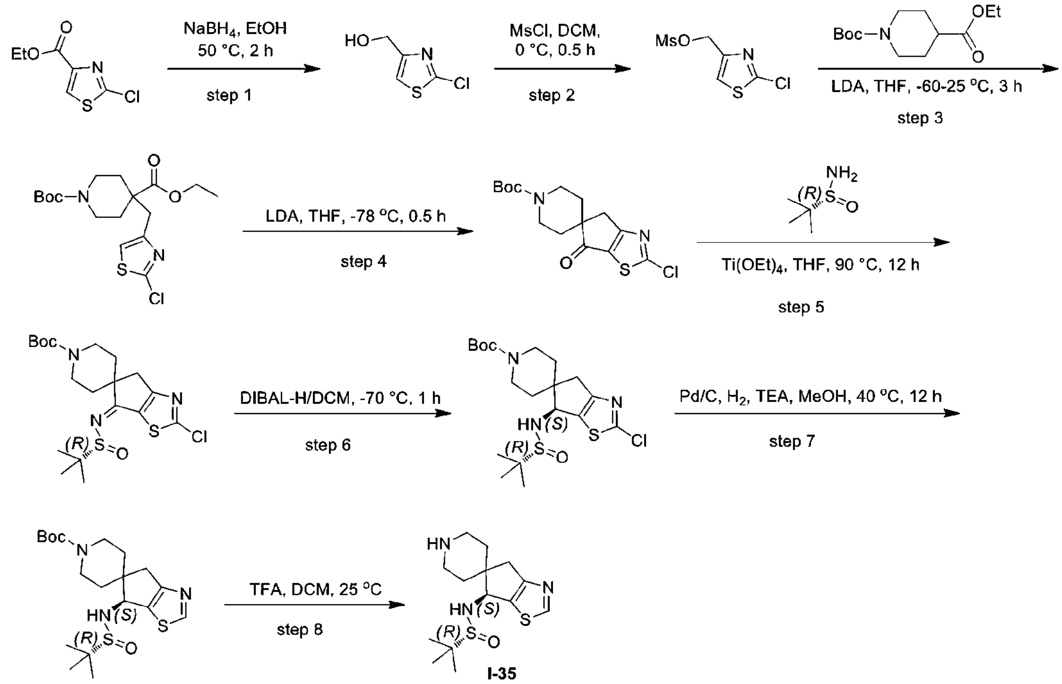


I-35

**Step** 1: **(2-chlorothiazol-4-yl)methanol**

To a solution of ethyl 2-chlorothiazole-4-carboxylate (90 g, 467 mmol) in EtOH (800 mL), $NaBH_4$ (88.8 g, 2.35 mol) was added, and the mixture was stirred at 50°C for 2 hours. Upon completion of stirring, the reaction mixture was quenched by addition of aqueous ammonium chloride (300 mL) at 0°C, and then diluted with water (500 mL) and extracted with ethyl acetate (3 x 800 mL). The collected organic layers were washed with brine (3 x 300 mL), dried over anhydrous sodium sulfate, and then filtered and concentrated under reduced pressure to obtain a residue. The residue was purified by MPLC ($SiO_2$, petroleum ether/ethyl acetate = 5/1 to 3/1) to obtain (2-chlorothiazol-4-yl)methanol (67 g, 95% yield) as a yellow oil.

$^1H$ NMR (400 MHz, $CDCl_3$) $\delta$ = 7.12 (s, 1H), 4.71 (s, 2H), 2.59 - 2.48 (m, 1H).

Step 2: **(2-chlorothiazol-4-yl)methyl methanesulfonate**

To a solution of (2-chlorothiazol-4-yl)methanol (33 g, 221 mmol) in DCM (350 mL), TEA (44.6 g, 441 mmol) and MsCl (35.5 g, 310 mmol) were added at 0°C. The mixture was stirred at 0°C for 0.5 hours. Upon completion of stirring, the reaction mixture was quenched by addition of aqueous $NaHCO_3$ (100 mL) at 25°C, and then diluted with water (100 mL) and extracted with DCM (3 x 200 mL). The combined organic layers were washed with brine (100 mL), dried over anhydrous sodium sulfate, and then filtered and concentrated under reduced pressure to obtain (2-chlorothiazol-4-yl) methyl methanesulfonate (42 g, crude product) as a yellow oil.

$^1H$ NMR (400 MHz, $CDCl_3$) $\delta$ 7.36 (s, 1H), 5.25 (s, 2H), 3.07 (s, 3H).

**Step 3: 1-(tert-butyl) 4-ethyl 4-((2-chlorothiazol-4-yl)methyl)piperidine-1,4-dicarboxylate**

O1-tert-butyl O4-ethyl piperidine-1,4-dicarboxylate (47 g, 183 mmol) was dissolved in THF (500 mL). The solution was cooled to -60°C under nitrogen atmosphere, and then LDA (2 M, 128 mL) was added thereto dropwise. Upon completion of the addition, the reaction solution was stirred at -60°C for 30 minutes. A solution of (2-chlorothiazol-4-yl) methyl methanesulfonate (41.6 g, 183 mmol) in THF (150 mL) was added dropwise. Upon completion of the addition, the reaction solution was stirred at -60°C for 30 minutes, then slowly warmed to 25°C and stirred for 2 hours. Upon completion of stirring, the reaction mixture was quenched by addition of aqueous ammonium chloride (100 mL) at 25°C, and then diluted with water (200 mL) and extracted with EA (3 x 300 mL). The combined organic layers were washed with brine (150 mL), dried over anhydrous sodium sulfate, and then filtered and concentrated under reduced pressure to obtain a residue. The residue was purified by MPLC ($SiO_2$, petroleum ether/ethyl acetate = 15/1 to 10/1) to obtain O1-tert-butyl O4-ethyl 4-[(2-chlorothiazol-4-yl)methyl]piperidine-1,4-dicarboxylate (31 g, 44% yield) as a yellow oil.

MS (EI) m/z: $[M-Boc+H]^+$ 289.3, $^1H$ NMR (400 MHz, $CDCl_3$) $\delta$ = 6.82 - 6.79 (m, 1H), 4.19 - 4.13 (m, 2H), 3.97 - 3.82 (m, 2H), 2.97 - 2.84 (m, 4H), 2.11 (d, $J$= 13.2 Hz, 2H), 1.51 (d, $J$ = 4.0 Hz, 2H), 1.45 (s, 9H), 1.26 - 1.22 (m, 3H).

Step 4: **tert-butyl 2-chloro-6-oxo-4,6-dihydrospiro[cyclopenta[d]thiazole-5,4'-piperidine]-1'-carboxylate**

To a solution of O1-tert-butyl O4-ethyl 4-[(2-chlorothiazol-4-yl)methyl]piperidine-1,4-dicarboxylate (62 g, 159 mmol) in THF (1100 mL), LDA (2 M, 199 mL) was added, and the mixture was stirred at -70°C for 0.5 hours. Upon completion of stirring, the reaction mixture was quenched by addition of aqueous ammonium chloride (100 mL) at 0°C, and then diluted with water (200 mL) and extracted with EA (3 x 300 mL). The combined organic layers were washed with brine (200 mL), dried over anhydrous sodium sulfate, and then filtered and concentrated under reduced pressure to obtain a residue. The residue was purified by MPLC ($SiO_2$, petroleum ether/ethyl acetate = 15/1 to 10/1) to obtain tert-butyl 2-chloro-6-oxo-spiro[4H-cyclopenta[d]thiazole-5,4'-piperidine]-1'-carboxylate (22.5 g, 41% yield) as a yellow solid.

$^1H$ NMR (400 MHz, $CDCl_3$) δ = 4.25 - 4.10 (m, 2H), 3.06 (s, 2H), 2.98-2.95 (m, 2H), 2.02 - 1.93 (m, 2H), 1.46-1.40 (m, 11H).

**Step 5: tert-butyl (R,Z)-6-((tert-butylsulfinyl)imino)-2-chloro-4,6-dihydrospiro[cyclopenta[d]thiazole-5,4'-piperidine]-1'-carboxylate**

To a solution of tert-butyl 2-chloro-6-oxo-spiro[4H-cyclopenta[d]thiazole-5,4'-piperidine]-1'-carboxylate (33 g, 96.3 mmol) in THF (100 mL), $Ti(OEt)_4$ (329 g, 1.44 mol) and (R)-2-methylpropane-2-sulfinamide (58.3 g, 481 mmol) were added. The mixture was stirred at 90°C for 12 hours. Upon completion of stirring, the reaction mixture was diluted with EA (1000 mL) and quenched by addition of water (500 mL). The combined organic layers were washed with brine (3 x 500 mL), dried over anhydrous sodium sulfate, and then filtered and concentrated under reduced pressure to obtain a residue. The residue was purified by MPLC ($SiO_2$, petroleum ether/ethyl acetate = 10/1 to 5/1) to obtain tert-butyl (6Z)-6-[(R)-tert-butylsulfinyl]imino-2-chloro-spiro[4H-cyclopenta[d]thiazole-5,4'-piperidine]-1'-carboxylate (35 g, 82% yield) as a yellow solid.

$^1H$ NMR (400 MHz, $CDCl_3$) δ = 4.27 - 4.12 (m, 2H), 2.99 - 2.85 (m, 4H), 2.06 - 1.88 (m, 2H), 1.58 - 1.51 (m, 2H), 1.49 (s, 9H), 1.28 (s, 9H).

**Step 6: tert-butyl (S)-6-(((R)-tert-butylsulfinyl)amino)-2-chloro-4,6-dihydrospiro[cyclopenta[d]thiazole-5,4'-piperidine]-1'-carboxylate**

To a solution of tert-butyl (6Z)-6-[(R)-tert-butylsulfinyl]imino-2-chloro-spiro[4H-cyclopenta[d]thiazole-5,4'-piperidine]-1'-carboxylate (34 g, 76.2 mmol) in DCM (500 mL), DIBAL-H (1 M, 229 mL) was added, and the mixture was stirred at -70°C for 1 hour. Upon completion of stirring, the reaction mixture was quenched by addition of MeOH (10 mL) at -70°C, and then diluted with DCM (1000 mL). The mixture was filtered, and the filtrate was concentrated under reduced pressure to obtain a residue. The residue was purified by column chromatography ($SiO_2$, petroleum ether/ethyl acetate = 3/1 to 2/1) to obtain tert-butyl (6S)-6-[[(R)-tert-butylsulfinyl]amino]-2-chloro-spiro[4,6-dihydrocyclopenta[d]thiazole-5,4'-piperidine]-1'-carboxylate (28 g, 82% yield) as a yellow solid.

MS (EI) m/z: $[M+H]^+$ 448.2, $^1H$ NMR (400 MHz, $CDCl_3$) δ = 4.52 (d, *J* = 8.8 Hz, 1H), 4.13 - 3.93 (m, 2H), 3.59 (d, *J*= 7.6 Hz, 1H), 3.07 - 2.74 (m, 4H), 1.90 - 1.77 (m, 2H), 1.67 - 1.54 (m, 2H), 1.46 (s, 9H), 1.22 (s, 9H)

**Step 7: tert-butyl (S)-6-(((R)-tert-butylsulfinyl)amino)-4,6-dihydrospiro[cyclopenta[d]thiazole-5,4'-piperidine]-1'-carboxylate**

To a solution of tert-butyl (6S)-6-[[(R)-tert-butylsulfinyl]amino]-2-chloro-spiro[4,6-dihydrocyclopenta[d]thiazole-5,4'-piperidine]-1'-carboxylate (12 g, 26.9 mmol) in MeOH (120 mL), Pd/C (6.00 g, 5.65 mmol, 10% purity) and TEA (8.13 g, 80.4 mmol) were added. The mixture was stirred under $H_2$ (50 psi) atmosphere at 40°C for 12 hours. Upon completion of stirring, the reaction mixture was filtered, and the filtrate was concentrated under reduced pressure to obtain tert-butyl (6S)-6-[[(R)-tert-butylsulfinyl]amino]spiro[4,6-dihydrocyclopenta[d]thiazole-5,4'-piperidine]-1'-carboxylate (12 g, crude product) as a yellow solid.

MS (EI) m/z: $[M+H]^+$ 414.2, $^1H$ NMR (400 MHz, $CDCl_3$) δ = 8.78 (s, 1H), 4.56 (d, *J* = 8.8 Hz, 1H), 4.12 - 3.94 (m, 2H), 3.64 - 3.54 (m, 1H), 3.05 - 2.93 (m, 2H), 2.87 (s, 2H), 1.86 - 1.78 (m, 2H), 1.61 (t, *J*= 13.6 Hz, 2H), 1.47 - 1.45 (s, 9H), 1.22 (s, 9H).

**Step 8: (R)-N-((S)-4,6-dihydrospiro[cyclopenta[d]thiazole-5,4'-piperidin]-6-yl)-2-methylpropane-2-sulfinamide**

To a solution of tert-butyl (6S)-6-[[(R)-tert-butylsulfinyl]amino]spiro[4,6-dihydrocyclopenta[d]thiazole-5,4'-piperidine]-1'-carboxylate (8 g, 19.3 mmol) in DCM (100 mL), TFA (17.6 g, 155 mmol) was added, and the mixture was stirred at 25°C for 1 hour. Upon completion of stirring, the reaction mixture was quenched by addition of aqueous $K_2CO_3$ (50 mL) at 25°C, and then diluted with water (100 mL) and extracted with DCM (3 x 150 mL). The combined organic layers were

washed with brine (200 mL), dried over anhydrous sodium sulfate, and then filtered and concentrated under reduced pressure to obtain **Intermediate I-35** (3.5 g, crude product) as a yellow solid.

$^1$H NMR (400 MHz, $CDCl_3$) δ = 8.80 - 8.75 (m, 1H), 4.60 (d, *J*= 8.8 Hz, 1H), 3.83 - 3.76 (m, 1H), 3.26 - 3.13 (m, 2H), 2.93 - 2.93 (m, 1H), 2.96 - 2.89 (m, 1H), 2.89 - 2.82 (m, 2H), 2.01 - 1.94 (m, 2H), 1.74 (dd, *J* = 2.0, 12.4 Hz, 1H), 1.67 - 1.60 (m, 1H), 1.25 - 1.22 (s, 9H).

**Preparation Example 36: sodium** 5-**((S)-4-(((R)-tert-butylsulfinyl)amino)-4.6-dihydrospiro[cyclopenta[d]thiazole-5,4'-piperidin]-1'-yl)pyrazine-2-thiolate (Intermediate I-36)**

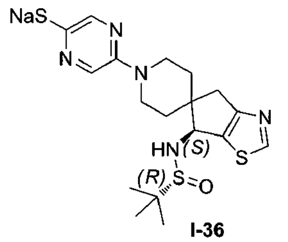

**I-36**

**Intermediate I-36** was prepared in the same method as in Preparation Example 26, except that **Intermediate I-35** was used instead of **Intermediate I-25** in Preparation Example 26.
**MS (EI) m/z: [M-Na]$^+$ 424.2**

**Preparation Example 37: (R)-2-methyl-N-[(4S)-spiro[4,6-dihydrocyclopenta[d]thiazole-5,4'-piperidin]-4-yl]propane-2-sulfinamide (Intermediate 1-37)**

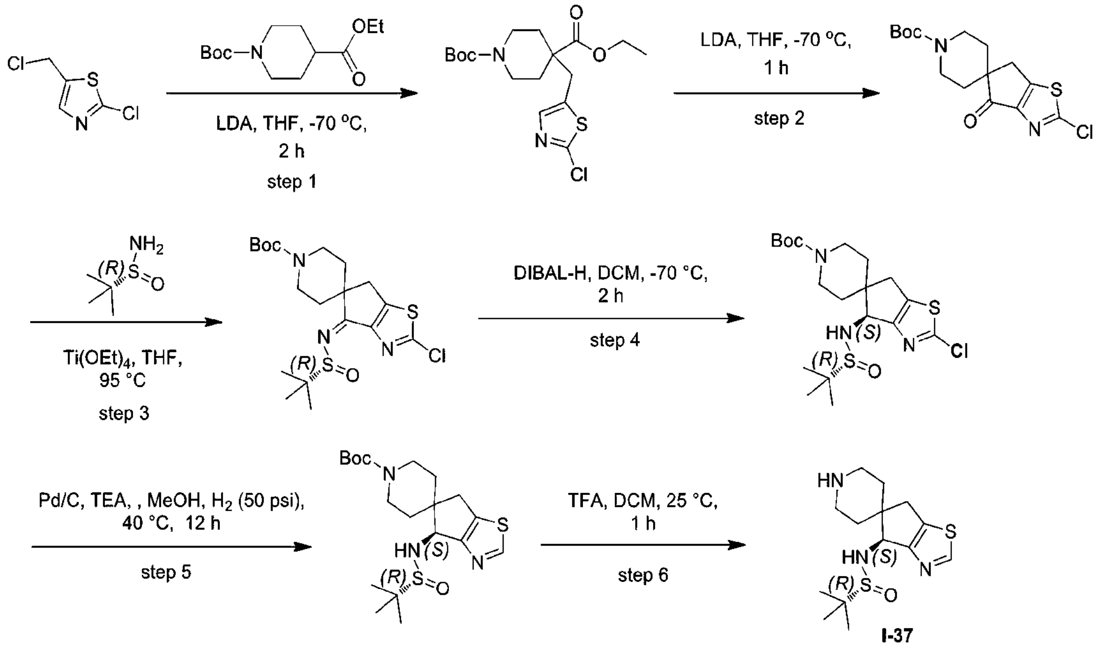


**I-37**

**Step 1: 1-(tert-butyl) 4-ethyl 4-((2-chlorothiazol-5-yl)methyl)piperidine-1,4-dicarboxylate**

To a mixture of O1-tert-butyl O4-ethyl piperidine-1,4-dicarboxylate (30.3 g, 118 mmol) in THF (200 mL), LDA (2 M, 64.3 mL) was added at -70°C. The mixture was stirred at -70°C for 1 hour. Thereafter, a solution of 2-chloro-5-(chloromethyl)thiazole (18 g, 107 mmol) in THF (40 mL) was added dropwise to the system. The reaction mixture was stirred at -70°C for 1 hour. Upon completion of stirring, the reaction mixture was poured into saturated $NH_4Cl$ (300 mL) solution, and then extracted with EA (3 x 200 mL). The organic layers were washed with brine (2 X 200 mL). The organic layers were

dried over anhydrous sodium sulfate and filtered. The filtrate was concentrated under reduced pressure. The residue was purified by silica gel chromatography (PE/EtOAc = 10/1) to obtain O1-tert-butyl O4-ethyl 4-[(2-chlorothiazol-5-yl)methyl] piperidine-1,4-dicarboxylate (25 g, 60% yield) as a yellow oil.

$^1$HNMR (400 MHz, $CDCl_3$) δ = 7.22 (s, 1H), 4.18 (q, *J*= 7.2 Hz, 2H), 3.97 - 3.77 (m, 2H), 3.00 (s, 2H), 2.95 (s, 2H), 2.11 (d, *J*= 13.2 Hz, 2H), 1.46 (s, 9H), 1.41 (d, *J*= *4.4* Hz, 2H), 1.26 (t, *J* = 7.2 Hz, 3H).

**Step 2: tert-butyl 2-chloro-4-oxo-4,6-dihydrospiro[cyclopenta[d]thiazole-5,4'-piperidine]-1'-carboxylate**

To a mixture of O1-tert-butyl O4-ethyl 4-[(2-chlorothiazol-5-yl)methyl]piperidine-1,4-dicarboxylate (15 g, 38.6 mmol) in THF (350 mL), LDA (2 M, 30.9 mL) was added at -70°C. The mixture was stirred at -70°C for 1 hour. Upon completion of stirring, the reaction mixture was poured into saturated $NH_4Cl$ (500 mL) solution at 0°C, and then extracted with EA (3 x 100 mL). The organic layers were washed with brine (2 X 200 mL). The organic layers were dried over anhydrous sodium sulfate and filtered. The filtrate was concentrated under reduced pressure. The residue was purified by reverse phase HPLC (0.1% $NH_3 \cdot H_2O$) to obtain tert-butyl 2-chloro-4-oxo-spiro[6H-cyclopenta[d]thiazole-5,4'-piperidine]-1'-carboxylate (4.0 g, 30% yield) as a yellow solid.

$^1$HNMR (400 MHz, $CDCl_3$) δ = 4.26 - 4.03 (m, 2H), 3.11 (s, 2H), 2.99 (t, *J* = 12.0 Hz, 2H), 2.03 - 1.92 (m, 2H), 1.55-1.51 (m, 1H), 1.48 (s, 9H), 1.47 - 1.44 (m, 1H).

**Step 3: tert-butyl (R,Z)-4-((tert-butylsulfinyl)imino)-2-chloro-4,6-dihydrospiro[cyclopenta[d]thiazole-5,4'-piperidine]-1'-carboxylate**

To a solution of tert-butyl 2-chloro-4-oxo-spiro[6H-cyclopenta[d]thiazole-5,4'-piperidine]-1'-carboxylate (4 g, 11.7 mmol) in THF (40 mL), (R)-2-methylpropane-2-sulfinamide (5.66 g, 46.7 mmol) and $Ti(OEt)_4$ (39.9 g, 175 mmol) were added, and the mixture was stirred at 95°C for 16 hours. Upon completion of stirring, the reaction mixture was diluted with EtOAc (150 mL) and quenched by addition of 200 ml of water. The mixture was filtered and extracted with EtOAc (2 x 100 mL). The combined organic layers were dried over $Na_2SO_4$, filtered and concentrated under reduced pressure to obtain a residue. The residue was purified by column chromatography ($SiO_2$, PE/EtOAc = 4/1) to obtain tert-butyl (4Z)-4-[(R)-tert-butylsulfinyl]imino-2-chloro-spiro[6H-cyclopenta[d]thiazole-5,4'-piperidine]-1'-carboxylate (4.3 g, 76% yield) as a light yellow solid.

MS (EI) m/z: $[M+H]^+$ 446.2, $^1$H NMR (400 MHz, $CDCl_3$) δ= 4.17 (d, *J* = 12.0 Hz, 2H), 3.12 - 3.06 (m, 2H), 2.97 - 2.86 (m, 2H), 2.17-2.11 (m,1H), 2.07 - 1.93 (m, 2H), 1.62 - 1.56 (m, 1H), 1.49 (s, 9H), 1.28 (s, 9H).

**Step 4: tert-butyl (S)-4-(((R)-tert-butylsulfinyl)amino)-2-chloro-4,6-dihydrospiro[cyclopenta[d]thiazole-5,4'-piperidine]-1'-carboxylate**

To a solution of tert-butyl (4Z)-4-[(R)-tert-butylsulfinyl]imino-2-chloro-spiro[6H-cyclopenta[d]thiazole-5,4'-piperidine]-1'-carboxylate (2.0 g, 4.48 mmol) in DCM (30 mL), DIBAL-H (1 M, 13.5 mL) was added, and the mixture was stirred at -70°C for 2 hours. Upon completion of stirring, the reaction mixture was quenched by addition of MeOH (2 mL) at 0°C, and then diluted with DCM (100 mL). The mixture was filtered, and the filtrate was concentrated under reduced pressure to obtain a residue. The residue was purified by column chromatography ($SiO_2$, petroleum ether/ethyl acetate = 1/1 to 1/3) to obtain tert-butyl (4S)-4-[[(R)-tert-butylsulfinyl]amino]-2-chloro-spiro[4,6-dihydrocyclopenta[d]thiazole-5,4'-piperidine]-1'-carboxylate (1.25 g, 62% yield) as a yellow solid.

$^1$H NMR (400 MHz, $CDCl_3$) δ = 4.40 (d, *J*= 9.2 Hz, 1H), 4.05 - 3.87 (m, 2H), 3.09 - 3.01 (m, 2H), 2.89 - 2.73 (m, 2H), 1.97 (t, *J* = 9.6 Hz, 1H), 1.91 - 1.83 (m, 1H), 1.65 - 1.61 (m, 1H), 1.59 - 1.54 (m, 1H), 1.46 (s, 9H), 1.26 (s, 9H).

**Step 5: tert-butyl (S)-4-(((R)-tert-butylsulfinyl)amino)-4,6-dihydrospiro[cyclopenta[d]thiazole-5,4'-piperidine]-1'-carboxylate**

To a solution of tert-butyl (4S)-4-[[(R)-tert-butylsulfinyl]amino]-2-chloro-spiro[4,6-dihydrocyclopenta[d]thiazole-5,4'-piperidine]-1'-carboxylate (1.2 g, 2.68 mmol) in MeOH (20 mL), Pd/C (853 mg, 804 μmol) and TEA (813 mg, 8.03 mmol) were added, and the mixture was stirred under $H_2$ (50 psi) atmosphere at 40°C for 12 hours. Upon completion of stirring, the reaction mixture was filtered, and the filtrate was concentrated under reduced pressure to obtain a residue. The residue was purified by reverse phase flash chromatography (0.1% FA) to obtain tert-butyl (4S)-4-[[(R)-tert-butylsulfinyl]amino]spiro[4,6-dihydrocyclopenta[d]thiazole-5,4'-piperidine]-1'-carboxylate (0.88 g, 79% yield) as a yellow solid.

$^1$H NMR (400 MHz, $CDCl_3$) δ = 8.72 (s, 1H), 4.47 (d, *J* = 9.0 Hz, 1H), 4.03 - 3.87 (m, 2H), 3.69 - 3.52 (m, 1H), 3.14 - 3.00 (m, 2H), 2.93 - 2.77 (m, 2H), 2.03 - 1.94 (m, 1H), 1.92 - 1.80 (m, 1H), 1.67 - 1.54 (m, 2H), 1.46 (s, 9H), 1.26 (s, 9H).

**Step 6: (R)-N-((S)-4,6-dihydrospiro[cyclopenta[d]thiazole-5,4'-piperidin]-4-yl)-2-methylpropane-2-sulfinamide**

To a solution of tert-butyl (4S)-4-[[(R)-tert-butylsulfinyl]amino]spiro[4,6-dihydrocyclopenta[d]thiazole-5,4'-piperidine]-1'-carboxylate (350 mg, 846 μmol) in DCM (6 mL), TFA (2.89 g, 25.4 mmol) was added, and the mixture was stirred at 25°C for 1 hour. Upon completion of stirring, the reaction mixture was concentrated under reduced pressure to obtain a residue. Thereafter, the residue was quenched by addition of aqueous $K_2CO_3$ (2 mL) solution at 25°C, diluted with water (5 mL) and extracted with DCM (3 x 10 mL). The combined organic layers were washed brine (3 x 20 mL), dried over anhydrous sodium sulfate, and then filtered and concentrated under reduced pressure to obtain **Intermediate I-37** (260 mg, crude product) as a yellow solid.

$^1$H NMR (400 MHz, $CDCl_3$) δ = 8.74 (s, 1H), 4.49 (d, *J* = 9.2 Hz, 1H), 3.84 (d, *J* = 8.8 Hz, 1H), 3.20 - 3.11 (m, 2H), 3.01 - 2.96 (m, 1H), 2.94 - 2.88 (m, 2H), 2.85 - 2.80 (m, 1H), 2.04 - 1.90 (m, 2H), 1.71 (d, *J*= 13.2 Hz, 1H), 1.57 (d, *J* = 14.0 Hz, 1H), 1.28 (s, 9H).

**Preparation Example 38: sodium 5-((S)-4-(((R)-tert-butylsulfinyl)amino)-4,6-dihydrospiro[cyclopenta[d]thiazole-5,4'-piperidin]-1'-yl)pyrazine-2-thiolate (Intermediate I-38)**

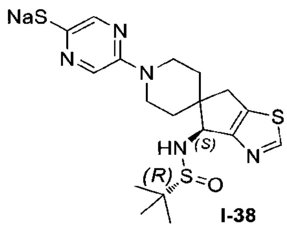

I-38

**Intermediate I-38** was obtained in the same method as in Preparation Example 26, except that **Intermediate I-37** was used instead of **Intermediate I-25** in Preparation Example 26.
MS (EI) m/z: $[M-Na]^+$ 424.5

**Preparation Example 39: sodium 3-((S)-1-(((R)-tert-butylsulfinyl)amino)-1,3-dihydrospiro[indene-2,4'-piperidin [-1'-yl)-1,2,4-triazin-6-thiolate (Intermediate 1-39)**

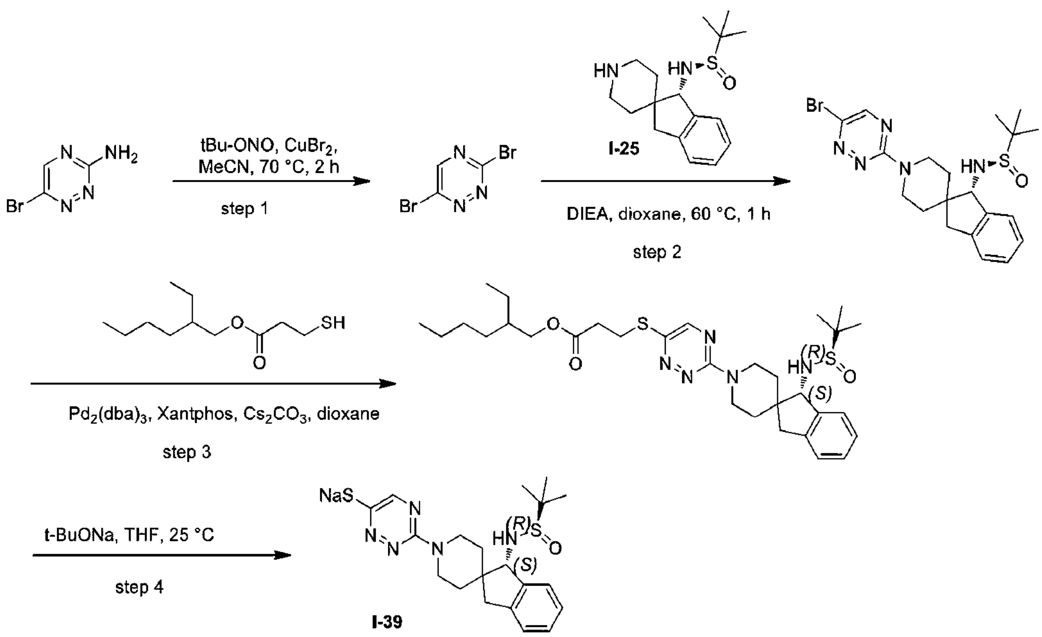


I-39

**Step 1: 3,6-dibromo-1,2,4-triazin**

To a solution of 6-bromo-1,2,4-triazin-3-amine (2 g, 11.4 mmol) in MeCN (20 mL), tert-butyl nitrite (1.89 g, 18.3 mmol) and $CuBr_2$ (3.32 g, 14.9 mmol) were added. The mixture was stirred at 70°C for 2 hours. Upon completion of stirring, the reaction mixture was concentrated under reduced pressure to obtain a residue. The residue was purified by column chromatography ($SiO_2$, petroleum ether/ethyl acetate=20/1 to 10/1) to obtain 3,6-dibromo-1,2,4-triazin (1.25 g, 46% yield) as a yellow solid.
$^1$H NMR (400 MHz, $CDCl_3$) $\delta$ = 8.55 (s, 1H)

**Step 2: (R)-N-((S)-1'-(6-bromo-1,2,4-triazin-3-yl)-1,3-dihydrospiro[indene-2,4'-piperidin]-1-yl)-2-methylpropane-2-sulfinamide**

To a solution of **Intermediate I-25** (500 mg, 1.19 mmol) in dioxane (8 mL), DIEA (1.54 g, 11.9 mmol) and 3,6-dibromo-1,2,4-triazin (284 mg, 1.19 mmol) were added. The mixture was stirred at 60°C for 1 hour. Upon completion of stirring, the reaction mixture was concentrated under reduced pressure to obtain a residue. The residue was purified by column chromatography ($SiO_2$, petroleum ether/ethyl acetate=3/1 to 2/1) to obtain (R)-N-[(1S)-1'-(6-bromo-1,2,4-triazin-3-yl)spiro[indane-2,4'-piperidin]-1-yl]-2-methyl-propane-2-sulfinamide (510 mg, 92% yield) as a yellow solid.

m/z ES+ $[M+H]^+$ 466.0
$^1$H NMR (400 MHz, $CDCl_3$) $\delta$ = 8.12 (s, 1H), 7.34 - 7.29 (m, 1H), 7.27 - 7.21 (m, 3H), 4.78 - 4.65 (m, 2H), 4.57 (d, *J*= 10.4 Hz, 1H), 3.58 (d, *J*= 10.4 Hz, 1H), 3.27 - 3.18 (m, 2H), 3.16 - 2.95 (m, 1H), 2.81 (d, *J* = 15.6 Hz, 1H), 2.31 (dt, *J* = 4.4, 12.4 Hz, 1H), 1.84 (dt, *J* = 4.4, 12.4 Hz, 1H), 1.70 (dd, *J* = 2.0, 13.6 Hz, 1H), 1.38 (dd, *J* = 2.0, 13.6 Hz, 1H), 1.30 (s, 9H)

**Step 3 and Step 4: sodium 3-((S)-1-(((R)-tert-butylsulfinyl)amino)-1,3-dihydrospiro [indene-2,4'-piperidin]-1'-yl)-1,2,4-triazin-6-thiolate**

**Intermediate I-39** (170 mg, 80%) was obtained as a yellow solid in the same method as Step 2 and Step 3 of Preparation Example 26, using (R)-N-((S)-1'-(6-bromo-1,2,4-triazin-3-yl)-1,3-dihydrospiro[indene-2,4'-piperidin]-1-yl)-2-methylpropane-2-sulfinamide as a starting material.
MS (EI) m/z: $[M+H]^+$ 418.1

**Preparation Example 40: N-((S)-1'-(3-(hydroxymethyl)-5-mercapto-6-methylpyrazin-2-yl)-1,3-dihydrospiro[indene-2,4'-piperidin]-1-yl)-2-methylpropane-2-sulfinamide (Intermediate 1-40)**

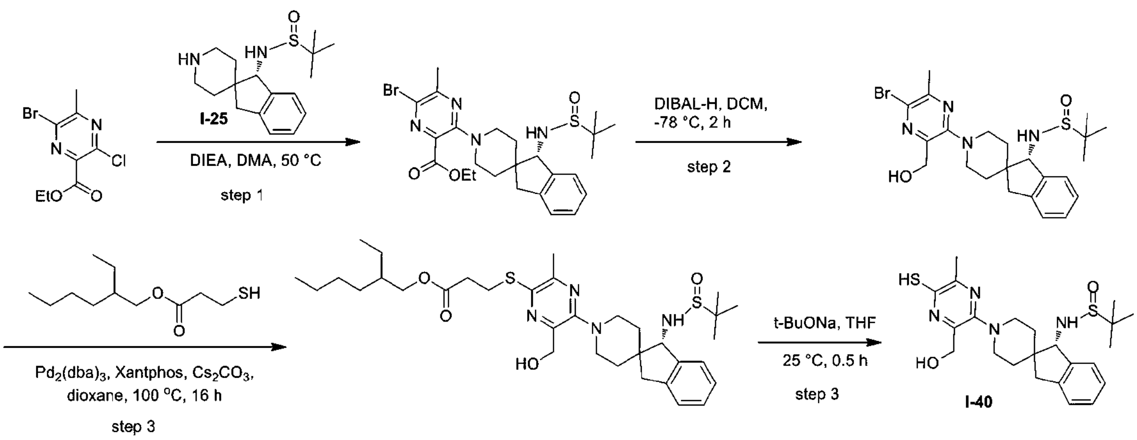


**Step 1: ethyl 6-bromo-3-((1S)-1-((tert-butylsulfinyl)amino)-1,3-dihydrospiro[indene-2,4'-piperidin]-1'-yl)-5-methylpyrazine-2-carboxylate**

To a solution of ethyl 6-bromo-3-chloro-5-methyl-pyrazine-2-carboxylate (0.3 g, 1.07 mmol) in DMA (6 mL), DIEA (694 mg, 5.37 mmol) and **Intermediate I-25** (361 mg, 859 $\mu$mol) were added. The mixture was stirred at 60°C for 4 hours. Upon completion of stirring, the reaction mixture was quenched by addition of aqueous ammonium chloride at 25°C, and then diluted with water (30 mL) and extracted with EA (3 x 50 mL). The combined organic layers were washed with brine (3 x

20 mL), dried over anhydrous sodium sulfate, and then filtered and concentrated under reduced pressure to obtain a residue. The residue was purified by column chromatography ($SiO_2$, petroleum ether/ethyl acetate = 3/1 to 2/1) to obtain ethyl 6-bromo-3-[(1S)-1-(tert-butylsulfinylamino)spiro[indane-2,4'-piperidin]-1'-yl]-5-methyl-pyrazine-2-carboxylate (450 mg, 76% yield) as a yellow solid.

MS (EI) m/z: $[M+H]^+$ 551.1.

$^1H$ NMR (400 MHz, $CDCl_3$) δ = 7.32 (d, *J* = 7.2 Hz, 1H), 7.29 - 7.26 (m, 1H), 7.25 - 7.21 (m, 2H), 4.53 (d, *J* = 9.6 Hz, 1H), 4.40 (q, *J* = 7.2 Hz, 2H), 3.94 (d, *J* = 13.2 Hz, 1H), 3.85 (d, *J* = 13.2 Hz, 1H), 3.54 (d, *J* = 9.6 Hz, 1H), 3.31 - 3.20 (m, 2H), 3.15 (d, *J* = 16.0 Hz, 1H), 2.80 (d, *J* = 16.0 Hz, 1H), 2.53 (s, 3H), 2.20 (dt, *J= 4.4,* 12.4 Hz, 1H), 1.86 (dt, *J* = 4.4, 12.4 Hz, 1H), 1.65 - 1.59 (m, 1H), 1.43 - 1.35 (m, 4H), 1.28 (s, 9H).

**Step 2: N-((S)-1'-(5-bromo-3-(hydroxymethyl)-6-methylpyrazin-2-yl)-1,3-dihydrospiro [indene-2,4'-piperidin]-1-yl)-2-methylpropane-2-sulfinamide**

To a solution of ethyl 6-bromo-3-[(1S)-1-(tert-butylsulfinylamino)spiro[indane-2,4'-piperidin]-1'-yl]-5-methyl-pyrazine-2-carboxylate (600 mg, 1.09 mmol) in DCM (15 mL), DIBAL-H (1 M, 4.37 mL) was added, and the mixture was stirred at -65°C for 3 hours. Upon completion of stirring, the reaction mixture was quenched by addition of MeOH (1 mL) at -60°C or below, and then extracted with EA (50 mL). The suspension was warmed to room temperature. Thereafter, the suspension was filtered through a celite pad and the filter cake was washed with EA (3 x 30 mL). The combined filtrate was concentrated and dried to obtain a residue. The residue was purified by column chromatography ($SiO_2$, petroleum ether/ethyl acetate = 2/1 to 1/1) to obtain N-[(1S)-1'-[5-bromo-3-(hydroxymethyl)-6-methyl-pyrazin-2-yl]spiro[indane-2,4'-piperidin]-1-yl]-2-methyl-propane-2-sulfinamide (410 mg, 74% yield) as a yellow solid.

MS (EI) m/z: $[M+H]^+$ 507.1
$^1H$ NMR (400 MHz, $CDCl_3$) δ = 7.33 (d, *J* = 7.6 Hz, 1H), 7.27 - 7.20 (m, 3H), 4.67 (s, 2H), 4.57 (d, *J*= 10.0 Hz, 1H), 3.59 (d, *J*= 10.0 Hz, 1H), 3.57 - 3.51 (m, 1H), 3.50 - 3.43 (m, 2H), 3.16 - 2.99 (m, 3H), 2.75 (d, *J*= 15.8 Hz, 1H), 2.55 (s, 3H), 2.36 (dt, *J* = 4.0, 12.4 Hz, 1H), 1.99 (dt, *J* = 4.0, 12.4 Hz, 1H), 1.66 (dd, *J* = 2.4, 13.2 Hz, 1H), 1.40 (dd, *J* = 2.4, 13.2 Hz, 1H), 1.30 (s, 9H).

**Step 3 and Step 4: N-((S)-1'-(3-(hydroxymethyl)-5-mercapto-6-methylpyrazin-2-yl)-1,3-dihydrospiro[indene-2,4'-piperidin]-1-yl)-2-methylpropane-2-sulfinamide**

**Intermediate I-40** (200 mg, 70%) was obtained as a yellow solid in the same method as in Step 2 and Step 3 of Preparation Example 26, using N-((S)-1'-(5-bromo-3-(hydroxymethyl)-6-methylpyrazin-2-yl)-1,3-dihydrospiro[indene-2,4'-piperidin]-1-yl)-2-methylpropane-2-sulfinamide as a starting material.
MS (EI) m/z: $[M+H]^+$ 461.1

**<u>Preparation Example 41: 6-((3-amino-5-chloropyrazin-2-yl)thio)-5-chloro-3-(2-methoxy-2-methylpropyl)quinazolin-4(3H)-one (Intermediate I-41)</u>**

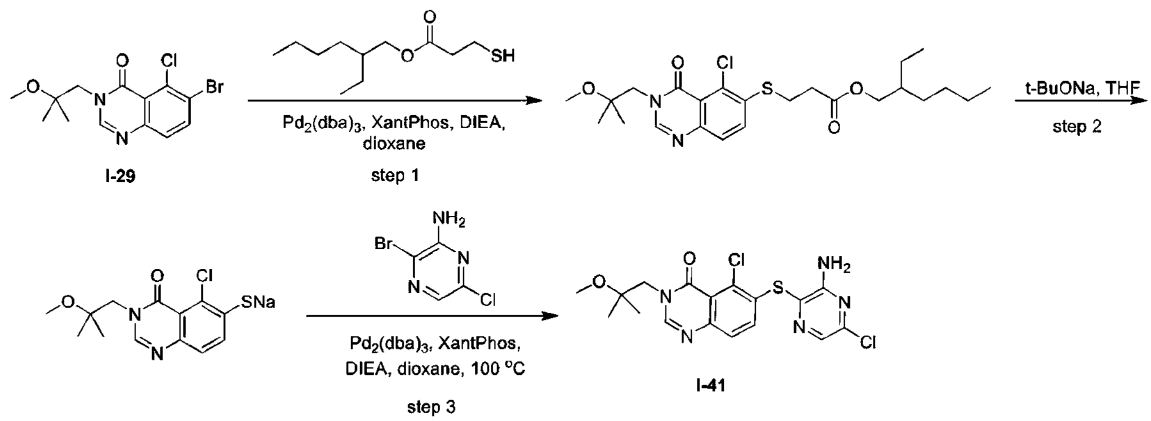

**Step 1 and Step 2: sodium 5-chloro-3-(2-methoxy-2-methylpropyl)-4-oxo-3,4-dihydroquinazolin-6-thiolate**

Sodium 5-chloro-3-(2-methoxy-2-methylpropyl)-4-oxo-3,4-dihydroquinazolin-6-thiolate (30mg, 50%) was obtained as a yellow solid in the same method as in Step 2 and Step 3 of Preparation Example 26, using **Intermediate I-29** as a starting material.
MS (EI) m/z: $[M+H]^+$ 299.1

**Step 3: 6-((3-amino-5-chloropyrazin-2-yl)thio)-5-chloro-3-(2-methoxy-2-methylpropyl)quinazolin-4(3H)-one**

A mixture of [3-(2-methoxy-2-methyl-propyl)-4-oxo-quinazolin-6-yl]sulfanyl sodium (330 mg, 1.15 mmol), 3-bromo-6-chloro-pyrazine-2-amine (216 mg, 1.04 mmol), $Pd_2(dba)_3$ (106 mg, 115 μmol), Xantphos (133 mg, 231 μmol) and DIEA (447 mg, 3.46 mmol) in dioxane (10 mL) was degassed and purged 3 times with $N_2$, and then the mixture was stirred under $N_2$ atmosphere at 100°C for 1 hour. Upon completion of stirring, the reaction mixture was filtered, and the filtrate was concentrated under reduced pressure to obtain a residue. The residue was purified by Prep-HPLC (column: Welch Ultimate XB-CN 250*50*10 μm; mobile phase: [Hexane-EtOH (0.1% $NH_3.H_2O$) ]; concentration gradient: 1%-40% B over 15 min) to obtain **Intermediate I-41** (150 mg, 31% yield) as a yellow solid.

MS (EI) m/z: $[M+H]^+$ 426.0
$^1$H NMR (400 MHz, $CDCl_3$) δ = 8.20 (s, 1H), 7.92 (s, 1H), 7.55 - 7.51 (m, 1H), 7.48 - 7.43 (m, 1H), 5.21 (s, 2H), 4.07 (s, 2H), 3.21 (s, 3H), 1.22 (s, 6H).

**Preparation Example 42: 7-bromo-8-chloroisoquinolin-1(2H)-one (Intermediate 1-42)**

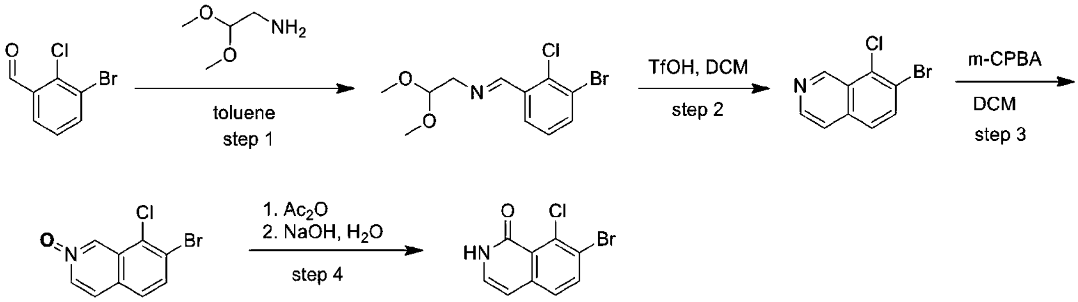


**I-42**

**Step 1: (E)-1-(3-bromo-2-chlorophenyl)-N-(2,2-dimethoxyethyl)methanimine**

To a mixture of 3-bromo-2-chloro-benzaldehyde (14 g, 63.7 mmol) in toluene (200 mL), 2,2-dimethoxyethanamine (6.71 g, 63.7 mmol) was added, and the mixture was stirred at 125°C for 64 hours. Upon completion of stirring, the reaction mixture was concentrated under reduced pressure to obtain (E)-1-(3-bromo-2-chloro-phenyl)-N-(2,2-dimethoxyethyl)methanimine (19 g, 97% yield) as a yellow oil.
$^1$H NMR (400 MHz, DMSO-$d_6$) δ = 8.68 (s, 1H), 7.94 - 7.86 (m, 2H), 7.34 (t, $J$ = 7.6 Hz, 1H), 4.64 (t, $J$ = 5.4 Hz, 1H), 3.78 (d, $J$ = 5.4 Hz, 2H), 3.30 (s, 6H).

**Step 2: 7-bromo-8-chloroisoquinoline**

To a mixture of (E)-1-(3-bromo-2-chloro-phenyl)-N-(2,2-dimethoxyethyl)methanimine (2g, 6.52 mmol) in DCM (20 mL), trifluoromethanesulfonic acid (20 mL) was added, and the mixture was stirred at 120°C for 0.5 hours. Thereafter, the reaction mixture was cooled to 25°C and stirred at 25°C for 0.5 hours. Upon completion of stirring, the reaction mixture was added with MeOH (200 mL). The mixture was poured into $NH_3 \cdot H_2O$ (200 mL) and extracted with EA (3 X 300 mL). The organic layers were concentrated under reduced pressure. The residue was triturated with water at 25°C for 30 minutes to obtain 7-bromo-8-chloro-isoquinoline (820 mg, 51% yield) as a brown solid.
$^1$H NMR (400 MHz, DMSO-$d_6$) δ = 9.56 (s, 1H), 8.68 (d, $J$= 5.6 Hz, 1H), 8.06 (d, $J$ = 8.8 Hz, 1H), 7.96 - 7.94 (m, 2H).

**Step 3: 7-bromo-8-chloroisoquinoline 2-oxide**

To a mixture of 7-bromo-8-chloro-isoquinoline (820 mg, 3.38 mmol) in DCM (20 mL), m-CPBA (1.37 g, 6.76 mmol, 85% purity) was added, and the mixture was stirred at 25°C for 16 hours. Upon completion of stirring, the mixture was added with $Na_2O_3S_2$ (50 mL). The mixture was concentrated under reduced pressure. The residue was extracted with EA (3 x 40 mL). The organic layers were concentrated under reduced pressure. The residue was purified by column chromatography ($SiO_2$, PE/EA/MeOH= 1/0/0 to 0/10/1) to obtain 7-bromo-8-chloro-2-oxido-isoquinolin-2-ium (690 mg, 78% yield) as a yellow solid.
$^1$H NMR (400 MHz, DMSO-$d_6$) $\delta$ = 8.88 (s, 1H), 8.32 (d, *J*= 7.2 Hz, 1H), 8.06 (d, *J* = 7.2 Hz, 1H), 7.90 (s, 2H)

**Step 4: 7-bromo-8-chloroisoquinolin-1(2H)-one**

A mixture of 7-bromo-8-chloro-2-oxido-isoquinolin-2-ium (640 mg, 2.48 mmol) in $Ac_2O$ (6 mL) was stirred at 120°C for 3 hours. Upon completion of stirring, the mixture was concentrated under reduced pressure to obtain (7-bromo-8-chloro-1-isoquinolyl) acetate (640 mg, crude product). A mixture of (7-bromo-8-chloro-1-isoquinolyl) acetate (640 mg, 2.13 mmol) in a mixture of NaOH (2 M, 32.00 mL) and $H_2O$ (8 mL) was stirred at 100°C for 1 hour. Upon completion of stirring, the mixture was acidified to pH 6 with citric acid (5% in water) and extracted with DCM (3 X 100 mL). The combined organic layers were concentrated under reduced pressure to obtain **Intermediate I-42** (320 mg, 58% yield) as a yellow solid.

$^1$H NMR (400 MHz, $CDCl_3$) $\delta$ = 10.76 - 10.32 (m, 1H), 7.86 (d, *J*= 8.4 Hz, 1H), 7.32 (d, *J*= 8.4 Hz, 1H), 7.19 (d, *J*= 6.8 Hz, 1H), 6.48 (d, *J* = 7.2 Hz, 1H).

**Preparation Example 43: 7-bromo-8-chloro-2-methylisoquinolin-1(2H)-one (Intermediate 1-43)**

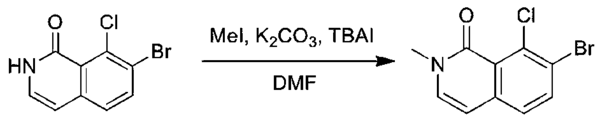


I-42

I-43

To a solution of **Intermediate I-42** (1.0 g, 3.87 mmol,) and $CH_3I$ (1.10 g, 7.74 mmol) in DMF (10 mL), $K_2CO_3$ (1.60 g, 11.6 mmol) and TBAI (143 mg, 387 $\mu$mol) were added. The mixture was stirred at 40°C for 2 hours. Upon completion of stirring, the reaction mixture was quenched by addition of aqueous ammonium chloride (20 mL) at 25°C, and then diluted with water (30 mL) and extracted with EA (3 x 50 mL). The combined organic layers were washed with brine (3 x 20 mL), dried over anhydrous sodium sulfate, and then filtered and concentrated under reduced pressure to obtain a residue. The residue was purified by column chromatography ($SiO_2$, petroleum ether/ethyl acetate = 2/1 to 1/1) to obtain **Intermediate I-43** (1.0 g, 95% yield) as a yellow solid.

MS (EI) m/z: $[M+H]^+$ 274.0.

$^1$H NMR (400 MHz, $CDCl_3$) $\delta$ = 7.83 (d, *J* = 8.8 Hz, 1H), 7.29 (s, 1H), 7.17 (d, *J* = 7.2 Hz, 1H), 6.41 (d, *J* = 7.2 Hz, 1H), 3.60 (s, 3H).

**Preparation Examples 44 to 46**

Intermediates I-44 to I-46 were prepared in the same method as in Preparation Example 43 using **Intermediate I-42** as a starting material, except that an appropriate alkyl halide was used.

[Table 1]

| No. | Structure/Name | Spectral Data |
|---|---|---|
| 44 | 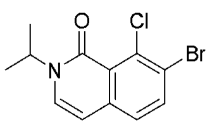 7-bromo-8-chloro-2-isopropylisoquinolin-1(2H)-one | MS (EI) m/z: $[M+H]^+$ 300.0 |

(continued)

| No. | Structure/Name | Spectral Data |
|---|---|---|
| 45 | 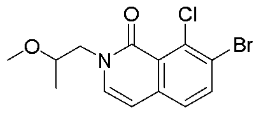 7-bromo-8-chloro-2-(2-methoxypropyl)isoquinolin-1 (2H)-one | MS (EI) m/z: $[M+H]^+$ 330.6 |
| 46 | 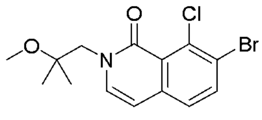 7-bromo-8-chloro-2-(2-methoxy-2-methylpropyl)isoquinolin-1 (2H)-one | MS (EI) m/z: $[M+H]^+$ 344.6 |

**Preparation Example 47: 7-bromo-2-(2-methoxy-2-methylpropyl)isoquinolin-1(2H)-one (Intermediate 1-47)**

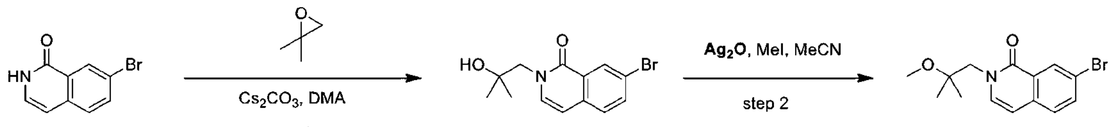


I-47

**Intermediate I-47** was obtained in the same method as in Preparation Example 31, except that 7-bromoisoquinolin-1(2H)-one was used instead of 6-bromo-3-(2-hydroxy-2-methylpropyl)quinazolin-4(3H)-one in Preparation Example 31.

m/z ES+ $[M+H]^+$ 310.0.
$^1$H NMR (400 MHz, $CDCl_3$) δ = 8.57 (d, *J* = 2.0 Hz, 1H), 7.71 (dd, *J* = 2.0, 8.4 Hz, 1H), 7.38 (d, *J*= 8.4 Hz, 1H), 7.30 (d, *J*= 7.6 Hz, 1H), 6.40 (d, *J*= 7.6 Hz, 1H), 4.10 (s, 2H), 3.23 (s, 3H), 1.21 (s, 6H).

**Preparation Example 48: 7-bromo-2-methylisoquinolin-1(2H)-one (Intermediate I-48)**

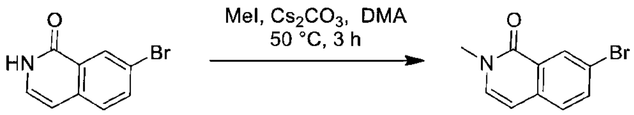


I-48

To a solution of 7-bromoisoquinoline-1-ol (600 mg, 2.68 mmol) in DMA (8 mL), $Cs_2CO_3$ (2.62 g, 8.03 mmol) and MeI (760 mg, 5.36 mmol) were added. The mixture was stirred at 50°C for 3 hours. Upon completion of stirring, the reaction mixture was quenched by addition of aqueous ammonium chloride (20 mL) at 25°C, and then diluted with water (30 mL) and extracted with EA (3 x 50 mL). The combined organic layers were washed with brine (3 x 20 mL), dried over anhydrous sodium sulfate, and then filtered and concentrated under reduced pressure to obtain a residue. The residue was purified by column chromatography ($SiO_2$, petroleum ether/ethyl acetate = 2/1 to 1/1) to obtain **Intermediate I-48** (600 mg, 94% yield) as a white solid.

MS (EI) m/z: $[M+H]^+$ 238.0
$^1$H NMR (400 MHz, $CDCl_3$) δ = 8.57 (d, *J* = 1.6 Hz, 1H), 7.71 (dd, *J* = 1.6, 8.4 Hz, 1H), 7.39 (d, *J* = 8.4 Hz, 1H), 7.09 (d, *J*= 7.2 Hz, 1H), 6.45 (d, *J*= 7.2 Hz, 1H), 3.61 (s, 3H).

**Preparation Example 49: 7-((3-amino-5-chloropyrazin-2-yl)thio)-8-chloro-2-methylisoquinolin-1(2H)-one (Intermediate 1-49)**

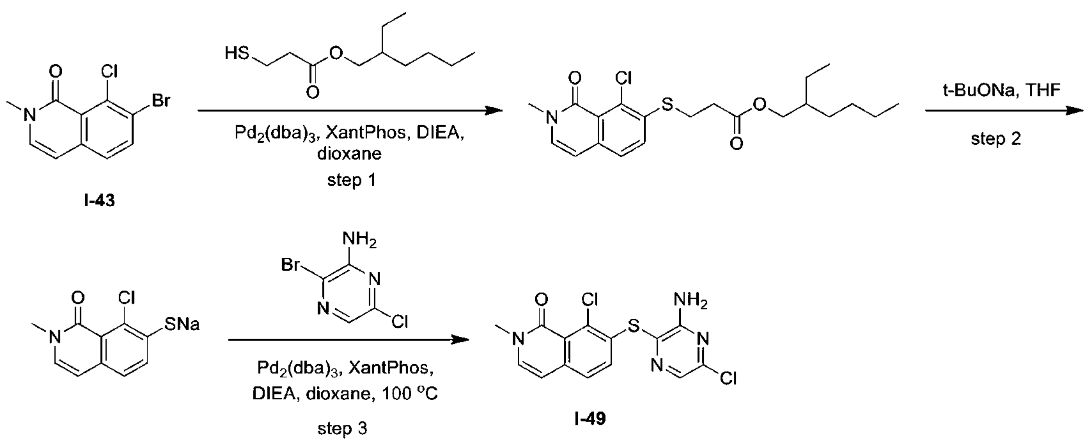


I-43

I-49

**Intermediate I-49** was obtained in the same method as in Preparation Example 41, except that **Intermediate I-43** was used instead of **Intermediate I-29** in Preparation Example 41.

MS (EI) m/z: $[M+H]^+$ 352.9

$^1$H NMR (400 MHz, $CDCl_3$) δ = 7.91 (s, 1H), 7.32 (s, 2H), 7.14 (d, *J* = 7.2 Hz, 1H), 6.40 (d, *J*= 7.2 Hz, 1H), 5.24 - 5.15 (m, 2H), 3.58 (s, 3H)

**Preparation Examples 50 and 51**

Intermediates I-50 and I-51 were prepared in the same method as in Preparation Example 49, using an appropriate intermediate as a starting material.

[Table 2]

| No. | Structure/Name | Preparation Example Used | Spectral Data |
|---|---|---|---|
| 50 | 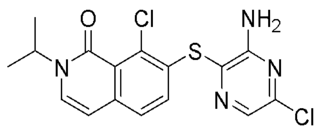 7-((3-amino-5-chloropyrazin-2-yl)thio)-8-chloro-2-isopropylisoquinolin-1(2H)-one | Intermediate I-44 | $^1$H NMR (400 MHz, $CDCl_3$) δ = 7.91 (s, 1H), 7.33 - 7.29 (m, 2H), 7.18 (s, 1H), 6.45 (d, *J* = 7.2 Hz, 1H), 5.39 - 5.33 (m, 1H), 5.20 - 5.16 (m, 2H), 1.39 (d, *J* = 6.8 Hz, 6H). |
| 51 | 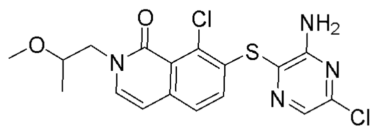 7-((3-amino-5-chloropyrazin-2-yl)thio)-8-chloro-2-(2-methoxypropyl)isoquinolin-1(2H)-one | Intermediate I-45 | MS (EI) m/z: $[M+H]^+$ 411.0 $^1$H NMR (400 MHz, $CDCl_3$) δ = 7.91 (s, 1H), 7.37 - 7.30 (m, 2H), 7.23 (d, *J* = 7.2 Hz, 1H), 6.37 (d, *J* = 7.2 Hz, 1H), 5.19 (s, 2H), 4.33 (dd, *J* = 2.8, 13.2 Hz, 1H), 3.82 - 3.71 (m, 1H), 3.27 (s, 3H), 1.23 (d, *J* = 6.4 Hz, 3H). |

**Preparation Example 52: (R)-2-methyl-N-((R)-3H-spiro[benzofuran-2,4'-piperidin]-3-yl)propane-2-sulfinamide (Intermediate I-52)**

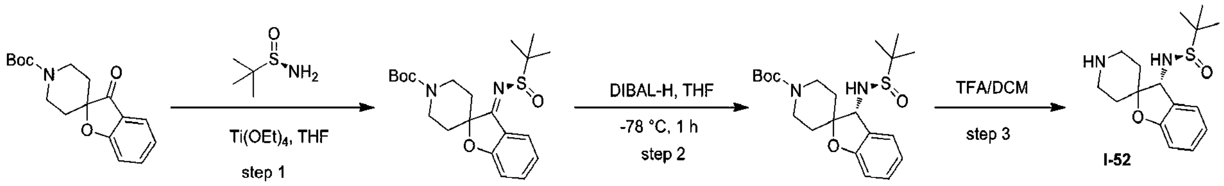


I-52

**Step 1: tert-butyl (R,E)-3-((tert-butylsulfinyl)imino)-3H-spiro[benzofuran-2,4'-piperidine]-1'-carboxylate**

Tert-butyl (R,E)-3-((tert-butylsulfinyl)imino)-3H-spiro[benzofuran-2,4'-piperidine]-1'-carboxylate (5.4 g, 80% yield) was obtained using tert-butyl 3-oxospiro[benzofuran-2,4'-piperidine]-1'-carboxylate instead of tert-butyl 1-oxospiro[indane-2,4'-piperidine]-1'-carboxylate in Step 1 of Preparation Example 25.

m/z ES+ $[M+Na]^+$ 429.2.

$^1$H NMR (400 MHz, DMSO-$d_6$) δ = 8.28 - 8.20 (m, 1H), 7.71 - 7.64 (m, 1H), 7.23 (d, *J* = 8.4 Hz, 1H), 7.20 - 7.10 (m, 1H), 3.36 - 3.33 (m, 2H), 3.18 - 2.98 (m, 2H), 1.76 - 1.70 (m, 4H), 1.43 (s, 9H), 1.22 (s, 9H).

**Step 2: tert-butyl (R)-3-(((R)-tert-butylsulfinyl)amino)-3H-spiro[benzofuran-2,4'-piperidine]-1'-carboxylate**

To a solution of tert-butyl rac-(3E)-3-[(R)-tert-butylsulfinyl]iminospiro[benzofuran-2,4'-piperidine]-1'-carboxylate (5.2 g, 12.7 mmol) in DCM (40 mL), DIBALH (1 M, 19.2 mL) was added. The mixture was stirred at -78°C for 1 hour. Upon completion of stirring, the reaction mixture was quenched by addition of MeOH (10 mL) at -78°C, and then slowly warmed to 25°C and stirred for 0.5 hours. The mixture was filtered, and the filtrate was concentrated under reduced pressure to obtain a residue. The residue was purified by column chromatography ($SiO_2$, petroleum ether/ethyl acetate = 1/0 to 3/1) to obtain tert-butyl rac-(3R)-3-[[(R)-tert-butylsulfinyl]amino]spiro[3H-benzofuran-2,4'-piperidine]-1'-carboxylate (3.4 g, 62% yield) as a yellow solid.

m/z ES+ $[M+Na]^+$ 431.1.

$^1$H NMR (400 MHz, $CDCl_3$) δ = 7.29 (s, 1H), 7.26 - 7.20 (m, 1H), 6.94 - 6.90 (m , 1H), 6.82 (d, *J*= 8.0 Hz, 1H), 4.68 - 4.60 (m, 1H), 4.21 - 3.99 (m, 2H), 3.72 - 3.60 (m, 1H), 3.26 - 3.10 (m, 2H), 1.98 - 1.65 (m, 4H), 1.47 (s, 9H), 1.26 (s, 9H).

**Step 3: (R)-2-methyl-N-((R)-3H-spiro[benzofuran-2,4'-piperidin]-3-yl)propane-2-sulfinamide**

**Intermediate I-52** (2.2g, 65% yield) was obtained in the same method as in Step 3 of Preparation Example 25. m/z ES+ $[M+H]^+$ 309.1

**Preparation Example 53: sodium 5-((R)-3-(((R)-tert-butylsulfinyl)amino)-3H-spiro[benzofuran-2,4'-piperidin]-1'-yl)pyrazine-2-thiolate (Intermediate I-53)**

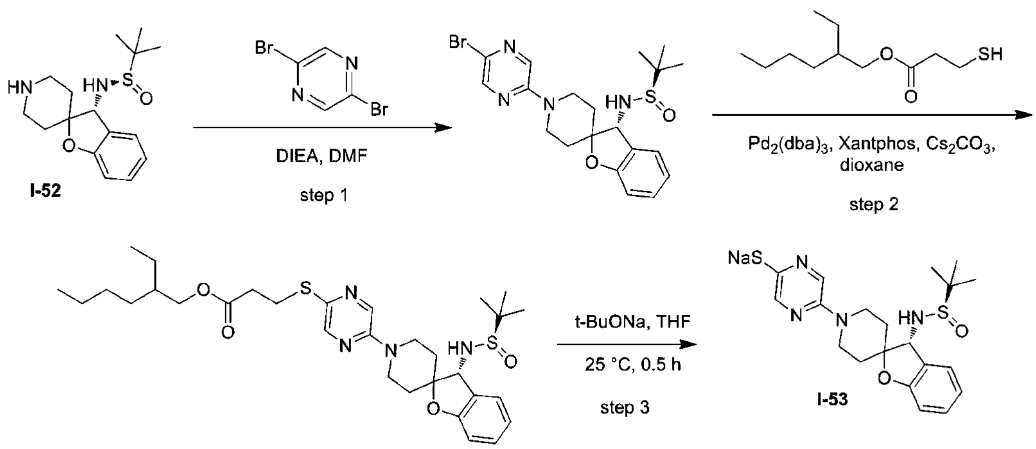


**Intermediate I-53** (3.15 g, 85% yield) was obtained using **Intermediate I-52** instead of **Intermediate I-25** in Step 1 of Preparation Example 26.
m/z ES+ $[M+Na]^+$ 441.1

**Preparation Example 54: 6-bromo-5-chloro-3-methylquinazolin-4(3H)-one (Intermediate I-54)**

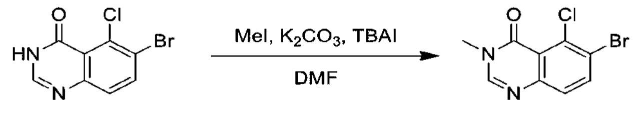


I-21 I-54

**Intermediate I-54** (2.5 g, 79% yield) was obtained using **Intermediate I-21** instead of **Intermediate I-42** in Preparation Example 43.

$^1H$ NMR (400 MHz, DMSO-$d_6$) δ= 8.45 (s, 1H), 8.10 (d, *J* = 8.8 Hz, 1H), 7.54 (d, *J* = 8.8 Hz, 1H), 3.45 (s, 3H).

**Preparation Example 55 : 6-bromo-5-chloro-3-(methyl-$d_3$)quinazolin-4(3H)-one**

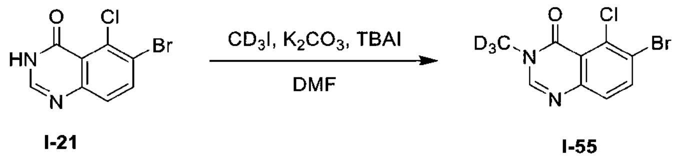


I-21 I-55

**Intermediate I-55** (100 mg, 75% yield) was obtained using $CD_3I$ instead of $CH_3I$ in Preparation Example 54.

$^1H$ NMR (400 MHz, DMSO-$d_6$) δ = 10.01 (s, 1H), 7.73 (d, *J* = 8.8 Hz, 1H), 7.52 (d, *J*= 8.8 Hz, 1H)
m/z ES+ $[M+H]^+$ 275.9

**Example 1: (S)-6-((5-(6-amino-4,6-dihydrospiro[cyclopenta[d]thiazole-5,4'-piperidin]-1'-yl)pyrazin-2-yl)thio)-5-chloroquinazolin-4(3H)-one**

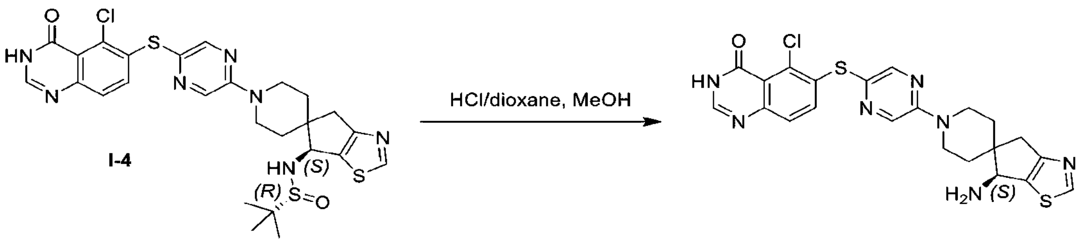


I-4

To a solution of Intermediate I-4 (50 mg, 83.0 μmol) in methanol (5 mL), HCl/dioxane (4 M, 1.04 mL) was added, and the mixture was stirred at 25 °C for 0.5 hours. Upon completion of the reaction, the reaction mixture was concentrated under reduced pressure. The filtrate was purified by column chromatography to obtain the compound of Example 1 (30 mg, 73% yield) as a yellow solid.

$^1H$ NMR (400 MHz, DMSO-$d_6$) δ= 9.03 (s, 1H), 8.50 (s, 1H), 8.31 (s, 1H), 8.22 (s, 1H), 8.04 (s, 1H), 7.51 (d, J = 8.8 Hz, 1H), 7.21 (d, J = 8.8 Hz, 1H), 4.32 - 4.18 (m, 3H), 4.12 (s, 1H), 3.39 - 3.35 (m, 1H), 3.31 - 3.25 (m, 2H), 2.88 (d, J = 13.2 Hz, 2H), 1.89 - 1.80 (m, 1H), 1.71 - 1.60 (m, 3H); MS (EI) m/z: 481.1 $[M-NH_2]^+$.

**Example 2: (S)-6-((5-(6-amino-4,6-dihydrospiro[cyclopenta[d]thiazole-5,4'-piperidin]-1'-yl)pyrazin-2-yl)thio)-5-chloro-3-methylquinazolin-4(3H)-one**

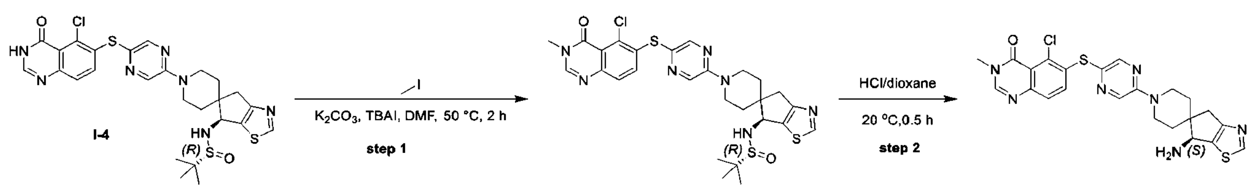


I-4

Step 1: **(R)-N-[(6S)-1'-[5-(5-chloro-3-methyl-4-oxo-quinazolin-6-yl)sulfanylpyrazin-2-yl]spiro[4,6-dihydrocyclopenta[d]thiazole-5,4'-piperidin]-6-yl]-2-methyl-propane-2-sulfinamide**

To a solution of Intermediate I-4 (45 mg, 74.7 μmol) in DMF (1 mL), methyl iodide (15.9 mg, 112 μmol), $K_2CO_3$ (31.0 mg, 224 μmol) and TBAI (2.76 mg, 7.47 mmol) were added, and the mixture was stirred at 25 °C for 2 hours. The reaction was terminated with aqueous $NaHCO_3$ solution, and the mixture was extracted with EA. The EA layer was dried over $MgSO_4$, filtered and then concentrated. The residue was purified by column chromatography to obtain (R)-N-[(6S)-1'-[5-(5-chloro-3-methyl-4-oxo-quinazolin-6-yl)sulfanylpyrazin-2-yl]spiro[4,6-dihydrocyclopenta[d]thiazole-5,4'-piperidin]-6-yl]-2-methyl-propane-2-sulfinamide (15 mg, 33% yield) as a yellow solid.

$^1H$ NMR (400 MHz, DMSO-$d_6$) δ= 9.06 (s, 1H), 8.50 (s, 1H), 8.35 (s, 1H), 8.31 (s, 1H), 7.50 (d, J = 8.8 Hz, 1H), 7.18 (d, J = 8.8 Hz, 1H), 5.97 (d, J = 10.0 Hz, 1H), 4.51 (d, J = 10.0 Hz, 1H), 4.39 - 4.26 (m, 2H), 3.45 (s, 3H), 3.28 - 3.17 (m, 2H), 3.00 - 2.92 (m, 1H), 2.89 - 2.85 (m, 1H), 1.93 - 1.84 (m, 1H), 1.82 - 1.66 (m, 3H), 1.13 (s, 9H).

**Step 2: (S)-6-((5-(6-amino-4,6-dihydrospiro[cyclopenta[d]thiazole-5,4'-piperidin]-1'-yl)pyrazin-2-yl)thio)-5-chloro-3-methylquinazolin-4(3H)-one**

The compound of Example 2 (5 mg, 40% yield) was synthesized in the same method as in Example 1.

$^1H$ NMR (400 MHz, DMSO-$d_6$) δ = 8.99 (s, 1H), 8.49 (d, J = 1.2 Hz, 1H), 8.36 (s, 1H), 8.30 (d, J = 1.2 Hz, 1H), 8.23 (s, 1H), 7.51 (d, J = 8.8 Hz, 1H), 7.20 (d, J = 8.8 Hz, 1H), 4.28 - 4.15 (m, 2H), 4.05 (s, 1H), 3.44 (s, 3H), 3.42 - 3.36 (m, 2H), 3.34 - 3.26 (m, 2H), 2.93 - 2.87 (m, 1H), 2.83 - 2.80 (m, 1H), 1.89 - 1.78 (m, 1H), 1.71 - 1.59 (m, 3H); MS (EI) m/z: 495.2 $[M-NH_2]^+$.

**Examples 3 to 5**

The compounds of Examples 3 to 5 were prepared in the same method as in Example 2, except that an appropriate alkyl halide compound was used instead of (1R)-1-(3-bromophenyl)ethanamine in Example 2.

[Table 3]

| No. | Structure/Name | Spectral Data |
|---|---|---|
| 3 | 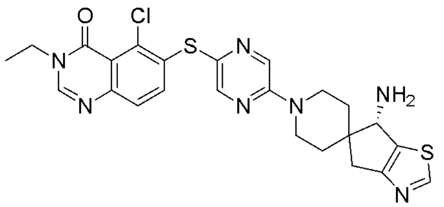 (S)-6-((5-(6-amino-4,6-dihydrospiro[cyclopenta[d]thiazole-5,4'-piperidin]-1'-yl)pyrazin-2-yl)thio)-5-chloro-3-ethylquinazolin-4(3H)-one | $^1H$ NMR (400 MHz, DMSO-$d_6$) δ 9.04 (s, 1H), 8.56 - 8.49 (m, 1H), 8.41 (s, 1H), 8.32 (s, 1H), 8.22 (s, 1H), 7.52 (d, J= 8.8 Hz, 1H), 7.22 (d, J= 8.8 Hz, 1H), 4.33 - 4.26 (m, 1H), 4.22 (d, J= 13.2 Hz, 1H), 4.13 (s, 1H), 3.98 - 3.96 (m, 2H), 3.46 - 3.25 (m, 4H), 2.94 - 2.81 (m, 2H), 1.92 - 1.80 (m, 1H), 1.75 - 1.58 (m, 3H), 1.28 (t, J= 7.6 Hz, 3H). MS (EI) m/z: 526.2 $[M+H]^+$. |
| 4 | 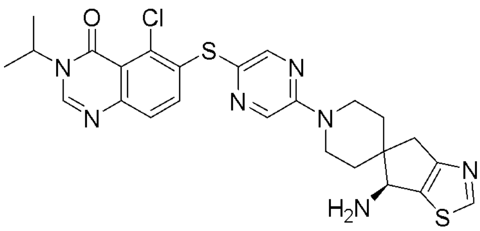 (S)-6-((5-(6-amino-4,6-dihydrospiro[cyclopenta[d]thiazole-5,4'-piperidin]-1'-yl)pyrazin-2-yl)thio)-5-chloro-3-isopropylquinazolin-4(3H)-one | $^1H$ NMR (400 MHz, $CDCl_3$) δ = 8.83 (s, 1H), 8.28 (s, 1H), 8.26 (s, 1H), 8.17 - 8.09 (m, 1H), 8.07 (s, 1H), 7.47 (d, J = 8.8 Hz, 1H), 7.31 - 7.29 (m, 1H), 5.22 - 5.13 (m, 1H), 4.34 (d, J = 13.2 Hz, 1H), 4.30 - 4.19 (m, 2H), 3.36 - 3.31 (m, 1H), 3.27 - 3.21 (m, 1H), 3.10 - 2.98 (m, 2H), 2.07 - 1.92 (m, 2H), 1.88 - 1.74 (m, 2H), 1.49 (d, J = 6.8 Hz, 6H). MS (EI) m/z: 540.1 $[M+H]^+$. |

(continued)

| No. | Structure/Name | Spectral Data |
|---|---|---|
| 5 | 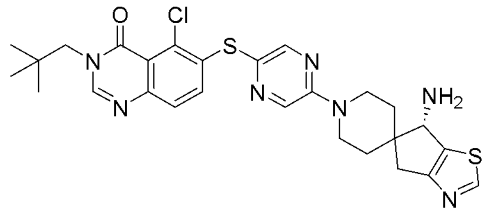 (S)-6-((5-(6-amino-4,6-dihydrospiro[cyclopenta[d]thiazole-5,4'-piperidin]-1'-yl)pyrazin-2-yl)thio)-5-chloro-3-neopentylquinazolin-4(3H)-one | $^1$H NMR (400 MHz, DMSO-$d_6$) δ = 9.00 (s, 1H), 8.51 (s, 1H), 8.30 (d, J = 12.4 Hz, 2H), 7.52 (d, J = 8.8 Hz, 1H), 7.22 (d, J = 8.8 Hz, 1H), 4.33 - 4.11 (m, 2H), 4.06 (s, 1H), 3.86 (s, 2H), 3.43 - 3.37 (m, 2H), 2.93 - 2.79 (m, 2H), 1.84 - 1.57 (m, 4H), 0.93 (s, 9H). MS (EI) m/z: 551.3 [M-$NH_2$]$^+$. |

**Example 6: (S)-6-((5-(5-amino-5,7-dihydrospiro[cyclopenta[b]pyridine-6,4'-piperidin]-1'-yl)pyrazin-2-yl)thio)-5-chloro-3-methylquinazolin-4(3H)-one**

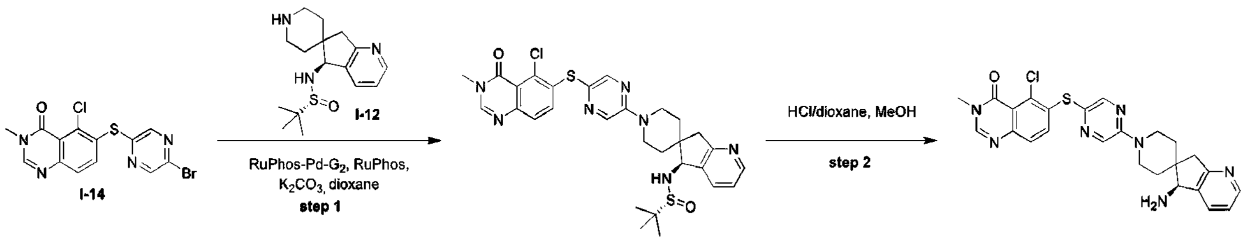


**Step 1: (R)-N-((S)-1'-(5-((5-chloro-3-methyl-4-oxo-3,4-dihydroquinazolin-6-ylthio)pyrazin-2-yl)-5,7-dihydrospiro[cyclopenta[b]pyridine-6,4'-piperidin]-5-yl)-2-methylpropane-2-sulfinamide**

To a solution of Intermediate I-14 (80.0 mg, 208 μmol) and Intermediate I-12 (64.1 mg, 208 μmol) in dioxane (3 mL), $K_2CO_3$ (86.4 mg, 625 μmol), [2-(2-aminophenyl)phenyl]-chloropalladium; dicyclohexyl-[2-(2,6-diisopropoxyphenyl)phenyl]phosphane (16.2 mg, 20.8 μmol) and RuPhos (19.4 mg, 41.7 μmol) were added. The mixture was stirred at 100 °C for 12 hours. Upon completion of the reaction, the reaction mixture was quenched by addition of aqueous ammonium chloride (20 mL) at 25 °C, and then diluted with water (20 mL) and extracted with EA (3 x 30 mL). The combined organic layers were washed with brine (20 mL), dried over anhydrous sodium sulfate, and filtered and concentrated under reduced pressure to obtain a residue. The residue was purified by column chromatography ($SiO_2$, petroleum ether/ethyl acetate = 1/1 to 1/4) to obtain (R)-N-((S)-1'-(5-((5-chloro-3-methyl-4-oxo-3,4-dihydroquinazolin-6-yl)thio)pyrazin-2-yl)-5,7-dihydrospiro[cyclopenta[b]pyridine-6,4'-piperidin]-5-yl)-2-methylpropane-2-sulfinamide (70.0 mg, 55% yield) as a yellow solid.

$^1$H NMR (400 MHz, $CDCl_3$) δ = 8.47 (d, J = 4.4 Hz, 1H), 8.29 (d, J = 1.2 Hz, 1H), 8.24 (d, J = 1.2 Hz, 1H), 8.03 - 8.00 (m, 2H), 7.46 (d, J = 8.8 Hz, 1H), 7.30 (s, 1H), 7.20 (dd, J = 5.2, 7.6 Hz, 1H), 4.33 - 4.24 (m, 2H), 3.57 (s, 4H), 3.34 - 3.24 (m, 4H), 2.94 (d, J = 16.4 Hz, 1H), 1.80 - 1.75 (m, 2H), 1.46 (d, J = 12.0 Hz, 2H), 1.27 (s, 9H).

**Step 2: (S)-6-((5-(5-amino-5,7-dihydrospiro[cyclopenta[b]pyridine-6,4'-piperidin]-1'-yl)pyrazin-2-yl)thio)-5-chloro-3-methylquinazolin-4(3H)-one**

The compound of Example 6 (25.6 mg, 62% yield) was synthesized in the same method as in Example 1.

$^1$H NMR (400 MHz, DMSO-$d_6$) δ= 8.49 (s, 1H), 8.38 - 8.29 (m, 3H), 7.68 (d, J = 7.6 Hz, 1H), 7.52 (d, J = 8.8 Hz, 1H), 7.24 - 7.16 (m, 2H), 4.34 - 4.27 (m, 2H), 3.95 (s, 1H), 3.45 (s, 3H), 3.28 - 3.21 (m, 2H), 3.14 (d, J = 16.4 Hz, 1H), 2.80 (d, J = 16.0 Hz, 1H), 1.85 - 1.70 (m, 2H), 1.62 - 1.53 (m, 1H), 1.20 (d, J = 13.2 Hz, 1H); MS (EI) m/z: 506.2 [M+H]$^+$.

**Example 7: (S)-6-((5-(1-amino-1,3-dihydrospiro[indene-2,4'-piperidin]-1'-yl)pyrazin-2-yl)thio)-5-chloro-3-methylquinazolin-4(3H)-one**

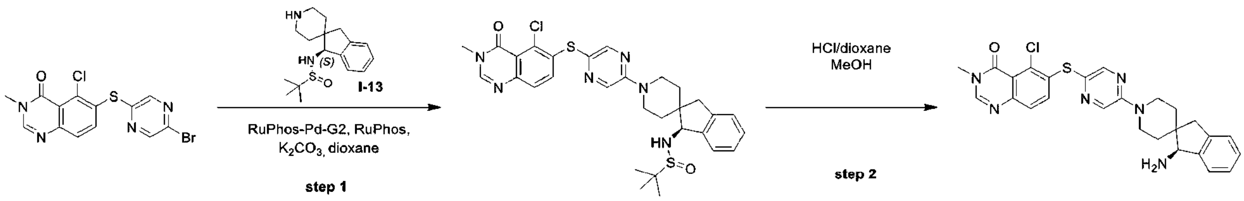


The compound of Example 7 (32 mg, 77% yield) was synthesized in the same method as in Example 6, using Intermediate I-13 as a starting material instead of Intermediate I-12 in Step 1 of Example 6.

$^1H$ NMR (400 MHz, DMSO-$d_6$) δ= 8.49 (s, 1H), 8.38 - 8.36 (m, 1H), 8.31 (d, J = 0.8 Hz, 1H), 8.27 (s, 1H), 7.52 (d, J = 8.8 Hz, 1H), 7.39 - 7.36 (m, 1H), 7.24 - 7.20 (m, 4H), 4.30 (d, J = 13.6 Hz, 2H), 4.01 (s, 1H), 3.45 (s, 3H), 3.24 (d, J = 12.0 Hz, 2H), 3.13 (d, J = 15.6 Hz, 1H), 2.76 (d, J = 15.6 Hz, 1H), 1.82 - 1.68 (m, 2H), 1.55 (d, J = 12.8 Hz, 1H), 1.29 - 1.24 (m, 1H); MS (EI) m/z: 506.1 $[M+H]^+$.

## Example 8: (S)-6-((3-amino-5-(1-amino-1,3-dihydrospiro[indene-2,4'-piperidin]-1'-yl)pyrazin-2-yl)thio)-5-chloro-3-(2-methoxyethyl)quinazolin-4(3H)-one

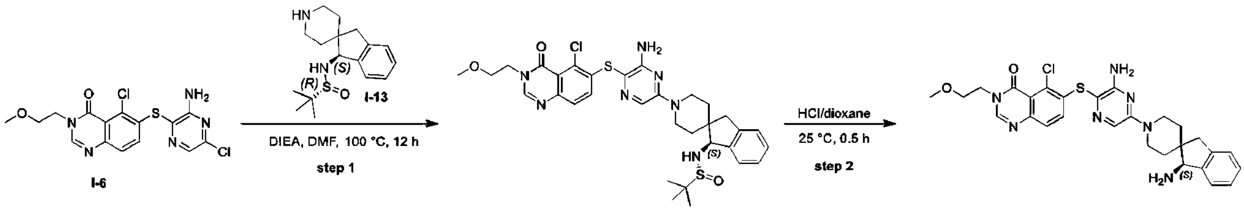


### Step 1: (R)-N-((S)-1'-(6-amino-5-((5-chloro-3-(2-methoxyethyl)-4-oxo-3,4-dihydroquinazolin-6-yl)thio)pyrazin-2-yl)-1,3-dihydrospiro [indene-2,4'-piperidin]-1-yl)-2-methylpropane-2-sulfinamide

To a solution of Intermediate I-6 (50 mg, 126 μmol) in DMSO (1 mL), DIEA (811 mg, 6.28 mmol) and Intermediate I-13 (38.5 mg, 126 μmol) were added, the mixture was stirred at 100 °C for 12 hours. the reaction mixture was added with aqueous ammonium chloride and extracted with EA. The EA layer was dried over $MgSO_4$, filtered, and then concentrated. The residue was purified by column chromatography to obtain (R)-N-((S)-1'-(6-amino-5-((5-chloro-3-(2-methoxyethyl)-4-oxo-3,4-dihydroquinazolin-6-yl)thio)pyrazin-2-yl)-1,3-dihydrospiro[indene-2,4'-piperidin]-1-yl)-2-methylpropane-2-sulfinamide (15 mg, 33% yield) as a yellow solid.

$^1H$ NMR (400 MHz, $CDCl_3$) δ= 8.02 (s, 1H), 7.67 (s, 1H), 7.50 - 7.46 (m, 1H), 7.35 - 7.31 (m, 1H), 7.27 - 7.22 (m, 3H), 7.15 (d, J = 8.8 Hz, 1H), 4.35 - 4.29 (m, 2H), 4.17 - 4.11 (m, 2H), 3.70 - 3.67 (m, 3H), 3.33 (s, 3H), 3.20 - 3.13 (m, 2H), 3.12 - 3.06 (m, 1H), 2.79 (d, J = 15.6 Hz, 1H), 1.89 - 1.86 (m, 1H), 1.75 - 1.69 (m, 1H), 1.42 - 1.36 (m, 2H), 1.31 (s, 9H).

### Step 2: (S)-6-((3-amino-5-(1-amino-1,3-dihydrospiro[indene-2,4'-piperidin]-1'-yl)pyrazin-2-yl)thio)-5-chloro-3-(2-methoxyethyl)quinazolin-4(3H)-one

The compound of Example 8 (25 mg, 59% yield) was synthesized in the same method as in Example 1.

$^1H$ NMR (400 MHz, $CDCl_3$) δ= 8.02 (s, 1H), 7.69 (s, 1H), 7.46 (d, J = 8.8 Hz, 1H), 7.34 (d, J = 5.2 Hz, 1H), 7.26 - 7.22 (m, 3H), 7.17 (d, J = 8.8 Hz, 1H), 4.86 (s, 2H), 4.27 - 4.19 (m, 2H), 4.18 - 4.13 (m, 2H), 4.01 (s, 1H), 3.69 (t, J = 4.8 Hz, 2H), 3.33 (s, 3H), 3.27 - 3.17 (m, 2H), 3.11 (d, J = 15.6 Hz, 1H), 2.75 (d, J = 15.6 Hz, 1H), 1.87 (d, J = 4.4 Hz, 1H), 1.86 - 1.73 (m, 2H), 1.39 (d, J = 12.0 Hz, 1H); MS (EI) m/z: 564.3 $[M+H]^+$.

## Example 9: 6-((3-amino-5-((S)-1-amino-1,3-dihydrospiro[indene-2,4'-piperidin]-1'-yl)pyrazin-2-yl)thio)-5-chloro-3-(2-methoxypropyl)quinazolin-4(3H)-one

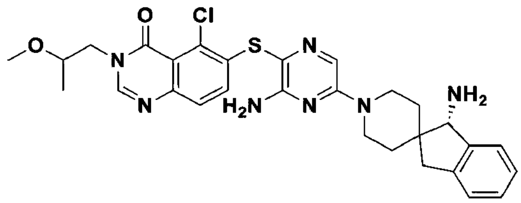

The compound of Example 9 (5.10 mg, 20% yield) was synthesized in the same method as in Example 6, using Intermediate I-7 as a starting material instead of Intermediate I-6 in Step 1 of Example 6.

$^1$H NMR (400 MHz, $CDCl_3$) δ= 8.03 (s, 1H), 7.68 (s, 1H), 7.42 (d, J = 7.2 Hz, 2H), 7.31 - 7.28 (m, 1H), 7.26 (s, 2H), 7.15 (d, J = 8.8 Hz, 1H), 4.96 (d, J = 11.6 Hz, 2H), 4.39 - 4.32 (m, 1H), 4.24 - 4.14 (m, 2H), 4.08 (s, 1H), 3.73 - 3.68 (m, 1H), 3.59 - 3.50 (m, 1H), 3.27 (d, J = 3.2 Hz, 3H), 3.22 - 3.16 (m, 2H), 3.12 (s, 1H), 2.87 - 2.80 (m, 1H), 1.62 - 1.58 (m, 2H), 1.52 - 1.46 (m, 2H), 1.26 (d, J = 2.0 Hz, 3H); MS (EI) m/z: 578.3 [M+H]$^+$.

**Example 10: (S)-6-((3-amino-5-(1-amino-1,3-dihydrospiro[indene-2,4'-piperidin]-1'-yl)pyrazin-2-ylthio)-5-chloro-3-methylquinazolin-4(3H)-one**

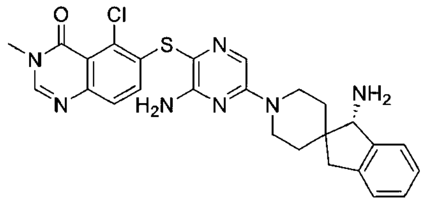

The compound of Example 10 (5.85 mg, 13% yield) was synthesized in the same method as in Example 6, using Intermediate I-15 as a starting material instead of Intermediate I-6 in Step 1 of Example 6.

$^1$H NMR (400 MHz, DMSO-$d_6$) δ= 8.48 (br s, 3H), 7.72 (s, 1H), 7.58 - 7.52 (m, 3H), 7.36 - 7.28 (m, 3H), 6.98 (d, J = 8.8 Hz, 1H), 6.18 (br s, 2H), 4.44 - 4.30 (m, 3H), 3.46 (s, 3H), 3.22 - 3.14 (m, 3H), 3.05 - 2.96 (m, 1H), 1.82 - 1.72 (m, 2H), 1.54 - 1.49 (m, 2H); MS (EI) m/z: 520.2 [M+H]$^+$.

**Example 11: (S)-6-((3-amino-5-(1-amino-1,3-dihydrospiro[indene-2,4'-piperidin]-1'-yl)pyrazin-2-yl)thio)-5-chloro-3-(2-hydroxy-2-methylpropyl)quinazolin-4(3H)-one**

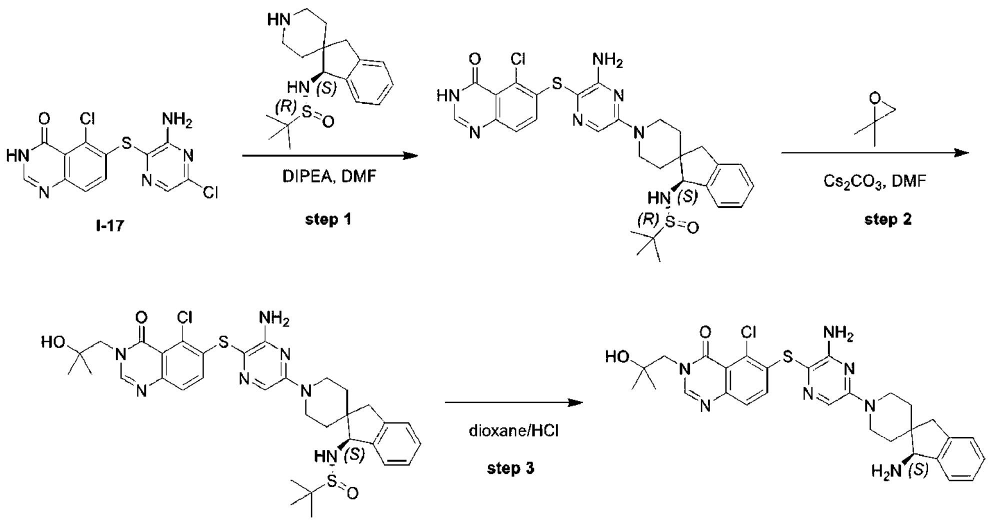

**Step 1: (R)-N-((S)-1'-(6-amino-5-((5-chloro-4-oxo-3,4-dihydroquinazolin-6-ylthio)pyrazin-2-yl)-1,3-dihydrospiro [indene-2,4'-piperidin]-1-yl)-2-methylpropane-2-sulfinamide**

(R)-N-((S)-1'-(6-amino-5-((5-chloro-4-oxo-3,4-dihydroquinazolin-6-yl)thio)pyrazin-2-yl)-1,3-dihydrospiro[indene-2,4'-piperidin]-1-yl)-2-methylpropane-2-sulfinamide (90 mg, 50% yield) was synthesized in the same method as in Example 8, using Intermediate I-17 as a starting material instead of Intermediate I-6 in Step 1 of Example 8.

$^1$H NMR (400 MHz, $CDCl_3$) δ = 7.86 (s, 1H), 7.54 (s, 1H), 7.39 (d, J = 8.8 Hz, 1H), 7.27 - 7.23 (m, 2H), 7.17 (s, 2H), 7.02 (d, J = 8.4 Hz, 1H), 4.52 (d, J = 10.0 Hz, 1H), 4.18 (dd, J = 4.0, 8.4 Hz, 2H), 3.80 (d, J = 10.0 Hz, 1H), 3.11 - 2.95 (m, 4H), 2.72 - 2.63 (m, 2H), 2.26 - 2.19 (m, 1H), 1.88 - 1.83 (m, 1H), 1.27 (s, 9H), 1.21 - 1.15 (m, 2H); MS (EI) m/z: 610.2 [M+H]$^+$.

**Step 2: (R)-N-((S)-1'-(6-amino-5-((5-chloro-3-(2-hydroxy-2-methylpropyl)-4-oxo-3,4-dihydroquinazolin-6-yl)thio) pyrazin-2-yl)-1,3-dihydrospiro[indene-2,4'-piperidin]-1-yl)-2-methylpropane-2-sulfinamide**

To a solution of (R)-N-((S)-1'-(6-amino-5-((5-chloro-4-oxo-3,4-dihydroquinazolin-6-yl)thio)pyrazin-2-yl)-1,3-dihydrospiro[indene-2,4'-piperidin]-1-yl)-2-methylpropane-2-sulfinamide (35.0 mg, 57.7 μmol) and 2,2-dimethyloxirane (74.9 mg, 1.04 mmol) in DMF (1 mL), $Cs_2CO_3$ (56.4 mg, 173 μmol) was added. The mixture was stirred at 80 °C for 12 hours. Upon completion of the reaction, the reaction mixture was quenched by addition of aqueous ammonium chloride (20 mL) at 25 °C, and then diluted with water (30 mL) and extracted with EA (3 x 20 mL). The combined organic layers were washed with brine (3 x 20 mL), dried over anhydrous sodium sulfate, and then filtered and concentrated under reduced pressure to obtain a residue. The residue was purified by Prep-HPLC to obtain (R)-N-((S)-1'-(6-amino-5-((5-chloro-3-(2-hydroxy-2-methylpropyl)-4-oxo-3,4-dihydroquinazolin-6-yl)thio)pyrazin-2-yl)-1,3-dihydrospiro[indene-2,4'-piperidin]-1-yl)-2-methylpropane-2-sulfinamide (15.0 mg, 38% yield) as a yellow solid.

$^1$H NMR (400 MHz, DMSO-$d_6$) δ= 8.20 (s, 1H), 7.68 (s, 1H), 7.51 (d, J = 8.8 Hz, 1H), 7.26 (s, 2H), 7.24 - 7.22 (m, 1H), 7.20 - 7.17 (m, 2H), 6.98 (d, J = 8.8 Hz, 1H), 6.17 (s, 2H), 4.82 (s, 1H), 4.06 (s, 2H), 3.96 (s, 2H), 3.25 (d, J = 11.2 Hz, 2H), 3.17 (d, J = 5.2 Hz, 1H), 3.12 - 3.07 (m, 1H), 2.83 - 2.61 (m, 2H), 2.55 (s, 2H), 1.61 - 1.56 (m, 2H), 1.42 (s, 9H), 1.24 (s, 2H), 1.12 (s, 6H).

**Step 3: (S)-6-((3-amino-5-(1-amino-1,3-dihydrospiro[indene-2,4'-piperidin]-1'-yl)pyrazin-2-yl)thio)-5-chloro-3-(2-hydroxy-2-methylpropyl)quinazolin-4(3H)-one**

The compound of Example 11 (4.63 mg, 36% yield) was synthesized in the same method as in Example 1.

$^1$H NMR (400 MHz, DMSO-$d_6$) δ= 8.20 - 8.17 (m, 1H), 7.69 (s, 1H), 7.52 (d, J = 8.8 Hz, 1H), 7.44 - 7.39 (m, 1H), 7.29 - 7.23 (m, 3H), 6.98 (d, J = 8.8 Hz, 1H), 6.16 (s, 2H), 4.82 (s, 1H), 4.25 (d J = 12.0 Hz, 2H), 4.12 (s, 1H), 3.95 (s, 2H), 3.15 (d, J = 2.4 Hz, 4H), 2.85 - 2.81 (m, 2H), 1.77 - 1.66 (m, 2H), 1.54 - 1.49 (m, 1H), 1.32 (d, J = 14.8 Hz, 1H), 1.11 (s, 6H); MS (EI) m/z: 578.3 [M+H]$^+$.

<u>**Example 12: 6-((3-amino-5-((3S,4S)-4-amino-3-methyl-2-oxa-8-azaspiro[4.5]decan-8-yl)pyrazin-2-yl)thio)-3-benzyl-5-chloroquinazolin-4(3H)-one**</u>

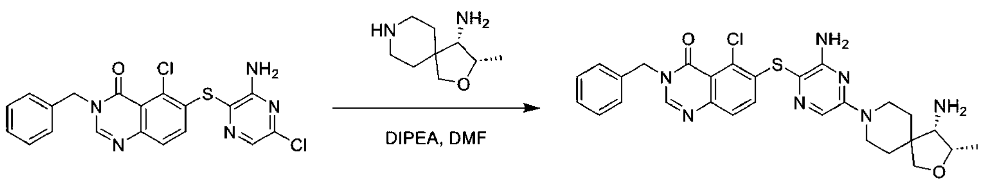


I-16

The compound of Example 12 (12.1 mg, 23% yield) was synthesized in the same method as in Step 1 of Example 6, using Intermediate I-16 instead of Intermediate I-6 and using (3S,4S)-3-methyl-2-oxa-8-azaspiro[4.5]decan-4-amine instead of Intermediate I-13 as starting materials in Step 1 of Example 6.

$^1$H NMR (400 MHz, DMSO-$d_6$) δ= 8.55 (s, 1H), 7.66 (s, 1H), 7.55 - 7.52 (m, 1H), 7.35 (d, J = 4.4 Hz, 4H), 7.30 (d, J = 4.4 Hz, 1H), 7.00 - 6.96 (m, 1H), 6.16 - 6.10 (m, 2H), 5.15 (s, 2H), 4.10 - 4.06 (m, 1H), 3.90 - 3.84 (m, 2H), 3.69 (d, J = 8.4 Hz, 2H), 3.51 (d, J = 8.4 Hz, 2H), 2.96 (d, J = 5.2 Hz, 1H), 1.76 - 1.69 (m, 1H), 1.67 - 1.60 (m, 1H), 1.56 - 1.45 (m, 2H), 1.09 (d, J = 6.4 Hz, 3H); MS (EI) m/z: 564.2 [M+H]$^+$.

**Example 13: 6-((3-amino-5-(4-(aminomethyl)-4-methylpiperidine-1-yl)pyrazin-2-yl)thio)-3-benzyl-5-chloroquinazolin-4(3H)-one**

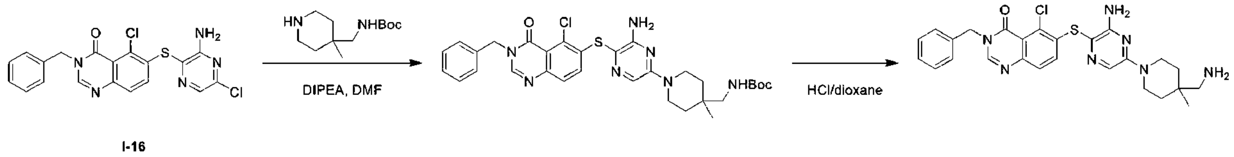


The compound of Example 13 (15.1 mg, 60% yield) was synthesized in the same method as in Example 6, using Intermediate I-16 instead of Intermediate I-6 and using tert-butyl N-[(4-methyl-4-piperidyl)methyl]carbamate instead of Intermediate I-13 as starting materials in Step 1 of Example 6.

$^1$H NMR (400 MHz, DMSO-$d_6$) δ= 8.55 (s, 1H), 7.65 (s, 1H), 7.52 (s, 1H), 7.38 - 7.33 (m, 4H), 7.32 - 7.27 (m, 1H), 6.98 (d, J = 8.8 Hz, 1H), 6.12 (s, 2H), 5.15 (s, 2H), 3.89 - 3.83 (m, 2H), 3.33 (s, 2H), 2.65 - 2.60 (m, 2H), 1.52 - 1.44 (m, 2H), 1.40 - 1.32 (m, 2H), 1.01 (s, 3H); MS (EI) m/z: 522.8 $[M+H]^+$.

**Example 14: 7-((5-((3S,4S)-4-amino-3-methyl-2-oxa-8-azaspiro[4.5]decan-8-yl)pyrazin-2-yl)thio)-8-chloro-2-(2-methoxypropyl)isoquinolin-1(2H)-one**

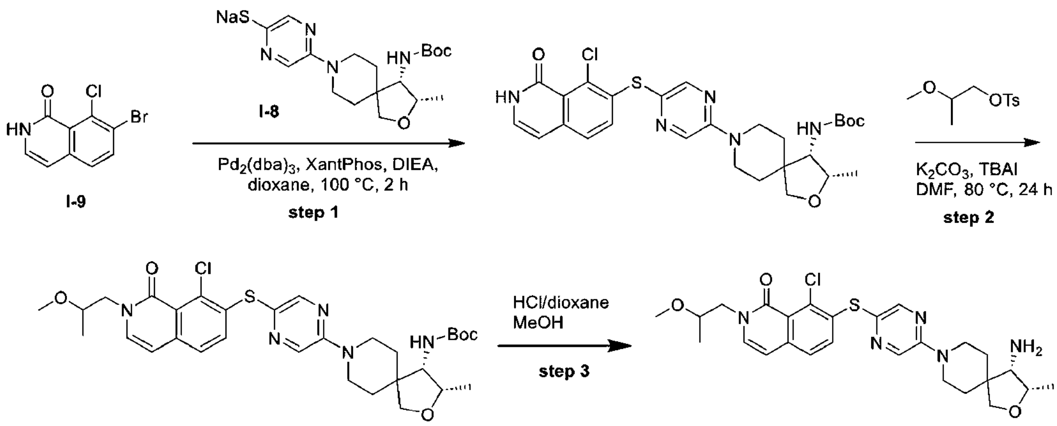


**Step 1: tert-butyl ((3S,4S)-8-(5-((8-chloro-1-oxo-1,2-dihydroisoquinolin-7-yl)thio)pyrazin-2-yl)-3-methyl-2-oxa-8-azaspiro[4.5]decan-4-yl)carbamate**

Intermediate I-9 (150 mg, 0.58 mmol), Intermediate I-8 (235 mg, 0.58 mmol), $Pd_2(dba)_3$ (53 mg, 0.058 mmol) and XantPhos (67.1 mg, 0.116 mmol) were dissolved in 1,4-dioxane (10 mL), and then DIPEA (224 mg, 1.74 mmol) was added thereto. The reaction mixture was purged with nitrogen, and then stirred at 100 °C for 2 hours. The reaction was terminated with $H_2O$, and the mixture was extracted with EA. The EA layer was dried over $MgSO_4$, filtered and then concentrated. The resulting product was separated by Prep-HPLC and concentrated to obtain tert-butyl ((3S,4S)-8-(5-((8-chloro-1-oxo-1,2-dihydroisoquinolin-7-yl)thio)pyrazin-2-yl)-3-methyl-2-oxa-8-azaspiro[4.5]decan-4-yl)carbamate (100 mg, 30% yield).

$^1$H NMR (400 MHz, $CDCl_3$) δ= 10.28 - 10.14 (m, 1H), 8.25 (s, 1H), 8.18 (s, 1H), 7.24 (s, 1H), 7.14 (d, J = 8.4 Hz, 1H), 7.06 (d, J = 4.0 Hz, 1H), 6.38 (d, J = 7.2 Hz, 1H), 4.28 - 4.22 (m, 2H), 4.16 (d, J = 13.6 Hz, 1H), 3.92 (d, J = 9.2 Hz, 1H), 3.68 (d, J = 9.2 Hz, 1H), 3.56 - 3.48 (m, 2H), 3.24 - 3.12 (m, 2H), 2.08 - 1.94 (m, 2H), 1.76 -1.72 (m, 2H), 1.28 (s, 9H), 1.24 (d, J = 6.4 Hz, 3H).

**Step 2: tert-butyl ((3S,4S)-8-(5-((8-chloro-2-(2-methoxypropyl)-1-oxo-1,2-dihydroisoquinolin-7-yl)thio)pyrazin-2-yl)-3-methyl-2-oxa-8-azaspiro[4.5]decan-4-yl)carbamate**

To a solution of tert-butyl ((3S,4S)-8-(5-((8-chloro-1-oxo-1,2-dihydroisoquinolin-7-yl)thio)pyrazin-2-yl)-3-methyl-2-oxa-8-azaspiro[4.5]decan-4-yl)carbamate (50 mg, 88.9 μmol) in DMF (2 mL), 2-methoxypropyl 4-methylbenzenesulfonate (65.1 mg, 266 μmol), $K_2CO_3$ (49.1 mg, 355 μmol) and TBAI (3.29 mg, 8.89 μmol) were added, and the

mixture was stirred at 80 °C for 24 hours. The reaction mixture was added with water and extracted with EA. The EA layer was dried over $MgSO_4$, filtered and then concentrated. The residue was purified by column chromatography to obtain tert-butyl ((3S,4S)-8-(5-((8-chloro-2-(2-methoxypropyl)-1-oxo-1,2-dihydroisoquinolin-7-yl)thio)pyrazin-2-yl)-3-methyl-2-oxa-8-azaspiro[4.5]decan-4-yl)carbamate (40 mg, 70% yield) as a yellow solid.

$^1H$ NMR (400 MHz, $CDCl_3$) δ = 8.22 (d, J = 14.8 Hz, 1H), 7.82 (d, J = 8.0 Hz, 2H), 7.36 (d, J = 8.0 Hz, 2H), 7.18 - 7.14 (m, 1H), 6.32 (d, J = 7.2 Hz, 1H), 4.32 (dd, J = 2.4, 13.2 Hz, 1H), 4.26 - 4.22 (m, 1H), 4.20 - 4.12 (m, 1H), 3.96 (d, J = 5.2 Hz, 2H), 3.92 (d, J = 9.2 Hz, 1H), 3.82 - 3.74 (m, 1H), 3.58 - 3.52 (m, 1H), 3.38 (d, J = 10.4 Hz, 1H), 3.30 (s, 3H), 3.26 (s, 2H), 2.10 - 1.96 (m, 1H), 1.82 - 1.76 (m, 2H), 1.74 - 1.62 (m, 1H), 1.26 (s, 9H), 1.24 (t, J = 6.8 Hz, 3H), 1.12 (d, J = 6.4 Hz, 3H).

**Step 3: 7-((5-((3S,4S)-4-amino-3-methyl-2-oxa-8-azaspiro[4.5]decan-8-yl)pyrazin-2-yl)thio)-8-chloro-2-(2-methoxypropyl)isoquinolin-1(2H)-one**

The compound of Example 14 (15.3 mg, 50% yield) was synthesized in the same method as in Example 1.

$^1H$ NMR (400 MHz, METHANOL-$d_4$) δ= 8.30 (d, J = 1.2 Hz, 1H), 8.26 (s, 1H), 7.40 (d, J = 8.8Hz, 1H), 7.34 (d, J = 7.4 Hz, 1H), 7.16 (d, J = 8.8 Hz, 1H), 6.54 (d, J = 7.2 Hz, 1H), 4.29 - 4.26 (m, 1H), 4.24 - 4.20 (m, 1H), 4.18 - 4.13 (m, 2H), 3.92 (d, J = 9.2 Hz, 1H), 3.82 - 3.74 (m, 3H), 3.40 - 3.34 (m, 1H), 3.26 (s, 3H), 3.26 - 3.24 (m, 1H), 3.20 (d, J = 4.8 Hz, 1H), 1.88 - 1.78 (m, 3H), 1.74 - 1.66 (m, 1H), 1.26 (d, J = 6.4 Hz, 3H), 1.20 (d, J = 5.6 Hz, 3H); MS (EI) m/z: 530.3 $[M+H]^+$.

**Example 15: 7-((5-((3S,4S)-4-amino-3-methyl-2-oxa-8-azaspiro[4.5]decan-8-yl)pyrazin-2-yl)thio)-8-chloro-2-methylisoquinolin-1(2H)-one**

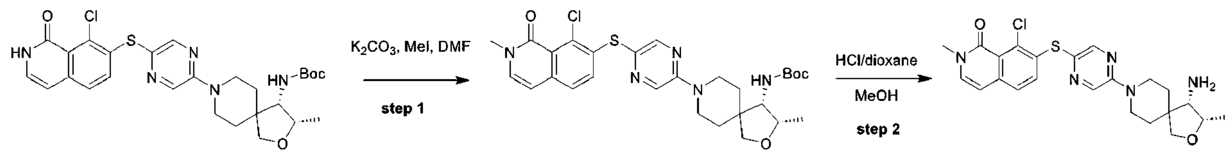


**Step 1: tert-butyl ((3S,4S)-8-(5-((8-chloro-2-methyl-1-oxo-1,2-dihydroisoquinolin-7-yl)thio)pyrazin-2-yl)-3-methyl-2-oxa-8-azaspiro[4.5]decan-4-yl)carbamate**

To a solution of tert-butyl ((3S,4S)-8-(5-((8-chloro-1-oxo-1,2-dihydroisoquinolin-7-yl)thio)pyrazin-2-yl)-3-methyl-2-oxa-8-azaspiro[4.5]decan-4-yl)carbamate (25.0 mg, 44.4 μmol) in DMF (2 mL), $K_2CO_3$ (15.3 mg, 111 μmol) and MeI (25.2 mg, 177 μmol) were added. The mixture was stirred at 25 °C for 12 hours. Upon completion of the reaction, the reaction mixture was partitioned between EA (30 mL) and $H_2O$ (20 mL). The organic layers were separated, washed with aqueous NaCl solution (20 mL), dried over $Na_2SO_4$, filtered and concentrated under reduced pressure to obtain a residue. The residue was purified by Prep-HPLC to obtain tert-butyl ((3S,4S)-8-(5-((8-chloro-2-methyl-1-oxo-1,2-dihydroisoquinolin-7-yl)thio)pyrazin-2-yl)-3-methyl-2-oxa-8-azaspiro[4.5]decan-4-yl)carbamate (6.00 mg, 23% yield) as a yellow solid.

$^1H$ NMR (400 MHz, $CDCl_3$) δ = 8.26 (d, J = 1.2 Hz, 1H), 8.21 - 8.17 (m, 1H), 7.25 - 7.23 (m, 1H), 7.12 (d, J = 8.4 Hz, 1H), 7.07 (d, J = 7.2 Hz, 1H), 6.35 (d, J = 7.2 Hz, 1H), 4.26 (s, 2H), 4.19 - 4.13 (m, 1H), 3.94 - 3.89 (m, 1H), 3.70 (d, J = 9.2 Hz, 1H), 3.57 (s, 3H), 3.55 - 3.50 (m, 1H), 3.24 - 3.14 (m, 2H), 2.07 - 1.97 (m, 2H), 1.78 - 1.70 (m, 2H), 1.27 (s, 9H), 1.25 - 1.23 (m, 3H).

**Step 2: 7-((5-((3S,4S)-4-amino-3-methyl-2-oxa-8-azaspiro[4.5]decan-8-yl)pyrazin-2-yl)thio)-8-chloro-2-methylisoquinolin-1(2H)-one**

The compound of Example 15 (3.12 mg, 37% yield) was synthesized in the same method as in Example 1.

$^1H$ NMR (400 MHz, MeOD) δ= 8.57 - 8.47 (m, 1H), 8.33 - 8.28 (m, 2H), 7.44 - 7.36 (m, 2H), 7.18 (d, J = 8.4 Hz, 1H), 6.58 (d, J = 7.2 Hz, 1H), 4.36 - 4.28 (m, 2H), 4.27 - 4.20 (m, 1H), 4.02 - 3.95 (m, 1H), 3.87 (d, J = 9.2 Hz, 1H), 3.58 (s, 3H), 3.38 - 3.35 (m, 2H), 3.28 - 3.21 (m, 1H), 1.91 - 1.81 (m, 3H), 1.77 - 1.69 (m, 1H), 1.32 (d, J = 6.4 Hz, 3H); MS (EI) m/z: 472.2 $[M+H]^+$.

**Example 16: (S)-7-((5-(6-amino-4,6-dihydrospiro[cyclopenta[d]thiazole-5,4'-pineridin]-1'-yl)pyrazin-2-yl)thio)-8-chloro-2-methylisoquinolin-1(2H)-one**

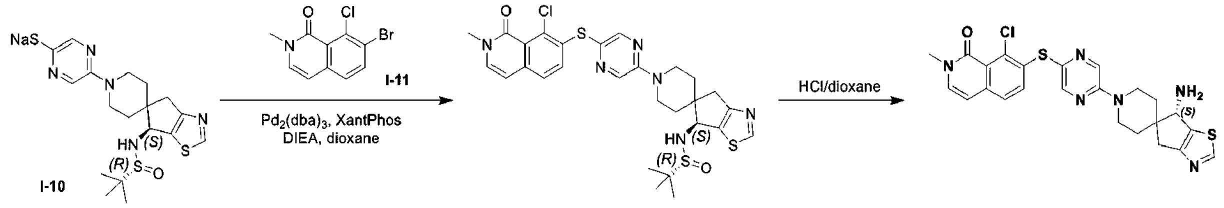


The compound of Example 16 (20.7 mg, 80% yield) was synthesized in the same method as in Example 14, using Intermediate I-11 instead of Intermediate I-9 and using Intermediate I-10 instead of Intermediate I-8 as starting materials in Step 1 of Example 14.

$^1$H NMR (400 MHz, MeOD) δ= 9.14 (s, 1H), 8.35 (d, J = 1.2 Hz, 1H), 8.29 (d, J = 1.2 Hz, 1H), 7.40 (dd, J = 8.0, 11.6 Hz, 2H), 7.17 (d, J = 8.0 Hz, 1H), 6.57 (d, J = 7.2 Hz, 1H), 4.54 - 4.48 (m, 1H), 4.48 - 4.45 (m, 1H), 4.37 (d, J = 14.2 Hz, 1H), 3.57 (s, 3H), 3.40-3.38 (m, 1H), 3.30 - 3.24 (m, 1H), 3.22 - 3.16 (m, 1H), 3.09 - 3.03 (m, 1H), 1.99 - 1.90 (m, 2H), 1.88 - 1.77 (m, 2H); MS (EI) m/z: 494.2 [M-NH$_2$]$^+$.

**Example 17: (S)-6-((5-(6-amino-4,6-dihydrospiro[cyclopenta[d]thiazole-5,4'-piperidin]-1'-yl)pyrazin-2-yl)thio)-3-methylquinazolin-4(3H)-one**

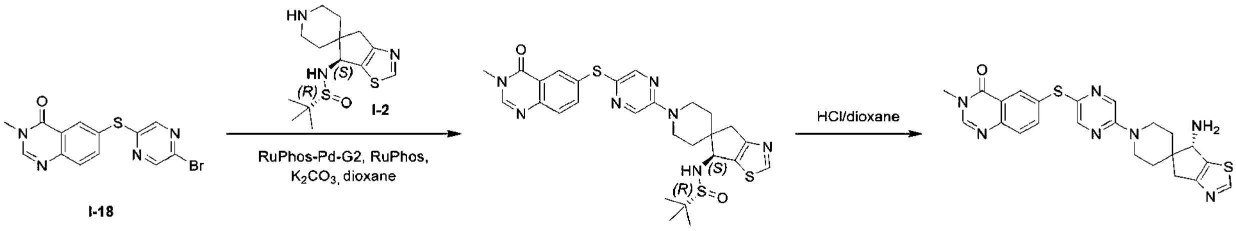


The compound of Example 17 (16 mg, 85% yield) was synthesized in the same method as in Example 6, using Intermediate 1-18 instead of Intermediate I-14 and using Intermediate I-2 instead of Intermediate I-12 as starting materials in Step 1 of Example 6.

$^1$H NMR (400 MHz, CDCl$_3$) δ= 8.78 (s, 1H), 8.23 (d, J = 1.4 Hz, 1H), 8.19 - 8.16 (m, 1H), 8.15 (d, J = 1.9 Hz, 1H), 8.07 - 8.05 (m, 1H), 7.71 - 7.66 (m, 1H), 7.64 - 7.60 (m, 1H), 4.29 - 4.22 (m, 1H), 4.20 - 4.12 (m, 2H), 3.59 - 3.56 (m, 3H), 3.39 - 3.25 (m, 2H), 3.03 - 2.89 (m, 2H), 1.84 - 1.78 (m, 4H); MS (EI) m/z: 461.1 [M-NH$_2$]$^+$.

**Example 18: 6-((5-((3S,4S)-4-amino-3-methyl-2-oxa-8-azaspiro[4.5]decan-8-yl)pyrazin-2-yl)thio)-5-chloro-3-(2-methoxy-2-methylpropyl)quinazolin-4(3H)-one**

[Synthesis Method 1]

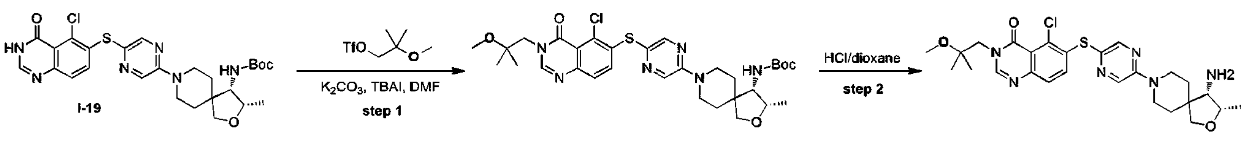


**Step 1: tert-butyl ((3S,4S)-8-(5-((5-chloro-3-(2-methoxy-2-methylpropyl)-4-oxo-3,4-dihydroquinazolin-6-yl)thio) pyrazin-2-yl)-3-methyl-2-oxa-8-azaspiro[4.5]decan-4-yl)carbamate**

To a mixture of **Intermediate I-19** (300 mg, 532 μmol), 2-methoxy-2-methylpropyl trifluoromethanesulfonate (136 mg, 586 μmol), TBAI (19.6 mg, 53.2 μmol) and K$_2$CO$_3$ (220 mg, 1.60 mmol) in DMF (1 mL), TBAI (19.6 mg, 53.2 μmol) was added at 25 °C. The mixture was stirred at 80 °C for 16 hours. Upon completion of the reaction, the mixture was filtered, and the filtrate was concentrated. The residue was purified by reverse phase HPLC (0.1% FA condition) to obtain tert-butyl ((3S,4S)-8-(5-((5-chloro-3-(2-methoxy-2-methylpropyl)-4-oxo-3,4-dihydroquinazolin-6-yl)thio)pyrazin-2-yl)-3-methyl-2-oxa-8-azaspiro[4.5]decan-4-yl)carbamate (300 mg, 87% yield) as a yellow solid.

$^1H$ NMR (400 MHz, $CDCl_3$) δ = 8.27 (d, *J* = 1.2 Hz, 1H), 8.19 (d, *J* = 1.2 Hz, 1H), 8.15 (s, 1H), 7.47 (d, *J* = 8.8 Hz, 1H), 7.25 (s, 1H), 4.64 (d, *J* = 10.8 Hz, 1H), 4.24 - 4.16 (m, 1H), 4.07 (s, 2H), 4.02 (dd, *J* = 4.4, 10.8 Hz, 1H), 3.86 (dd, *J* = 3.6, 9.6 Hz, 1H), 3.82 - 3.76 (m, 1H), 3.73 - 3.67 (m, 2H), 3.66 - 3.60 (m, 1H), 3.56 - 3.47 (m, 1H), 1.94 - 1.68 (m, 4H), 1.46 (s, 9H), 1.23 (s, 3H), 1.21 (s, 6H).

**Step 2: 6-((5-((3S,4S)-4-amino-3-methyl-2-oxa-8-azaspiro[4.5]decan-8-yl)pyrazin-2-yl)thio)-5-chloro-3-(2-methoxy-2-methylpropyl)quinazolin-4(3H)-one**

**The compound of Example 18** (27.1 mg, 93% yield) was synthesized in the same method as in Example 1.
$^1H$ NMR (400 MHz, $CDCl_3$) δ= 8.26 (s, 1H), 8.21 - 8.18 (m, 1H), 8.15 (s, 1H), 7.42 (d, J = 8.8 Hz, 1H), 7.21 (d, J = 8.8 Hz, 1H), 4.28 - 4.20 (m, 2H), 4.20 - 4.17 (m, 1H), 4.06 (s, 2H), 3.98 (d, J = 9.2 Hz, 1H), 3.79 (d, J = 9.2 Hz, 1H), 3.27 (d, J = 4.4 Hz, 1H), 3.21 (s, 3H), 3.16 - 3.14 (m, 1H), 3.13 - 3.05 (m, 1H), 1.97 - 1.75 (m, 4H), 1.33 (d, J = 6.4 Hz, 3H), 1.21 (s, 6H).
MS (EI) m/z: 545.3 $[M+H]^+$.

**[Synthesis Method 2]**

**The compound of Example 18** (88.5 mg, 57% yield) was synthesized in the same method as in Example 25, using **Intermediate I-24** instead of **Intermediate I-26** and using **Intermediate I-29** instead of **Intermediate I-28** as starting materials in Step 1 of Example 25.
$^1H$ NMR (400 MHz, $CDCl_3$) δ = 8.27 (d, *J* = 1.2 Hz, 1H), 8.21 (d, *J* = 1.2 Hz, 1H), 8.15 (s, 1H), 7.45 (d, *J* = 8.8 Hz, 1H), 7.28 - 7.26 (m, 1H), 4.25 - 4.17 (m, 1H), 4.07 (s, 2H), 4.02 - 3.89 (m, 2H), 3.84 (d, *J* = 8.8 Hz, 1H), 3.71 (d, *J* = 8.8 Hz, 1H), 3.55 - 3.45 (m, 1H), 3.44 - 3.35 (m, 1H), 3.21 (s, 3H), 3.02 (d, *J* = 4.4 Hz, 1H), 1.97 - 1.87 (m, 1H), 1.85 - 1.67 (m, 3H), 1.26 (d, *J* = 6.4 Hz, 3H), 1.21 (s, 6H).
MS (EI) m/z: 545.3 $[M+H]^+$.

**Examples 19 to 21**

The compounds of Examples 19 to 21 were prepared in the same method as in Example 18, except that a compound having an appropriate trifluoromethanesulfonate functional group was used instead of 2-methoxy-2-methylpropyl trifluoromethanesulfonate in Synthesis Method 1 of Example 18.

[Table 4]

| No. | Structure/Name | Spectral Data |
|---|---|---|
| 19 | 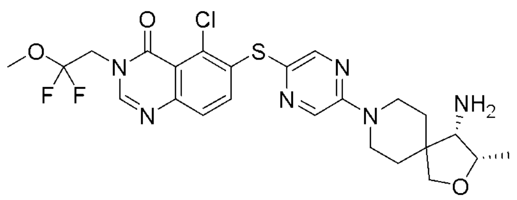 6-((5-((3S,4S)-4-amino-3-methyl-2-oxa-8-azaspiro[4.5]decan-8-yl) pyrazin-2-yl)thio)-5-chloro-3-(2,2-difluoro-2-methoxyethyl)quinazolin-4(3H)-one | MS m/z: 553.1 $[M+H]^+$. |
| 20 | 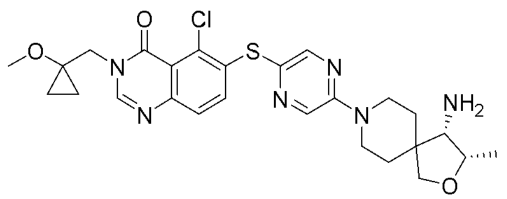 6-((5-((3S,4S)-4-amino-3-methyl-2-oxa-8-azaspiro[4.5]decan-8-yl) pyrazin-2-yl)thio)-5-chloro-3-((1-methoxycyclopropyl)methyl)quinazolin-4(3H)-one | MS m/z: 543.1 $[M+H]^+$. |

(continued)

| No. | Structure/Name | Spectral Data |
| --- | --- | --- |
| 21 | 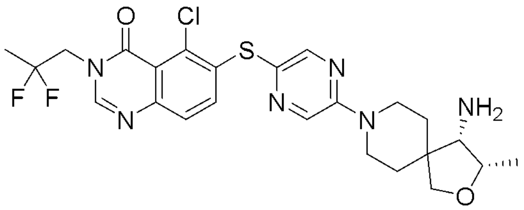 6-((5-((3S,4S)-4-amino-3-methyl-2-oxa-8-azaspiro[4.5]decan-8-yl) pyrazin-2-yl)thio)-5-chloro-3-(2,2-difluoropropyl)quinazolin-4(3H)-one | $^1$H NMR (500 MHz, DMSO) δ 8.49 (s, 1H), 8.30 (d, J = 21.8 Hz, 2H), 7.98 (s, 3H), 7.54 (d, J = 8.8 Hz, 1H), 7.27 (d, J = 8.8 Hz, 1H), 4.53 (t, J = 14.3 Hz, 2H), 4.27 - 4.18 (m, 3H), 3.90 (d, J = 9.1 Hz, 2H), 3.16 (dd, J = 24.3, 13.0 Hz, 3H), 1.75-1.67 (m, 6H), 1.59 (d, J = 12.5 Hz, 1H), 1.22 (d, J = 6.5 Hz, 3H). MS m/z: 538.0 [M+H]$^+$. |

**Example 22: (R)-6-((5-(3-amino-3H-spiro[benzofuran-2,4'-piperidin]-1'-yl)pyrazin-2-yl)thio)-5-chloro-3-(2-methoxyethyl)quinazolin-4(3H)-one**

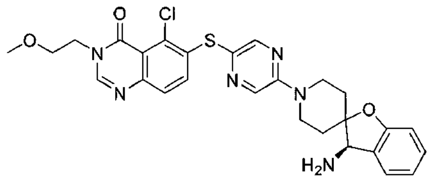

The compound of Example 22 (27 mg, 58% yield) was synthesized in the same method as in Example 18, using Intermediate I-20 instead of Intermediate I-19 and using 2-bromoethyl methyl ether instead of 2-methoxy-2-methylpropyl trifluoromethanesulfonate as starting materials in Step 1 of Example 18.

$^1$H NMR (500 MHz, DMSO) δ 8.67(bs, 2H), 8.57(s, 1H), 8.36(s, 1H), 8.29(s, 1H), 7.63(d, J = 7.4 Hz, 1H), 7.53(d, J = 8.8 Hz, 1H), 7.37(t, J = 8.0 Hz, 1H), 7.27(d, J = 8.8 Hz, 2H), 7.04-6.98(m, 2H), 4.68(d, J = 4.7 Hz, 1H), 4.56-4.53(m, 1H), 4.41-4.39(m, 1H), 4.13(t, J = 5.0 Hz, 2H), 3.61(t, J = 5.0 Hz, 2H), 3.26(s, 3H), 2.13-2.00(m, 2H), 1.91-1.80(m, 2H); MS m/z: 552.0 [M+H]$^+$.

**Example 23: 6-((5-((3S,4S)-4-amino-3-methyl-2-oxa-8-azaspiro[4.5]decan-8-yl)pyrazin-2-yl)thio)-5-chloro-3-((4-methyltetrahydro-2H-pyran-4-yl)methyl)quinazolin-4(3H)-one**

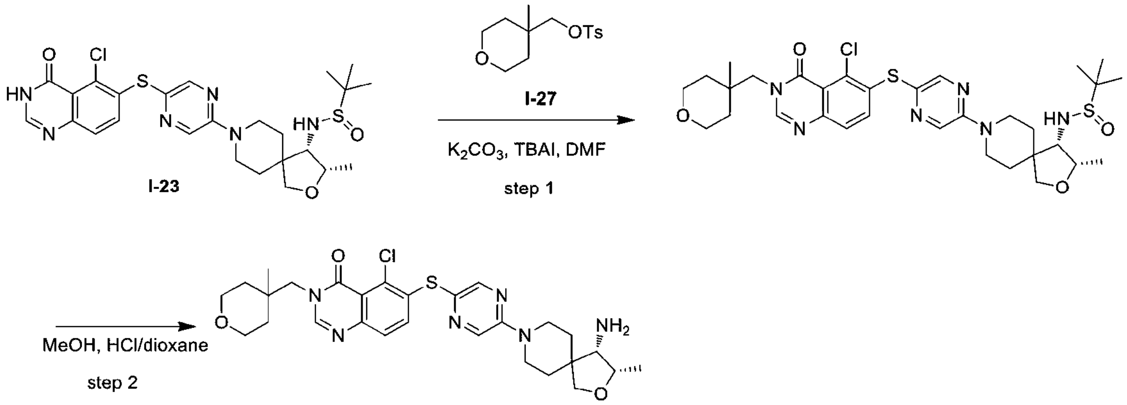

**Step 1: N-((3S,4S)-8-(5-((5-chloro-3-((4-methyltetrahydro-2H-pyran-4-yl)methyl)-4-oxo-3,4-dihydroquinazolin-6-yl)thio)pyrazin-2-yl)-3-methyl-2-oxa-8-azaspiro[4.5]decan-4-yl)-2-methylpropane-2-sulfinamide**

To a solution of **Intermediate I-23** (100 mg, 177 μmol) and **Intermediate I-27** (101 mg, 355 μmol) in DMF (6 mL), $K_2CO_3$ (73.6 mg, 532 μmol) and TBAI (9.84 mg, 26.6 μmol) were added. The mixture was stirred at 100°C for 12 hours. Upon completion of stirring, the reaction mixture was partitioned between EA (40 mL) and $H_2O$ (20 mL). The organic layers were separated, washed with aqueous NaCl solution (20 mL), dried over $Na_2SO_4$, filtered and concentrated under reduced pressure to obtain a residue. The residue was purified by Prep-HPLC (column: Phenomenex luna C18 150*25mm*10 μm; mobile phase: [water(FA)-ACN]; concentration gradient: 41%-71% B over 10 min) to obtain N-[(3S,4S)-8-[5-[5-chloro-3-[(4-methyltetrahydropyran-4-yl)methyl]-4-oxo-quinazolin-6-yl]sulfanylpyrazin-2-yl]-3-methyl-2-oxa-8-azaspiro[4.5]decan-4-yl]-2-methyl-propane-2-sulfinamide (40.0 mg, 33% yield) as a white solid.

$^1H$ NMR (400 MHz, $CDCl_3$) δ = 8.19 (d, *J* = 1.2 Hz, 1H), 8.12 (d, *J* = 1.2 Hz, 1H), 7.85 (s, 1H), 7.38 (d, *J* = 8.8 Hz, 1H), 7.19 - 7.16 (m, 1H), 4.22 - 4.14 (m, 2H), 4.10 (dd, *J* = 4.0, 13.6 Hz, 1H), 3.88 - 3.86 (m, 2H), 3.85 (s, 1H), 3.78 - 3.73 (m, 2H), 3.64 - 3.54 (m, 3H), 3.46 (dd, *J* = 5.6, 10.4 Hz, 1H), 3.30 (d, *J* = 10.4 Hz, 1H), 3.18 - 3.13 (m, 1H), 3.12 - 3.07 (m, 1H), 2.03 - 1.96 (m, 1H), 1.95 - 1.86 (m, 1H), 1.70 - 1.61 (m, 1H), 1.62 - 1.57 (m, 1H), 1.30 (d, *J* = 13.6 Hz, 2H), 1.20 - 1.19 (m, 9H), 1.16 (d, *J* = 6.4 Hz, 3H), 1.03 (s, 3H).

**Step 2: 6-((5-((3S,4S)-4-amino-3-methyl-2-oxa-8-azaspiro[4.5]decan-8-yl)pyrazin-2-yl)thio)-5-chloro-3-((4-methyltetrahydro-2H-pyran-4-yl)methyl)quinazolin-4(3H)-one**

To a solution of N-[(3S,4S)-8-[5-[5-chloro-3-[(4-methyltetrahydropyran-4-yl)methyl]-4-oxo-quinazolin-6-yl]sulfanylpyrazin-2-yl]-3-methyl-2-oxa-8-azaspiro[4.5]decan-4-yl]-2-methylpropane-2-sulfinamide (40.0 mg, 59.2 μmol) in MeOH (4 mL), HCl/dioxane (4M, 1 mL) was added. The mixture was stirred at 25°C for 0.5 hours. Upon completion of stirring, the reaction mixture was filtered and concentrated under reduced pressure to obtain a residue. The residue was purified by Prep-HPLC (column: Phenomenex luna C18 150*25mm*10 μm; mobile phase: [water(FA)-ACN]; concentration gradient: 18%-48% B over 7 min) to obtain **the compound of Example 23** (24.6 mg, 69% yield) as a white solid.

MS (EI) m/z: $[M+H]^+$ 571.2.

$^1H$ NMR (400 MHz, MeOD) δ = 8.35 (d, *J* = 1.2 Hz, 1H), 8.31 (d, *J* = 1.2 Hz, 1H), 8.24 (s, 1H), 7.49 (d, *J* = 8.8 Hz, 1H), 7.30 (d, *J* = 8.8 Hz, 1H), 4.38 - 4.30 (m, 2H), 4.30 - 4.23 (m, 1H), 4.04 (s, 2H), 4.02 - 3.98 (m, 1H), 3.89 (d, *J* = *9.2* Hz, 1H), 3.83 (d, *J* = 4.4, 11.6 Hz, 2H), 3.72 - 3.65 (m, 2H), 3.40 (d, *J* = 4.4 Hz, 1H), 3.31 - 3.19 (m, 2H), 1.91 - 1.87 (m, 2H), 1.86 - 1.81 (m, 1H), 1.75 (s, 1H), 1.71 - 1.63 (m, 2H), 1.42 (d, *J* = 2.8 Hz, 1H), 1.38 (s, 1H), 1.33 (d, *J* = 6.4 Hz, 3H), 1.11 (s, 3H).

**Example 24: 3-(6-((5-((3S,4S)-4-amino-3-methyl-2-oxa-8-azaspiro[4.5]decan-8: yl)pyrazin-2-yl)thio)-5-chloro-4-oxoquinazolin-3(4H)-yl)propanenitrile**

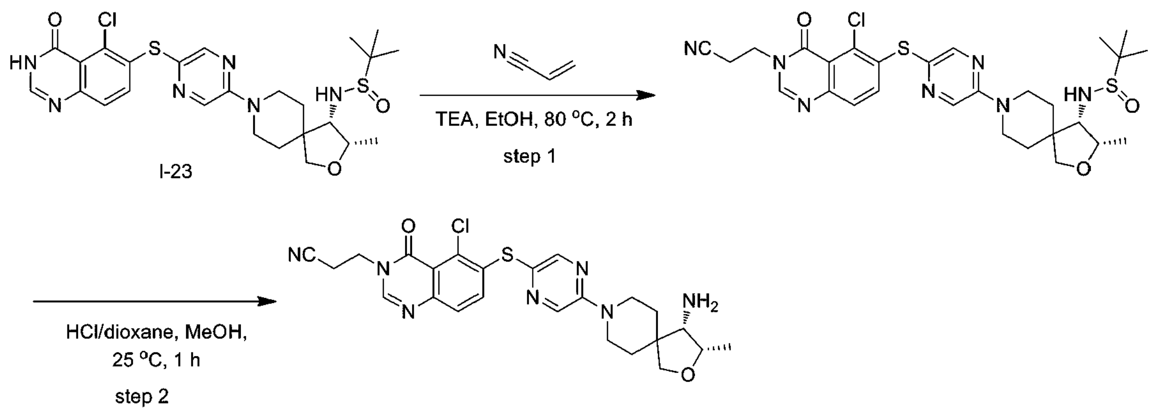


**Step 1: N-((3S,4S)-8-(5-((5-chloro-3-(2-cyanoethyl)-4-oxo-3,4-dihydroquinazolin-6-yl)thio)pyrazin-2-yl)-3-methyl-2-oxa-8-azaspiro[4.5]decan-4-yl)-2-methylpropane-2-sulfinamide**

To a solution of **Intermediate I-23** (50 mg, 88.7 μmol) and TEA (44.9 mg, 443 μmol) in EtOH (1 mL), prop-2-ennitrile (47.1 mg, 887 μmol) was added. The reaction mixture was stirred at 80°C for 2 hours. Upon completion of stirring, the reaction mixture was added with water (20 ml) and extracted with EA (3 x 30 ml). The combined organic layers were dried under reduced pressure. The residue was purified by Prep-HPLC (column: Phenomenex luna C18 150*25mm*10

μm; mobile phase: [water(FA)-ACN]; concentration gradient: 28%-68% B over 10 min) to obtain N-[(3S,4S)-8-[5-[5-chloro-3-(2-cyanoethyl)-4-oxo-quinazolin-6-yl]sulfanylpyrazin-2-yl]-3-methyl-2-oxa-8-azaspiro[4.5]decan-4-yl]-2-methyl-propane-2-sulfinamide (45 mg, 85% yield) as a white solid.

$^1$H NMR (400 MHz, DMSO-$d_6$) δ = 8.48 (d, *J* = 1.2 Hz, 1H), 8.40 (s, 1H), 8.30 (d, *J* = 1.2 Hz, 1H), 7.53 (d, *J* = 8.8 Hz, 1H), 7.21 (d, *J* = 8.8 Hz, 1H), 5.13 (d, *J* = 12.0 Hz, 1H), 4.24 - 4.20 (m, 3H), 4.19 - 4.07 (m, 2H), 3.89 (d, *J* = 8.8 Hz, 1H), 3.52 (d, *J* = 8.8 Hz, 1H), 3.46 - 3.40 (m, 1H), 3.23 - 3.12 (m, 2H), 3.03 (t, *J* = 6.4 Hz, 2H), 1.85 - 1.71 (m, 2H), 1.68 - 1.53 (m, 2H), 1.16 (s, 9H), 1.10 (d, *J* = 6.4 Hz, 3H).

**Step 2: 3-(6-((5-((3S,4S)-4-amino-3-methyl-2-oxa-8-azaspiro[4.5]decan-8-yl)pyrazin-2-yl)thio)-5-chloro-4-oxo-quinazolin-3(4H)-yl)propanenitrile**

To a solution of N-[(3S,4S)-8-[5-[5-chloro-3-(2-cyanoethyl)-4-oxo-quinazolin-6-yl]sulfanylpyrazin-2-yl]-3-methyl-2-oxa-8-azaspiro[4.5]decan-4-yl]-2-methyl-propane-2-sulfinamide (60 mg, 97.3 μmol) in MeOH (2 mL), HCl/dioxane (0.5 mL) was added. The reaction mixture was stirred at 25°C for 1 hour. Upon completion of stirring, the reaction mixture was concentrated under reduced pressure. The residue was purified by reverse phase HPLC (0.1% FA) to obtain **the compound of Example 24** (42.92 mg, 78% yield) as a white solid.

MS (EI) m/z: [M+H]$^+$ 512.3.

$^1$H NMR (400 MHz, DMSO-$d_6$) δ = 8.47 (s, 1H), 8.41 (s, 1H), 8.30 (d, *J* = 1.2 Hz, 1H), 8.24 (s, 1H), 7.53 (d, *J* = 8.8 Hz, 1H), 7.23 (d, *J* = 8.8 Hz, 1H), 4.24 - 4.20 (m 2H), 4.12 - 4.07 (m, 1H), 4.01 - 3.91 (m, 3H), 3.74 - 3.70 (m, 2H), 3.55 - 3.46 (m, 2H), 3.39 - 3.34 (m, 1H), 3.07 - 3.00 (m, 3H), 1.78 - 1.65 (m, 2H), 1.61 - 1.49 (m, 2H), 1.10 (d, *J* = 6.4 Hz, 3H).

**Example 25: (S)-6-((5-(1-amino-1,3-dihydrospiro[indene-2,4'-piperidin]-1'-yl)pyrazin-2-yl)thio)-5-chloro-3-(2-hydroxy-2-methylpropyl)quinazolin-4(3H)-one**

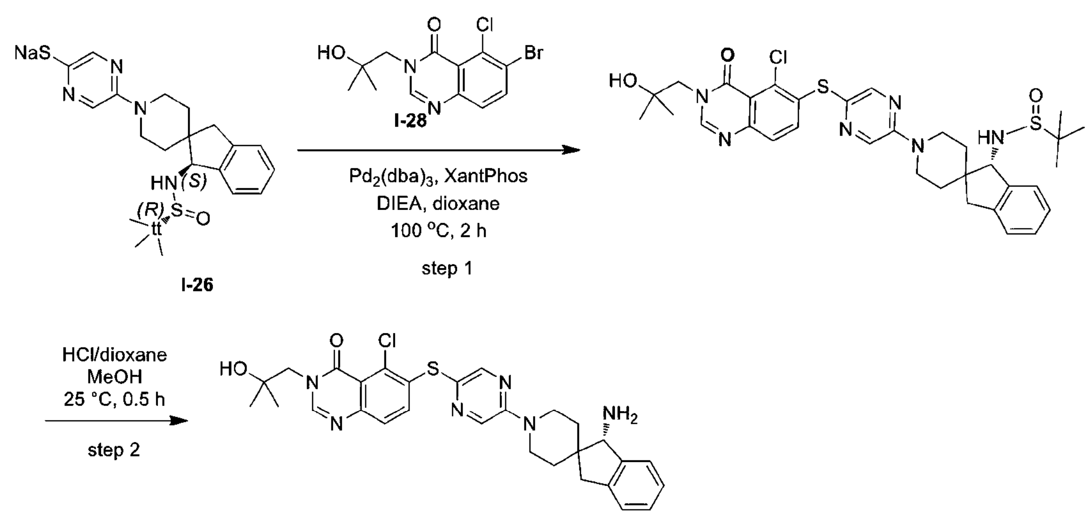


**Step 1: N-((S)-1'-(5-((5-chloro-3-(2-hydroxy-2-methylpropyl)-4-oxo-3,4-dihydroquinazolin-6-yl)thio)pyrazin-2-yl)-1,3-dihydrospiro[indene-2,4'-piperidin]-1-yl)-2-methylpropane-2-sulfinamide**

A mixture of **Intermediate I-28** (75.0 mg, 226 μmol), **Intermediate I-26** (99.2 mg, 226 μmol), DIEA (87.7 mg, 678 μmol), Xantphos (26.1 mg, 45.2 μmol) and $Pd_2(dba)_3$ (31.0 mg, 33.9 μmol) in dioxane (5 mL) was degassed and purged 3 times with $N_2$, and then the mixture was stirred under $N_2$ atmosphere at 100°C for 2 hours. Upon completion of stirring, the reaction mixture was filtered, and the filtrate was concentrated under reduced pressure to obtain a residue. The residue was purified by Prep-TLC (petroleum ether/ethyl acetate = 0/1) to obtain N-[(1S)-1'-[5-[5-chloro-3-(2-hydroxy-2-methylpropyl)-4-oxo-quinazolin-6-yl]sulfanylpyrazin-2-yl]spiro[indane-2,4'-piperidin]-1-yl]-2-methyl-propane-2-sulfinamide (90.0 mg, 60% yield) as a white solid.

MS (EI) m/z: [M+H]$^+$ 667.2.

$^1$H NMR (400 MHz, DMSO-$d_6$) δ = 8.51 (s, 1H), 8.32 (s, 1H), 8.22 (s, 1H), 7.51 (d, *J* = 8.8 Hz, 1H), 7.28 - 7.18 (m, 5H), 5.69 (d, *J* = 10.8 Hz, 1H), 4.82 (s, 1H), 4.47 (s, 1H), 4.49 - 4.43 (m, 1H), 4.39 (d, *J* = 12.8 Hz, 2H), 4.12 - 4.08 (m, 2H), 3.96 (s, 1H), 3.14 - 3.10 (m, 1H), 2.78 - 2.72 (m, 1H), 2.15 - 2.06 (m, 1H), 1.78 - 1.64 (m, 2H), 1.33 - 1.28 (m, 1H), 1.24 - 1.19 (m, 9H), 1.12 (s, 6H).

**Step 2: (S)-6-((5-(1-amino-1,3-dihydrospiro[indene-2,4'-piperidin]-1'-yl)pyrazin-2-yl)thio)-5-chloro-3-(2-hydroxy-2-methylpropyl)quinazolin-4(3H)-one**

To a solution of N-[(1S)-1'-[5-[5-chloro-3-(2-hydroxy-2-methyl-propyl)-4-oxo-quinazolin-6-yl]sulfanylpyrazin-2-yl]spiro[indane-2,4'-piperidin]-1-yl]-2-methyl-propane-2-sulfinamide (120 mg, 179 μmol) in MeOH (5 mL), HCl/dioxane (3 mL) was added. The mixture was stirred at 25°C for 0.5 hours. Upon completion of stirring, the reaction mixture was concentrated under reduced pressure to obtain a residue. The residue was purified by Prep-HPLC (column: C18 150×30mm; mobile phase: [water(FA)-ACN]; concentration gradient: 10%-40% B over 7 min) to obtain **the compound of Example 25** (84.7 mg, 80% yield) as a white solid.

MS (EI) m/z: $[M+H]^+$ 563.3.

$^1H$ NMR (400 MHz, DMSO-$d_6$) δ = 8.48 (d, *J* = 1.2 Hz, 1H), 8.30 (d, *J* = 1.2 Hz, 1H), 8.21 (d, *J* = 4.4 Hz, 2H), 7.52 (d, *J* = 8.8 Hz, 1H), 7.37 - 7.34 (m, 1H), 7.23 (s, 1H), 7.21 (d, *J* = 3.6 Hz, 3H), 4.81 (s, 1H), 4.31 (s, 1H), 4.28 (d, *J* = 1.6 Hz, 1H), 3.97 (s, 1H), 3.95 (s, 2H), 3.13-3.09 (m, 2H), 2.73 (d, *J* = 15.6 Hz, 1H), 1.83 - 1.77 (m, 1H), 1.72 - 1.66 (m, 1H), 1.55 (d, *J* = 13.6 Hz, 1H), 1.25 - 1.21 (m, 1H), 1.11 (s, 6H).

**Example 26: 6-((5-((3S,4S)-4-amino-3-methyl-2-oxa-8-azaspiro[4.5]decan-8-yl)pyrazin-2-yl)thio)-5-chloro-3-((1-methylpiperidin-4-yl)methyl)quinazolin-4(3H)-one**

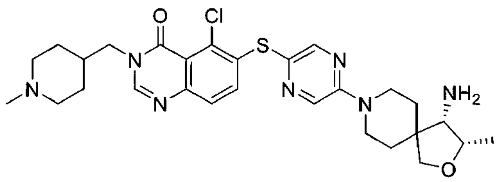

The compound of Example 26 (33.3 mg, 78% yield) was synthesized in the same method as in Example 25, using **Intermediate I-24** instead of **Intermediate I-26** and using **Intermediate I-34** instead of **Intermediate I-28** as starting materials in Step 1 of Example 25.

MS (EI) m/z: $[M+H]^+$ 570.1, $^1H$ NMR (400 MHz, MeOD) δ = 8.52 - 8.50 (m, 1H), 8.35 - 8.32 (m, 1H), 8.28 (s, 1H), 8.26 (s, 1H), 7.44 (d, *J* = 8.8 Hz, 1H), 7.23 (d, *J* = 8.8 Hz, 1H), 4.37 - 4.21 (m, 3H), 4.02 - 3.94 (m, 3H), 3.86 (d, *J* = 9.2 Hz, 1H), 3.50 - 3.43 (m, 2H), 3.41 (d, *J* = 4.2 Hz, 1H), 3.30 - 3.16 (m, 2H), 2.91 (t, *J* = 11.6 Hz, 2H), 2.80 (s, 3H), 2.24 - 2.12 (m, 1H), 1.94 (d, *J* = 13.6 Hz, 2H), 1.90 - 1.80 (m, 3H), 1.78 - 1.56 (m, 3H), 1.31 (d, *J* = 6.4 Hz, 3H).

**Example 27: (S)-6-((5-(1-amino-1,3-dihydrospiro[indene-2,4'-piperidin]-1'-yl)pyrazin-2-yl)thio)-5-chloro-3-(2-methoxy-2-methylpropyl)quinazolin-4(3H)-one**

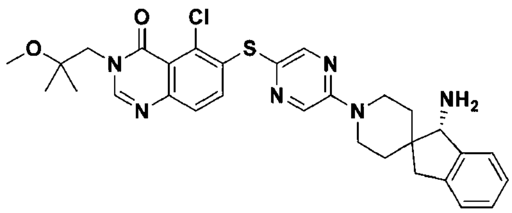

**The compound of Example 27** (45 mg, 65% yield) was synthesized in the same method as in Example 25, using **Intermediate I-29** instead of **Intermediate I-28** as a starting material in Step 1 of Example 25.

MS (EI) m/z: $[M+H]^+$ 577.3

$^1H$ NMR (400 MHz, $CDCl_3$) δ = 8.28 (d, *J* = 1.2 Hz, 1H), 8.23 (d, *J* = 1.2 Hz, 1H), 8.15 (s, 1H), 7.46 (d, *J* = 8.8 Hz, 1H), 7.34 (d, *J* = 5.6 Hz, 1H), 7.29 (s, 1H), 7.26 - 7.21 (m, 3H), 4.32 - 4.21 (m, 2H), 4.07 (s, 2H), 4.01 (s, 1H), 3.34 - 3.23 (m, 2H), 3.21 (s, 3H), 3.12 (d, *J* = 15.6 Hz, 1H), 2.76 (d, *J* = 15.6 Hz, 1H), 1.96 - 1.78 (m, 2H), 1.67-1.63 (m, 1H), 1.45 - 1.40 (m, 1H), 1.22 (s, 6H).

**Example 28: 6-((5-((3S,4S)-4-amino-3-methyl-2-oxa-8-azaspiro[4.5]decan-8-yl)pyrazin-2-yl)thio)-5-chloro-3-(1-fluoro-2-methoxy-2-methylpropyl)quinazolin-4(3H)-one**

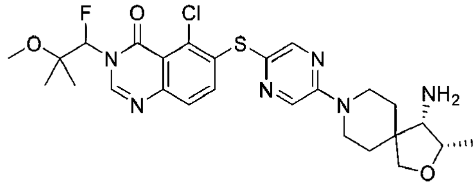

The compound of Example 28 (11.38 mg, 44% yield) was synthesized in the same method as in Example 25, using **Intermediate I-30** instead of **Intermediate I-28** and **Intermediate I-24** instead of **Intermediate I-26** as starting materials in Step 1 of Example 25.

MS (EI) m/z: $[M+H]^+$ 563.3.

$^1H$ NMR (400 MHz, MeOD) δ = 8.37 (s, 1H), 8.32 (s, 1H), 8.29 (s, 1H), 7.48 (d, *J* = 8.8 Hz, 1H), 7.28 (d, *J* = 8.8 Hz, 1H), 6.79 - 6.61 (m, 1H), 4.30 - 4.23 (m, 1H), 4.22 - 4.08 (m, 2H), 3.92 (d, *J* = 8.8 Hz, 1H), 3.77 (d, *J* = 8.8 Hz, 1H), 3.48 - 3.34 (m, 2H), 3.34 (s, 3H), 3.20 - 3.12 (m, 1H), 1.90 - 1.69 (m, 4H), 1.49 (s, 3H), 1.25 (d, *J* = 6.4 Hz, 3H), 1.14 (d, *J* = 1.6 Hz, 3H)

**Example 29: (S)-6-((5-(1-amino-1,3-dihydrospiro[indene-2,4'-piperidin]-1'-yl)pyrazin-2-yl)thio)-3-(2-methoxy-2-methylpropyl)quinazolin-4(3H)-one**

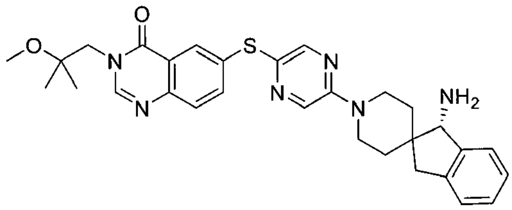

**The compound of Example 29** (60.3 mg, 58% yield) was synthesized in the same method as in Example 25, using **Intermediate I-31** instead of **Intermediate I-28** as a starting material in Step 1 of Example 25.

MS (EI) m/z: $[M+H]^+$ 543.5.

$^1H$ NMR (400 MHz, $CDCl_3$) δ = 8.23 - 8.21 (m, 1H), 8.21 - 8.18 (m, 1H), 8.18 - 8.13 (m, 2H), 7.70 - 7.64 (m, 1H), 7.64 - 7.59 (m, 1H), 7.40 - 7.32 (m, 1H), 7.26 - 7.21 (m, 3H), 4.26 - 4.11 (m, 2H), 4.09 - 4.04 (m, 2H), 4.03 (s, 1H), 3.31 - 3.22 (m, 2H), 3.21 (s, 3H), 3.15 - 3.06 (m, 1H), 2.81 - 2.73 (m, 1H), 1.90 - 1.80 (m, 2H), 1.80 - 1.77 (m, 1H), 1.46 - 1.40 (m, 1H), 1.20 (s, 6H).

**Example 30: (S)-6-((5-(1-amino-1,3-dihydrospiro[indene-2,4'-piperidin]-1'-yl)pyrazin-2-yl)thio)-5-chloro-3-phenylquinazolin-4(3H)-one**

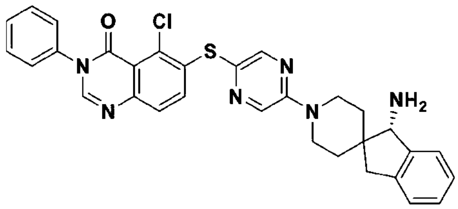

**The compound of Example 30** (101 mg, 79% yield) was synthesized in the same method as in Example 25, using **Intermediate I-32** instead of **Intermediate I-28** as a starting material in Step 1 of Example 25.

m/z ES+ $[M+H]^+$ 567.1

$^1$H NMR (400 MHz, $CDCl_3$) δ = 8.31 (d, *J* = 1.2 Hz, 1H), 8.25 (s, 1H), 8.06 (s, 1H), 7.57 - 7.49 (m, 5H), 7.44 - 7.40 (m, 2H), 7.33 - 7.27 (m, 3H), 7.27 - 7.25 (m, 1H), 4.25 - 4.15 (m, 3H), 3.36 - 3.25 (m, 2H), 3.22 (s, 1H), 2.89 (d, *J* = 16.0 Hz, 1H), 1.93 - 1.75 (m, 2H), 1.65 - 1.57 (m, 2H).

**Example 31: (S)-6-((5-(1-amino-1,3-dihydrospiro[indene-2,4'-piperidin]-1'-yl)pyrazin-2-yl)thio)-5-chloro-3-(pyridin-3-yl)quinazolin-4(3H)-one**

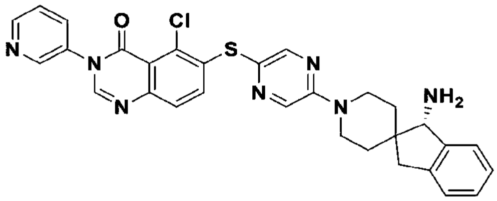

**The compound of Example 31** (45 mg, 52% yield) was synthesized in the same method as in Example 25, using **Intermediate I-32** instead of **Intermediate I-28** as a starting material in Step 1 of Example 25.

m/z ES+ $[M+H]^+$ 568.3.

$^1$H NMR (400 MHz, $CDCl_3$) δ = 8.31 (d, *J* = 1.2 Hz, 1H), 8.25 (s, 1H), 8.06 (s, 1H), 7.57 - 7.49 (m, 5H), 7.44 - 7.40 (m, 2H), 7.33 - 7.27 (m, 3H), 7.27 - 7.25 (m, 1H), 4.25 - 4.15 (m, 3H), 3.36 - 3.25 (m, 2H), 3.22 - 3.19 (m, 1H), 2.91 - 2.86 (m, 1H), 1.93 - 1.75 (m, 2H), 1.64 - 1.59 (m, 2H).

**Example 32: (S)-6-((5-(6-amino-4,6-dihydrospiro[cyclopenta[d]thiazole-5,4'-piperidin]-1'-yl)pyrazin-2-yl)thio)-5-chloro-3-(2-methoxy-2-methylpropyl)quinazolin-4(3H)-one**

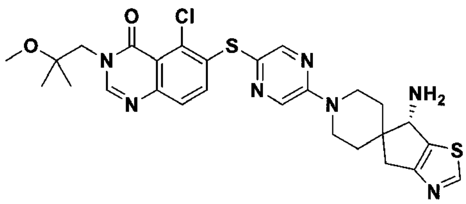

**The compound of Example 32** (55 mg, 58% yield) was synthesized in the same method as in Example 29, using **Intermediate I-36** instead of Intermediate **I-26** as a starting material in Step 1 of Example 29.

m/z ES+ $[M+H]^+$ 584.2;

$^1$H NMR (400 MHz, $CDCl_3$) δ = 8.78 (s, 1H), 8.28 (s, 1H), 8.24 (s, 1H), 8.15 (s, 1H), 7.46 (d, *J* = 8.8 Hz, 1H), 7.29 (d, *J* = 8.8 Hz, 1H), 4.29 (d, *J* = 13.2 Hz, 1H), 4.23 - 4.16 (m, 2H), 4.07 (s, 2H), 3.41 - 3.27 (m, 2H), 3.21 (s, 3H), 3.05 - 2.91 (m, 2H), 1.99 - 1.92 (m, 1H), 1.87 - 1.77 (m, 3H), 1.21 (s, 6H).

**Examples 33 to 35**

The compounds of Examples 33 to 35 were prepared in the same method as in Example 32, except that appropriate Intermediates were used instead of Intermediate I-36 in Example 32.

[Table 5]

| No. | Structure/Name | Intermediate Used | Spectral Data |
|---|---|---|---|
| 33 | 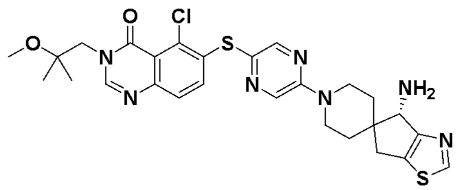 (S)-6-((5-(4-amino-4,6-dihydrospiro[cyclopenta[d] thiazole-5,4'-piperidin]-1'-yl)pyrazin-2-yl)thio)-5-chloro-3-(2-methoxy-2-methylpropyl)quinazolin-4(3H)-one | Intermediate I-38 | m/z ES+ $[M+H]^+$ 584.2. $^1H$ NMR (400 MHz, $CDCl_3$) $\delta$ = 8.69 (s, 1H), 8.26 (d, $J$ = 1.2 Hz, 1H), 8.22 (d, $J$ = 1.2 Hz, 1H), 8.13 (s, 1H), 7.44 (d, $J$ = 8.8 Hz, 1H), 7.25 (d, $J$ = 4.0 Hz, 1H), 4.12 - 4.05 (m, 2H), 4.05 (s, 2H), 4.02 (d, $J$ = 4.4 Hz, 1H), 3.55 - 3.41 (m, 2H), 3.19 (s, 3H), 3.00 - 2.94 (m, 1H), 2.86 - 2.79 (m, 1H), 2.06 - 2.00 (m, 1H), 1.92 - 1.82 (m, 1H), 1.81 - 1.72 (m, 2H), 1.19 (s, 6H). |
| 34 | 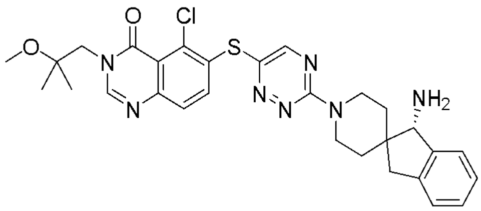 (S)-6-((3-(1-amino-1,3-dihydrospiro[indene-2,4'-piperidin]-1'-yl)-1,2,4-triazin-6-yl)thio)-5-chloro-3-(2-methoxy-2-methylpropyl)quinazolin-4(3H)-one | Intermediate I-39 | m/z ES+ $[M+H]^+$ 578.3 $^1H$ NMR (400 MHz, $CDCl_3$) $\delta$ = 8.19 (s, 1H), 8.18 (s, 1H), 7.53 (d, $J$ = 1.2 Hz, 2H), 7.34 (d, $J$ = 5.2 Hz, 1H), 7.26 - 7.24 (m, 1H), 7.23 (d, $J$ = 2.0 Hz, 2H), 4.68 (s, 2H), 4.07 (s, 2H), 4.02 (s, 1H), 3.42 - 3.29 (m, 2H), 3.22 (s, 3H), 3.14 (d, $J$ = 15.6 Hz, 1H), 2.78 (d, $J$ = 15.6 Hz, 1H), 1.93 - 1.78 (m, 2H), 1.67 (s, 1H), 1.44 - 1.39 (m, 1H), 1.22 (s, 6H) |
| 35 | 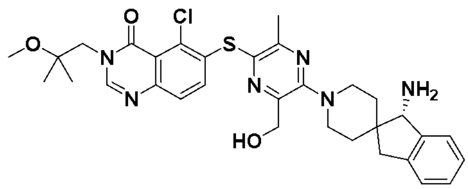 (S)-6-((5-(1-amino-1,3-dihydrospiro[indene-2,4'-piperidin]-1'-yl)-6-(hydroxymethyl)-3-methylpyrazin-2-yl)thio)-5-chloro-3-(2-methoxy-2-methylpropyl)quinazolin-4(3H)-one | Intermediate I-40 | MS (EI) m/z: $[M+H]^+$ 621.3. $^1H$ NMR (400 MHz, $CDCl_3$) $\delta$ = 8.19 (s, 1H), 7.50 (d, $J$ = 8.8 Hz, 1H), 7.39 - 7.33 (m, 2H), 7.26 - 7.19 (m, 3H), 4.61 (s, 2H), 4.08 (s, 2H), 4.02 (s, 1H), 3.58 - 3.48 (m, 2H), 3.22 (s, 3H), 3.20 - 3.11 (m, 2H), 3.11 - 3.06 (m, 1H), 2.72 (d, $J$ = 15.6 Hz, 1H), 2.56 (s, 3H), 1.95 (d, $J$ = 2.8 Hz, 1H), 1.86 (d, $J$ = 4.0 Hz, 1H), 1.66 (s, 1H), 1.40 (d, $J$ = 13.6 Hz, 1H), 1.23 (s, 6H). |

**Example 36: (S)-6-((3-amino-5-(1-amino-1,3-dihydrospiro[indene-2,4'-piperidin]-1'-yl)pyrazin-2-yl)thio)-5-chloro-3-(2-methoxy-2-methylpropyl)quinazolin-4(3H)-one**

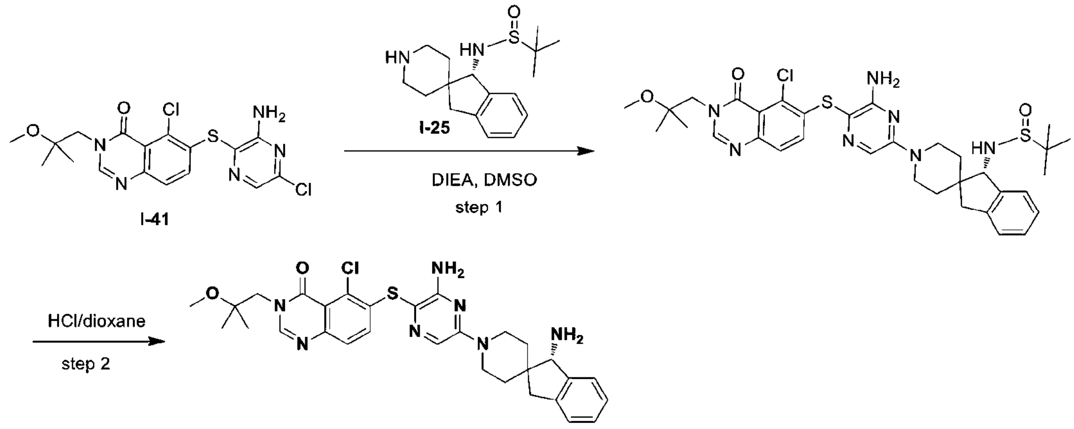


**Step 1: N-((S)-1'-(6-amino-5-((5-chloro-3-(2-methoxy-2-methylpropyl)-4-oxo-3,4-dihydroquinazolin-6-yl)thio) pyrazin-2-yl)-1,3-dihydrospiro[indene-2,4'-piperidin]-1-yl)-2-methylpropane-2-sulfinamide**

To a solution of 6-(3-amino-5-chloro-pyrazin-2-yl)sulfanyl-5-chloro-3-(2-methoxy-2-methyl-propyl)quinazolin-4-one (80 mg, 188 μmol) in DMSO (3 mL), DIEA (2.43 g, 18.8 mmol) and 2-methyl-N-[(1S)-spiro[indane-2,4'-piperidin]-1-yl] propane-2-sulfinamide (57.5 mg) were added. The mixture was stirred at 100°C for 12 hours. Upon completion of stirring, the reaction mixture was quenched by addition of aqueous ammonium chloride (20 mL) at 25°C, and then diluted with water (30 mL) and extracted with EA (3 x 50 mL). The combined organic layers were washed with brine (3 x 20 mL), dried over anhydrous sodium sulfate, and then filtered and concentrated under reduced pressure to obtain a residue. The residue was purified by Prep-HPLC (column: Phenomenex luna C18 150*25mm*10 μm; mobile phase: [water(FA)-ACN]; concentration gradient: 55%-85% B over 10 min) to obtain N-[(1S)-1'-[6-amino-5-[5-chloro-3-(2-methoxy-2-methyl-propyl)-4-oxo-quinazolin-6-yl]sulfanyl-pyrazin-2-yl]spiro[indane-2,4'-piperidin]-1-yl]-2-methyl-propane-2-sulfinamide (60 mg, 46% yield) as a yellow solid.

$^1$H NMR (400 MHz, $CDCl_3$) δ = 8.14 (s, 1H), 7.67 (s, 1H), 7.47 (d, *J* = 8.8 Hz, 1H), 7.34 - 7.31 (m, 1H), 7.29 - 7.27 (m, 1H), 7.27 - 7.22 (m, 2H), 7.14 (d, *J* = 8.8 Hz, 1H), 4.84 (br.s, 2H), 4.58 (d, *J* = 10.0 Hz, 1H), 4.37 - 4.28 (m, 2H), 4.07 (s, 2H), 3.60 (d, *J* = 10.0 Hz, 1H), 3.21 (s, 3H), 3.20 - 3.16 (m, 1H), 3.15 - 3.06 (m, 2H), 2.79 (d, *J* = 15.6 Hz, 1H), 2.33 (dt, *J* = 4.4, 12.8 Hz, 1H), 1.92 - 1.84 (m, 1H), 1.49 - 1.36 (m, 2H), 1.31 (s, 9H), 1.22 (s, 6H).

**Step 2: (S)-6-((3-amino-5-(1-amino-1,3-dihydrospiro[indene-2,4'-piperidin]-1'-yl)pyrazin-2-yl)thio)-5-chloro-3-(2-methoxy-2-methylpropyl)quinazolin-4(3H)-one**

To a solution of N-[(1S)-1'-[6-amino-5-[5-chloro-3-(2-methoxy-2-methyl-propyl)-4-oxo-quinazolin-6-yl]sulfanyl-pyrazin-2-yl]spiro[indane-2,4'-piperidin]-1-yl]-2-methyl-propane-2-sulfinamide (60 mg, 86.2 μmol) in MeOH (3 mL), HCl/dioxane (4 M) was added, and the mixture was stirred at 25°C for 0.5 hours. Upon completion of stirring, the reaction mixture was concentrated under reduced pressure to obtain a residue. The residue was purified by Prep-HPLC (column: Waters Xbridge 150*25mm*5 μm; mobile phase: [water (ammonium hydroxide v/v)-ACN]; concentration gradient:35%-65% B over min) to obtain **the compound of Example 36** (34 mg, 66% yield) as an off-white solid.

MS (EI) m/z: $[M+H]^+$ 592.3.

$^1$H NMR (400 MHz, $CDCl_3$) δ = 8.14 (s, 1H), 7.69 (s, 1H), 7.44 (d, *J* = 8.8 Hz, 1H), 7.40 - 7.33 (m, 1H), 7.26 - 7.19 (m, 3H), 7.16 (d, *J* = 8.8 Hz, 1H), 4.88 (s, 2H), 4.27 - 4.17 (m, 2H), 4.07 (s, 2H), 4.04 - 4.01 (m, 1H), 3.26 - 3.24 (m, 1H), 3.21 (s, 3H), 3.20 - 3.16 (m, 1H), 3.12 (d, *J* = 15.6 Hz, 1H), 2.77 (d, *J* = 15.6 Hz, 1H), 1.90 - 1.77 (m, 2H), 1.55-1.50 (m, 1H), 1.45 - 1.39 (m, 1H), 1.22 (s, 6H).

**Example 37: 6-((3-amino-5-((3S,4S)-4-amino-3-methyl-2-oxa-8-azaspiro[4.5]decan-8-yl)pyrazin-2-yl)thio)-5-chloro-3-(2-methoxy-2-methylpropyl)quinazolin-4(3H)-one**

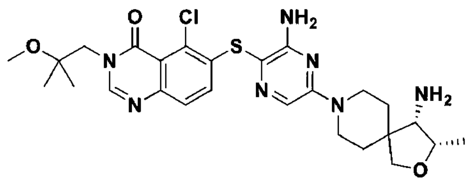

**The compound of Example 37** (41 mg, 69% yield) was synthesized in the same method as in Example 25, using the resulting product from Step 1 of Preparation Example 23 instead of **Intermediate I-25** as a starting material in Step 1 of Example 36.

m/z ES+ $[M+H]^+$ 560.2.

$^1H$ NMR (400 MHz, $CDCl_3$) δ = 8.14 (s, 1H), 7.67 (s, 1H), 7.43 (d, *J* = 8.8 Hz, 1H), 7.14 (d, *J* = 8.8 Hz, 1H), 4.87 (s, 2H), 4.20-4.17 (m, 1H), 4.07 (s, 2H), 3.98 - 3.87 (m, 2H), 3.83 (d, *J* = 8.8 Hz, 1H), 3.71 (d, *J* = 8.8 Hz, 1H), 3.43-3.40 (m, 1H), 3.36 - 3.29 (m, 1H), 3.21 (s, 3H), 3.02 (d, *J* = 4.4 Hz, 1H), 1.93 - 1.86 (m, 1H), 1.80 - 1.70 (m, 3H), 1.26 (d, *J* = 6.4 Hz, 3H), 1.22 (s, 6H).

**Example 38: (S)-6-((5-(6-amino-4,6-dihydrospiro[cyclopenta[d]thiazole-5,4'-piperidin]-1'-yl)pyrazin-2-yl)thio)-5-chloro-3-phenylquinazolin-4(3H)-one**

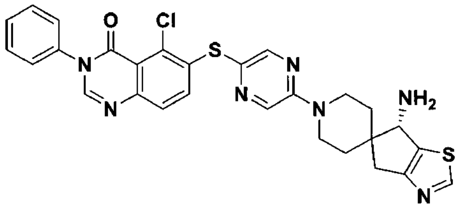

**The compound of Example 38** (115 mg, 75% yield) was synthesized in the same method as in Example 25, using **Intermediate I-36** instead of **Intermediate I-26** and using **Intermediate I-32** instead of **Intermediate I-28** as starting materials in Step 1 of Example 25.

m/z ES+ $[M+H]^+$ 573.1

$^1H$ NMR (400 MHz, $CDCl_3$) δ = 8.81 (s, 1H), 8.27 (d, *J* = 9.6 Hz, 2H), 8.05 (s, 1H), 7.58 - 7.45 (m, 4H), 7.41 (d, *J* = 7.2 Hz, 2H), 7.30 (d, *J* = 8.8 Hz, 1H), 4.40 - 4.17 (m, 3H), 3.38 - 3.19 (m, 2H), 3.11 - 2.91 (m, 2H), 2.09 - 1.89 (m, 2H), 1.89 - 1.69 (m, 2H).

**Example 39: 6-((5-((3S,4S)-4-amino-3-methyl-2-oxa-8-azaspiro[4.5]decan-8-yl)pyrazin-2-yl)thio)-5-chloro-3-(2-methoxypropyl)-2-methylquinazolin-4(3H)-one**

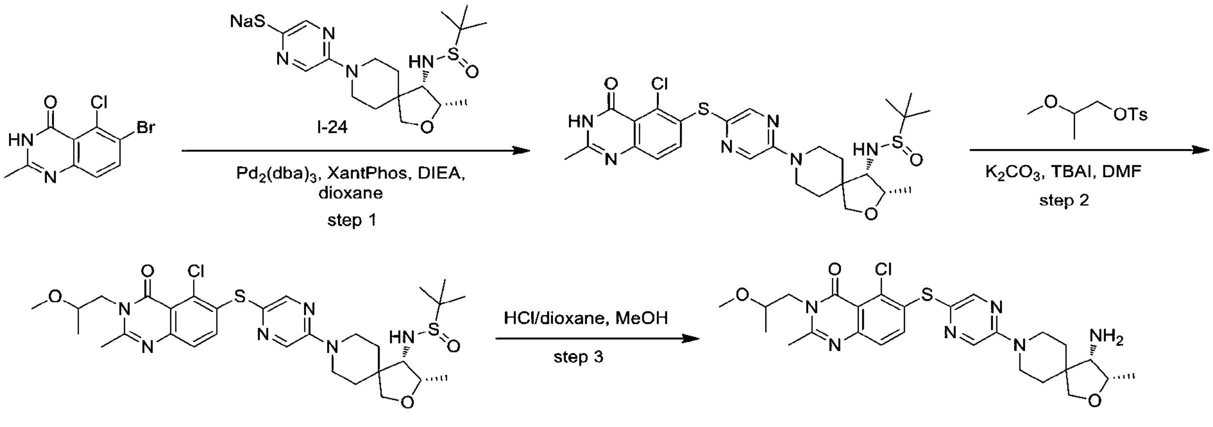

**Step 1: N-((3S,4S)-8-(5-((5-chloro-2-methyl-4-oxo-3,4-dihydroquinazolin-6-yl)thio)pyrazin-2-yl)-3-methyl-2-oxa-8-azaspiro[4.5]decan-4-yl)-2-methylpropane-2-sulfinamide**

N-((3S,4S)-8-(5-((5-chloro-2-methyl-4-oxo-3,4-dihydroquinazolin-6-yl)thio)pyrazin-2-yl)-3-methyl-2-oxa-8-azaspiro[4.5]decan-4-yl)-2-methylpropane-2-sulfinamide (90 mg, 38% yield) was synthesized in the same method as in Example 25, using **Intermediate I-24** instead of **Intermediate I-26** and using 6-bromo-5-chloro-2-methylquinazolin-4(3H)-one instead of **Intermediate I-28** as starting materials in Step 1 of Example 25.

$^1$H NMR (400 MHz, DMSO-$d_6$) δ = 12.81 - 11.83 (m, 1H), 8.46 (d, *J* = 1.2 Hz, 1H), 8.28 (d, *J* = 1.2 Hz, 1H), 7.40 (d, *J* = 8.8 Hz, 1H), 7.14 (d, *J* = 8.8 Hz, 1H), 5.13 (d, *J* = 10.8 Hz, 1H), 4.26 - 4.19 (m, 1H), 4.18 - 4.11 (m, 2H), 3.88 (d, *J* = 8.8 Hz, 1H), 3.52 (d, *J* = 8.8 Hz, 1H), 3.45 (s, 1H), 3.17 - 3.06 (m, 2H), 2.29 (s, 3H), 1.83 - 1.73 (m, 2H), 1.67 - 1.53 (m, 2H), 1.16 (s, 9H), 1.10 (d, *J* = 6.4 Hz, 3H).

**Step 2: N-((3S,4S)-8-(5-((5-chloro-3-(2-methoxypropyl)-2-methyl-4-oxo-3,4-dihydroquinazolin-6-yl)thio)pyrazin-2-yl)-3-methyl-2-oxa-8-azaspiro[4.5]decan-4-yl)-2-methylpropane-2-sulfinamide**

A solution of N-[(3S,4S)-8-[5-[(5-chloro-2-methyl-4-oxo-3H-quinazolin-6-yl)sulfanyl]pyrazin-2-yl]-3-methyl-2-oxa-8-azaspiro[4.5]decan-4-yl]-2-methyl-propane-2-sulfinamide (70 mg, 121 μmol), 2-methoxypropyl 4-methylbenzenesulfonate (88.8 mg, 363 μmol), $K_2CO_3$ (50.2 mg, 363 μmol), and TBAI (4.48 mg, 12.1 μmol) in DMF (2 mL) was stirred at 60°C for 2 hours. Upon completion of stirring, the reaction mixture was added with water (40 mL) and extracted with EA (3 X 80 mL). The combined organic layers were concentrated under reduced pressure. The residue was purified by Prep-TLC ($SiO_2$, EA: MeOH = 10: 1) to obtain N-[(3S,4S)-8-[5-[5-chloro-3-(2-methoxypropyl)-2-methyl-4-oxo-quinazolin-6-yl]sulfanylpyrazin-2-yl]-3-methyl-2-oxa-8-azaspiro[4.5]decan-4-yl]-2-methyl-propane-2-sulfinamide (30 mg, 38% yield) as a yellow solid.

$^1$H NMR (400 MHz, $CDCl_3$) δ = 8.27 - 8.23 (m, 1H), 8.19 (d, *J* = 1.2 Hz, 1H), 8.03 (s, 1H), 7.40 (d, *J* = 8.8 Hz, 1H), 4.40 - 4.20 (m, 1H), 4.28 - 4.19 (m, 2H), 4.19 - 4.12 (m, 1H), 3.91 (d, *J* = 9.2 Hz, 1H), 3.86 - 3.77 (m, 1H), 3.69 (d, *J* = 9.2 Hz, 1H), 3.56 - 3.50 (m, 1H), 3.38 (d, *J* = 10.4 Hz, 1H), 3.22 (s, 2H), 2.96 (s, 3H), 2.89 (s, 3H), 2.66 (s, 3H), 2.12 - 1.94 (m, 2H), 1.80 - 1.65 (m, 2H), 1.64 - 1.60 (m, 1H), 1.27 (s, 9H), 1.24 (d, *J* = 6.4 Hz, 3H).

**Step 3: 6-((5-((3S,4S)-4-amino-3-methyl-2-oxa-8-azaspiro[4.5]decan-8-yl)pyrazin-2-yl)thio)-5-chloro-3-(2-methoxypropyl)-2-methylquinazolin-4(3H)-one**

**The compound of Example 39** (7.41 mg, 26% yield) was synthesized in the same method as in Step 2 of Example 25.

MS (EI) m/z: $[M+H]^+$ 545.3,
$^1$H NMR (400 MHz, MeOD) δ = 8.56 - 8.50 (m, 1H), 8.30 (s, 1H), 8.27 (s, 1H), 7.37 (d, *J* = 8.8 Hz, 1H), 7.25 (d, *J* = 8.8 Hz, 1H), 4.33 - 4.28 (m, 2H), 4.27 - 4.18 (m, 2H), 3.99 - 3.89 (m, 2H), 3.85 (d, *J* = 9.2 Hz, 1H), 3.82 - 3.70 (m, 1H), 3.36 (d, *J* = 4.4 Hz, 1H), 3.29 - 3.24 (m, 1H), 3.23 (s, 3H), 3.22 - 3.17 (m, 1H), 2.66 (s, 3H), 1.88 - 1.81 (m, 3H), 1.76 - 1.67 (m, 1H), 1.30 (d, *J* = 6.4 Hz, 3H), 1.26 (d, *J* = 6.4 Hz, 3H).

**Example 40: (S)-7-((5-(6-amino-4,6-dihydrospiro[cyclopenta[d]thiazole-5,4'-piperidin]-1'-yl)pyrazin-2-yl)thio)-8-chloro-2-isopropylisoquinolin-1(2H)-one**

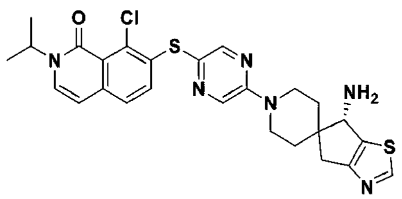

**The compound of Example 40** (2.34 mg, 25% yield) was synthesized in the same method as in Example 25, using **Intermediate I-36** instead of **Intermediate I-26** and **Intermediate I-44** instead of **Intermediate I-28** as starting materials in Step 1 of Example 25.

MS (EI) m/z: $[M+H]^+$ 539.0,
$^1$H NMR (400 MHz, MeOD) δ = 9.01 (s, 1H), 8.58 - 8.45 (m, 1H), 8.33 (s, 1H), 8.28 (s, 1H), 7.45 (d, *J* = 7.6 Hz, 1H), 7.40

(d, *J* = 8.8 Hz, 1H), 7.15 (d, *J* = 8.8 Hz, 1H), 6.63 (d, *J* = 7.6 Hz, 1H), 5.33 - 5.18 (m, 1H), 4.46 - 4.36 (m, 1H), 4.35 - 4.27 (m, 1H), 4.23 (s, 1H), 3.47 - 3.34 (m, 2H), 3.02 (s, 2H), 2.05 - 1.87 (m, 2H), 1.84 - 1.76 (m, 2H), 1.40 (d, *J* = 6.8 Hz, 6H).

**Example 41: (S)-7-((5-(1-amino-1,3-dihydrospiro[indene-2,4'-piperidin]-1'-yl)pyrazin-2-yl)thio)-8-chloro-2-methylisoquinolin-1(2H)-one**

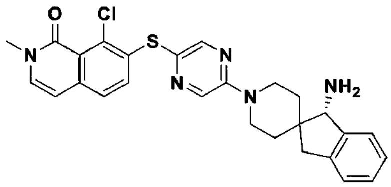

**The compound of Example 41** (19 mg, 73% yield) was synthesized in the same method as in Example 25, using **Intermediate I-43** instead of **Intermediate I-28** as a starting material in Step 1 of Example 25.

MS (EI) m/z: $[M+H]^+$ 504.3
$^1$H NMR (400 MHz, MeOD) δ = 8.32 (s, 1H), 8.27 (s, 1H), 7.54 - 7.40 (m, 1H), 7.48 - 7.30 (m, 2H), 7.36 - 7.33 (m, 2H), 7.32 - 7.28 (m, 1H), 7.16 (d, *J* = 8.8 Hz, 1H), 6.56 (d, *J* = 7.2 Hz, 1H), 4.50 - 4.30 (m, 1H), 4.34 - 4.27 (m, 2H), 3.56 (s, 3H), 3.40 - 3.30 (m, 2H), 3.25 - 3.15 (m, 1H), 3.13 - 3.05 (m, 1H), 1.88 - 1.80 (m, 2H), 1.71 - 1.62 (m, 2H).

**Examples 42 to 45**

The compounds of Examples 42 to 45 were prepared in the same method as in Example 41, except that **Intermediate I-43** was replaced with an appropriate intermediate in Example 41.

[Table 6]

| No. | Structure/Name | Intermediate Used | Spectral Data |
|---|---|---|---|
| 42 | 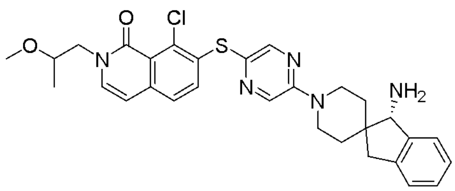 7-((5-((S)-1-amino-1,3-dihydrospiro[indene-2,4'-piperidin]-1'-yl)pyrazin-2-yl)thio)-8-chloro-2-(2-methoxypropyl)isoquinolin-1(2H)-one | Intermediate I-45 | MS (EI) m/z: $[M+H]^+$ 562.3. $^1$H NMR (400 MHz, $CDCl_3$) δ = 8.26 (d, *J* = 1.2 Hz, 1H), 8.21 (d, *J* = 1.2 Hz, 1H), 7.38 - 7.33 (m, 1H), 7.26 - 7.21 (m, 4H), 7.21 - 7.15 (m, 2H), 6.37 - 6.29 (m, 1H), 4.35 - 4.29 (m, 1H), 4.29 - 4.18 (m, 2H), 4.07 - 3.96 (m, 1H), 3.84 - 3.73 (m, 1H), 3.61 - 3.50 (m, 1H), 3.33 - 3.29 (m, 1H), 3.29 - 3.27 (m, 3H), 3.27 - 3.21 (m, 1H), 3.17 - 3.06 (m, 1H), 2.82 - 2.71 (m, 1H), 1.95 - 1.86 (m, 1H), 1.85 - 1.76 (m, 1H), 1.66 (dd, *J* = 2.0, 12.8 Hz, 1H), 1.45 - 1.39 (m, 1H), 1.25 - 1.19 (m, 3H). |
| 43 | 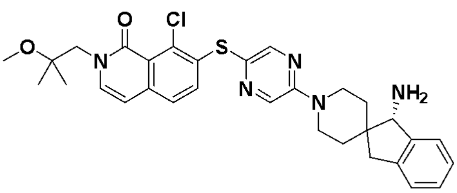 (S)-7-((5-(1-amino-1,3-dihydrospiro[indene-2,4'-piperidin]-1'-yl)pyrazin-2-yl)thio)-8-chloro-2-(2-methoxy-2-methylpropyl)isoquinolin-1(2H)-one | Intermediate I-46 | m/z ES+ $[M+H]^+$ 576.2 $^1$H NMR (400 MHz, $CDCl_3$) δ = 8.26 (d, *J* = 1.2 Hz, 1H), 8.21 (d, *J* = 1.2 Hz, 1H), 7.34 ( d, *J* = 5.2 Hz, 1H), 7.29 (s, 1H), 7.25 - 7.22 (m, 3H), 7.22 - 7.17 (m, 2H), 6.30 (d, *J* = 7.6 Hz, 1H), 4.29 - 4.19 (m, 2H), 4.09 (s, 2H), 4.02 (s, 1H), 3.33 - 3.24 (m, 2H), 3.23 (s, 3H), 3.12 (d, *J* = 15.6 Hz, 1H), 2.76 (d, *J* = 15.6 Hz, 1H), 1.95 - 1.77 (m, 2H), 1.66 (dd, *J* = 1.6, 13.6 Hz, 1H), 1.44-1.40 (m, 1H), 1.21 (s, 6H) |

(continued)

| No. | Structure/Name | Intermediate Used | Spectral Data |
|---|---|---|---|
| 44 | 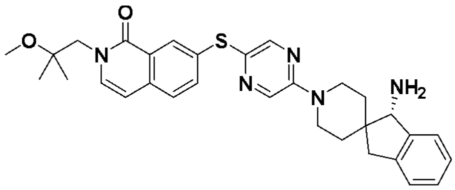 (S)-7-((5-(1-amino-1,3-dihydrospiro[indene-2,4'-piperidin]-1'-yl)pyrazin-2-yl)thio)-2-(2-methoxy-2-methylpropyl)isoquinolin-1(2H)-one | Intermediate I-47 | m/z ES+ $[M+H]^+$ 542.2. $^1H$ NMR (400 MHz, $CDCl_3$) δ = 8.35 (d, *J* = 1.6 Hz, 1H), 8.18 (d, *J* = 0.8 Hz, 1H), 8.13 (s, 1H), 7.58 (dd, *J* = 1.6, 8.4 Hz, 1H), 7.42 (d, *J* = 8.4 Hz, 2H), 7.26 - 7.22 (m, 4H), 6.39 (d, *J* = 7.2 Hz, 1H), 4.14 (d, *J* = 13.6 Hz, 2H), 4.09 - 4.04 (m, 3H), 3.25 (d, *J* = 2.8 Hz, 1H), 3.23 (s, 3H), 3.21 - 3.17 (m, 1H), 3.13 (d, *J* = 15.6 Hz, 1H), 2.79 (d, *J* = 15.6 Hz, 1H), 1.86 - 1.77 (m, 2H), 1.60 (d, *J* = 13.2 Hz, 1H), 1.49-1.44 (m, 1H), 1.19 (s, 6H). |
| 45 | 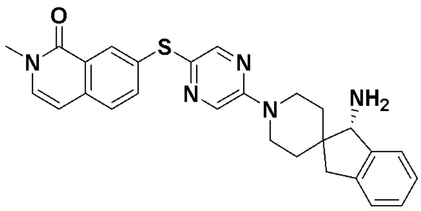 (S)-7-((5-(1-amino-1,3-dihydrospiro[indene-2,4'-piperidin]-1'-yl)pyrazin-2-yl)thio)-2-methylisoquinolin-1(2H)-one | Intermediate I-48 | MS (EI) m/z: $[M+H]^+$ 470.2. $^1H$ NMR (400 MHz, $CDCl_3$) δ = 8.32 (d, *J* = 1.6 Hz, 1H), 8.19 (d, *J* = 1.6 Hz, 1H), 8.14 (d, *J* = 1.6 Hz, 1H), 7.58 (dd, *J* = *1.6,* 8.4 Hz, 1H), 7.43 (d, *J* = 8.4 Hz, 1H), 7.37 - 7.33 (m, 1H), 7.26 - 7.21 (m, 3H), 7.04 (d, *J*= 7.2 Hz, 1H), 6.44 (d, *J*= 7.2 Hz, 1H), 4.24 - 4.14 (m, 2H), 4.01 (s, 1H), 3.58 (s, 3H), 3.29 - 3.18 (m, 2H), 3.10 (d, *J* = 15.6 Hz, 1H), 2.75 (d, *J* = 15.6 Hz, 1H), 1.92 - 1.76 (m, 2H), 1.65 (d, *J* = 2.0 Hz, 1H), 1.40 (dd, *J* = 2.0, 13.6 Hz, 1H). |

**Example 46: 7-((5-((3S,4S)-4-amino-3-methyl-2-oxa-8-azaspiro[4.5]decan-8-yl)pyrazin-2-yl)thio)-8-chloro-2-isopropylisoquinolin-1(2H)-one**

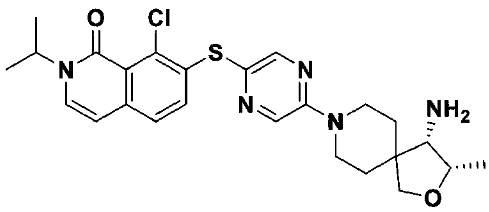

**The compound of Example 46** (10 mg, 66% yield) was synthesized in the same method as in Example 40, using **Intermediate I-24** instead of **Intermediate I-36** as a starting material in Example 40.

MS (EI) m/z: $[M+H]^+$ 500.2
$^1H$ NMR (400 MHz, MeOD) δ = 8.30 (s, 1H), 8.26 (d, *J* = 1.2 Hz, 1H), 7.45 (d, *J* = 7.6 Hz, 1H), 7.39 (d, *J* = 8.4 Hz, 1H), 7.15 (d, J = 8.4 Hz, 1H), 6.62 (d, *J* = 7.6 Hz, 1H), 5.25-5.20 (m, 1H), 4.37 - 4.20 (m, 3H), 3.98 (d, *J* = 9.2 Hz, 1H), 3.86 (d, *J* = 9.2 Hz, 1H), 3.740 (d, *J* = 4.0 Hz, 1H), 3.29 - 3.14 (m, 2H), 1.90 - 1.80 (m, 3H), 1.76 - 1.68 (m, 1H), 1.40 (d, *J* = 6.8 Hz, 6H), 1.31 (d, *J* = 6.4 Hz, 3H).

**Example 47: (S)-7-((3-amino-5-(1-amino-1,3-dihydrospiro[indene-2,4'-piperidin]-1'-yl)pyrazin-2-yl)thio)-8-chloro-2-methylisoquinolin-1(2H)-one**

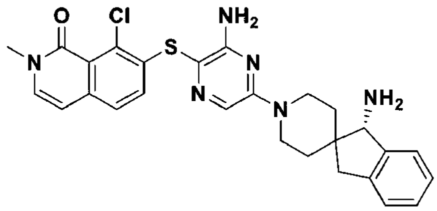

**The compound of Example 47** (20 mg, 59% yield) was synthesized in the same method as in Example 36, using **Intermediate I-49** instead of **Intermediate I-41** as a starting material in Example 36.

MS (EI) m/z: $[M+H]^+$ 519.2
$^1H$ NMR (400 MHz, $CDCl_3$) δ = 7.68 (s, 1H), 7.38 (s, 1H), 7.26 - 7.19 (m, 4H), 7.08 - 7.04 (m, 2H), 6.34 (d, *J* = 7.2 Hz, 1H), 4.87 (s, 2H), 4.24 - 4.15 (m, 2H), 4.04 (s, 1H), 3.57 (s, 3H), 3.26 - 3.17 (m, 2H), 3.13 (d, *J* = 15.2 Hz, 1H), 2.78 (d, *J* = 15.2 Hz, 1H), 1.89 - 1.80 (m, 2H), 1.66-1.63 (m, 1H), 1.47-1.45 (m, 1H)

**Example 48: (S)-7-((3-amino-5-(1-amino-1,3-dihydrospiro[indene-2,4'-piperidin]-1'-yl)pyrazin-2-yl)thio)-8-chloro-2-isopropylisoquinolin-1(2H)-one**

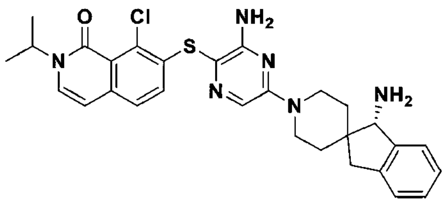

**The compound of Example 48** (20 mg, 59% yield) was synthesized in the same method as in Example 36, using **Intermediate I-50** instead of **Intermediate I-41** as a starting material in Example 36.

MS (ESI) m/z: $[M+H]^+$ 547.2;
$^1H$ NMR (400 MHz, $CDCl_3$) δ = 7.67 (s, 1H), 7.39 - 7.35 (m, 1H), 7.24 (d, *J* = 2.8 Hz, 3H), 7.21 (d, *J* = 8.4 Hz, 1H), 7.12 (d, *J* = 7.2 Hz, 1H), 7.05 (d, *J* = 8.4 Hz, 1H), 6.41 (d, *J* = 7.2 Hz, 1H), 5.42 - 5.30 (m, 1H), 4.87 (s, 2H), 4.25 - 4.16 (m, 2H), 4.03 (s, 1H), 3.26 - 3.16 (m, 2H), 3.12 (d, *J* = 15.6 Hz, 1H), 2.78 (d, *J* = 15.6 Hz, 1H), 1.90 - 1.80 (m, 1H), 1.86 - 1.70 (m, 1H), 1.63 - 1.59 (m, 1H), 1.46 - 1.40 (m, 1H), 1.37 (d, *J* = 6.8 Hz, 6H)

**Example 49: 7-((3-amino-5-((S)-1-amino-1,3-dihydrospiro[indene-2,4'-piperidin]-1'-ylpyrazin-2-yl)thio)-8-chloro-2-(2-methoxypropyl)isoquinolin-1(2H)-one**

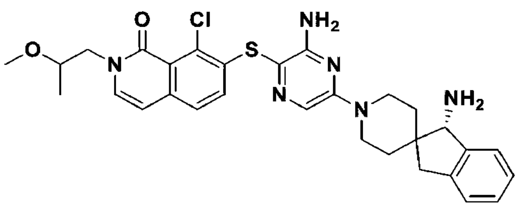

**The compound of Example 49** (32 mg, 62% yield) was synthesized in the same method as in Example 36, using **Intermediate I-51** instead of **Intermediate I-41** as a starting material in Example 36.
MS (EI) m/z: $[M+H]^+$ 577.3.
$^1H$ NMR (400 MHz, $CDCl_3$) δ = 7.72 (s, 1H), 7.39 (d, *J* = 4.4 Hz, 1H), 7.33 - 7.29 (m, 2H), 7.27 - 7.21 (m, 2H), 7.18 (d, *J* = 7.2 Hz, 1H), 7.10 (d, *J* = 8.4 Hz, 1H), 6.36 (d, *J* = 7.2 Hz, 1H), 4.89 (s, 2H), 4.35 (dd, *J* = 2.8, 13.6 Hz, 1H), 4.29 - 4.20 (m, 2H), 4.05 (s, 1H), 3.87 - 3.78 (m, 1H), 3.59 (dd, *J* = 8.4, 13.6 Hz, 1H), 3.31 (s, 3H), 3.29 - 3.19 (m, 2H), 3.15 (d, *J* = 15.6 Hz, 1H), 2.79 (d, *J* = 15.6 Hz, 1H), 1.95 - 1.83 (m, 2H), 1.82 - 1.77 (m, 1H), 1.46 - 1.41 (m, 1H), 1.26 (d, *J* = 6.4 Hz, 3H).

**Example 50: (R)-6-((5-(3-amino-3H-spiro[benzofuran-2,4'-piperidin]-1'-yl)pyrazin-2-yl)thio)-5-chloro-3-(2-methoxy-2-methylpropyl)quinazolin-4(3H)-one**

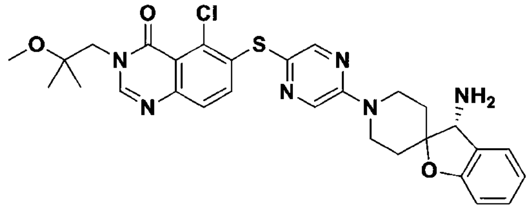

**The compound of Example 50** (49 mg, 48% yield) was synthesized in the same method as in Example 25, using **Intermediate I-53** instead of **Intermediate I-26** as a starting material in Step 1 of Example 25.

m/z ES+ $[M-NH_2]^+$ 562.3.

$^1H$ NMR (400 MHz, $CDCl_3$) δ = 8.29 (d, *J* = 1.2 Hz, 1H), 8.26 (d, *J* = 1.2 Hz, 1H), 8.15 (s, 1H), 7.47 (d, *J* = 8.8 Hz, 1H), 7.34 (d, *J* = 7.2 Hz, 1H), 7.29 (d, *J* = 8.8 Hz, 1H), 7.25 - 7.21 (m, 1H), 6.95 (t, *J* = 7.2 Hz, 1H), 6.84 (d, *J* = 8.0 Hz, 1H), 4.36 (d, *J* = 13.6 Hz, 1H), 4.26 (d, *J* = 13.6 Hz, 1H), 4.16 (s, 1H), 4.07 (s, 2H), 3.60 - 3.50 (m, 2H), 3.22 (s, 3H), 1.98 (dd, *J* = 4.0, 8.0 Hz, 2H), 1.92 (d, *J* = 13.6 Hz, 1H), 1.82 (dd, *J* = 4.8, 12.0 Hz, 1H), 1.22 (s, 6H).

**Example 51: (R)-6-((5-(3-amino-3H-spiro[benzofuran-2,4'-piperidin]-1'-yl)pyrazin-2-yl)thio)-5-chloro-3-methylquinazolin-4(3H)-one**

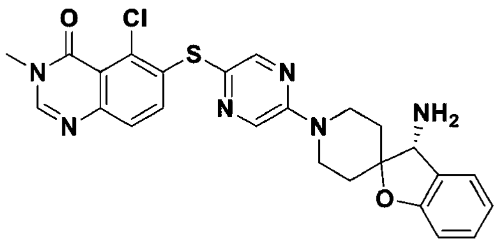

**The compound of Example 51** (44 mg, 47% yield) was synthesized in the same method as in Example 50, using **Intermediate I-54** instead of **Intermediate I-28** as a starting material in Example 50.

m/z ES+ $[M+Na]^+$ 529.1.

$^1H$ NMR ($CDCl_3$, 400 MHz) δ= 8.30 (d, *J* = 1.2 Hz, 1H), 8.27 (d, *J* = 1.2 Hz, 1H), 7.99 (s, 1H), 7.47 (d, *J* = 8.8 Hz, 1H), 7.38 (d, *J* = 7.6 Hz, 1H), 7.29 (s, 1H), 7.25 - 7.21 (m, 1H), 7.05 - 6.90 (m, 1H), 6.85 (d, *J* = 8.0 Hz, 1H), 4.46 - 4.30 (m, 1H), 4.34 - 4.20 (m, 1H), 4.21 (s, 1H), 3.57 (s, 3H), 3.56 - 3.49 (m, 2H), 1.98 - 1.90 (m, 2H), 1.86 - 1.80 (m, 2H).

**Example 52: (R)-6-((5-(3-amino-3H-spiro[benzofuran-2,4'-piperidin]-1'-yl)pyrazin-2-yl)thio)-5-chloro-3-phenylquinazolin-4(3H)-one**

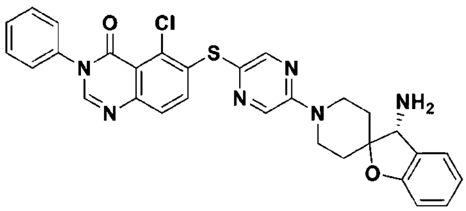

**The compound of Example 52** (54 mg, 63% yield) was synthesized in the same method as in Example 50, using **Intermediate I-32** instead of **Intermediate I-28** as a starting material in Example 50.

m/z ES+ $[M+Na]^+$ 591.2.

$^1H$ NMR (400 MHz, $CDCl_3$) δ= 8.32 (d, *J* = 1.2 Hz, 1H), 8.29 (d, *J* = 1.2 Hz, 1H), 8.07 (s, 1H), 7.62 - 7.48 (m, 5H),

7.43 (d, $J$ = 1.6 Hz, 1H), 7.41 (s, 1H), 7.37 - 7.32 (m, 2H), 7.25 - 7.20 (m, 1H), 7.00 - 6.92 (m, 1H), 6.85 (d, $J$ = 8.0 Hz, 1H), 4.39 (m, 1H), 4.32 - 4.25 (m, 1H), 4.19 (s, 1H), 3.61 - 3.52 (m, 2H), 2.00 - 1.90 (m, 2H), 1.89 - 1.76 (m, 2H).

**Example 53: (R)-6-((5-(3-amino-3H-spiro[benzofuran-2,4'-piperidin]-1'-yl)pyrazin-2-yl)thio)-5-chloro-3-(methyl-$d_3$)quinazolin-4(3H)-one**

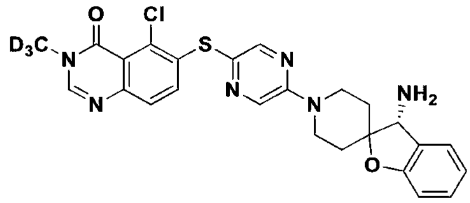

**The compound of Example 53** (25 mg, 40% yield) was synthesized in the same method as in Example 50, using **Intermediate I-55** instead of **Intermediate I-28** as a starting material in Example 50.

m/z ES+ $[M+H]^+$ 510.2.

$^1$H NMR (DMSO, 400 MHz) $\delta$= 8.77 (s, 1H), 8.64 (s, 1H), 8.36 (s, 1H), 8.09 (s, 1H), 7.40 (d, $J$ = 8.8 Hz, 1H), 7.31 (d, $J$ = 7.6 Hz, 1H), 7.25 (s, 1H), 7.23 - 7.19 (m, 1H), 7.05 - 6.87 (m, 1H), 6.82 (d, $J$ = 8.0 Hz, 1H), 4.44 - 4.30 (m, 1H), 4.30 - 4.20 (m, 1H), 4.18 (s, 1H), 3.54 - 3.48 (m, 2H), 1.99 - 1.90 (m, 2H), 1.86 - 1.80 (m, 2H).

**Experimental Example**

**Experimental Example 1: Phosphatase assay ($IC_{50}$)**

$IC_{50}$ values were measured using 6,8-difluoro-4-methylumbelliferyl phosphate (DiFMUP) as a substrate. The reaction was performed in a 384-well plate, and fluorescence measurement values were obtained using an EnVision plate reader from PerkinElmer.

SHP2 (full length, diluted to 100 pM final concentration in reaction buffer, BPS bioscience, US) was incubated with the SHP2 activating peptide in reaction buffer (60 mM HEPES (pH 7.2), 75 mM NaCl, 75 mM KCl, 1 mM EDTA, 5 mM DTT, 0.05% P-20) for 30 minutes to activate SHP2. After activation, DMSO [1% (V/V) or the compound according to the Examples (concentration ranging from 0.6 nM to 10 $\mu$M)] was added. As a positive control group, the selective SHP2 inhibitors SHP099 (6-(4-amino-4-methylpiperidin-1-yl)-3-(2,3-dichlorophenyl)pyrazin-2-amine, FOCUS bioscience, Australia; concentration ranging from 0.6 nM to 10 $\mu$M) and TNO155 ((3S,4S)-8-(6-amino-5-((2-amino-3-chloropyridin-4-yl)thio)pyrazin-2-yl)-3-methyl-2-oxa-8-azaspiro[4.5]decan-4-amine, Chemietek, US; concentration ranging from 0.6 nM to 10 $\mu$M) were used.

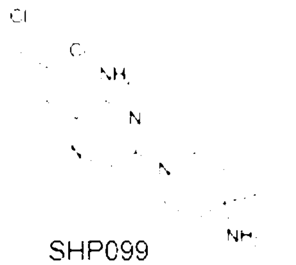

SHP099

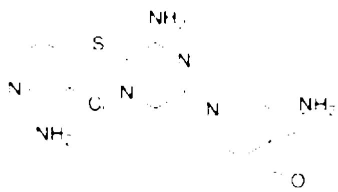

TNO155

DiFMUP (80 $\mu$M) was added to the reaction and incubated for a total of 2 hours to allow for the reaction to proceed. Thereafter, fluorescence (340 nm excitation, 450 nm emission) values were measured. $IC_{50}$ values were obtained using Graphad's Prism from the measured values depending on the concentration. The $IC_{50}$ values of the compounds according to the Examples as obtained are summarized in Table 7 below.

In the phosphatase assay (PTPase assay), when an $IC_{50}$ value was 100 nM or more, + was indicated, when an $IC_{50}$ value was 3 nM or more and less than 100 nM, ++ was indicated, and when an $IC_{50}$ value was less than 3 nM, +++ was indicated.

[Table 7]

| Example No. | PTPase assay | Example No. | PTPase assay |
|---|---|---|---|
| 1 | +++ | 28 | +++ |
| 2 | +++ | 29 | ++ |
| 3 | +++ | 30 | NA |
| 4 | +++ | 31 | NA |
| 5 | +++ | 32 | NA |
| 6 | +++ | 33 | NA |
| 7 | +++ | 34 | +++ |
| 8 | +++ | 35 | +++ |
| 9 | +++ | 36 | +++ |
| 10 | +++ | 37 | ++ |
| 11 | +++ | 38 | NA |
| 12 | ++ | 39 | +++ |
| 13 | ++ | 40 | ++ |
| 14 | +++ | 41 | +++ |
| 15 | +++ | 42 | +++ |
| 16 | NA | 43 | +++ |
| 17 | +++ | 44 | ++ |
| 18 | +++ | 45 | +++ |
| 19 | +++ | 46 | +++ |
| 20 | ++ | 47 | +++ |
| 21 | +++ | 48 | +++ |
| 22 | ++ | 49 | +++ |
| 23 | +++ | 50 | NA |
| 24 | +++ | 51 | NA |
| 25 | +++ | 52 | NA |
| 26 | ++ | 53 | NA |
| 27 | +++ | | |

Referring to Table 7, it was confirmed that the compounds according to the present disclosure have an excellent SHP2 inhibitory effect.

**Experimental Example 2: phospho-ERK assay**

H358 [20,000 cell/well] was contained in 40 μL of the cell culture solution (RPMI, 10% FBS, penicillin, streptomycin) and placed on a 384-well plate. After 24 hours, the serially diluted compound according to the Examples (concentration of minimum 0.13 nM, maximum 10 μM) was contained in 10 μL of the cell culture solution, put into wells containing cells, and incubated in a cell incubator for 1.5 hours. As a positive control group, the selective SHP2 inhibitor, TNO155 (Chemietek, US; concentration ranging from 0.13 nM to 10 μM) was used.

Thereafter, phospho-ERK was labeled using an AlphaLisa system from PerkinElmer according to the manufacturer's instructions, and then fluorescence (680 nm excitation wavelength, 615 nm emission wavelength) values were measured using a Varioskan equipment from Thermo. $IC_{50}$ values were obtained using Graphad's Prism from the measured values depending on the concentration. The $IC_{50}$ values of the compounds according to the Examples as obtained are summarized in Table 8 below.

In the phospho-ERK assay, when an $IC_{50}$ value was 1.5 μM or more, + was indicated, when an $IC_{50}$ value was 45

nM or more and less than 1.5 μM, ++ was indicated, and when an $IC_{50}$ value was less than 45 nM, +++ was indicated.

[Table 8]

| Example No. | pERK assay | Example No. | pERK assay |
|---|---|---|---|
| 1 | +++ | 28 | ++ |
| 2 | +++ | 29 | ++ |
| 3 | +++ | 30 | NA |
| 4 | +++ | 31 | ++ |
| 5 | NA | 32 | +++ |
| 6 | NA | 33 | NA |
| 7 | +++ | 34 | NA |
| 8 | +++ | 35 | ++ |
| 9 | +++ | 36 | +++ |
| 10 | +++ | 37 | ++ |
| 11 | NA | 38 | NA |
| 12 | NA | 39 | ++ |
| 13 | NA | 40 | NA |
| 14 | NA | 41 | ++ |
| 15 | NA | 42 | NA |
| 16 | NA | 43 | ++ |
| 17 | NA | 44 | ++ |
| 18 | +++ | 45 | ++ |
| 19 | NA | 46 | NA |
| 20 | NA | 47 | +++ |
| 21 | NA | 48 | ++ |
| 22 | NA | 49 | +++ |
| 23 | NA | 50 | NA |
| 24 | NA | 51 | NA |
| 25 | +++ | 52 | NA |
| 26 | NA | 53 | NA |
| 27 | +++ | | |

Intracellular SHP2 activity can be confirmed by measuring the degree of phosphorylation of ERK (extracellular signal regulated kinase), which represents Ras/Raf/ERK, a downstream signal pathway of SHP2. Inhibition of ERK phosphorylation by SHP2 inhibitors was confirmed through phospho-ERK analysis, which confirmed that the SHP2 inhibitors of the present disclosure have excellent activity in inhibiting ERK phosphorylation mediated by SHP2.

**Experimental Example 3: Cell viability**

Human non-small cell lung cancer cell line H358 cells were purchased from the Korean Cell Line Bank. Cancer cell lines were cultured in the recommended RPMI-1640 medium supplemented with 10% fetal bovine serum (FBS) and 1% penicillin/streptomycin. Monolayer-cultured cancer cell lines were detached by treatment with Trypsin/EDTA, and 100 μL of cell suspension containing 4 x $10^3$ cells was each dispensed into SPL3D™ Cell Floater, 96-well round plate, through cell line counting. After culturing for 72 hours to form spheroid, the cells were individually treated with the compounds according to the Examples, which had been serially diluted (minimum 1.5 nM, maximum 10 μM in concentration), and then cultured in a cell incubator for 5 days. To analyze cell viability, the cells were treated with 50 uL of 3D CellTiter-Glo, stirred for

5 minutes at 500 rpm in a plate shaker from Thermo Fisher Scientific, and incubated for 25 minutes at room temperature. Then, the luminescent values were measured using a Varioskan equipment from Thermo Fisher Scientific. $IC_{50}$ values were obtained using GraphPad's Prism from the measured values depending on the concentration. The $IC_{50}$ values of the compounds according to the Examples are summarized in Table 9 below.

In the cell viability assay, when an $IC_{50}$ value was 50 nM or more, + was indicated, when an $IC_{50}$ value was 10 nM or more and less than 50 nM, ++ was indicated, and when an $IC_{50}$ value was less than 10 nM, +++ was indicated.

[Table 9]

| Example No. | Cell viability | Example No. | Cell viability |
|---|---|---|---|
| 1 | NA | 28 | ++ |
| 2 | +++ | 29 | +++ |
| 3 | +++ | 30 | +++ |
| 4 | +++ | 31 | +++ |
| 5 | NA | 32 | +++ |
| 6 | +++ | 33 | ++ |
| 7 | +++ | 34 | +++ |
| 8 | NA | 35 | +++ |
| 9 | NA | 36 | +++ |
| 10 | NA | 37 | ++ |
| 11 | +++ | 38 | +++ |
| 12 | NA | 39 | ++ |
| 13 | NA | 40 | +++ |
| 14 | +++ | 41 | ++ |
| 15 | ++ | 42 | +++ |
| 16 | +++ | 43 | +++ |
| 17 | +++ | 44 | +++ |
| 18 | +++ | 45 | +++ |
| 19 | NA | 46 | ++ |
| 20 | NA | 47 | +++ |
| 21 | NA | 48 | +++ |
| 22 | NA | 49 | +++ |
| 23 | ++ | 50 | +++ |
| 24 | ++ | 51 | NA |
| 25 | +++ | 52 | NA |
| 26 | +++ | 53 | NA |
| 27 | +++ | | |

**Experimental Example 4: Brain-plasma Pharmacokinetics**

Nine male CD-1 mice (ICR mouse) aged 6 to 9 weeks were treated with the compounds according to the Examples at a concentration of 20 mg/kg. 0.025 mL of blood and brain tissue were collected at 1 hour, 8 hours, and 24 hours post-treatment, respectively. The collected blood was placed in an EDTA-K2 tube, centrifuged at 4°C at 3,200 x g for 10 minutes, and the supernatant plasma was collected. The brain tissue samples were collected immediately after euthanizing the CD-1 mice after blood collection and performing cardiac perfusion with cold saline. After collection, the brain tissues were washed with cold saline, wiped and dried, weighed, and homogenized using a homogenization buffer (15 mM PBS (pH 7.4): MeOH = 2:1) at a ratio of 1:3 (1 g of tissue to 3 mL of buffer, dilution ratio 4). Plasma and homogenized

brain tissue samples were each transferred to a polypropylene microcentrifuge tube and stored at -60°C or lower. The concentration of compounds distributed in plasma and brain tissue was analyzed using LC-MS/MS, and the distribution in brain tissue compared to plasma was calculated as follows.

$$\text{Brain-to-plasma ratio} = \text{AUC(0-t) brain tissue} / \text{AUC(0-t) plasma}$$

In the Brain-plasma Pharmacokinetics assay, when a brain-to-plasma ratio value was 0.05 or more and less than 0.1, + was indicated, when a brain-to-plasma ratio value was 0.1 or more and less than 0.25, ++ was indicated, and when a brain-to-plasma ratio value was more than 0.25, +++ was indicated.

[Table 10]

| Example No. | Brain-to-plasma ratio | Example No. | Brain-to-plasma ratio |
|---|---|---|---|
| 2 | ND | 34 | ++ |
| 3 | ND | 35 | ++ |
| 4 | ND | 36 | + |
| 6 | ND | 37 | + |
| 7 | ++ | 41 | +++ |
| 14 | ND | 43 | +++ |
| 15 | + | 44 | +++ |
| 16 | ND | 45 | ++ |
| 18 | +++ | 47 | ++ |
| 25 | + | 48 | ++ |
| 27 | +++ | 49 | ++ |
| 29 | +++ | 50 | +++ |
| 31 | ++ | 51 | +++ |
| 32 | ++ | 52 | +++ |

Referring to Table 10, it was confirmed that the compounds of the present disclosure have excellent blood-brain barrier penetration properties. Specifically, it was confirmed that the compounds of the present disclosure have a blood-brain concentration ratio of at least 0.05 or more, at least 0.1 or more, at least 0.2 or more, or at least 0.25 or more.

## Claims

1. A compound represented by Formula 1A, a stereoisomer, solvate or isotope-labeled compound thereof, or a pharmaceutically acceptable salt thereof:

[Formula 1A]

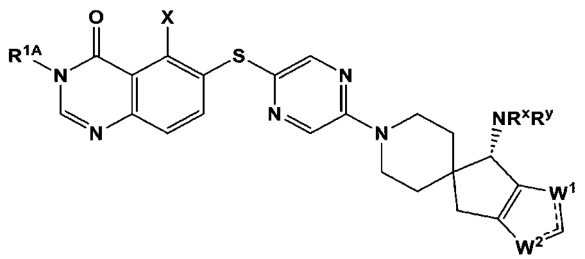

wherein,

X is H or halogen;
one of $W^1$ and $W^2$ is S, and the other is N;
$R^{1A}$ is H, $C_{6-10}$ aryl, or $C_{1-6}$ alkyl optionally substituted with $C_{1-6}$ alkoxyl; and
$R^x$ and $R^y$ are each independently H or $C_{1-6}$ alkyl.

**2.** The compound according to claim 1, or a stereoisomer, solvate or isotope-labeled compound thereof, or a pharmaceutically acceptable salt thereof, wherein

X is H or Cl,
$R^{1A}$ is selected from the group consisting of H, phenyl, $CH_3$, $CH_2CH_3$,

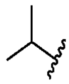 , 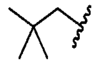 ,

and

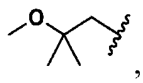 ,

and
$R^x$ and $R^y$ are each H.

**3.** The compound according to claim 1, or a stereoisomer, solvate or isotope-labeled compound thereof, or a pharmaceutically acceptable salt thereof, wherein the compound is selected from:

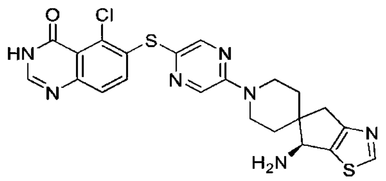

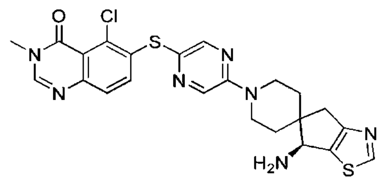

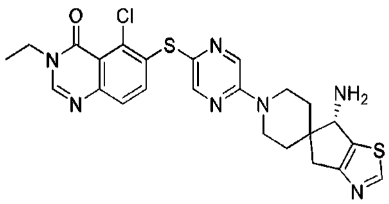

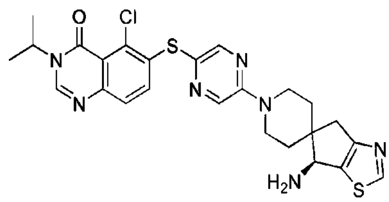

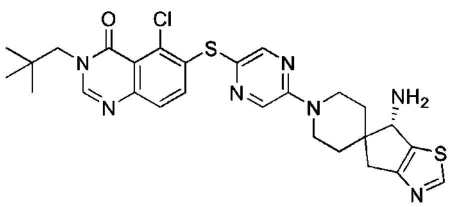

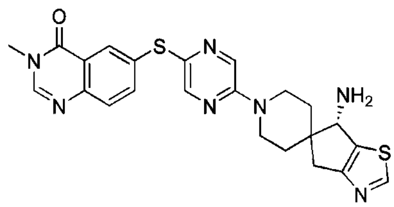

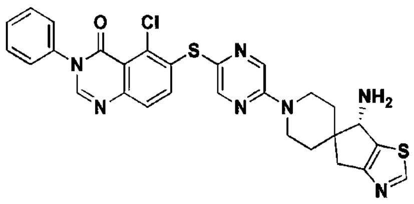

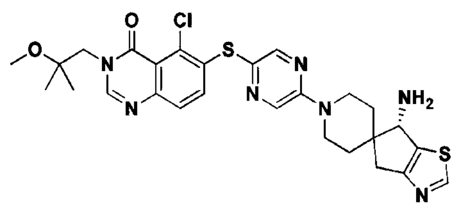

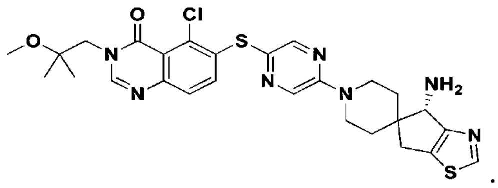

4. A compound represented by Formula 1B, a stereoisomer, solvate or isotope-labeled compound thereof, or a pharmaceutically acceptable salt thereof:

[Formula 1B]

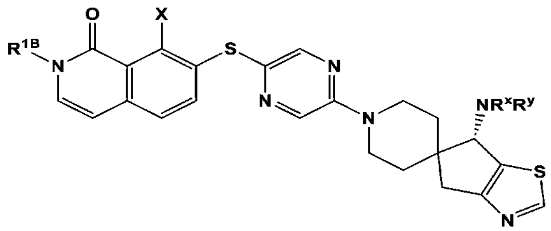

wherein,

X is H or halogen;
$R^{1B}$ is H or $C_{1-6}$ alkyl; and
$R^x$ and $R^y$ are each independently H or $C_{1-6}$ alkyl.

5. The compound according to claim 4, or a stereoisomer, solvate or isotope-labeled compound thereof, or a pharmaceutically acceptable salt thereof, wherein

X is halogen,
$R^{1B}$ is $C_{1-6}$ alkyl, and
$R^x$ and $R^y$ are each H.

6. The compound according to claim 4, or a stereoisomer, solvate or isotope-labeled compound thereof, or a pharmaceutically acceptable salt thereof, wherein the compound is selected from:

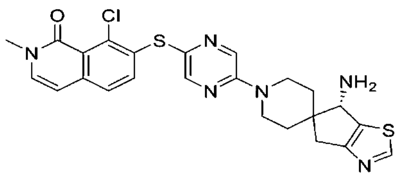

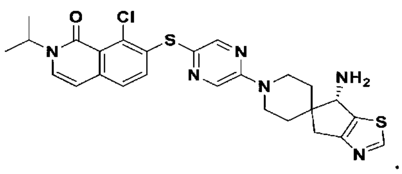

7. A compound represented by Formula 2A, a stereoisomer, solvate or isotope-labeled compound thereof, or a pharmaceutically acceptable salt thereof:

[Formula 2A]

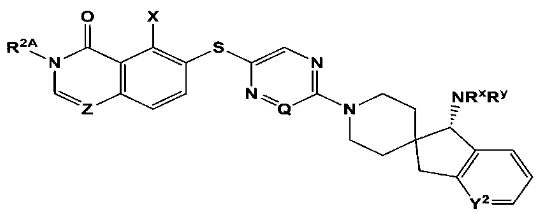

wherein,

Z is N or CH;
Q is N or CH;
X is H or halogen;
$Y^2$ is N or CH;
$R^{2A}$ is H, $C_{6-10}$ aryl, 5- to 7-membered heteroaryl containing a heteroatom selected from N and O, or $C_{1-6}$ alkyl optionally substituted with OH or $C_{1-6}$ alkoxy; and
$R^x$ and $R^y$ are each independently H or $C_{1-6}$ alkyl.

**8.** The compound according to claim 7, or a stereoisomer, solvate or isotope-labeled compound thereof, or a pharmaceutically acceptable salt thereof, wherein

X is halogen,
$R^{2A}$ is methyl, phenyl, pyridinyl,

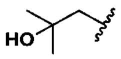, 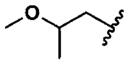 or 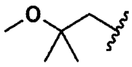,

and
$R^x$ and $R^y$ are each independently H.

**9.** The compound according to claim 7, or a stereoisomer, solvate or isotope-labeled compound thereof, or a pharmaceutically acceptable salt thereof, wherein the compound is selected from:

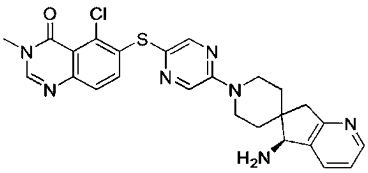
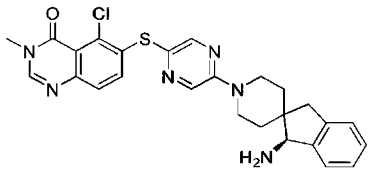

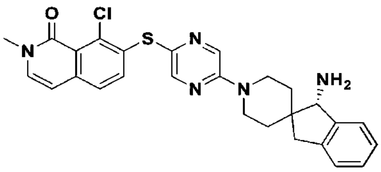
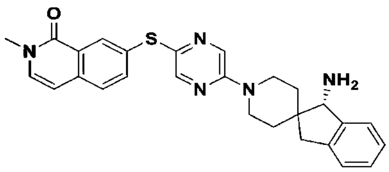

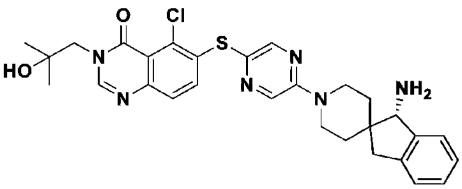
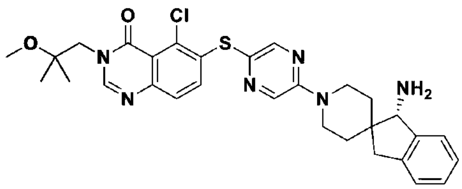

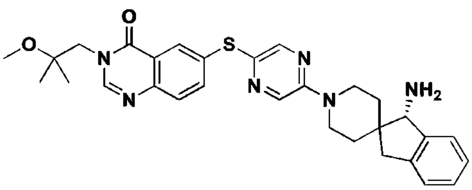
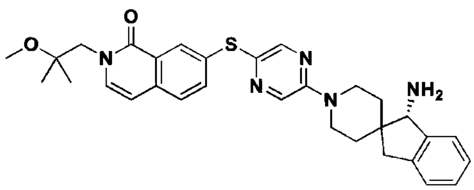

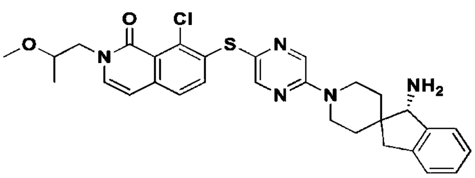
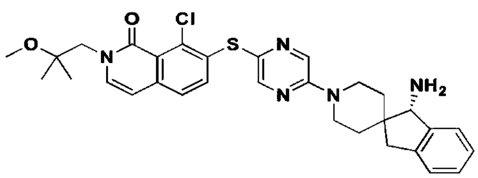

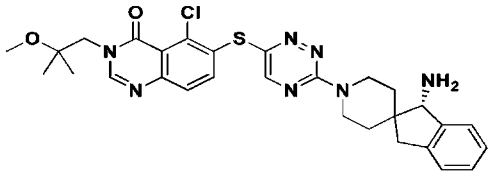

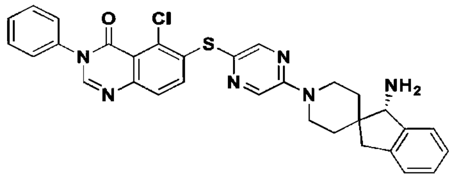

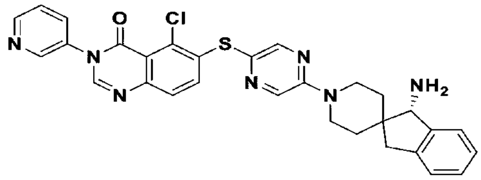
.

**10.** A compound represented by Formula 2B, a stereoisomer, solvate or isotope-labeled compound thereof, or a pharmaceutically acceptable salt thereof:

[Formula 2B]

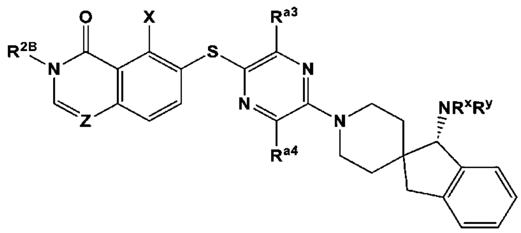

wherein,

Z is N or CH;
X is H or halogen;
$R^{a3}$ is $NR^iR^{ii}$ or $C_{1-6}$ alkyl;
$R^{a4}$ is H, or $C_{1-6}$ alkyl substituted with OH or $C_{1-6}$ alkoxy;
$R^{2B}$ is selected from H, and $C_{1-6}$ alkyl optionally substituted with OH or $C_{1-6}$ alkoxy;
$R^i$ and $R^{ii}$ are each independently H or $C_{1-6}$ alkyl; and
$R^x$ and $R^y$ are each independently H or $C_{1-6}$ alkyl.

**11.** The compound according to claim 10, or a stereoisomer, solvate or isotope-labeled compound thereof, or a pharmaceutically acceptable salt thereof, wherein

X is halogen,
$R^{2B}$ is selected from the group consisting of $CH_3$,

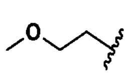
,
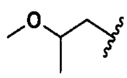
,
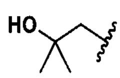

and

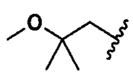
,

and
$R^i$, $R^{ii}$, $R^x$ and $R^y$ are each independently H.

**12.** The compound according to claim 10, or a stereoisomer, solvate or isotope-labeled compound thereof, or a

pharmaceutically acceptable salt thereof, wherein the compound is selected from:

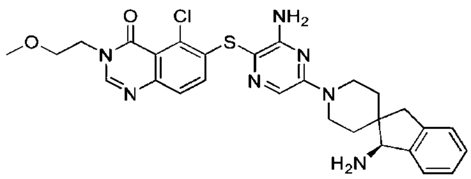

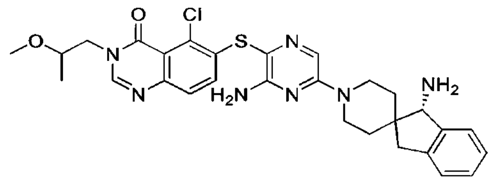

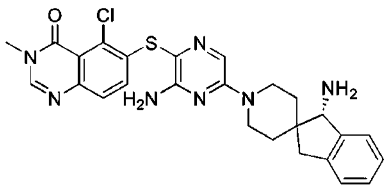

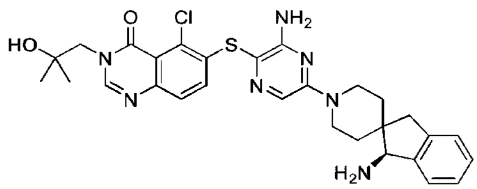

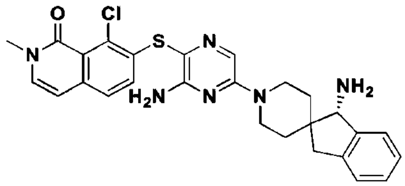

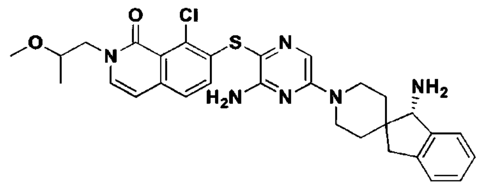

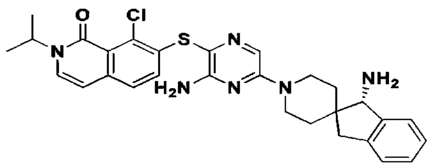

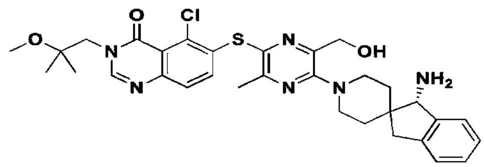

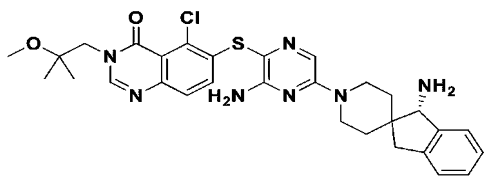
.

**13.** A compound represented by Formula 2C, a stereoisomer, solvate or isotope-labeled compound thereof, or a pharmaceutically acceptable salt thereof:

[Formula 2C]

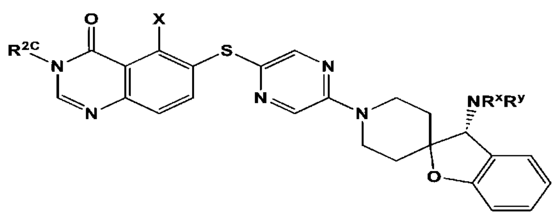

wherein,

X is H or halogen;
$R^{2C}$ is selected from H, $C_{6-10}$ aryl, and $C_{1-6}$ alkyl optionally substituted with OH or $C_{1-6}$ alkoxy; and
$R^x$ and $R^y$ are each independently H or $C_{1-6}$ alkyl.

**14.** The compound according to claim 13, or a stereoisomer, solvate or isotope-labeled compound thereof, or a pharmaceutically acceptable salt thereof, wherein

X is halogen,
$R^{2C}$ is selected from $C_{6-10}$ aryl, and $C_{1-6}$ alkyl optionally substituted with OH or $C_{1-6}$ alkoxy, and
$R^x$ and $R^y$ are each independently H.

**15.** The compound according to claim 13, or a stereoisomer, solvate or isotope-labeled compound thereof, or a pharmaceutically acceptable salt thereof, wherein the compound is selected from:

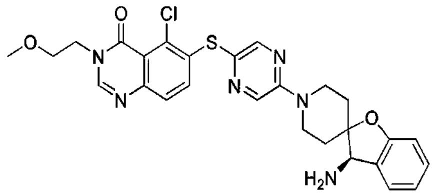

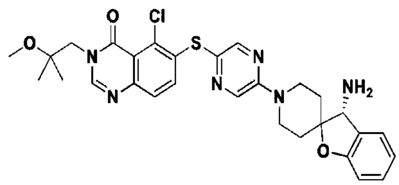

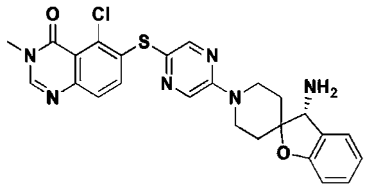

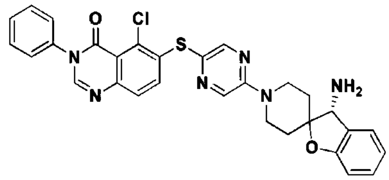

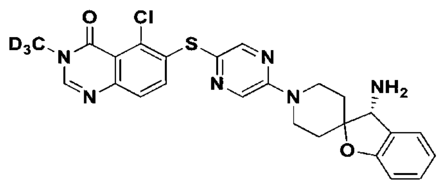

.

**16.** A compound represented by Formula 3A, a stereoisomer, solvate or isotope-labeled compound thereof, or a pharmaceutically acceptable salt thereof:

[Formula 3A]

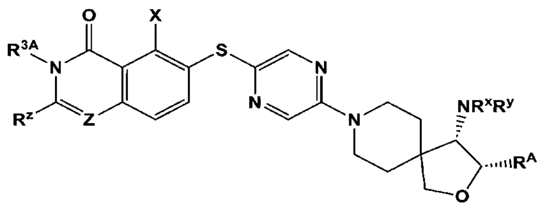

wherein,

Z is N or CH;
X is H or halogen;
$R^z$ is H or $C_{1-6}$ alkyl;
$R^{3A}$ is H, -($C_{1-6}$ alkylene)-oxanyl substituted with $C_{1-6}$ alkyl, or $C_{1-6}$ alkyl, -($C_{1-6}$ alkylene)-($C_{3-6}$ cycloalkyl) or -($C_{1-6}$ alkylene)-piperidinyl, each optionally substituted with one to three occurences of $R^{31A}$;
$R^{31A}$ is halogen, cyano, or $C_{1-6}$ alkoxy;
$R^A$ is H or $C_{1-6}$ alkyl; and
$R^x$ and $R^y$ are each independently H or $C_{1-6}$ alkyl.

**17.** The compound according to claim 16, or a stereoisomer, solvate or isotope-labeled compound thereof, or a pharmaceutically acceptable salt thereof, wherein

X is halogen,
$R^{3A}$ is selected from the group consisting of $CH_3$,

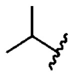, 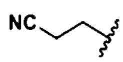, 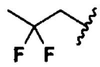,

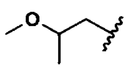, 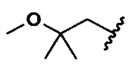, 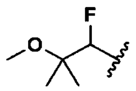, 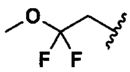, 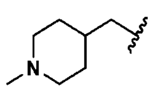, 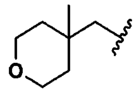 and 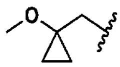,

$R^A$ is $C_{1-6}$ alkyl, and
$R^x$ and $R^y$ are each independently H.

**18.** The compound according to claim 16, or a stereoisomer, solvate or isotope-labeled compound thereof, or a pharmaceutically acceptable salt thereof, wherein the compound is selected from:

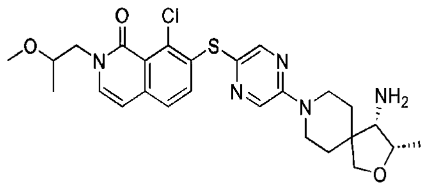 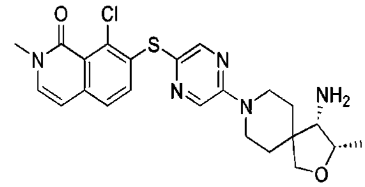

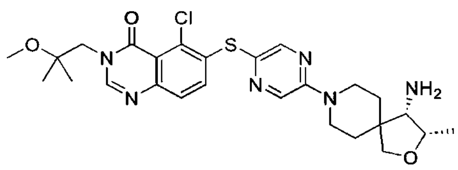 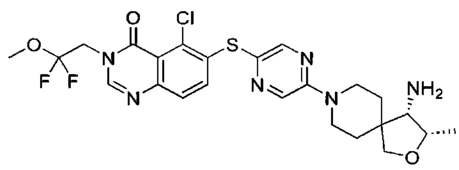

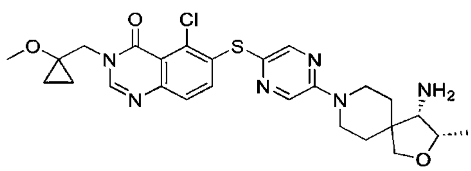 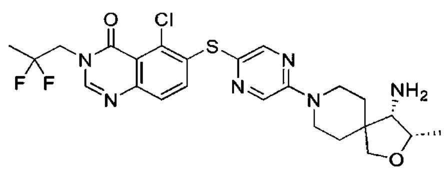

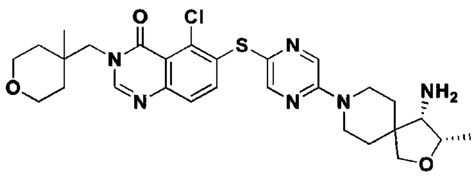 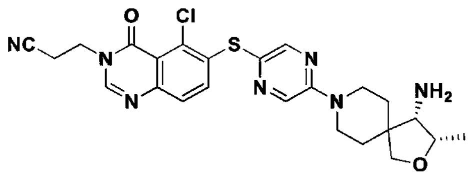

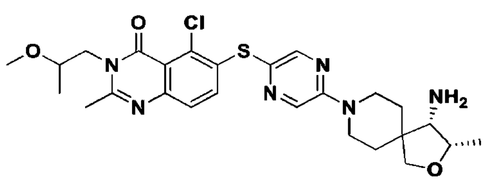 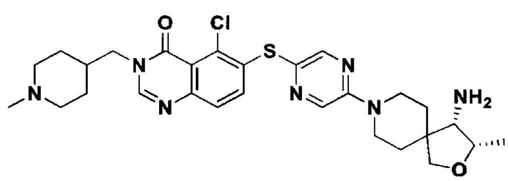

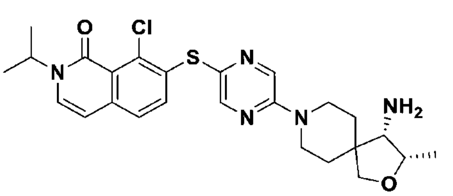 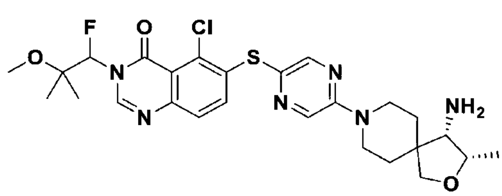 .

**19.** A compound represented by Formula 3B, a stereoisomer, solvate or isotope-labeled compound thereof, or a pharmaceutically acceptable salt thereof:

[Formula 3B]

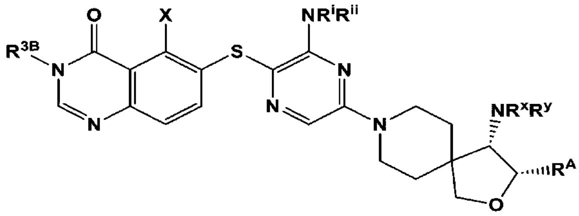

wherein,

X is H or halogen;
$R^{3B}$ is $C_{1-6}$ alkyl optionally substituted with OH, $C_{1-6}$ alkoxy or $C_{6-10}$ aryl;
$R^A$ is H or $C_{1-6}$ alkyl;
$R^i$ and $R^{ii}$ are each independently H or $C_{1-6}$ alkyl; and
$R^x$ and $R^y$ are each independently H or $C_{1-6}$ alkyl.

**20.** The compound according to claim 19, or a stereoisomer, solvate or isotope-labeled compound thereof, or a pharmaceutically acceptable salt thereof, wherein

X is halogen,
$R^{3B}$ is $C_{1-6}$ alkyl substituted with $C_{1-6}$ alkoxy or $C_{6-10}$ aryl,
$R^A$ is $C_{1-6}$ alkyl, and
$R^i$, $R^{ii}$, $R^x$ and $R^y$ are each independently H.

**21.** The compound according to claim 19, or a stereoisomer, solvate or isotope-labeled compound thereof, or a pharmaceutically acceptable salt thereof, wherein the compound is selected from:

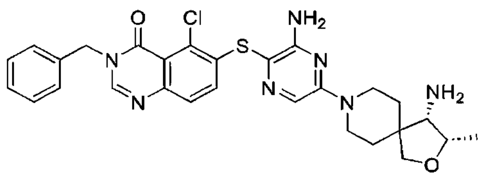

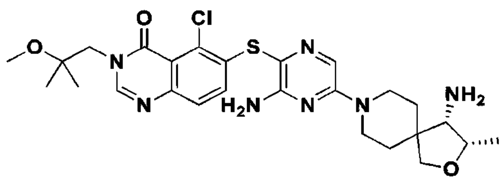
.

**22.** A compound represented by Formula 3C, a stereoisomer, solvate or isotope-labeled compound thereof, or a pharmaceutically acceptable salt thereof:

[Formula 3C]

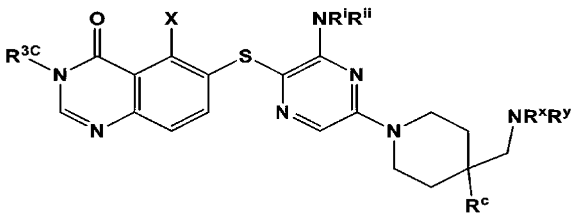

wherein

X is H or halogen;
$R^{3C}$ is $C_{1-6}$ alkyl optionally substituted with $C_{6-10}$ aryl;
$R^c$ is H or $C_{1-6}$ alkyl;
$R^i$ and $R^{ii}$ are each independently H or $C_{1-6}$ alkyl; and
$R^x$ and $R^y$ are each independently H or $C_{1-6}$ alkyl.

**23.** The compound according to claim 22, or a stereoisomer, solvate or isotope-labeled compound thereof, or a pharmaceutically acceptable salt thereof, wherein

X is halogen,
$R^{3C}$ is $C_{1-6}$ alkyl substituted with $C_{6-10}$ aryl,
$R^c$ is $C_{1-6}$ alkyl,
$R^i$, $R^{ii}$, $R^x$ and $R^y$ are each independently H.

**24.** The compound according to claim 22, or a stereoisomer, solvate or isotope-labeled compound thereof, or a pharmaceutically acceptable salt thereof, wherein the compound is represented by:

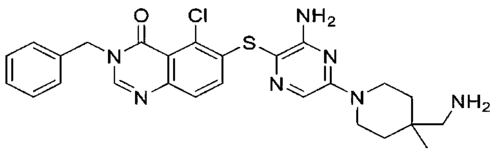
.

**25.** A pharmaceutical composition for preventing or treating a disease associated with the abnormal activity of Src homology region 2 domain-containing phosphatase-2 (SHP2), comprising the compound of any one of claims 1 to 24, or a stereoisomer, solvate, isotope-labeled compound thereof, or a pharmaceutically acceptable salt thereof.

**26.** The pharmaceutical composition according to claim 25, wherein the disease associated with the abnormal activity of SHP2 is selected from a group consisting of cancer, cancer metastasis, cardiovascular disease, immune disorder, fibrosis, and ocular disorder.

**27.** The pharmaceutical composition according to claim 25, wherein the disease associated with the abnormal activity of SHP2 is selected from a group consisting of Noonan syndrome, Leopard syndrome, juvenile myelomonocytic leukemia, neuroblastoma, melanoma, acute myeloid leukemia, breast cancer, esophageal cancer, lung cancer, large intestinal cancer, head cancer, malignant brain tumor, squamous cell carcinoma of the head and neck, gastric carcinoma, anaplastic large cell lymphoma, pancreatic cancer, biliary tract cancer, uterine cancer, endometrial cancer, liver cancer and neurofibromatosis type 1.

**28.** A method for preventing or treating a disease associated with the abnormal activity of SHP2, comprising administering to a subject the compound of any one of claims 1 to 24, or a stereoisomer, solvate or isotope-labeled compoud thereof, or pharmaceutically acceptable salt thereof.

**29.** A pharmaceutical composition comprising the compound of any one of claims 1 to 24, or a stereoisomer, solvate or isotope-labeled compound thereof, or a pharmaceutically acceptable salt thereof.

**30.** A compound of any one of claims 1 to 24, or a stereoisomer, solvate or isotope-labeled compound thereof, or a pharmaceutically acceptable salt thereof, for use in preventing or treating a disease associated with the abnormal activity of SHP2.

**31.** Use of the compound of any one of claims 1 to 24, or a stereoisomer, solvate or isotope-labeled compound thereof, or a pharmaceutically acceptable salt thereof, for use in the manufacture of a medicament for the prevention or treatment of a disease associated with the abnormal activity of SHP2.

# INTERNATIONAL SEARCH REPORT

International application No.
**PCT/KR2024/000298**

## A. CLASSIFICATION OF SUBJECT MATTER

**C07D 513/10**(2006.01)i; **A61K 31/517**(2006.01)i; **A61K 31/497**(2006.01)i; **A61P 35/00**(2006.01)i; **A61P 35/04**(2006.01)i; **A61P 9/00**(2006.01)i; **A61P 37/00**(2006.01)i; **A61P 27/02**(2006.01)i; **C07D 471/10**(2006.01)i; **C07D 401/14**(2006.01)i

According to International Patent Classification (IPC) or to both national classification and IPC

## B. FIELDS SEARCHED

Minimum documentation searched (classification system followed by classification symbols)

C07D 513/10(2006.01); A61K 31/444(2006.01); A61K 31/4965(2006.01); A61K 31/497(2006.01); C07D 241/18(2006.01); C07D 401/04(2006.01); C07D 401/14(2006.01); C07D 491/107(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus), Google & keywords: SHP2 억제제(Src homology region 2 domain-containing phosphatase-2), 용매화물(solvate), 동위원소(isotope), 암(cancer), 심혈관질환(cardiovascular disease), 면역장애 (immune disorder)

## C. DOCUMENTS CONSIDERED TO BE RELEVANT

| Category* | Citation of document, with indication, where appropriate, of the relevant passages | Relevant to claim No. |
|---|---|---|
| A | KR 10-2021-0075110 A (JIANGSU HANSOH PHARMACEUTICAL GROUP CO., LTD. et al.) 22 June 2021 (2021-06-22)<br>See claims 1-31. | 1-27,29-31 |
| A | WO 2021-043077 A1 (SICHUAN KELUN-BIOTECH BIOPHARMACEUTICAL CO., LTD.) 11 March 2021 (2021-03-11)<br>See claims 1-25. | 1-27,29-31 |
| A | KR 10-2020-0070295 A (REVOLUTION MEDICINES, INC.) 17 June 2020 (2020-06-17)<br>See claims 1-39. | 1-27,29-31 |
| A | KR 10-2019-0015756 A (JACOBIO PHARMACEUTICALS CO., LTD.) 14 February 2019 (2019-02-14)<br>See claims 1-85. | 1-27,29-31 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **23 April 2024** | **24 April 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.
**PCT/KR2024/000298**

| C. DOCUMENTS CONSIDERED TO BE RELEVANT | | |
|---|---|---|
| Category* | Citation of document, with indication, where appropriate, of the relevant passages | Relevant to claim No. |
| PX | WO 2023-282702 A1 (KANAPH THERAPEUTICS INC. et al.) 12 January 2023. See paragraphs [0030]-[0042]; and claims 1-29. | 1-27,29-31 |

Form PCT/ISA/210 (second sheet) (July 2022)

**INTERNATIONAL SEARCH REPORT**

International application No.
**PCT/KR2024/000298**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **28**
because they relate to subject matter not required to be searched by this Authority, namely:

Claim 28 pertains to a method for treatment of the human body by surgery or therapy, and thus pertains to a subject matter on which the International Searching Authority is not required to carry out an international search under the provisions of PCT Article 17(2)(a)(i) and PCT Rule 39.1(iv).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/KR2024/000298**

| Patent document cited in search report | Publication date (day/month/year) | Patent family member(s) | Publication date (day/month/year) |
|---|---|---|---|
| KR 10-2021-0075110 A | 22 June 2021 | AU 2019-357433 A1 | 08 April 2021 |
| | | BR 112021004966 A2 | 08 June 2021 |
| | | CA 3114160 A1 | 16 April 2020 |
| | | CN 111295374 A | 16 June 2020 |
| | | CN 111295374 B | 04 November 2022 |
| | | CN 115353509 A | 18 November 2022 |
| | | EP 3868742 A1 | 25 August 2021 |
| | | JP 2022-504352 A | 13 January 2022 |
| | | MX 2021003442 A | 15 June 2021 |
| | | TW 202028183 A | 01 August 2020 |
| | | US 2021-0355105 A1 | 18 November 2021 |
| | | WO 2020-073949 A1 | 16 April 2020 |
| WO 2021-043077 A1 | 11 March 2021 | CN 111339855 A | 26 June 2020 |
| | | CN 111339855 B | 23 May 2023 |
| | | CN 113271684 A | 17 August 2021 |
| | | CN 114127053 A | 01 March 2022 |
| | | CN 114127053 B | 13 June 2023 |
| | | EP 4106474 A1 | 21 December 2022 |
| | | JP 2021-089761 A | 10 June 2021 |
| | | JP 2023-515402 A | 13 April 2023 |
| | | JP 7146977 B2 | 04 October 2022 |
| | | KR 10-2021-0038446 A | 07 April 2021 |
| | | KR 10-2022-0137976 A | 12 October 2022 |
| | | US 2021-0191611 A1 | 24 June 2021 |
| | | US 2022-0369169 A1 | 17 November 2022 |
| | | US 2022-0383518 A1 | 01 December 2022 |
| | | WO 2021-159554 A1 | 19 August 2021 |
| | | WO 2021-160084 A1 | 19 August 2021 |
| | | WO 2021-160093 A1 | 19 August 2021 |
| KR 10-2020-0070295 A | 17 June 2020 | AR 113762 A1 | 10 June 2020 |
| | | AU 2018-347516 A1 | 07 May 2020 |
| | | BR 112020007058 A2 | 06 October 2020 |
| | | CA 3078565 A1 | 18 April 2019 |
| | | CN 111212834 A | 29 May 2020 |
| | | CN 111212834 B | 19 January 2024 |
| | | CO 2020003714 A2 | 24 April 2020 |
| | | EP 3694848 A1 | 19 August 2020 |
| | | IL 273756 A | 31 May 2020 |
| | | JP 2020-536881 A | 17 December 2020 |
| | | MX 2020003579 A | 22 July 2020 |
| | | PH 12020550216 A1 | 15 February 2021 |
| | | RU 2020115095 A | 12 November 2021 |
| | | SG 11202002941 A | 29 April 2020 |
| | | TW 201930292 A | 01 August 2019 |
| | | US 11702411 B2 | 18 July 2023 |
| | | US 2020-0339552 A1 | 29 October 2020 |
| | | US 2024-0067636 A1 | 29 February 2024 |
| | | WO 2019-075265 A1 | 18 April 2019 |
| KR 10-2019-0015756 A | 14 February 2019 | AU 2017-276457 A1 | 24 January 2019 |
| | | AU 2017-276457 B2 | 03 October 2019 |

Form PCT/ISA/210 (patent family annex) (July 2022)

**INTERNATIONAL SEARCH REPORT**
**Information on patent family members**

International application No.
**PCT/KR2024/000298**

| Patent document cited in search report | Publication date (day/month/year) | Patent family member(s) | Publication date (day/month/year) |
|---|---|---|---|
| | | CA 3026784 A1 | 14 December 2017 |
| | | CN 109311848 A | 05 February 2019 |
| | | CN 109311848 B | 01 February 2022 |
| | | CN 112250670 A | 22 January 2021 |
| | | CN 112250670 B | 08 June 2021 |
| | | CN 114539273 A | 27 May 2022 |
| | | CY 1125042 T1 | 24 March 2023 |
| | | DK 3464272 T3 | 21 February 2022 |
| | | EA 201990001 A1 | 31 May 2019 |
| | | EA 202092441 A1 | 21 May 2021 |
| | | EA 202092442 A2 | 30 June 2021 |
| | | EA 202092442 A3 | 31 August 2021 |
| | | EP 3464272 A1 | 10 April 2019 |
| | | EP 3464272 B1 | 08 December 2021 |
| | | EP 4108659 A1 | 28 December 2022 |
| | | ES 2908801 T3 | 04 May 2022 |
| | | HR P20220251 T1 | 29 April 2022 |
| | | HU E057838 T2 | 28 June 2022 |
| | | JP 2019-521181 A | 25 July 2019 |
| | | JP 2020-189868 A | 26 November 2020 |
| | | JP 2022-153616 A | 12 October 2022 |
| | | JP 6751203 B2 | 02 September 2020 |
| | | KR 10-2021-0019607 A | 22 February 2021 |
| | | KR 10-2021-0141778 A | 23 November 2021 |
| | | KR 10-2022-0124275 A | 13 September 2022 |
| | | KR 10-2023-0109185 A | 19 July 2023 |
| | | KR 10-2218333 B1 | 22 February 2021 |
| | | LT 3464272 T | 10 March 2022 |
| | | MA 45189 A | 10 April 2019 |
| | | MX 2018015297 A | 12 August 2019 |
| | | MX 2021015073 A | 18 January 2022 |
| | | MX 2021015074 A | 18 January 2022 |
| | | PH 12018550202 A1 | 21 October 2019 |
| | | PL 3464272 T3 | 28 March 2022 |
| | | PT 3464272 T | 11 March 2022 |
| | | RS 62987 B1 | 31 March 2022 |
| | | SG 10202110874 A | 29 November 2021 |
| | | SG 11201810983 A | 30 January 2019 |
| | | SI 3464272 T1 | 31 May 2022 |
| | | US 10858359 B2 | 08 December 2020 |
| | | US 2019-0127378 A1 | 02 May 2019 |
| | | WO 2017-211303 A1 | 14 December 2017 |
| WO 2023-282702 A1 | 12 January 2023 | AU 2024-309195 A1 | 25 January 2024 |
| | | CA 3226206 A1 | 12 January 2023 |
| | | IL 309984 A | 01 March 2024 |
| | | KR 10-2023-0011245 A | 20 January 2023 |
| | | TW 202319056 A | 16 May 2023 |

Form PCT/ISA/210 (patent family annex) (July 2022)